# EUROPEAN PATENT SPECIFICATION

(11) **EP 4 188 379 B1**
(45) Date of publication and mention of the grant of the patent: **25.02.2026**
(21) Application number: 21848925.0
(22) Date of filing: 22.07.2021
(51) Int. Cl.: A61K 31/4738, A61K 31/53, C07D 253/10, C07D 471/04

(54) **POLYCYCLIC CAP-DEPENDENT ENDONUCLEASE INHIBITORS FOR TREATING OR PREVENTING INFLUENZA**
POLYCYCLISCHE CAP-ABHÄNGIGE ENDONUKLEASEHEMMER ZUR BEHANDLUNG ODER PRÄVENTION VON INFLUENZA
INHIBITEURS POLYCYCLIQUES DE L'ENDONUCLÉASE DÉPENDANTS DE CAP POUR LE TRAITEMENT OU LA PRÉVENTION DE LA GRIPPE

(30) Priority: 27.07.2020 US 202063056861 P
(43) Date of publication of application: 07.06.2023
(73) Proprietor: Merck Sharp & Dohme LLC, Rahway, New Jersey 07065 (US); Cocrystal Pharma, Inc., Bothell, WA 98011 (US)
(72) Inventor: ZHANG, Yonglian, Kenilworth, New Jersey 07033 (US); MCCAULEY, John A., West Point, Pennsylvania 19486 (US); LO, Michael Man-Chu, Kenilworth, New Jersey 07033 (US); GUO, Liangqin, Kenilworth, New Jersey 07033 (US); ZHAO, Kake, Kenilworth, New Jersey 07033 (US); BENNETT, Frank, Kenilworth, New Jersey 07033 (US); KIM, Ronald M., Kenilworth, New Jersey 07033 (US); DEJESUS, Reynalda Keh, Kenilworth, New Jersey 07033 (US); SHURTLEFF, Valerie W., West Point, Pennsylvania 19486 (US); DE LERA RUIZ, Manuel, West Point, Pennsylvania 19486 (US); PLOTKIN, Michael A., West Point, Pennsylvania 19486 (US); SU, Hua, West Point, Pennsylvania 19486 (US); FELLS, James, West Point, Pennsylvania 19486 (US); CROWLEY, Brendan M., West Point, Pennsylvania 19486 (US); CHOBANIAN, Harry R., Kenilworth, New Jersey 07033 (US); EMBREY, Mark, W., West Point, PA 19486 (US); MORRIELLO, Gregori, J., Kenilworth, NJ 07033 (US)
(74) Representative: Merck Sharp & Dohme LLC
(86) International application number: PCT/US2021/042711
(87) International publication number: WO 2022/026285

(56) References cited:
- WO-A1-2019/230858
- WO-A1-2020/055858
- US-A1- 2017 349 587
- DATABASE PubChem substance NCBI; ANONYMOUS : "SID 393093638", XP055905862, Database accession no. SID 393093638

## Description

### BACKGROUND OF THE INVENTION

Influenza viruses, members of the *Orthomyxoviridae* family, are categorized by type: Influenza A, B, C or D. Seasonal epidemic disease caused by Influenza A and Influenza B, which co-circulate throughout the world, is the biggest concern for human public health. Influenza A viruses are characterized by the combination of surface proteins, hemagglutinin (HA, H) and neuraminidase (NA, N), present on the virion. Both H1N1 and H3N2 viruses are capable of infecting and causing disease in humans. Influenza B viruses fall into one of two lineages, Victoria-like or Yamagata-like, both of which cause human disease.

Influenza A and B virus particles consist of a cell-derived lipid membrane lined with the viral M1 matrix protein. This envelope encompasses 8 segments of negative strand RNA genome, each encoding one or more viral proteins. Surface-exposed hemagglutinin, M2 and neuraminidase proteins mediate host cell entry, uncoating and release of nascent virus particles from infected cells, respectively. The segmented genome is packaged as a ribonucleoprotein complex made up of nucleoprotein-coated RNA associated with the heterotrimeric polymerase. The polymerase, made up of PA, PB1 and PB2 subunits, is critical for both viral genome replication and mRNA transcription. The PB 1 subunit harbors the polymerase active site, while the PB2 and PA subunits, in addition to their role in genome replication, work together to capture (PB2) and remove the cap (PA) from host cell pre-mRNAs, facilitating transcription of viral mRNA.

Seasonal influenza is a respiratory disease characterized by sudden onset fever, cough, sore throat, headache, myalgia, and malaise. Symptoms range from mild to severe and may lead to death of the infected person. Worldwide, 3 - 5 million people each year suffer from severe influenza disease and approximately 0.5 million die. Those who are immunecompromised, including the very young and those over the age of 65, are at highest risk for influenza-related morbidity and mortality.

Vaccines for prevention of influenza disease are available; however, the effectiveness of such vaccines varies from year to year with an estimated pooled effectiveness of 59% for healthy adults (Osterholm et al, CIDRAP report (2012)). Influenza virus strains capable of escaping host immunity are selectively transmitted; thus, to provide protection against currently circulating virus, seasonal influenza vaccines must be reformulated and readministered annually. Vaccines that provide durable, multi-season or broad-spectrum protection, are not currently available.

Several small molecules targeting influenza virus have been approved for therapeutic and/or limited prophylactic use in one or more countries, including M2 ion channel inhibitors, NA inhibitors, a nucleoside analog and a recently approved inhibitor targeting the endonuclease activity of the PA protein. As therapy, small molecule inhibitors of influenza must be administered within 48 hours of symptom onset to be effective, and shorten the duration of virus shedding and respiratory symptoms. Currently circulating influenza virus strains are resistant to approved M2 inhibitors, such that use of M2 inhibitors is no longer recommended. The purine analog favipiravir is approved for use only in Japan, and safety concerns restrict its use. In the past, increasing levels of circulating drug-resistant virus variants had limited the effectiveness of several neuraminidase inhibitors; however, since the 2009 H1N1 pandemic, the level of drug resistance has been low. Resistance to the cap-dependent endonuclease inhibitor baloxavir marboxil was high in late stage clinical trials wherein virus harboring a mutation at amino acid 38 of the PA protein was isolated from 9.7% of adult and 23.4 % of pediatric trial participants (Hayden et al, N Engl J Med., 379(10):913-923 (2018); Hirotsu et al, Clin Infect Dis., ciz908 (2019)). Whether these mutant viruses, which significantly reduce the in vitro antiviral potency, can efficiently transmit from human to human is not known (Omoto et al, Sci Rep., 8(1):9633 (2018); Noshi et al, Antiviral Res., 160:109-117 (2018)).

Currently, there are limited options for the treatment and prevention of influenza disease and significant concerns about drug resistance with approved therapies. Furthermore, the inability of seasonal vaccines to provide consistent, robust, durable, broad spectrum protection against influenza disease, combined with the threat of emergence of novel zoonotic influenza viruses with pandemic potential, necessitate continued development of both prophylactic and therapeutic agents targeting the influenza virus. WO 2020/055858 and US 2017/349587 describe compounds for treating influenza disease.

### SUMMARY OF THE INVENTION

The present invention relates to compounds of Formula I: 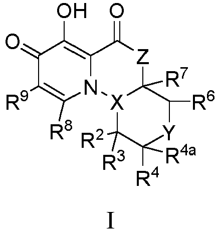 and pharmaceutically acceptable salts thereof. The compounds of Formula I are cap-dependent endonuclease inhibitors, and as such may be useful in the treatment, inhibition or amelioration of one or more disease states that could benefit from inhibition of a virus having a cap-dependent endonuclease, including influenza. The compounds of this invention could further be used in combination with other therapeutically effective agents, including but not limited to, other drugs useful for the treatment of influenza. The invention furthermore relates to processes for preparing compounds of Formula I, and pharmaceutical compositions which comprise compounds of Formula I and pharmaceutically acceptable salts thereof.

Any references to methods of treatment in the subsequent paragraphs of this description are to be interpreted as references to the compounds, pharmaceutical compositions and medicaments of the present invention for use in a method for treatment of the human (or animal) body by therapy (or for diagnosis).

### DETAILED DESCRIPTION OF THE INVENTION

The present invention relates to compounds of Formula I: 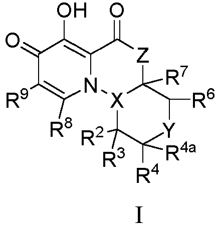 wherein X is N or CH;
Y is absent, CHR⁵*,* -CH₂-CHR⁵-, -CH₂-CHR⁵-CH₂-, S, SO or SO₂;
Z is NR¹ or CR¹R^{1a};
R¹ is selected from the group consisting of hydrogen, C₁₋₆ alkyl, C₁₋₃ alkyl(C₃₋₇ cycloalkyl), C₁₋₃ alkyl(heterocycyl), (C₁₋₆ alkyl)OR^{x} and C₁₋₃ haloalkyl, wherein said cycloalkyl group can be monocyclic or bicyclic, and is optionally substituted with one or two substituents independently selected from the group consisting of halo and R^{x};
R^{1a} is selected from the group consisting of hydrogen, C₁₋₆ alkyl, C₁₋₃ alkyl(cyclopropyl), (C₁₋₆ alkyl)OR^{x} and C₁₋₃ haloalkyl;
R² is aryl, heteroaryl, cycloalkyl or heterocyclyl, wherein said aryl may be monocyclic or bicyclic, wherein said aryl, heteroaryl, cycloalkyl and heterocyclyl are optionally substituted with one to three substituents independently selected from the group consisting of halo, cyano, cyclopropyl, R^{x}, R^{y}, SR^{x} and OR^{x};
R³ is hydrogen, hydroxy, C₁₋₆ alkyl, OR^{x} or C₁₋₃ haloalkyl;
or R² and R³ can be taken with the carbon atom or heteroatom to which they are attached to form dihydroindene, 1,2,3,4-tetrahydronaphthalene, chromane, 2,3-dihydrobenzo[b]thiophene, dihydrobenzofuran or thiochromane;
R⁴ is selected from the group consisting of hydrogen, hydroxy, N₃, NH(C=O)R^{x}, SR^{x}, C₁₋₆ alkyl, C₂₋₆ alkenyl, O(C₁₋₆ alkyl), O(C₂₋₆ alkenyl), (C₁₋₃ alkyl)R^{y}, O(C₁₋₃ alkyl)R^{y}, R^{y} and heteroaryl, wherein said alkyl groups are optionally substituted with one to three substituents independently selected from the group consisting of halo, R^{x}, R^{y}, OR^{x}, cyano and phenyl, wherein said heteroaryl and alkenyl groups are optionally substituted with one to three substituents independently selected from the group consisting of halo, R^{x} and (C₁₋₃ alkyl)OR^{x};
or R² and R⁴ can be taken with the carbon atom or heteroatom to which they are attached to form dihydroindene, 1,2,3,4-tetrahydronaphthalene, 2,3-dihydrobenzo[b]thiophene, dihydrobenzofuran, chromane or thiochromane;
R^{4a} is selected from the group consisting of hydrogen and C₁₋₆ alkyl, wherein said alkyl group is optionally substituted with one to three substituents independently selected from the group consisting of halo and OR^{x};
or R⁴ and R^{4a} can be taken with the carbon atom or heteroatom to which they are attached to form an oxo group or a C₄₋₆ cycloalkyl group, which can be monocyclic or bicyclic, which is optionally substituted with one to three substituents independently selected from the group consisting of halo, C₁₋₃ alkyl and C₁₋₃ haloalkyl;
R⁵ is selected from the group consisting of hydrogen, C₁₋₆ alkyl, C₁₋₃ alkyl(cyclopropyl) and (C₁₋₆ alkyl)OR^{x};
R⁶ is hydrogen, C₁₋₆ alkyl, SR^{x}, NR^{x}(C=O)C₁₋₆ alkyl or (C₂₋₃ alkenyl)R^{y};
or R⁴ and R⁶ can be taken with the carbon atoms to which they are attached to form a C₄₋₆ cycloalkyl group;
R⁷ is hydrogen or C₁₋₃ alkyl;
R⁸ is hydrogen, C₁₋₆ alkyl or (C₁₋₆ alkyl)OR^{x};
R⁹ is hydrogen, cyano or C₁₋₆ alkyl, wherein said alkyl is optionally substituted with one to three halo;
R^{x} is selected from the group consisting of hydrogen and C₁₋₆ alkyl, wherein said alkyl is optionally substituted with one to three halo;
R^{y} is selected from the group consisting of phenyl and C₃₋₆ cycloalkyl, wherein said phenyl and cycloalkyl groups are optionally substituted with one to three substituents independently selected from the group consisting of halo, cyano and R^{x};
or a pharmaceutically acceptable salt thereof.

In an embodiment of the invention, X is N. In another embodiment of the invention, X is CH.

In an embodiment of the invention, Y is absent. In another embodiment of the invention, Y is CHR⁵. In a class of the embodiment, Y is CH₂. In another embodiment of the invention, Y is -CH₂-CHR⁵*-.* In a class of the embodiment, Y is -CH₂-CH₂-. In another embodiment of the invention, Y is -CH₂-CHR⁵-CH₂-. In a class of the embodiment, Y is -CH₂-CH₂-CH₂-. In another embodiment of the invention, Y is S. In another embodiment of the invention, Y is SO. In another embodiment of the invention, Y is SO₂.

In an embodiment of the invention, R¹ is methyl.

In an embodiment of the invention, R² is phenyl, which is optionally substituted with one or two substituents independently selected from the group consisting of halo, CH₃, CF₃, OCHF₂, and OCH₃.

In an embodiment of the invention, R³ is hydrogen, methyl, ethyl or hydroxy.

In an embodiment of the invention, R⁴ is hydrogen, methyl, ethyl, propyl, trifluoroethyl, CH₂CH₂OH, CH₂CH₂OCH₃ or cycloproylmethyl.

In an embodiment of the invention, R⁵ is hydrogen. In another embodiment of the invention, R⁵ is methyl.

In an embodiment of the invention, R⁶ is hydrogen.

In an embodiment of the invention, R⁴ and R⁶ can be taken with the carbon atoms to which they are attached to form a C₄₋₆ cycloalkyl group.

In an embodiment of the invention, R⁷ is hydrogen. In another embodiment of the invention, R⁷ is methyl.

Reference to the preferred classes and subclasses set forth above is meant to include all combinations of particular and preferred groups unless stated otherwise.

Specific embodiments of the present invention include, but are not limited to compounds 1A to 167 identified herein as Examples 1 to 40, or pharmaceutically acceptable salts thereof.

Also included within the scope of the present invention is a pharmaceutical composition which is comprised of a compound of Formula I as described above and a pharmaceutically acceptable carrier. The invention is also contemplated to encompass a pharmaceutical composition which is comprised of a pharmaceutically acceptable carrier and any of the compounds specifically disclosed in the present application. These and other aspects of the invention will be apparent from the teachings contained herein.

The invention also includes compositions for inhibiting cap-dependent endonuclease in a virus, treating a disease caused by a virus having a cap-dependent endonuclease, treating influenza and preventing influenza, in a mammal, comprising a compound of the invention in a pharmaceutically acceptable carrier. These compositions may optionally include other antiviral agents. The compositions can be added to blood, blood products, or mammalian organs in order to affect the desired inhibitions.

The compounds of the present invention may be administered in the form of a pharmaceutically acceptable salt. The term "pharmaceutically acceptable salt" refers to salts prepared from pharmaceutically acceptable non-toxic bases or acids including inorganic or organic bases and inorganic or organic acids. Salts of basic compounds encompassed within the term "pharmaceutically acceptable salt" refer to non-toxic salts of the compounds of this invention which are generally prepared by reacting the free base with a suitable organic or inorganic acid. Representative salts of basic compounds of the present invention include, but are not limited to, the following: acetate, ascorbate, adipate, alginate, aspirate, benzenesulfonate, benzoate, bicarbonate, bisulfate, bitartrate, borate, bromide, butyrate, camphorate, camphorsulfonate, camsylate, carbonate, chloride, clavulanate, citrate, cyclopentane propionate, diethylacetic, digluconate, dihydrochloride, dodecylsulfanate, edetate, edisylate, estolate, esylate, ethanesulfonate, formic, fumarate, gluceptate, glucoheptanoate, gluconate, glutamate, glycerophosphate, glycollylarsanilate, hemisulfate, heptanoate, hexanoate, hexylresorcinate, hydrabamine, hydrobromide, hydrochloride, 2-hydroxyethanesulfonate, hydroxynaphthoate, iodide, isonicotinic, isothionate, lactate, lactobionate, laurate, malate, maleate, mandelate, mesylate, methylbromide, methylnitrate, methylsulfate, methanesulfonate, mucate, 2-naphthalene sulfonate, napsylate, nicotinate, nitrate, N-methylglucamine ammonium salt, oleate, oxalate, pamoate (embonate), palmitate, pantothenate, pectinate, persulfate, phosphate/diphosphate, pimelic, phenylpropionic, polygalacturonate, propionate, salicylate, stearate, sulfate, subacetate, succinate, tannate, tartrate, teoclate, thiocyanate, tosylate, triethiodide, trifluoroacetate, undeconate, valerate and the like. Furthermore, where the compounds of the invention carry an acidic moiety, suitable pharmaceutically acceptable salts thereof include, but are not limited to, salts derived from inorganic bases including aluminum, ammonium, calcium, copper, ferric, ferrous, lithium, magnesium, manganic, mangamous, potassium, sodium, zinc, and the like. Also included are the ammonium, calcium, magnesium, potassium, and sodium salts. Salts derived from pharmaceutically acceptable organic non-toxic bases include salts of primary, secondary, and tertiary amines, cyclic amines, dicyclohexyl amines and basic ion-exchange resins, such as arginine, betaine, caffeine, choline, N,N-dibenzylethylenediamine, diethylamine, 2-diethylaminoethanol, 2-dimethylaminoethanol, ethanolamine, ethylamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and the like. Also, included are the basic nitrogen-containing groups that may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others.

These salts can be obtained by known methods, for example, by mixing a compound of the present invention with an equivalent amount and a solution containing a desired acid, base, or the like, and then collecting the desired salt by filtering the salt or distilling off the solvent. The compounds of the present invention and salts thereof may form solvates with a solvent such as water, ethanol, or glycerol. The compounds of the present invention may form an acid addition salt and a salt with a base at the same time according to the type of substituent of the side chain.

If the compounds of Formula I simultaneously contain acidic and basic groups in the molecule the invention also includes, in addition to the salt forms mentioned, inner salts or betaines (zwitterions).

The present invention encompasses all stereoisomeric forms of the compounds of Formula I. Unless a specific stereochemistry is indicated, the present invention is meant to comprehend all such isomeric forms of these compounds. Centers of asymmetry that are present in the compounds of Formula I can all independently of one another have (R) configuration or (S) configuration. When bonds to the chiral carbon are depicted as straight lines in the structural Formulas of the invention, it is understood that both the (R) and (S) configurations of the chiral carbon, and hence both each individual enantiomer and mixtures thereof, are embraced within the Formula. When a particular configuration is depicted, that entantiomer (either (R) or (S), at that center) is intended. Similarly, when a compound name is recited without a chiral designation for a chiral carbon, it is understood that both the (R) and (S) configurations of the chiral carbon, and hence individual enantiomers and mixtures thereof, are embraced by the name. The production of specific stereoisomers or mixtures thereof may be identified in the Examples where such stereoisomers or mixtures were obtained, but this in no way limits the inclusion of all stereoisomers and mixtures thereof from being within the scope of this invention.

Unless a specific enantiomer or diastereomer is indicated, the invention includes all possible enantiomers and diastereomers and mixtures of two or more stereoisomers, for example mixtures of enantiomers and/or diastereomers, in all ratios. Thus, enantiomers are a subject of the invention in enantiomerically pure form, both as levorotatory and as dextrorotatory antipodes, in the form of racemates and in the form of mixtures of the two enantiomers in all ratios. In the case of a cis/trans isomerism the invention includes both the cis form and the transform as well as mixtures of these forms in all ratios. The preparation of individual stereoisomers can be carried out, if desired, by separation of a mixture by customary methods, for example by chromatography or crystallization, by the use of stereochemically uniform starting materials for the synthesis or by stereoselective synthesis. Optionally a derivatization can be carried out before a separation of stereoisomers. The separation of a mixture of stereoisomers can be carried out at an intermediate step during the synthesis of a compound of Formula I or it can be done on a final racemic product. Absolute stereochemistry may be determined by X-ray crystallography of crystalline products or crystalline intermediates which are derivatized, if necessary, with a reagent containing a stereogenic center of known configuration. Where compounds of this invention are capable of tautomerization, all individual tautomers as well as mixtures thereof are included in the scope of this invention. The present invention includes all such isomers, as well as salts, solvates (including hydrates) and solvated salts of such racemates, enantiomers, diastereomers and tautomers and mixtures thereof.

In the compounds of the invention, the atoms may exhibit their natural isotopic abundances, or one or more of the atoms may be artificially enriched in a particular isotope having the same atomic number, but an atomic mass or mass number different from the atomic mass or mass number predominantly found in nature. The present invention is meant to include all suitable isotopic variations of the specifically and generically described compounds. For example, different isotopic forms of hydrogen (H) include protium (1_{H}) and deuterium (2_{H}). Protium is the predominant hydrogen isotope found in nature. Enriching for deuterium may afford certain therapeutic advantages, such as increasing *in vivo* half-life or reducing dosage requirements, or may provide a compound useful as a standard for characterization of biological samples. Isotopically-enriched compounds can be prepared without undue experimentation by conventional techniques well known to those skilled in the art or by processes analogous to those described in the general process schemes and examples herein using appropriate isotopically-enriched reagents and/or intermediates.

When any variable occurs more than one time in any constituent, its definition on each occurrence is independent at every other occurrence. Also, combinations of substituents and variables are permissible only if such combinations result in stable compounds. Lines drawn into the ring systems from substituents represent that the indicated bond may be attached to any of the substitutable ring atoms. If the ring system is bicyclic, it is intended that the bond be attached to any of the suitable atoms on either ring of the bicyclic moiety.

It is understood that one or more silicon (Si) atoms can be incorporated into the compounds disclosed herein in place of one or more carbon atoms by one of ordinary skill in the art to provide compounds that are chemically stable and that can be readily synthesized by techniques known in the art from readily available starting materials. Carbon and silicon differ in their covalent radius leading to differences in bond distance and the steric arrangement when comparing analogous C-element and Si-element bonds. These differences lead to subtle changes in the size and shape of silicon-containing compounds when compared to carbon. One of ordinary skill in the art would understand that size and shape differences can lead to subtle or dramatic changes in potency, solubility, lack of off-target activity, packaging properties, and so on. (Diass, J. O. et al. Organometallics (2006) 5:1188-1198; Showell, G.A. et al. Bioorganic & Medicinal Chemistry Letters (2006) 16:2555-2558).

It is understood that substituents and substitution patterns on the compounds of the instant invention can be selected by one of ordinary skill in the art to provide compounds that are chemically stable and that can be readily synthesized by techniques known in the art, as well as those methods set forth below, from readily available starting materials. If a substituent is itself substituted with more than one group, it is understood that these multiple groups may be on the same carbon or on different carbons, so long as a stable structure results. The phrase "optionally substituted" (with one or more substituents) should be understood as meaning that the group in question is either unsubstituted or may be substituted with one or more substituents.

Furthermore, compounds of the present invention may exist in amorphous form and/or one or more crystalline forms, and as such all amorphous and crystalline forms and mixtures thereof of the compounds of Formula I are intended to be included within the scope of the present invention. In addition, some of the compounds of the instant invention may form solvates with water (i.e., a hydrate) or common organic solvents. Such solvates and hydrates, particularly the pharmaceutically acceptable solvates and hydrates, of the instant compounds are likewise encompassed within the scope of this invention, along with un-solvated and anhydrous forms.

Accordingly, the compounds within the generic structural formulas, embodiments and specific compounds described and claimed herein encompass salts, all possible stereoisomers and tautomers, physical forms (e.g., amorphous and crystalline forms), solvate and hydrate forms thereof and any combination of these forms, where such forms are possible unless specified otherwise.

The terms used herein have their ordinary meaning and the meaning of such terms is independent at each occurrence thereof. That notwithstanding and except where stated otherwise, the following definitions apply throughout the specification and claims. Chemical names, common names, and chemical structures may be used interchangeably to describe the same structure. These definitions apply regardless of whether a term is used by itself or in combination with other terms, unless otherwise indicated. Hence, the definition of "alkyl" applies to "alkyl" as well as the "alkyl" portions of "hydroxyalkyl," "haloalkyl," "-O-alkyl," etc.

As used herein, and throughout this disclosure, the following terms, unless otherwise indicated, shall be understood to have the following meanings:
A "subject" is a human or non-human mammal. In one embodiment, a subject is a human. In another embodiment, a subject is a primate. In another embodiment, a subject is a monkey. In another embodiment, a subject is a chimpanzee. In still another embodiment, a subject is a rhesus monkey.

As used herein, the terms "treatment" and "treating" refer to all processes in which there may be a slowing, interrupting, arresting, controlling, or stopping of the progression of a disease or disorder described herein. The terms do not necessarily indicate a total elimination of all disease or disorder symptoms.

The terms "preventing," or "prophylaxis," as used herein, refers to reducing the likelihood of contracting disease or disorder described herein, or reducing the severity of a disease or disorder described herein.

The term "alkyl," as used herein, refers to an aliphatic hydrocarbon group having one of its hydrogen atoms replaced with a bond. An alkyl group may be straight or branched and contain from about 1 to about 20 carbon atoms. In one embodiment, an alkyl group contains from about 1 to about 12 carbon atoms. In different embodiments, an alkyl group contains from 1 to 6 carbon atoms (C₁-C₆ alkyl) or from about 1 to about 4 carbon atoms (C₁-C₄ alkyl). Non-limiting examples of alkyl groups include methyl, ethyl, n-propyl, isopropyl, n-butyl, sec-butyl, isobutyl, tert-butyl, n-pentyl, neopentyl, isopentyl, n-hexyl, isohexyl and neohexyl. In one embodiment, an alkyl group is linear. In another embodiment, an alkyl group is branched. Unless otherwise indicated, an alkyl group is unsubstituted.

The term "haloalkyl," as used herein refers to an alkyl group as defined above, wherein one or more of the alkyl group's hydrogen atoms has been replaced with a halogen. In one embodiment, a haloalkyl group has from 1 to 6 carbon atoms. In another embodiment, a haloalkyl group is substituted with from 1 to 3 F atoms. Non-limiting examples of haloalkyl groups include -CH₂F, -CHF₂, -CF₃, -CH₂Cl and -CCl₃. The term "C₁-C₆ haloalkyl" refers to a haloalkyl group having from 1 to 6 carbon atoms.

The term "halo," as used herein, means -F, -Cl, -Br or -I.

The term "cycloalkyl" means a monocyclic or bicyclic saturated aliphatic hydrocarbon group having the specified number of carbon atoms. For example, "cycloalkyl" includes cyclopropyl, cyclobutyl, cyclopentyl, cyclohexyl, and so on. Bicyclic cycloalkyl ring systems include fused ring systems, where two rings share two atoms, and spiro ring systems, where two rings share one atom.

The term "aryl", as used herein, represents a stable bicyclic or tricyclic ring system of up to 10 atoms in each ring, wherein at least one ring is aromatic, and all of the ring atoms are carbon. Bicyclic and tricyclic ring systems include fused ring systems, where two rings share two atoms, and spiro ring systems, where two rings share one atom.

The term "heteroaryl", as used herein, represents a stable monocyclic or bicyclic ring system of up to 10 atoms in each ring, wherein at least one ring is aromatic, and at least one ring contains from 1 to 4 heteroatoms selected from the group consisting of O, N and S. Bicyclic heteroaryl ring systems include fused ring systems, where two rings share two atoms, and spiro ring systems, where two rings share one atom. Heteroaryl groups within the scope of this definition include but are not limited to: azaindolyl, benzoimidazolyl, benzisoxazolyl, benzofuranyl, benzofurazanyl, benzopyrazolyl, benzotriazolyl, benzothiophenyl, benzoxazolyl, carbazolyl, carbolinyl, cinnolinyl, dihydroindenyl, furanyl, indolinyl, indolyl, indolazinyl, indazolyl, isobenzofuranyl, isoindolyl, isoquinolyl, isothiazolyl, isoxazolyl, naphthalenyl, naphthpyridinyl, oxadiazolyl, oxazolyl, oxazoline, isoxazoline, pyranyl, pyrazinyl, pyrazolyl, pyrazolopyrimidinyl, pyridazinyl, pyridopyridinyl, pyridyl, pyrimidinyl, pyrrolyl, quinazolinyl, quinolyl, quinoxalinyl, tetrazolyl, tetrazolopyridyl, thiadiazolyl, thiazolyl, thienyl, triazolyl, dihydrobenzoimidazolyl, dihydrobenzofuranyl, dihydrobenzothiophenyl, dihydrobenzoxazolyl, dihydroindolyl, dihydroquinolinyl, dihydrobenzodioxinyl, dihydropyrazoloxazinyl, dihydropyrazolyothiazinedioxidyl, methylenedioxybenzene, benzothiazolyl, benzothienyl, quinolinyl, isoquinolinyl, oxazolyl, tetra-hydroquinoline and 3-oxo-3,4dihydro-2N-benzo[b][1,4]thiazine. If the heteroaryl contains nitrogen atoms, it is understood that the corresponding N-oxides thereof are also encompassed by this definition.

The term "heterocycle," "heterocycloalkyl" gor "heterocyclyl" as used herein is intended to mean a stable nonaromatic monocyclic or bicyclic ring system of up to 10 atoms in each ring, unless otherwise specified, containing from 1 to 4 heteroatoms selected from the group consisting of O, N, S, SO, or SO₂. Bicyclic heterocyclic ring systems include fused ring systems, where two rings share two atoms, and spiro ring systems, where two rings share one atom. "Heterocyclyl" therefore includes, but is not limited to the following: azaspirononanyl, azaspirooctanyl, azetidinyl, dioxanyl, oxadiazaspirodecenyl, oxaspirooctanyl, oxazolidinonyl, piperazinyl, piperidinyl, pyrrolidinyl, morpholinyl, thiomorpholinyl, tetrahydrofuranyl, tetrahydropyranyl, dihydropiperidinyl, tetrahydrothiophenyl and the like. If the heterocycle contains a nitrogen, it is understood that the corresponding N-oxides thereof are also encompassed by this definition.

"Celite^{®}" (Fluka) diatomite is diatomaceous earth, and can be referred to as "celite".

The term "substituted" means that one or more hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds. By "stable compound' or "stable structure" is meant a compound that is sufficiently robust to survive isolation to a useful degree of purity from a reaction mixture, and formulation into an efficacious therapeutic agent.

The term "in substantially purified form," as used herein, refers to the physical state of a compound after the compound is isolated from a synthetic process (e.g., from a reaction mixture), a natural source, or a combination thereof. The term "in substantially purified form," also refers to the physical state of a compound after the compound is obtained from a purification process or processes described herein or well-known to the skilled artisan (e.g., chromatography, recrystallization and the like), in sufficient purity to be characterizable by standard analytical techniques described herein or well-known to the skilled artisan.

It should also be noted that any carbon as well as heteroatom with unsatisfied valences in the text, schemes, examples and tables herein is assumed to have the sufficient number of hydrogen atom(s) to satisfy the valences.

When a functional group in a compound is termed "protected", this means that the group is in modified form to preclude undesired side reactions at the protected site when the compound is subjected to a reaction. Suitable protecting groups will be recognized by those with ordinary skill in the art as well as by reference to standard textbooks such as, for example, T. W. Greene et al, Protective Groups in Organic Synthesis (1991), Wiley, New York.

When any substituent or variable (e.g., R^{x}) occurs more than one time in any constituent or in Formula I, its definition on each occurrence is independent of its definition at every other occurrence, unless otherwise indicated.

As used herein, the term "composition" is intended to encompass a product comprising the specified ingredients in the specified amounts, as well as any product which results from combination of the specified ingredients in the specified amounts.

The invention also relates to medicaments containing at least one compound of the Formula I and/or of a pharmaceutically acceptable salt of the compound of the Formula I and/or an optionally stereoisomeric form of the compound of the Formula I or a pharmaceutically acceptable salt of the stereoisomeric form of the compound of Formula I, together with a pharmaceutically suitable and pharmaceutically acceptable vehicle, additive and/or other active substances and auxiliaries.

The term "patient" used herein is taken to mean mammals such as primates, humans, sheep, horses, cattle, pigs, dogs, cats, rats, and mice.

The term "influenza" includes seasonal influenza, pandemic influenza, avian influenza, swine influenza and influenza disease in humans or animals. Seasonal influenza is caused by Influenza A and/or Influenza B viruses.

The medicaments according to the invention can be administered by oral, inhalative, rectal or transdermal administration or by subcutaneous, intraarticular, intraperitoneal or intravenous injection. Oral administration is preferred. Coating of stents with compounds of the Formula (I) and other surfaces which come into contact with blood in the body is possible.

The invention also relates to a process for the production of a medicament, which comprises bringing at least one compound of the Formula (I) into a suitable administration form using a pharmaceutically suitable and pharmaceutically acceptable carrier and optionally further suitable active substances, additives or auxiliaries.

Suitable solid or galenical preparation forms are, for example, granules, powders, coated tablets, tablets, (micro)capsules, suppositories, syrups, juices, suspensions, emulsions, drops or injectable solutions and preparations having prolonged release of active substance, in whose preparation customary excipients such as vehicles, disintegrants, binders, coating agents, swelling agents, glidants or lubricants, flavorings, sweeteners and solubilizers are used. Frequently used auxiliaries which may be mentioned are magnesium carbonate, titanium dioxide, lactose, mannitol and other sugars, talc, lactose, gelatin, starch, cellulose and its derivatives, animal and plant oils such as cod liver oil, sunflower, peanut or sesame oil, polyethylene glycol and solvents such as, for example, sterile water and mono- or polyhydric alcohols such as glycerol.

The dosage regimen utilizing the cap-dependent endonuclease inhibitors of the instant invention is selected in accordance with a variety of factors including type, species, age, weight, sex and medical condition of the patient; the severity of the condition to be treated; the route of administration; the renal and hepatic function of the patient; and the particular compound or salt thereof employed. An ordinarily skilled physician or veterinarian can readily determine and prescribe the effective amount of the drug required to prevent, counter, or arrest the progress of the condition.

Oral dosages of the cap-dependent endonuclease inhibitors, when used for the indicated effects, will range between about 0.01 mg per kg of body weight per day (mg/kg/day) to about 30 mg/kg/day, preferably 0.025-7.5 mg/kg/day, more preferably 0.1-2.5 mg/kg/day, and most preferably 0.1-0.5 mg/kg/day (unless specified otherwise, amounts of active ingredients are on free base basis). For example, an 80 kg patient would receive between about 0.8 mg/day and 2.4 g/day, preferably 2-600 mg/day, more preferably 8-200 mg/day, and most preferably 8-40 mg/kg/day. A suitably prepared medicament for once a day administration would thus contain between 0.8 mg and 2.4 g, preferably between 2 mg and 600 mg, more preferably between 8 mg and 200 mg, and most preferably 8 mg and 40 mg, e.g., 8 mg, 10 mg, 20 mg and 40 mg. Advantageously, the cap-dependent endonuclease inhibitors may be administered in divided doses of two, three, or four times daily. For administration twice a day, a suitably prepared medicament would contain between 0.4 mg and 4 g, preferably between 1 mg and 300 mg, more preferably between 4 mg and 100 mg, and most preferably 4 mg and 20 mg, e.g., 4 mg, 5 mg, 10 mg and 20 mg.

Intravenously, the patient would receive the active ingredient in quantities sufficient to deliver between 0.025-7.5 mg/kg/day, preferably 0.1-2.5 mg/kg/day, and more preferably 0.1-0.5 mg/kg/day. Such quantities may be administered in a number of suitable ways, e.g. large volumes of low concentrations of active ingredient during one extended period of time or several times a day, low volumes of high concentrations of active ingredient during a short period of time, e.g. once a day. Typically, a conventional intravenous formulation may be prepared which contains a concentration of active ingredient of between about 0.01-1.0 mg/mL, e.g. 0.1 mg/mL, 0.3 mg/mL, and 0.6 mg/mL, and administered in amounts per day of between 0.01 mL/kg patient weight and 10.0 mL/kg patient weight, e.g. 0.1 mL/kg, 0.2 mL/kg, 0.5 mL/kg. In one example, an 80 kg patient, receiving 8 mL twice a day of an intravenous formulation having a concentration of active ingredient of 0.5 mg/mL, receives 8 mg of active ingredient per day. Glucuronic acid, L-lactic acid, acetic acid, citric acid or any pharmaceutically acceptable acid/conjugate base with reasonable buffering capacity in the pH range acceptable for intravenous administration may be used as buffers. The choice of appropriate buffer and pH of a formulation, depending on solubility of the drug to be administered, is readily made by a person having ordinary skill in the art.

Compounds of Formula I can be administered both as a monotherapy and in combination with other therapeutic agents, including other antivirals or treatments of influenza.

The cap-dependent endonuclease inhibitors can also be co-administered with suitable antivirals, including, but not limited to, M2 ion channel inhibitors, neuraminidase inhibitors, nucleoside analogs and inhibitors targeting the endonuclease activity of the PA protein.

Alternatively or additionally, one or more additional pharmacologically active agents may be administered in combination with a compound of the invention. The additional active agent (or agents) is intended to mean a pharmaceutically active agent (or agents) that is active in the body, including pro-drugs that convert to pharmaceutically active form after administration, which is different from the compound of the invention, and also includes freeacid, free-base and pharmaceutically acceptable salts of said additional active agents when such forms are sold commercially or are otherwise chemically possible. Generally, any suitable additional active agent or agents, including but not limited to M2 ion channel inhibitors, neuraminidase inhibitors, nucleoside analogs and inhibitors targeting the endonuclease activity of the PA protein may be used in any combination with the compound of the invention in a single dosage formulation (a fixed dose drug combination), or may be administered to the patient in one or more separate dosage formulations which allows for concurrent or sequential administration of the active agents (co-administration of the separate active agents).

Typical doses of the cap-dependent endonuclease inhibitors of the invention in combination with other suitable M2 ion channel inhibitors, neuraminidase inhibitors, nucleoside analogs and inhibitors targeting the endonuclease activity of the PA protein may be the same as those doses of the cap-dependent endonuclease inhibitors administered without coadministration of additional M2 ion channel inhibitors, neuraminidase inhibitors, nucleoside analogs and inhibitors targeting the endonuclease activity of the PA protein, or may be substantially less that those doses of thrombin inhibitors administered without coadministration of M2 ion channel inhibitors, neuraminidase inhibitors, nucleoside analogs and inhibitors targeting the endonuclease activity of the PA protein, depending on a patient's therapeutic needs.

The compounds are administered to a mammal in a therapeutically effective amount. By "therapeutically effective amount" it is meant an amount of a compound of the present invention that, when administered alone or in combination with an additional therapeutic agent to a mammal, is effective to treat (i.e., prevent, inhibit or ameliorate) the viral condition or treat the progression of the disease in a host.

The compounds of the invention are preferably administered alone to a mammal in a therapeutically effective amount. However, the compounds of the invention can also be administered in combination with an additional therapeutic agent, as defined below, to a mammal in a therapeutically effective amount. When administered in a combination, the combination of compounds is preferably, but not necessarily, a synergistic combination. Synergy, as described for example by Chou and Talalay, Adv. Enzyme Regul. 1984, 22, 27-55, occurs when the effect (in this case, inhibition of the desired target) of the compounds when administered in combination is greater than the additive effect of each of the compounds when administered individually as a single agent. In general, a synergistic effect is most clearly demonstrated at suboptimal concentrations of the compounds. Synergy can be in terms of lower cytotoxicity, increased anticoagulant effect, or some other beneficial effect of the combination compared with the individual components.

By "administered in combination" or "combination therapy" it is meant that the compound of the present invention and one or more additional therapeutic agents are administered concurrently to the mammal being treated. When administered in combination each component may be administered at the same time or sequentially in any order at different points in time. Thus, each component may be administered separately but sufficiently closely in time so as to provide the desired therapeutic effect.

The present invention is not limited in scope by the specific embodiments disclosed in the examples which are intended as illustrations of a few aspects of the invention and any embodiments that are functionally equivalent are within the scope of this invention. Indeed, various modifications of the invention in addition to those shown and described herein will become apparent to those skilled in the relevant art and are intended to fall within the scope of the appended claims.

### GENERAL METHODS

Several methods for preparing the compounds of this invention are illustrated in the following Schemes and Examples. Starting materials and the requisite intermediates are in some cases commercially available, or can be prepared according to literature procedures or as illustrated herein. The compounds of this invention may be prepared by employing reactions as shown in the following schemes, in addition to other standard manipulations that are known in the literature or exemplified in the experimental procedures. Substituent numbering as shown in the schemes does not necessarily correlate to that used in the claims and often, for clarity, a single substituent is shown attached to the compound where multiple substituents are allowed under the definitions hereinabove. Reactions used to generate the compounds of this invention are carried out by employing reactions as shown in the schemes and examples herein, in addition to other standard manipulations such as ester hydrolysis, cleavage of protecting groups, etc., as may be known in the literature or exemplified in the experimental procedures. Starting materials are made according to procedures known in the art or as illustrated herein.

The compounds of the present invention can be prepared in a variety of fashions. In some cases, the final product may be further modified, for example, by manipulation of substituents. These manipulations may include, but are not limited to, reduction, oxidation, alkylation, acylation, and hydrolysis reactions which are commonly known to those skilled in the art. In some cases, the order of carrying out the foregoing reaction schemes may be varied to facilitate the reaction or to avoid unwanted reaction products. Because the schemes are an illustration, the invention should not be construed as being limited by the chemical reactions and conditions expressed. The preparation of the various starting materials used herein is well within the skill of a person versed in the art. The following examples are provided so that the invention might be more fully understood. These examples are illustrative only and should not be construed as limiting the invention in any way. Absolute stereochemistry of separate stereoisomers in the examples and intermediates are not determined unless stated otherwise in an example or explicitly in the nomenclature.

When chiral resolution was achieved by chromatography using chiral columns, the chiral columns used for SFC chiral resolutions are listed in tables. Some of the chiral columns used were CHIRALPAK AD, CHIRALCEL OJ, CHIRALPAK AS, CHIRALPAK AY, CHIRALPAK IA, CHIRALPAK AD-H, and CHIRALPAK AS-H. Henceforth, they will be referred by their two or three letter abbreviations. As a convention, the fast-eluting isomer from a chiral resolution is always listed first in this table followed immediately by the slower-eluting isomer from the same resolution. If more than two isomers were separated, they will be always listed in the tables in order they were eluted, such as Peak 1 followed by Peak 2, Peak 3 and so on.

Catalysts are used in the following procedures. "Grubbs II" is also known as "Grubbs catalyst 2^{nd} generation" and (1,3-Bis(2,4,6-trimethylphenyl)-2-imidazolidinylidene)dichloro(phenylmethylene)(tricyclohexylphosphine)ruthenium; Carbonyltris(triphenylphosphine)rhodium(I) hydride; Wilkinson's catalyst is also known as chloridotris(triphenylphosphine)rhodium(I); all of which are available from Millipore Sigma.

### GENERAL SCHEME

### Methods for Making the Compounds of Formula (I)

The Compounds of Formula (I) may be prepared from known or readily prepared starting materials, following methods known to one skilled in the art of organic synthesis. Methods useful for making the Compounds of Formula (I) are set forth in the Examples below and generalized in Schemes 1, 2, 3, 4, 5 and 6 below. Alternative synthetic pathways and analogous structures will be apparent to those skilled in the art of organic synthesis.

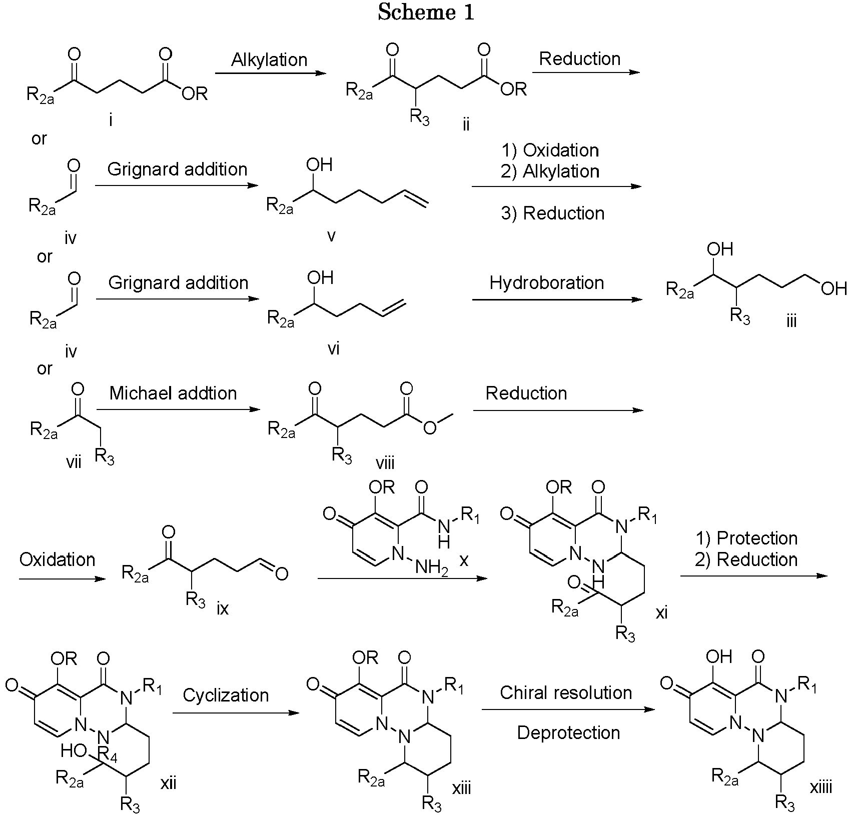

Diol **iii** was prepared from multiple-steps syntheses, either from commercially available **i,** aldehyde **iv** or ketone **vii.** It was then oxidized via Dess-Martin periodinane to provide aldehyde **ix.** The aldehyde may be reacted with a compound of formula **x** to provide a compound of formula **xi.** Direct reduction of compound **xi** followed by cyclization affords a compound of formula **xiii.** Alternatively, protection of compound **xi** followed by a two-step sequence also allows the formation of a compound of formula **xiii.** Chiral resolution to separate the diastereomers of a compound of formula **xiii,** followed by deprotection provides a compound of formula **xiiii** which correspond to the compounds of formula (I).

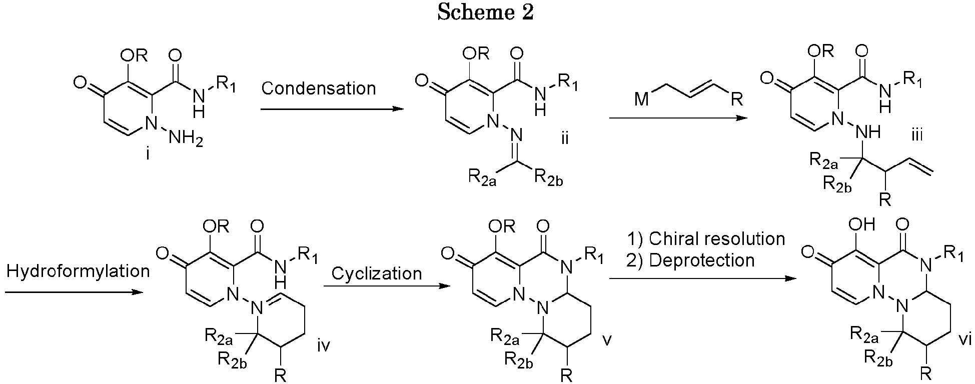

The hydrazone compound of formula ii may be formed by condensation of a compound of formula i and an appropriate ketone or aldehyde which, in turn, may subsequently be reacted with an organometallic reagent to provide a compound of formula **iii.** Hydroformylation of the compound of formula **iii** followed by cyclization to provide a compound of formula v. Chiral resolution to separate the diastereomers of a compound of formula v, followed by deprotection provides compound of formula vi which correspond to the compounds of formula (I).

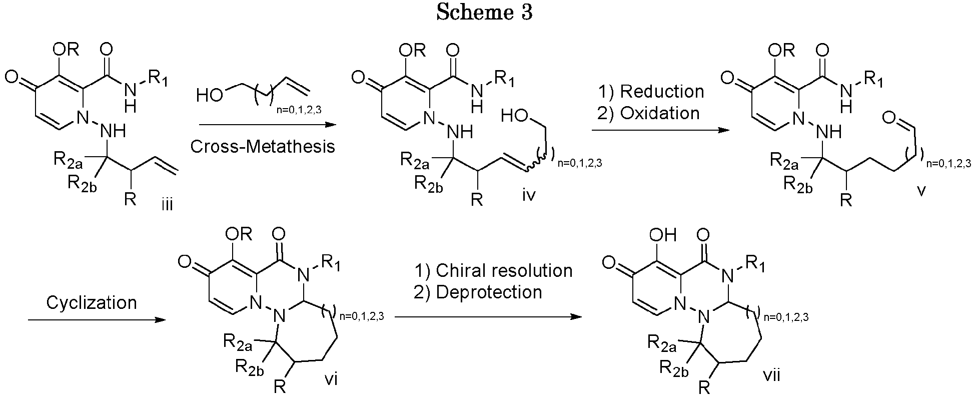

A compound of formula **iii** may be made following essentially the method employed in **Scheme 2.** Two-, three- or four-carbon extensions of compound **iii** through crossmetathesis provides a compound of formula **iv.** Sequentially reduction and oxidation of compound **iv** affords aldehyde compound of formula **v** which allows further cyclization to provide compound **vi.** Chiral resolution to separate the diastereomers of a compound of formula **vi,** followed by deprotection provides a compound of formula **vii** which correspond to the compounds of formula (I).

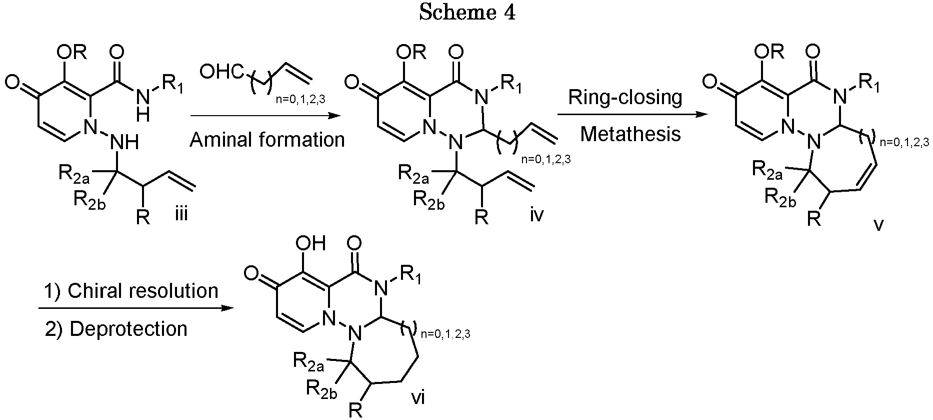

A compound of formula iii may be made following essentially the method employed in **Scheme 2.** It was then reacted with the corresponding aldehyde to form aminal iv which allows ring-closing metathesis to provide a compound of formula v. Reduction of the carbon-carbon double bond and subsequent chiral resolution to separate the diastereomers followed by deprotection provides a compound of formula vi which corresponds to the compounds of formula (I).

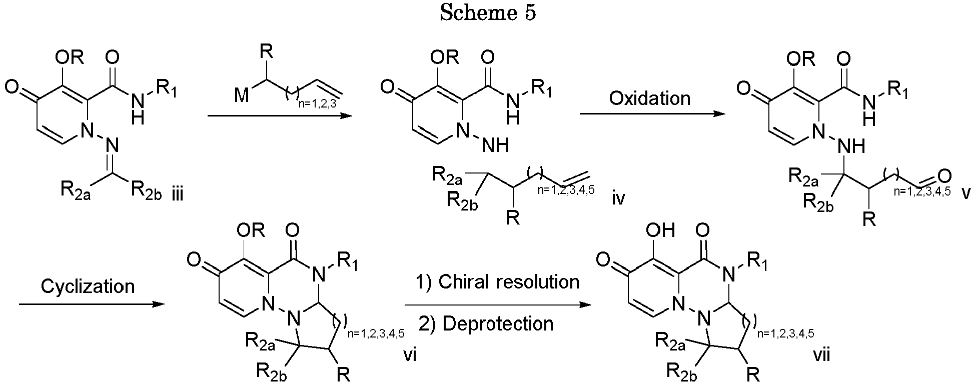

A compound of formula iii may be made following essentially the method employed in **Scheme 2.** It was then reacted with the corresponding organometallic reagent to form aminal **iv** which allows oxidation to provide compound of formula **v.** Cyclization and subsequent chiral resolution to separate the diastereomers followed by deprotection provides a compound of formula **vii** which correspond to the compounds of formula (I).

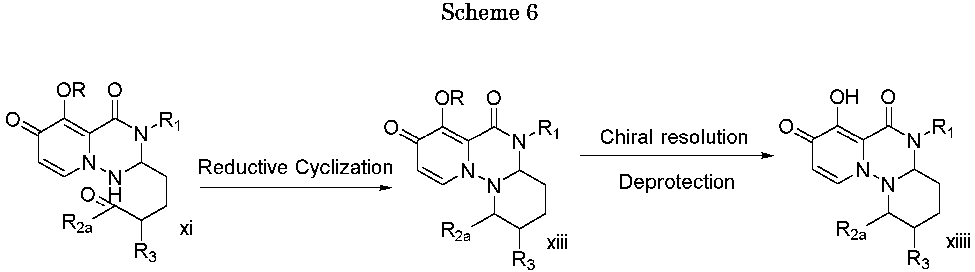

A compound of formula xiii described in **Scheme 1** may be made from a compound of formula xi through reductive cyclization. Chiral resolution to separate the diastereomers of a compound of formula **xiii,** followed by deprotection provides compound of formula **xiiii** which corresponds to the compounds of formula (I).

In the methods for preparing compounds of the present invention set forth in the foregoing schemes, functional groups in various moieties and substituents (in addition to those already explicitly noted in the foregoing schemes) may be sensitive or reactive under the reaction conditions employed and/or in the presence of the reagents employed. Such sensitivity/reactivity can interfere with the progress of the desired reaction to reduce the yield of the desired product, or possibly even preclude its formation. Accordingly, it may be necessary or desirable to protect sensitive or reactive groups on any of the molecules concerned. Protection may be achieved by means of conventional protecting groups, such as those described in Protective Groups in Organic Chemistry, ed. J.F.W. McOmie, Plenum Press, 1973 and in T.W. Greene & P.G.M. Wuts, Protective Groups in Organic Synthesis, John Wiley & Sons, 3rd edition, 1999, and 2nd edition, 1991. The protecting groups may be removed at a convenient subsequent stage using methods known in the art. Alternatively, the interfering group may be introduced into the molecule subsequent to the reaction Step of concern.

One skilled in the art of organic synthesis will recognize that the synthesis of compounds with multiple reactive functional groups, such as -OH and NH₂, may require protection of certain functional groups (*i.e.,* derivatization for the purpose of chemical compatibility with a reaction condition). Suitable protecting groups for the various functional groups of these compounds and methods for their installation and removal are well-known in the art of organic chemistry. A summary of many of these methods may be found in Greene & Wuts, Protecting Groups in Organic Synthesis, John Wiley & Sons, 3rd edition (1999).

One skilled in the art of organic synthesis will also recognize that one route for the synthesis of the Compounds of Formula (I) may be more desirable depending on the choice of appendage substituents. Additionally, one skilled in the relevant art will recognize that in some cases the order of reactions may differ from that presented herein to avoid functional group incompatibilities and thus adjust the synthetic route accordingly.

Compounds of formula **vii, x, xv** and **xvii** may be further elaborated using methods that would be well-known to those skilled in the art of organic synthesis or, for example, the methods described in the Examples below, to make the full scope of the Compounds of Formula (I).

The starting materials used, and the intermediates prepared using the methods set forth in Schemes 1 to 6 may be isolated and purified if desired using conventional techniques, including but not limited to filtration, distillation, crystallization, chromatography and alike. Such materials may be characterized using conventional means, including physical constants and spectral data.

The following abbreviations may be used in the following experimentals:

| | |
|---|---|
| °C | Degrees Celsius |
| ACN | Acetonitrile |
| AcOH | Acetic acid |
| aq. | Aqueous |
| Atm | Atmospheres |
| BiPhePhos | 6-[2-(2-benzo[d][1,3,2]benzodioxaphosphepin-6-yloxy-3-*tert*-butyl-5-methoxyphenyl)-6- *tert-*butyl-4-methoxyphenoxy]benzo[d][1,3,2]benzodioxaphosphepine |
| 9-BBN | 9-Borabicyclo [3.3.1]nonane |
| BHT | 3,5-Di-tert-4-butylhydroxytoluene |
| BSA | *N*,*O*-Bis(trimethylsilyl)acetamide |
| CDI | 1,1'-Carbonyldiimidazole |
| CD₃OD | Deuterated Methanol-*d4* |
| CPME | Cyclopentyl methyl ether |
| DBU | Diazabicycloundecene |
| DCC | N,N'-Dicyclohexylcarbodiimide |
| DCE | 1,2-Dichloroethane |
| DCM | Dichloromethane |
| DDQ | 2,3-Dichloro-5,6-dicyano-p-benzoquinone |
| DEA | Diethylamine |
| DEAD | Diethyl azodicarboxylate |
| DIAD | Diisopropyl azodicarboxylate |
| DIBAL | Diisobutylaluminium hydride |
| DIEA | *N,N-*Diisopropylethylamine |
| DIPA | Diisopropylamine |
| DIPEA | *N,N-*Diisopropylethylamine |
| DMA | *N,N*-Dimethylacetamide |
| DMAP | 4-Dimethylaminopyridine |
| DME | Dimethoxyethane |
| DMF | Dimethylformamide |
| DMP | Dess-Martin periodinane |
| DMPU | 1,3-Dimethyl-3,4,5,6-tetrahydro-2(1H)-pyrimidinone |
| DMSO | Dimethyl Sulfoxide |
| DMSO*-d6* | Deuterated Dimethyl Sulfoxide |
| DPP | Diphenylphosphine |
| Dppf | Bis(diphenylphosphino)ferrocene |
| ESI | Electrospray Ionization |
| Et₃N | Triethylamine |
| Et₂O | Diethylether |
| EtOAc | Ethyl Acetate |
| EtOH | Ethanol |
| h | Hours |
| HATU | (1-[Bis(dimethylamino)methylene]-1H-1,2,3-triazolo[4,5-b]pyridinium 3-oxide hexafluorophosphate |
| HPLC | High Performance Liquid Chromatography |
| IPA | Isopropyl alcohol |
| LAH | Lithium Aluminum Hydride |
| LED | Light-emitting diode |
| LiHMDS | Lithium bis(trimethylsilyl)amide |
| M | Molar |
| MeOD-*d4* | Deuterated Methanol |
| MeOH | Methanol |
| MHz | Megahertz |
| Min | Minutes |
| mL | Milliliters |
| MOMCl | Methoxymethyl chloride |
| MP TMT resin | Macroporous polystyrene-bound trimercaptotriazine, a resin bound equivalent of 2,4,6-trimercaptotriazine |
| MS | Mass Spectroscopy |
| MTBE | Methyl tert-butyl ether |
| TsCl | *p*-Toluenesulfonyl chloride |
| NaHMDS | Sodium bis(trimethylsilyl)amide |
| NBS | *N*-Bromosuccinimide |
| Nm | Nanometers |
| NMP | *N*-Methyl-2-pyrrolidone |
| NMR | Nuclear Magnetic Resonance |
| N-XantPhos | 4,6-Bis(diphenylphosphino)-10H-phenoxazine |
| Pd/C | Palladium on Carbon |
| Prep SFC | Preparative Super Critical Fluid (CO2) |
| *p*-TsOH | 4-Methylbenzenesulfonic acid |
| RT | Room temperature |
| sat. | Saturated |
| TBAF | Tetrabutylammonium fluoride |
| TBME | Methyl *tert*-butyl ether |
| TEA | Triethanolamine |
| TFA | Trifluoroacetic acid |
| TFE | 2,2,2-Trifluoroethanol |
| Tf₂O | Trifluoromethanesulfonic anhydride |
| THF | Tetrahydrofuran |
| TLC | Thin Layer Chromatography |
| TMS | Trimethylsilyl |
| PE | Petroleum ether |
| 2-Me-THF | 2-Methyltetrahydrofuran |
| SEMCl | 2-(Trimethylsilyl)ethoxymethyl chloride |
| T₃P | Propanephosphonic acid anhydride |

### EXAMPLES

The following examples serve only to illustrate the invention and its practice. The examples are not to be construed as limitations on the scope or spirit of the invention. In these examples, all temperatures are degrees Celsius unless otherwise noted, and "room temperature" refers to a temperature in a range of from about 20 °C to about 25 °C. Reactions sensitive to moisture or air were performed under nitrogen using anhydrous solvents and reagents. The progress of reactions was determined by either analytical thin layer chromatography (TLC) performed with E. Merck precoated TLC plates, silica gel 60F-254, layer thickness 0.25 mm or liquid chromatography-mass spectrum (LC-MS). For HPLC/MS data, the two HPLC conditions used were as follows: 1) LC2 (Waters C18 XTerra^{™} 3.5 µm 2.1x20 mm column with gradient 10:90-98:2 v/v CH₃CN/H₂O + v 0.05 % TFA over 1.25 min then hold at 98:2 v/v CH₃CN/H₂O + v 0.05% TFA for 0.75 min; flow rate 1.5 mL/min, UV wavelength 254 nm); and 2) LC4 (Waters C18 XTerra 3.5 µm 2.1x20 mm column with gradient 10:90-98:2 v/v CH₃CN/H₂O + v 0.05 % TFA over 3.25 min then hold at 98:2 v/v CH₃CN/H₂O + v 0.05 % TFA for 0.75 min; flow rate 1.5 mL/min, UV wavelength 254 nm).

Mass analysis was performed with electrospray ionization in positive ion detection mode. ¹H NMR spectra were recorded on Varian or Bruker instruments at 400-500 MHz. Chemical shifts are reported in ppm downfield and up field from tetramethylsilane (TMS) and referenced to either internal TMS or solvent resonances (¹H NMR: δ 7.27 for C*D*Cl₃, δ 2.50 for (CD₃)(C*H*D₂)SO, and ¹³C NMR: δ 77.02 for *C*DCl₃, δ 39.51 for (*C*D₃)₂SO. Coupling constants (J) are expressed in hertz (Hz), and spin multiplicities are given as s (singlet), d (doublet), dd (double doublet), t (triplet), m (multiplet), and br (broad). Chiral resolutions were performed on either Waters Thar 80 SFC or Berger MG II preparative SFC systems.

Concentration of solutions was carried out on a rotary evaporator under reduced pressure or by lyophilization. Flash chromatography was performed on pre-packed silica gel columns using a commercial MPLC system. Compounds described herein were synthesized as racemic mixtures unless otherwise stated in the experimental procedures.

### Example 1

### Preparation of Compounds 1A and 1B

### 7-hydroxy-5-methyl-1-phenyl-1,2,3,4,4a,5-hexahydrodipyrido[1,2-b:2',1'-f)[1,2,4]triazine-6,8-dione

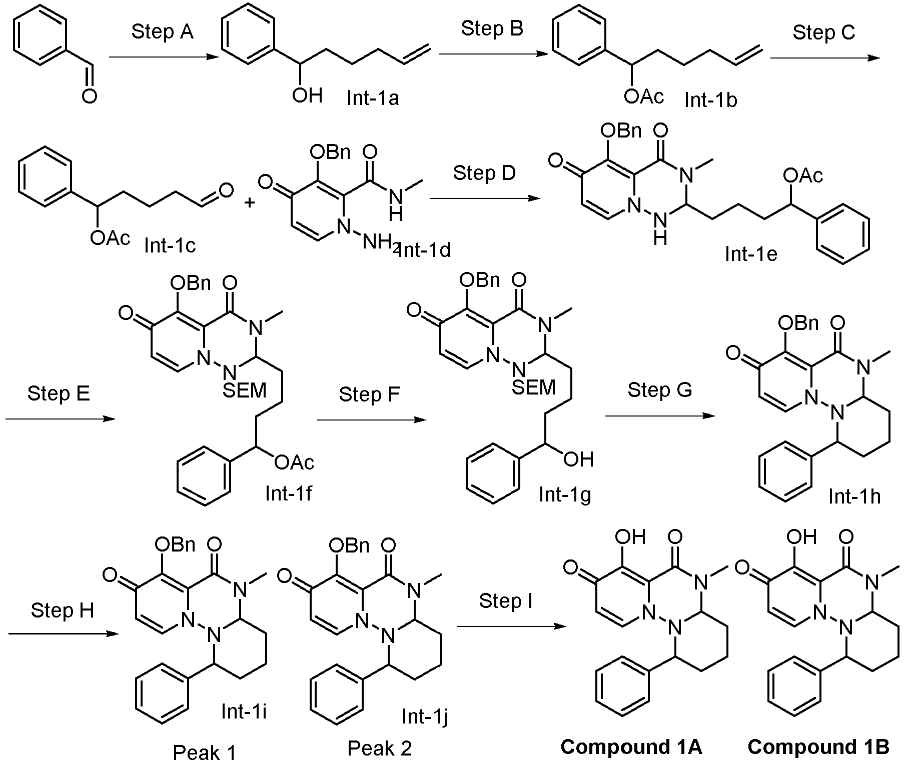

### Step A - Synthesis of Compound Int-1a

To a solution of benzaldehyde (10 g, 94 mmol) in THF (100 mL) at 0 °C was added pent-4-en-1-ylmagnesium bromide (23.23 g, 134 mmol) under N₂ atmosphere. The mixture was slowly warmed up to RT for 4 h. Upon completion, to above mixture was added 100 mL aqueous NH₄Cl solution. The mixture was extracted with EtOAc (3x60 mL). The combined organic layer was washed with brine (100 mL), dried over anhydrous Na₂SO₄, filtered and concentrated. The residue was purified by a 120 g flash silica gel column with 0-100% EtOAc in hexane as eluting solvent to afford ***Int-1a.*** ¹H NMR (500 MHz, Chloroform-d) *δ* 7.34-7.38 (m, 4H), 7.27-7.31 (m, 1H), 5.79 (tdd, *J =* 6.68, 10.22, 17.01 Hz, 1H), 5.74-5.84 (m, 1H), 4.93-5.03 (m, 2H), 4.69 (br t, J=6.56 Hz, 1H), 2.09 (q, *J =* 7.12 Hz, 2H), 1.70-1.87 (m, 3H), 1.49-1.59 (m, 1H), 1.34-1.45 (m, 1H).

### Step B - Synthesis of Compound Int-1b

To a solution of ***Int-1a*** (8 g, 45.4 mmol) in DCM (50 mL) was added acetyl chloride (5.34 g, 68.1 mmol) and TEA (18.98 mL, 136 mmol) at 0 °C. The mixture was slowly warmed up to room temperature for 16 h. Upon completion, to above reaction was added 50 mL aqueous NH₄Cl solution. The mixture was extracted with EtOAc (3x50 mL). The combined organic layer was washed with brine (100 mL), dried over anhydrous Na₂SO₄, filtered and concentrated. The residue was purified by an 80 g flash silica gel Column with 0~5% EtOAc/PE as eluting solvent to afford ***Int-1b.*** ¹H NMR (500 MHz, Chloroform-d) *δ* 7.27-7.38 (m, 5H), 5.72-5.81 (m, 2H), 4.93-5.04 (m, 2H), 2.06-2.10 (m, 5H), 1.88-1.97 (m, 1H), 1.75-1.84 (m, 1H), 1.45 (ttd, *J =* 5.23, 7.51, 18.18 Hz, 1H), 1.30-1.39 (m, 1H).

### Step C - Synthesis of Compound Int-1c

To a solution of ***Int-1b*** (6 g, 27.5 mmol) in t-BuOH (120 mL) and water (30 mL) was added NaHCO₃ (23.09 g, 275 mmol) and sodium periodate (35.3 g, 165 mmol) at 20 °C. Then osmium tetroxide (6.99 ml, 0.275 mmol) was added at 20 °C. The reaction mixture was stirred at 20 °C for 3 h. Upon completion, 200 mL EtOAc was added to the reaction. The mixture was filtered. The filtrate was washed with water (3x50 mL), brine (100 mL), dried over Na₂SO₄, filtered and concentrated. The residue was purified by an 80 g flash silica gel column with 0-10% EtOAc/PE as eluting solvent to afford ***Int-1c**.* ¹H NMR (400 MHz, DMSO-d⁶) *δ* 9.63 (t, *J =* 1.37 Hz, 1H), 7.26-7.39 (m, 5H), 5.65 (dd, *J =* 5.87, 7.83 Hz, 1H), 2.42-2.48 (m, 2H), 2.05 (s, 3H), 1.67-1.89 (m, 2H), 1.36-1.59 (m, 2H).

### Step D - Synthesis of Compound Int-1e

To a solution of ***Int-1d*** (1 g, 3.66 mmol) in DMF (10 mL) and AcOH (1 mL) was added ***Int-1c*** (1.3 g, 5.90 mmol) at 25 °C in a 40 mL sealed tube. The reaction mixture was stirred at 120 °C for 5 h. Upon completion, the reaction was concentrated and purified by an 80 g flash silica gel column with 1-10% MeOH in DCM as eluting solvent to provide ***Int-1e.*** LCMS anal. calcd. for C₂₇H₂₉N₃O₅: 475.21; Found: 476.2 (M+H)⁺.

### Step E - Synthesis of Compound Int-1f

To a solution of ***Int-1e*** (1.11 g, 2.334 mmol) in DCM (10 mL) was added SEMCl (0.828 mL, 4.67 mmol) and TEA (0.976 mL, 7.00 mmol) at 25 °C. The reaction was stirred at 40 °C for 16 h. Upon completion, the reaction was purified directly by an 80 g silica gel column with 0-5% MeOH in DCM as eluting solvent to provide ***Int-1f.*** LCMS anal. calcd. for C₃₃H₄₃N₃O₆Si: 605.3; Found: 606.4 (M+H)⁺.

### Step F - Synthesis of Compound Int-1g

To a solution of ***Int-1f*** (110 mg, 0.182 mmol) in MeOH (5 mL) was added K₂CO₃ (25.10 mg, 0.182 mmol) at 25 °C. The reaction was stirred at 25 °C for 3 h. Upon completion, it was concentrated and purified by prep silica gel TLC plate with 6% MeOH in DCM as eluting solvent to provide ***Int-1g.*** LCMS anal. calcd. for C₃₁H₄₁N₃O₅Si: 563.3; Found: 564.2 (M+H)⁺.

### Step G - Synthesis of Compound Int-1h

To a solution of ***Int-1g*** (100 mg, 0.177 mmol) in DCM (5 mL) was added methanesulfonyl chloride (0.429 mL, 5.50 mmol) and triethylamine (0.099 mL, 0.710 mmol) at 25 °C. The reaction was stirred at 25 °C for 16 h. Upon completion, the reaction was concentrated to afford the crude product.

To a solution of above crude (103 mg, 0.177 mmol) in THF (1 mL) was added TBAF (1 mL, 1.000 mmol) at 25 °C. The reaction was stirred at 50 °C for 16 h. Upon completion, the reaction was concentrated. The residue was purified by prep silica gel TLC plate with 6% MeOH in DCM as eluting solvent to provide ***Int-1h.*** LCMS anal. calcd. for C₂₅H₂₅N₃O₃: 415.2; Found: 416.3 (M+H)⁺.

### Step H - Synthesis of Compound Int-1i and Int-1j

***Int-1h*** (150 mg, 0.361 mmol) was resolved with SFC to provide the first eluting peak ***Int-1i*** and the second eluting peak ***Int-1j.*** SFC condition: Daicel^{@} chiralpak AS column (250*30 mm, 10 µm); 45% EtOH with 0.1%NH₃H₂O; 70 mL/min. LCMS anal. calcd. for C₂₅H₂₅N₃O₃: 415.2; Found: 416.3 (M+H)⁺.

### Step I - Synthesis of Compound 1A and 1B

To a stirred mixture of ***Int-1i*** (15 mg, 0.036 mmol) in ACN (1 mL) was added magnesium bromide (6.65 mg, 0.036 mmol) at 20 °C. The mixture was stirred at 20 °C for 2 h under N₂ atmosphere. Upon completion, it was purified by HPLC with a C18 column (YMC-Actus Triart 150*30mm*5um) using 0-100% ACN in water (with 0.1% TFA modifier) as eluting solvent to provide compound ***1A.*** LCMS anal. calcd. for C₁₈H₁₉N₃O₃: 325.1; Found: 326.0 (M+H)⁺. ¹H NMR (500 MHz, methanol-d⁴) *δ* 7.08-7.73 (m, 5H), 7.03 (d, *J =* 7.32 Hz, 1H), 5.84 (d, *J* = 7.32 Hz, 1H), 5.30 (t, *J =* 2.90 Hz, 1H), 4.22 (dd, *J =* 2.75, 11.60 Hz, 1H), 3.24 (s, 3H), 2.53 (td, *J =* 2.75, 15.26 Hz, 1H), 2.16-2.33 (m, 2H), 1.85-2.01 (m, 2H), 1.66-1.79 (m, 1H).

Following essentially the method employed to produce compound ***1A*** of example **1**, compound ***1B*** was prepared from ***Int-1j.*** LCMS anal. calcd. for C₁₃H₁₉N₃O₃: 325.1; Found: 326.0 (M+H)⁺. ¹H NMR (500 MHz, methanol-d⁴) *δ* 7.29 (br s, 5H), 6.96 (d, *J =* 7.53 Hz, 1H), 5.73 (d, *J =* 7.53 Hz, 1H), 5.28 (s, 1H), 4.20 (dd, *J =* 2.76, 11.80 Hz, 1H), 3.23 (s, 3H), 2.49-2.54 (m, 1H), 2.17-2.27 (m, 2H), 1.86-1.97 (m, 2H), 1.74 (br t, *J =* 13.55 Hz, 1H).

### Example 2

### Preparation of Compounds 2A and 2B

### 1-(4-fluorophenyl)-7-hydroxy-5-methyl-1,2,3,4,4a,5-hexahydrodipyrido[1,2-b:2',1'-f][1,2,4]triazine-6,8-dione

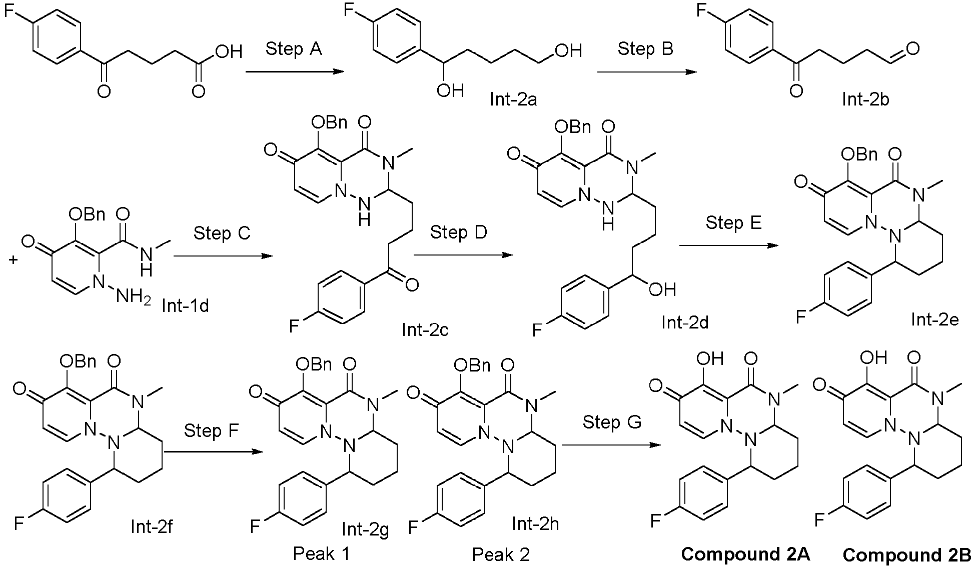

### Step A - Synthesis of Compound Int-2a

The solution of 5-(4-fluorophenyl)-5-oxopentanoic acid (8 g, 38.1 mmol) in DCM (140 mL) and MeOH (50 mL) was added to (diazomethyl)trimethylsilane (34.3 ml, 68.5 mmol) dropwise. The resulting reaction was concentrated. To the residue was added 200 mL DCM at 0 °C, followed by adding diisobutylaluminum hydride (133 mL, 133 mmol). The resulting mixture was stirred at this temperature for 1 hour followed by the addition of 10 mL MeOH and 5 mL water at 0 °C. It was further stirred at room temperature for 30 minutes and filtered through celite. The filtrate was concentrated to provide ***Int-2a.*** LCMS anal. calcd. for C₁₁H₁₅FO₂: 198.1; Found: 181.3 (M-OH)⁺.

### Step B - Synthesis of Compound Int-2b

The solution of ***Int-2a*** (2 g, 10.09 mmol) in DCM (50 mL) was added to Dess Martin periodinane (8.56 g, 20.18 mmol). The resulting mixture was stirred at room temperature for 1 h. It was concentrated to remove most of the DCM. To the above residue was added 20 mL DCM and the mixture was filtered. The filtrate was loaded onto an 80 g silica gel column with 0-100% EtOAc in hexane as eluting solvent to provide ***Int-2b.*** LCMS anal. calcd. for C₁₁H₁₁FO₂: 194.1; Found: 195.3 (M+H)⁺.

### Step C - Synthesis of Compound Int-2c

To a solution of ***Int-2b*** (1.8 g, 6.59 mmol) in DMF (5 mL) and AcOH (0.5 mL) was added ***Int-1d*** (1.4 g, 7.21 mmol) at 25 °C. The reaction was stirred at 120 °C for 2 h. Upon completion, it was concentrated. The residue was purified by an 80 g silica gel column with 1-5% MeOH in DCM as eluting solvent to provide ***Int-2c.*** LCMS anal. calcd. for C₂₅H₂₄FN₃O₄: 449.2; Found: 450.4 (M+H)⁺.

### Step D - Synthesis of Compound Int-2d

To a stirred solution of ***Int-2c*** (500 mg, 1.112 mmol) in DCM (8 mL) and MeOH (40 mL) was added sodium borohydride (42.1 mg, 1.112 mmol) at room temperature. It was stirred at this temperature for 30 minutes. Upon completion, the reaction was quenched with 1 mL 1 N HCl aqueous solution and concentrated. The residue was loaded onto an 80 g silica gel column with 0-100% 30% EtOH in EtOAc/hexane as eluting solvent to provide ***Int-2d.*** LCMS anal. calcd. for C₂₅H₂₆FN₃O₄: 451.5; Found: 452.4 (M+H)⁺.

### Step E - Synthesis of Compound Int-2e

The mixture of ***Int-2d*** (410 mg, 0.908 mmol) and 2-(tributyl-15-phosphaneylidene)acetonitrile (438 mg, 1.816 mmol) in toluene (10 mL) was heated at 120 °C for 4 h. Upon completion, it was cooled down, and the resulting reaction was loaded onto an 80 g silica gel column with 0-100% 30% EtOH in EtOAc/hexane as eluent to remove phosphine impurity. The mixture was then loaded onto a C18 column (100 g) with 0-100% ACN in water as eluent to provide minor isomer ***Int-2e*** and major isomer ***Int-2f.*** LCMS anal. calcd. for C₂₅H₂₄FN₃O₃: 433.2; Found: 434.4 (M+H)⁺.

### Step F - Synthesis of Compound Int-2g and Int-2h

***Int-2h*** (185 mg, 0.427 mmol) was resolved with SFC to provide the first eluting peak ***Int-2g*** and the second eluting peak ***Int-2h.*** SFC condition: Daicel^{@} chiralpak AS-H column (250*21 mm, 10 µm); 45% EtOH with 0.2% DIPA; 70 mL/min. LCMS anal. calcd. for C₂₅H₂₄FN₃O₃: 433.2; Found: 434.4 (M+H)⁺.

### Step G - Synthesis of Compound 2A and 2B

The solution of ***Int-2g*** (52 mg, 0.120 mmol) in MeOH (3 mL) was added palladium on carbon (25.5 mg, 0.024 mmol). The resulting mixture was stirred under hydrogen balloon at room temperature for 30 minutes. Upon completion, it was filtered through celite and the filtrate was loaded onto a 50 g ISCO^{@} C18 column with 0-100% ACN in water (with 0.01% TFA modifier) as eluting solvent to provide compound **2*A***. LCMS anal. calcd. for C₁₈H₁₈FN₃O₃: 343.1; Found: 344.3 (M+H)⁺. ¹H NMR (500 MHz, Methanol-d⁴) *δ* 7.19 (d, *J =* 7.3 Hz, 1H), 7.05 (broad, 5 H), 6.14 (d, *J* = 7.3 Hz, 1H), 5.35 (s, 1H), 4.30 (dd, *J =* 11.7, 2.7 Hz, 1H), 3.27 (s, 3H), 2.59 - 2.52 (m, 1H), 2.32 - 2.19 (m, 2H), 1.96 (dd, *J* = 22.6, 14.9 Hz, 2H), 1.90 (s, 1H), 1.82 - 1.70 (m, 1H).

Following essentially the method employed to produce compound ***2A*** of example **2,** compound ***2B*** was prepared from ***Int-2h.*** LCMS anal. calcd. for C₁₈H₁₈FN₃O₃: 343.1; Found: 344.3 (M+H)⁺. ¹H NMR (500 MHz, Methanol-d⁴) *δ* 7.20 (d, *J =* 7.3 Hz, 1H), 7.04 (broad, 5H), 6.15 (d, *J* = 7.3 Hz, 1H), 5.36 (s, 1H), 4.29 (dd, *J =* 11.7, 2.5 Hz, 1H), 3.26 (s, 2H), 2.56 (d, *J =* 15.4 Hz, 1H), 2.31 - 2.19 (m, 1H), 2.01 - 1.94 (m, 1H), 1.91 (d, *J =* 13.9 Hz, 1H), 1.81 - 1.69 (m, 1H).

The examples in **Table 1** were prepared using procedures similar to those described in **Example 2,** from the appropriate starting material.

**Table 1**

| **Example** | **Structure** | **Observed *m*/*z* [M + H]⁺** |
|---|---|---|
| | **Name** | |
| | **Precursor SFC Separation Condition** | |
| **3A (early peak)** | 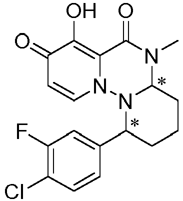 | 378.3 |
| **3B (late peak)** | 1-(4-chloro-3-fluorophenyl)-7-hydroxy-5-methyl-1,2,3,4,4a,5-hexahydrodipyrido[1,2-b:2', 1'-f][1,2,4]triazine-6,8-dione | |
| | Precursor was resolved by SFC. SFC conditions: Daicel^{@} chiralpak AD-H column (250*21 mm, 10 µm); 45% EtOH with 0.2% DIPA; 70 mL/min | |
| **4A (early peak)** | 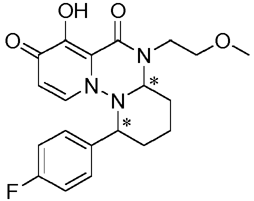 | 388.2 |
| **4B (late peak)** | 1-(4-fluorophenyl)-7-hydroxy-5-(2-methoxyethyl)-1,2,3,4,4a,5-hexahydrodipyrido[1,2-b:2', 1'-f][1,2,4]triazine-6,8-dione | |
| | Precursor was resolved by SFC. SFC conditions: Daicel^{@} chiralpak OD-H column (250*21 mm, 10 µm); 45% EtOH with 0.2% DIPA; 70 mL/min | |
| **5A (early peak)** | 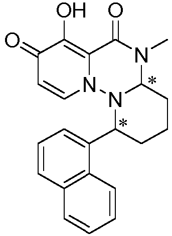 | 376.4 |
| **5B (late peak)** | 7-hydroxy-5-methyl-1-(naphthalen-1-yl)-1,2,3,4,4a,5-hexahydrodipyrido[1,2-b:2',1'-f][1,2,4]triazine-6,8-dione | |
| | Precursor was resolved SFC. SFC conditions: Daicel^{@} chiralpak OJ-H column (250*21 mm, 10 µm); 25% EtOH with 0.2% DIPA; 70 mL/min | |
| **6A (Third peak)** | 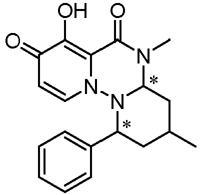 | 340.3 |
| **6B (Fourth peak)** | 7-hydroxy-3,5-dimethyl-1-phenyl-1,2,3,4,4a,5-hexahydrodipyrido[1,2-b :2',1'-f][1,2,4]triazine-6,8-dione | |
| | Precursor was resolved by SFC. SFC conditions: Daicel^{@} chiralpak AD-H column (250*21 mm, 10 µm); 45% EtOH with 0.2% DIPA; 70 mL/min | |
| **7A (early peak)** | 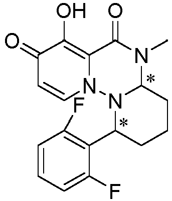 | 362.3 |
| **7B (late peak)** | 1-(2,6-difluorophenyl)-7-hydroxy-5-methyl-1,2,3,4,4a,5-hexahydrodipyrido[1,2-b:2', 1'-f][1,2,4]triazine-6,8-dione | |
| | Precursor was resolved by SFC. SFC conditions: Daicel^{@} chiralpak AD-H column (250*21 mm, 10 µm); 45% EtOH with 0.2% DIPA; 70 mL/min | |
| **8A (early peak)** | 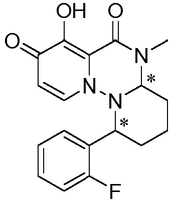 | 344.3 |
| **8B (late peak)** | 1-(2-fluorophenyl)-7-hydroxy-5-methyl-1,2,3,4,4a,5-hexahydrodipyrido[1,2-b:2',1'-f][1,2,4]triazine-6,8-dione | |
| | Precursor was resolved by SFC. SFC conditions: Daicel^{@} chiralpak AS-H column (250*21 mm, 10 µm); 35% EtOH with 0.2% DIPA; 70 mL/min | |
| **9A (First peak)** | 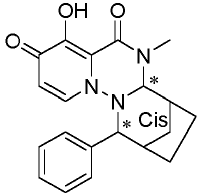 | 352.3 |
| **9B (Second peak)** | | |
| **9C (Third peak)** | | |
| **9D (Fourth peak)** | | |
| | 4-hydroxy-6-methyl-11-phenyl-6a,7,8,9,10,11-hexahydro-3H-7,10-methanopyrido[1',2':1,6][1,2,4]triazino[2,3-a]azepine-3,5(6H)-dione | |
| **10A (early peak)** | 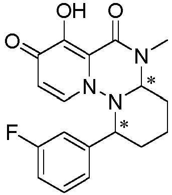 | 344.3 |
| **10B (late peak)** | 1-(3-fluorophenyl)-7-hydroxy-5-methyl-1,2,3,4,4a,5-hexahydrodipyrido[1,2-b:2',1'-f][1,2,4]triazine-6,8-dione | |
| | Precursor was resolved by SFC. SFC conditions: Daicel^{@} chiralpak AD-H column (250*21 mm, 10 µm); 45% EtOH with 0.2% DIPA; 70 mL/min | |
| **11A (early peak)** | 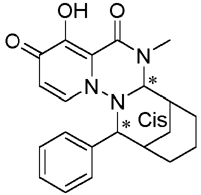 | 366.2 |
| **11B (late peak)** | | |
| | 4-hydroxy-6-methyl-12-phenyl-6,6a,7,8,9,10,11,12-octahydro-7,11-methanopyrido[1',2':1,6][1,2,4]triazino[2,3-a]azocine-3,5-dione | |
| **12A (early peak)** | 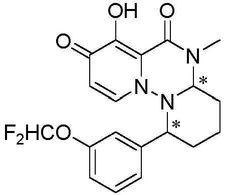 | 392.2 |
| **12B (late peak)** | | |
| | 1-(3-(difluoromethoxy)phenyl)-7-hydroxy-5-methyl-1,2,3,4,4a,5-hexahydrodipyrido[1,2-b:2',1'-f][1,2,4]triazine-6,8-dione | |
| | Precursor was resolved by SFC. SFC conditions: Daicel^{@} chiralpak AS-H column (250*21 mm, 10 µm); 30% EtOH; 70 mL/min | |
| **13A (early peak)** | 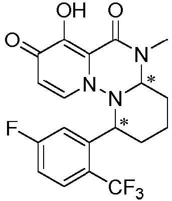 | 412.3 |
| **13B (late peak)** | 1-(5-fluoro-2-(trifluoromethyl)phenyl)-7-hydroxy-5-methyl-1,2,3,4,4a,5-hexahydrodipyrido[1,2-b:2',1'-f][1,2,4]triazine-6,8-dione | |
| | Precursor was resolved by SFC. SFC conditions: Daicel^{@} chiralpak AS-H column (250*21 mm, 10 µm); 35% EtOH; 70 mL/min | |
| **14A (early peak)** | 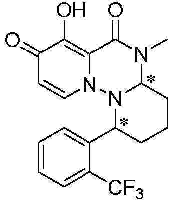 | 394.3 |
| **14B (late peak)** | 7-hydroxy-5-methyl-1-(2-(trifluoromethyl)phenyl)-1,2,3,4,4a,5-hexahydrodipyrido[1,2-b:2', 1'-f][1,2,4]triazine-6,8-dione | |
| | Precursor was resolved by SFC. SFC conditions: Daicel^{@} chiralpak AD-H column (250*21 mm, 10 µm); 35% EtOH; 70 mL/min | |
| **15A (early peak)** | 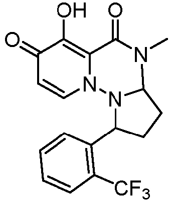 | 380.3 |
| **15B (late peak)** | 6-hydroxy-4-methyl-1-(2-(trifluoromethyl)phenyl)-2,3,3a,4-tetrahydro-1H-pyrido[2,1-f]pyrrolo[1,2-b][1,2,4]triazine-5,7-dione | |
| | Precursor was resolved by SFC. SFC conditions: Daicel^{@} chiralpak AD-H column (250*21 mm, 10 µm); 40% EtOH; 50 mL/min | |
| **16** | 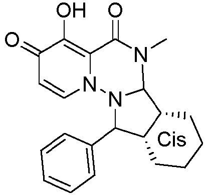 | 366.4 |
| | (6bR,10aS)-4-hydroxy-6-methyl-11-phenyl-6a,6b,7,8,9,10,10a,11-octahydro-3H-pyrido[1',2':1,6][1,2,4]triazino[3,2-a]isoindole-3,5(6H)-dione | |

### Example 3

### Preparation of Compounds 17A and 17B

### 1-(2-(difluoromethoxy)-5-fluorophenyl)-7-hydroxy-5-methyl-1,2,3,4,4a,5-hexahydrodipyrido[1,2-b:2',1'-f][1,2,4]triazine-6,8-dione

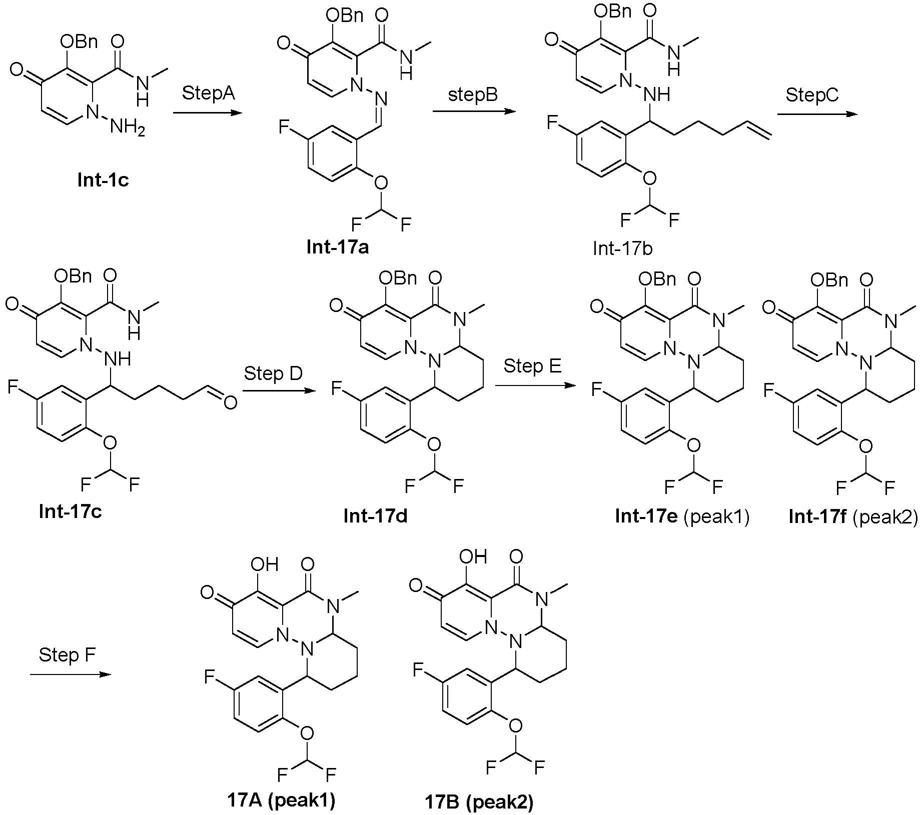

### Step A - Synthesis of Compound Int-17a

To a stirred solution of ***Int-1c*** (500 mg, 1.830 mmol) and 2-(difluromethoxy)-5-fluorobenzaldehyde (417 mg, 2.195 mmol) in dry dioxane (8 mL) at room temperature was added TFA (141 µL, 1.830 mmol). The mixture was stirred at RT for 4 h. Upon completion, the mixture was concentrated. The residue was re-dissolved in DCM, and TEA (306 µL, 2.195 mmol) was added. The mixture was purified by a 120 g silica gel column using Isco system with 0-20% MeOH in DCM as eluting solvent to provide ***Int-17a.*** LCMS anal. calcd. for C₂₂H₁₈F₃N₃O₄: 445.1; found 446.3 (M+H)⁺

### Step B - Synthesis of Compound Int-17b

To a stirred solution of ***Int-17a*** (500 mg, 1.123 mmol) in dry THF (18 mL) was added 4-pentene-1-magnesium bromide (0.5 M in 2-Me-THF) (6736 µL, 3.37 mmol) at 0 °C. The mixture was stirred at this temperature for 14 min. Upon completion, the reaction was quenched with the addition of water (~ 30 mL) and extracted with EtOAc (3x60 mL). The combined organic phases were washed with brine, dried over Na₂SO₄, filtered, and concentrated. The residue was purified by an 80 g silica gel column using Isco system with 0-100%EtOH-EtOAc (3:1)/hexane as eluting solvent to provide ***Int-17b.*** LCMS anal. calcd. for C₂₇H₂₈F₃N₃O₄: 515.2; found 516.4 (M+H)⁺.

### Step C - Synthesis of Compound Int-17c

To a 40 mL-vial with a stirred solution of ***Int-17b*** (210 mg, 0.407 mmol) in THF (3 mL) and water (1 mL) was added osmium tetroxide (2.5 % in t-BuOH) (102 µL, 8.15 µmol) at room temperature, followed by the addition of sodium periodate (261 mg, 1.222 mmol). The vial was sealed with a pressure relief cap and stirred at room temperature for 7 h. Upon completion, the mixture was diluted with EtOAc (~ 100 mL), the solid was filtered and washed with EtOAc (~ 20 mL). The filtrate was washed with 10% Na₂S₂O₃ aqueous solution twice (30 mL x 2). The organic phase was separated, dried over Na₂SO₄, filtered and concentrated in vacuo to provide ***Int-17c.*** LCMS anal. calcd. for C₂₆H₂₆F₃N₃O₅: 517.2; found 518.4 (M+H)⁺.

### Step D - Synthesis of Compound Int-17d

To a 40-ml vial with a stirred solution of ***Int-17c*** (184 mg, 0.356 mmol) in DMF (5 mL) was added AcOH (539 µL) at room temperature. The vial was sealed with a pressure relief cap and stirred at 120 °C for 8 h. The mixture was cooled to room temperature, then purified by a reverse phase 100 g Gold C18 column on Isco system with 0-100% acetonitrile-water (with 0.05 % TFA buffer) as eluting solvent. The fractions with desired mass were combined and lyophilized, then redissolved in DCM (~ 15 mL), TEA (49.6 µL, 0.356 mmol) was added, and the mixture was put through a 40 g silica gel column using Isco system with EtOH/EtOAc (3:1)/hexane as eluting solvent to provide ***Int-17d.*** LCMS anal. calcd. for C₂₆H₂₄F₃N₃O₄: 499.2; found 500.3 (M+H)⁺.

### Step E - Synthesis of Compound Int-17e and Int-17f

Compound ***Int-17d*** (48.5 mg, 0.097 mmol) was resolved by chiral SFC to provide the first eluting peak ***Int-17e*** (21.5 mg, 0.043 mmol) and the second eluting peak ***Int17f*** (21.8 mg, 0.044 mmol). SFC condition: Daicel^{@} chiralpak AD-H column (250*21 mm, 10 µm); 20% IPA with 0.2% DIPA; 70 mL/min. LCMS anal. calcd. for C₂₆H₂₄F₃N₃O₄: 499.2; Found: 500.3 (M+H)⁺.

### Step F - Synthesis of Compound 17A and 17B

To the round bottom flask (50 mL) was added Pd on carbon (22.37 mg, 0.021 mmol), followed by addition of ***Int-17f*** (21 mg, 0.042 mmol) in EtOH (12 mL). The flask was equipped with a hydrogen balloon. The system was degassed and refilled with H₂ twice. The mixture was then stirred at room temperature under H₂ for 45 min. Upon completion, the catalyst was filtered off. The filtrate was concentrated in vacuo. The residue was purified by a 30 g Isco C18 gold column with 0-100% acetonitrile/water (buffered with 0.05% TFA) as eluent to afford compound *17A.* LCMS anal. calcd. for C₁₉H₁₈F₃N₃O₄: 409.1; found 410.2 (M+H)⁺. ¹H NMR (500 MHz, Methanol-*d*₄) *δ* 7.61 (d, *J* = 9.1 Hz, 1H), 7.18 (d, *J =* 7.3 Hz, 1H), 7.12 (d, *J =* 7.7 Hz, 1H), 7.05 (d, *J* = 4.2 Hz, 1H), 6.58 (t, *J =* 73.2 Hz, 1H), 6.05 (d, *J =* 7.6 Hz, 1H), 5.33 (s, 1H), 4.84 (s, 1H), 3.26 (s, 3H), 2.55 (d, *J =* 15.4 Hz, 1H), 2.24 (t, *J =* 14.6 Hz, 2H), 1.95-1.93 (m, 2H), 1.77-1.74 (m, 1H).

Following essentially the method employed to produce compound ***17B*** of example **3,** compound ***17A*** was prepared from ***Int-17e.*** LCMS anal. calcd. for C₁₉H₁₈F₃N₃O₄: 409.1; found 410.2 (M+H)⁺. ¹H NMR (500 MHz, Methanol-d⁴) _{δ} 7.61 (d, *J =* 8.6 Hz, 1H), 7.31 - 6.96 (m, 3H), 6.57 (t, *J* = 73.2 Hz, 1H), 6.03 (d, *J=* 7.2 Hz, 1H), 5.33 (s, 1H), 4.84 (s, 1H), 3.26 (s, 3H), 2.55 (d, *J =* 15.7 Hz, 1H), 2.24 (t, *J =* 14.1 Hz, 2H), 2.0 - 1.9 (m, 2H), 1.77-1.74 (m, 1H).

### Example 4

### Preparation of Compounds 18A and 18B

### 7-hydroxy-1,5-dimethyl-1-phenyl-1,2,3,4,4a,5-hexahydrodipyrido[1,2-b:2',1'-f][1,2,4]triazine-6,8-dione

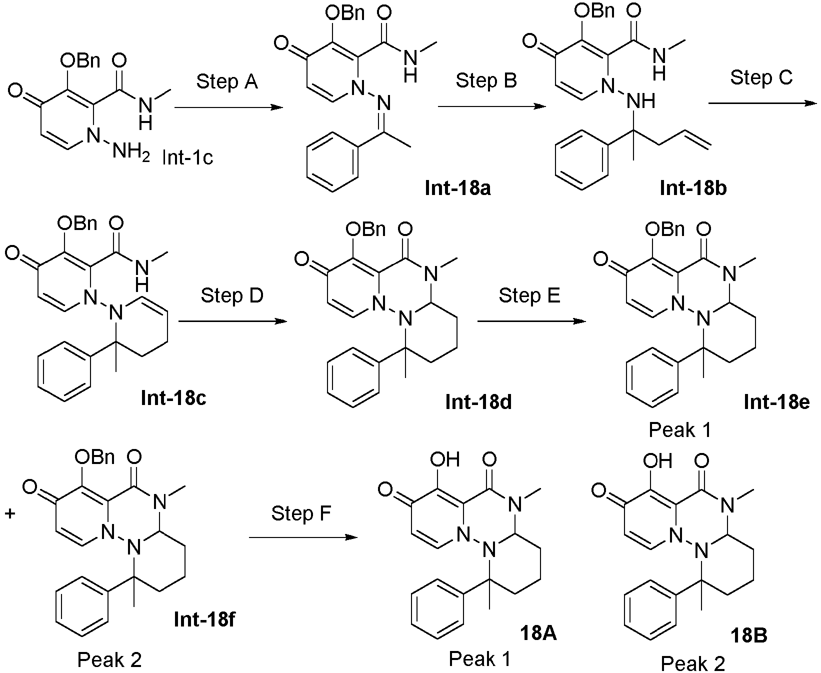

### Step A - Synthesis of Compound Int-18a

To the stirred suspension of ***Int-1c*** (5670 mg, 20.75 mmol) and acetophenone (5 mL, 41.5 mmol) in dry dioxane (40 mL) at RT was added TFA (1758 µL, 22.82 mmol). The mixture was stirred at 80°C overnight. Upon completion, the mixture was concentrated. The residue was redissolved in 40 mL DCM, followed by the addition of TEA (3181 µL, 22.82 mmol). The mixture was loaded onto a 330 g silica gel flash column with 0-20% MeOH in DCM as eluting solvent to afford ***Int-18a.*** LCMS anal. calcd. for C₂₂H₂₁N₃O₃: 375.2; Found: 376.2 (M+H)⁺.

### Step B - Synthesis of Compound Int-18b

To a rigorously stirred solution of ***Int-18a*** (5380 mg, 14.33 mmol) in dry THF (18 mL) was added allylmagnesium bromide (1.0 M in diethyl ether) (4.01E+04 µL, 40.1 mmol) at 0°C. The mixture was stirred at this temperature for 15 min. Upon completion, the reaction was quenched with the addition of saturated NH₄Cl (~ 50mL) and partitioned between EtOAc (100 mL) and water (~50 mL). The aqueous was extracted with EtOAc two more times (2x80 mL). The combined organic layer was dried over Na₂SO₄, filtered and concentrated. The residue was purified by a 220 g silica gel flash column with 0-100%EtOH-EtOAc (3:1)/hexane as eluting solvent to provide ***Int-18b.*** LCMS anal. calcd. for C₂₅H₂₇N₃O₃: 417.2; Found: 418.2 (M+H)⁺.

### Step C - Synthesis of Compound Int-18c

To a 350 mL high pressure vessel with dicyclohexyl(2',6'-dimethoxy-[1,1'-biphenyl]-2-yl)phosphane (S-phos) (703 mg, 1.731 mmol), chloro(1,5-cyclooctadiene)iridium(I) dimer [Ir(cod)Cl]₂ (240 mg, 0.357 mmol) and DCC (3682 mg, 17.84 mmol) was added ***Int-18b*** (2980 mg, 7.14 mmol) and formic acid (1.1 mL, 28.6 mmol) in dry acetonitrile (70 mL) under N₂ at room temperature. The vial was sealed with a pressure relief cap. The mixture was warmed up to 75 °C overnight and then 90 °C for three days. Upon completion, the reaction mixture was cooled to room temperature, diluted with EtOAc (~ 60 mL), then filtered. The solid was washed with EtOAc/DCM (20 mL/20 mL). The combined filtrate was concentrated. The residue was purified by a 120 g silica gel flash column with 0-100%EtOH-EtOAc (3:1) /hexane as eluting solvent to provide ***Int-18c*** and ***Int-18d.*** LCMS anal. calcd. for C₂₆H₂₇N₃O₃: 429.2; Found: 430.2 (M+H)⁺.

### Step D - Synthesis of Compound Int-18d

To a 40-mL vial with stirred solution of ***Int-18c*** (230 mg, 0.535 mmol) in DMF (6 mL) was added AcOH (609 µL) at room temperature. The vial was sealed with a pressure relief cap. The mixture was stirred at 120°C for 3 days. Upon completion, the mixture was cooled to RT, then purified by a 100 g reverse phase C18 column with 0-100% acetonitrile-water (with 0.05 % TFA buffer) to ***Int-18d.*** LCMS anal. calcd. for C₂₆H₂₇N₃O₃: 429.2; Found: 430.3 (M+H)⁺.

### Step E - Synthesis of Compound Int-18e and Int-18f

***Int-18d*** (974 mg, 2.268 mmol) was resolved with SFC to provide the first eluting peak ***Int-18e*** and the second eluting peak ***Int-18f.*** SFC condition: Chiral OD-H column (250*21 mm, 10 µm); 45% EtOH; 70 mL/min. LCMS anal. calcd. for C₂₆H₂₇N₃O₃: 429.2; Found: 430.3 (M+H)⁺.

### Step F - Synthesis of Compound 18A and 18B

To a round bottom flask (150 mL) with ***Int-18e*** (420 mg, 0.978 mmol) was added palladium on carbon (10%) (312 mg, 0.293 mmol), followed by addition of EtOH (80 mL) under N₂. The flask was equipped with a hydrogen balloon. The system was degassed and refilled with H₂ twice. Then the mixture was stirred at room temperature for 50 min. Upon completion, the catalyst was filtered off. The filtrate was concentrated in vacuo. The residue was purified by a 150 g C18 reverse phase column with 0-100% ACN in water as eluting solvent to provide ***18A.*** LCMS anal. calcd. for C₁₉H₂₁N₃O₃: 339.2; Found: 340.6 (M+H)⁺. ¹H NMR (500 MHz, Methanol-d⁴) *δ* 7.58 (d, *J =* 5.7 Hz, 2H), 7.38 (d, *J =* 7.3 Hz, 3H), 6.52 (d, *J =* 7.1 Hz, 1H), 5.73 (d, *J* = 7.1 Hz, 1H), 5.30 (s, 1H), 3.23 (s, 3H), 2.60 (d, *J =* 13.3 Hz, 2H), 2.30 (t, *J =* 13.0 Hz, 1H), 1.89 (dd, *J =* 24.2, 13.9 Hz, 3H), 1.65 (s, 3H).

Following essentially the method employed to produce compound ***18A*** of example **4,** compound ***18B*** was prepared from ***Int-18f.*** LCMS anal. calcd. for C₁₉H₂₁N₃O₃: 339.2; Found: 340.6 (M+H)⁺. ¹H NMR (500 MHz, Methanol-d⁴) *δ* 7.59 (d, *J =* 5.7 Hz, 2H), 7.40 (d, *J =* 7.8 Hz, 3H), 6.71 (d, *J =* 6.9 Hz, 1H), 6.13 (d, *J =* 7.0 Hz, 1H), 5.36 (s, 1H), 3.33 (s, 3H), 3.26 (s, 2H), 2.67 - 2.59 (m, 1H), 2.34 (t, *J =* 14.0 Hz, 1H), 1.99 - 1.87 (m, 3H), 1.65 (s, 3H).

The examples in **Table 2** were prepared using procedures similar to those described in **Example 4,** from the appropriate starting material.

**Table 2**

| **Example** | **Structure** | **Observed *m*/*z* [M + H]⁺** |
|---|---|---|
| | **Name** | |
| | **Precursor SFC Separation Condition** | |
| **19A (early peak)** | 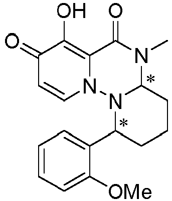 | 356.2 |
| **19B (late peak)** | 7-hydroxy-1-(2-methoxyphenyl)-5-methyl-1,2,3,4,4a,5-hexahydrodipyrido[1,2-b:2',1'-f][1,2,4]triazine-6,8-dione | |
| | Precursor was resolved by SFC. SFC conditions: Daicel^{@} chiralpak AS-H column (250*21 mm, 10 µm); 25% MeOH with 0.2% DIPA; 70 mL/min | |
| **20A (early peak)** | 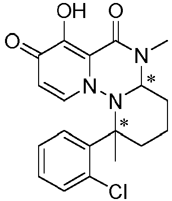 | 374.3 |
| **20B (late peak)** | | |
| | 1-(2-chlorophenyl)-7-hydroxy-1,5-dimethyl-1,2,3,4,4a,5-hexahydrodipyrido[1,2-b:2',1'-f][1,2,4]triazine-6,8-dione | |
| | Precursor was resolved by SFC. SFC conditions: Daicel^{@} chiralpak AS-H column (250*21 mm, 10 µm); 45% EtOH; 70 mL/min | |
| **21A (peak1)** | 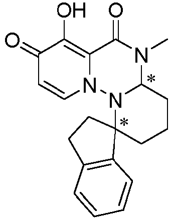 | 352.2 |
| **21B (peak2)** | | |
| **21A (peak3)** | 7-hydroxy-5-methyl-2',3,3',4,4a, 5-hexahydro-2H-spiro[dipyrido[1,2-b:2',1'-f][1,2,4]triazine-1,1'-indene]-6,8-dione | |
| **21B (peak4)** | | |
| | Precursor was resolved by SFC. SFC conditions: Daicel^{@} chiralpak AD-H column (250*30 mm, 5 µm); 45% EtOH with 0.1%NH₃H₂O; 70 mL/min. | |
| **22A (Peak1)** | 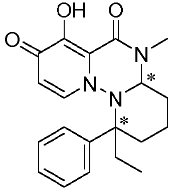 | 354.2 |
| **22B (peak2)** | 1-ethyl-7-hydroxy-5-methyl-1-phenyl-1,2,3,4,4a,5-hexahydrodipyrido[1,2-b:2',1'-f][1,2,4]triazine-6,8-dione | |
| **22C (peak 3)** | | |
| **22D (peak 4)** | | |
| | Precursor was separated SFC. SFC conditions: Daicel^{@} chiralpak OD-H column and chiralpak AD-H column (250*30 mm, 10 µm); 45% EtOH with 0.1%NH₃H₂O; 70 mL/min | |
| **23A (Peak 1)** | 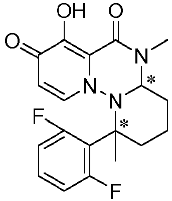 | 376.1 |
| **23B (Peak 2)** | 1-(2,6-difluorophenyl)-7-hydroxy-1,5-dimethyl-1,2,3,4,4a,5-hexahydrodipyrido[1,2-b:2', 1'-f][1,2,4]triazine-6,8-dione | |
| | Precursor was separated SFC. SFC conditions: Daicel@ chiralpak AS-H column (250*21 mm, 10 µm); 50% EtOH; 50 mL/min | |
| **24A (Peak 1)** | 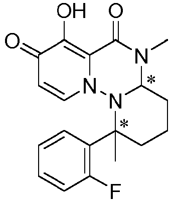 | 358.2 |
| **24B (Peak 2)** | 1-(2-fluorophenyl)-7-hydroxy-1,5-dimethyl-1,2,3,4,4a,5-hexahydrodipyrido[1,2-b:2',1'-f][1,2,4]triazine-6,8-dione | |
| | Precursor was separated SFC. SFC conditions: 2xWhelko-1 column (250*21 mm, 10 µm); 35% MeOH/ACN(1:1) with 0.2% DIPA; 70 mL/min | |
| **25A (Peak 1)** | 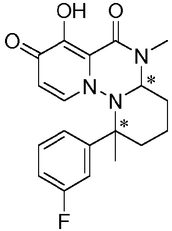 | 358.2 |
| **25B (Peak 2)** | | |
| | 1-(3-fluorophenyl)-7-hydroxy-1,5-dimethyl-1,2,3,4,4a,5-hexahydrodipyrido[1,2-b:2',1'-f][1,2,4]triazine-6,8-dione | |
| | Precursor was separated SFC. SFC conditions: 2xWhelko-1 column (250*21 mm, 10 µm); 55% MeOH/ACN(1:1) with 0.2% DIPA; 50 mL/min | |
| **26A (Peak 1)** | 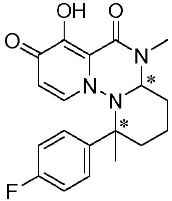 | 358.3 |
| **26B (Peak 2)** | 1-(4-fluorophenyl)-7-hydroxy-1,5-dimethyl-1,2,3,4,4a,5-hexahydrodipyrido[1,2-b:2',1'-f][1,2,4]triazine-6,8-dione | |
| | Precursor was separated SFC. SFC conditions: Daicel@ chiralpak AS-H column (250*21 mm, 10 µm); 25% EtOH, 70 mL/min | |
| **27A (Peak 1)** | 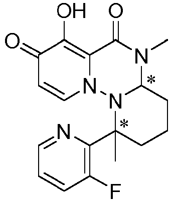 | 359.2 |
| **27B (Peak 2)** | 1-(3-fluoropyridin-2-yl)-7-hydroxy-1,5-dimethyl-1,2,3,4,4a,5-hexahydrodipyrido[1,2-b:2', 1'-f][1,2,4]triazine-6,8-dione | |
| | Precursor was separated SFC. SFC conditions: Daicel@ chiralpak AS-H column (250*21 mm, 10 µm); 30% MeOH, 50 mL/min | |
| **28A (Peak 1)** | 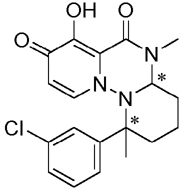 | 374.2 |
| **28B (Peak 2)** | | |
| | 1-(3-chlorophenyl)-7-hydroxy-1,5-dimethyl-1,2,3,4,4a,5-hexahydrodipyrido[1,2-b:2',1'-f][1,2,4]triazine-6,8-dione | |
| | Precursor was separated SFC. SFC conditions: Daicel@ chiralcel OD-H column (250*21 mm, 10 µm); 25% IPA with 0.2% DIPA; 70 mL/min | |
| **29A (Peak 1)** | 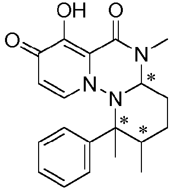 | 354.3 |
| **29B (Peak 2)** | 7-hydroxy-1,2,5-trimethyl-1-phenyl-1,2,3,4,4a,5-hexahydrodipyrido[1,2-b:2',1'-f][1,2,4]triazine-6,8-dione | |
| | Precursor was separated SFC. SFC conditions: Daicel@ chiralcel AD-H column (250*21 mm, 10 µm); 25% IPA with 0.2% DIPA; 70 mL/min | |
| **30A (Peak 1)** | 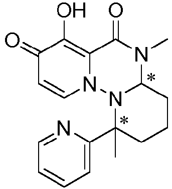 | 341.2 |
| **30B (Peak 2)** | 7-hydroxy-1,5-dimethyl-1-(pyridin-2-yl)-1,2,3,4,4a,5-hexahydrodipyrido[1,2-b:2',1'-f][1,2,4]triazine-6,8-dione | |
| | Precursor was separated SFC. SFC conditions: Daicel@ chiralcel OD-H column (250*21 mm, 10 µm); 25% IPA with 0.2% DIPA; 70 mL/min | |
| **31A (Peak 1)** | 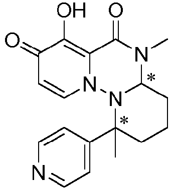 | |
| **31B (Peak 2)** | | |
| | 7-hydroxy-1,5-dimethyl-1-(pyridin-4-yl)-1,2,3,4,4a,5-hexahydrodipyrido[1,2-b:2',1'-f][1,2,4]triazine-6,8-dione | |
| | Precursor was separated SFC. SFC conditions: Daicel@ chiralpak AD-H column (250*21 mm, 10 µm); 25% IPA with 0.2% DIPA; 70 mL/min | |
| **32A (Peak 1)** | 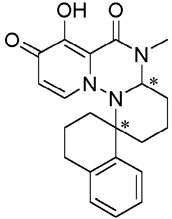 | 366.4 |
| **32B (Peak 2)** | 7-hydroxy-5-methyl-3,3',4,4a,4',5-hexahydro-2H,2'H-spiro[dipyrido[1,2-b:2',1'-f][1,2,4]triazine-1,1'-naphthalene]-6,8-dione | |
| | Precursor was separated SFC. SFC conditions: Daicel@ chiralpak AS-H column (250*30 mm, 10 µm); 30% MeOH with 0.2% DIPA; 70 mL/min | |
| **33A (Peak 1)** | 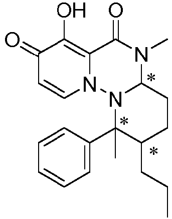 | |
| **33B (Peak 2)** | | |
| **33C (Peak 3)** | 7-hydroxy-1,5-dimethyl-1-phenyl-2-propyl-1,2,3,4,4a,5-hexahydrodipyrido[1,2-b:2',1'-f][1,2,4]triazine-6,8-dione | |
| **33D (Peak 4)** | | |
| | Precursor was separated SFC. SFC conditions: Daicel@ chiralpak IG column (250*30 mm, 10 µm); 30% EtOH with 0.2% DIPA; 60 mL/min | |
| **34A (Peak 1)** | 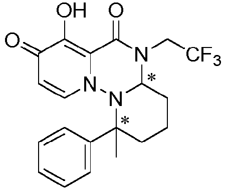 | 408.2 |
| **34B (Peak 2)** | 7-hydroxy-1-methyl-1-phenyl-5-(2,2,2-trifluoroethyl)-1,2,3,4,4a,5-hexahydrodipyrido[1,2-b:2', 1'-f][1,2,4]triazine-6,8-dione | |
| | Precursor was separated SFC. SFC conditions: Daicel@ chiralpak AS column (250*30 mm, 10 µm); 40% EtOH with 0.05% DEA; 60 mL/min | |
| **35A (Peak 1)** | 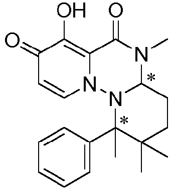 | 368.2 |
| **35B (Peak 2)** | 7-hydroxy-1,2,2,5-tetramethyl-1-phenyl-1,2,3,4,4a,5-hexahydrodipyrido[1,2-b:2',1'-f][1,2,4]triazine-6,8-dione | |
| | Precursor was separated SFC. SFC conditions: Daicel@ chiralpak OD-H column (250*30 mm, 5 µm); 55% MeOH with 0.1% NH₃H₂O; 60 mL/min | |
| **36A (Peak 1)** | 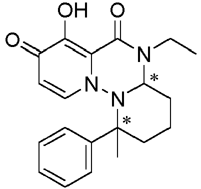 | 354.2 |
| **36B (Peak 2)** | | |
| | 5-ethyl-7-hydroxy-1-methyl-1-phenyl-1,2,3,4,4a,5-hexahydrodipyrido[1,2-b:2',1'-f][1,2,4]triazine-6,8-dione | |
| | Precursor was separated SFC. SFC conditions: WHELK-O1 column (250*30 mm, 5 µm); 50% EtOH with 0.1% | |
| | NH₃H₂O; 60 mL/min | |
| **37A (Peak 1)** | 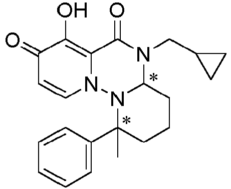 | 448.2 |
| **37B (Peak 2)** | 5-(cyclopropylmethyl)-7-hydroxy-1-methyl-1-phenyl-1,2,3,4,4a,5-hexahydrodipyrido[1,2-b:2',1'-f][1,2,4]triazine-6,8-dione | |
| | Precursor was separated SFC. SFC conditions: WHELK-O1 column (250*30 mm, 5 µm); 50% EtOH with 0.1% NH₃H₂O; 60 mL/min | |
| **38A (Peak 1)** | 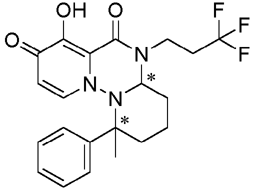 | 422.2 |
| **38B (Peak 2)** | 7-hydroxy-1-methyl-1-phenyl-5-(3,3,3-trifluoropropyl)-1,2,3,4,4a,5-hexahydrodipyrido[1,2-b:2',1'-f][1,2,4]triazine-6,8-dione | |
| | Precursor was separated SFC. SFC conditions: Daicel@ chiralpak OD-H column (250*30 mm, 5 µm); 30% EtOH with 0.1% NH₃H₂O; 60 mL/min | |
| **39A (Peak 1)** | 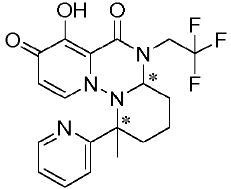 | 409.2 |
| **39B (Peak 2)** | | |
| | 7-hydroxy-1-methyl-1-(pyridin-2-yl)-5-(2,2,2-trifluoroethyl)-1,2,3,4,4a,5-hexahydrodipyrido[1,2-b:2',1'- | |
| | f][1,2,4]triazine-6,8-dione | |
| | Precursor was separated SFC. SFC conditions: Daicel@ chiralpak IC column (250*30 mm, 5 µm); 60% EtOH with 0.1% NH₃H₂O; 60 mL/min | |
| **40A (Peak 1)** | 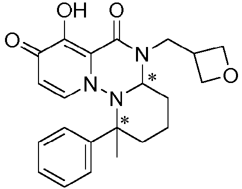 | 396.2 |
| **40B (Peak 2)** | 7-hydroxy-1-methyl-5-(oxetan-3-ylmethyl)-1-phenyl-1,2,3,4,4a,5-hexahydrodipyrido[1,2-b:2',1'-f][1,2,4]triazine-6,8-dione | |
| | Precursor was separated SFC. SFC conditions: Daicel@ chiralpak OD-H column (250*30 mm, 5 µm); 50% EtOH with 0.1% NH₃H₂O; 60 mL/min | |
| **41A (Peak 1)** | 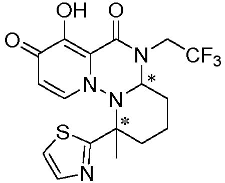 | 415.1 |
| **41B (Peak 2)** | 7-hydroxy-1-methyl-1-(thiazol-2-yl)-5-(2,2,2-trifluoroethyl)-1,2,3,4,4a,5-hexahydrodipyrido[1,2-b:2',1'-f][1,2,4]triazine-6,8-dione | |
| | Precursor was separated SFC. SFC conditions: Daicel@ chiralpak AS column (250*30 mm, 5 µm); 35% EtOH with 0.1% NH₃H₂O; 70 mL/min | |
| **Example** | **Structure** | **Observed *m*/*z* [M + H]⁺** |
| | **Name** | |
| | **Precursor SFC Separation Condition** | |
| **42A (Peak 1)** | 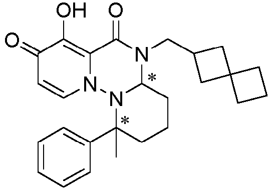 | 434.2 |
| **42B (Peak 2)** | 7-hydroxy-1-methyl-1-phenyl-5-(spiro[3.3]heptan-2-ylmethyl)-1,2,3,4,4a,5-hexahydrodipyrido[1,2-b:2',1'-f][1,2,4]triazine-6,8-dione | |
| | Precursor was separated SFC. SFC conditions: Daicel@ chiralpak OD-H column (250*30 mm, 5 µm); 35% EtOH with 0.1% NH₃H₂O; 70 mL/min | |
| **43A (Peak 1)** | 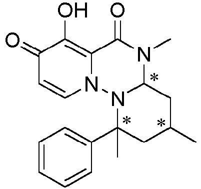 | 354.3 |
| **43B (Peak 2)** | 7-hydroxy-1,3,5-trimethyl-1-phenyl-1,2,3,4,4a,5-hexahydrodipyrido[1,2-b:2',1'-f][1,2,4]triazine-6,8-dione | |
| **43C (Peak 3)** | | |
| **43D (Peak 4)** | Precursor was separated SFC. SFC conditions: Daicel@ chiralpak AD-H column (250*30 mm, 5 µm); 25% MeOH with 0.1% DEA 70 mL/min and then chiralpak OD-H column (250*30 mm, 5 µm); 30% MeOH with 0.1% DEA 70 mL/min | |
| **44A (Peak 1)** | 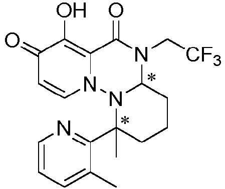 | 423.3 |
| **44B (Peak 2)** | | |
| | 7-hydroxy-1-methyl-1-(3-methylpyridin-2-yl)-5-(2,2,2-trifluoroethyl)-1,2,3,4,4a,5-hexahydrodipyrido[1,2-b:2',1'- | |
| | f][1,2,4]triazine-6,8-dione | |
| | Precursor was separated SFC. SFC conditions: ChromegaChiral COO OD column (250*20 mm, 5 µm); 20% MeOH with 0.1% DEA; 60 mL/min | |
| **45A (Peak 1)** | 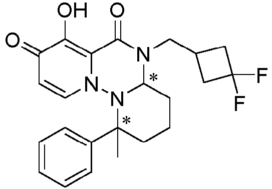 | 430.2 |
| **45B (Peak 2)** | 5-((3,3-difluorocyclobutyl)methyl)-7-hydroxy-1-methyl-1-phenyl-1,2,3,4,4a,5-hexahydrodipyrido[1,2-b:2',1'-f][1,2,4]triazine-6,8-dione | |
| | Precursor was separated SFC. SFC conditions: Daicel@ chiralpak AD column (250*30 mm, 5 µm); 45% EtOH with 0.1% NH₃H₂O; 70 mL/min | |
| **46A (Peak 1)** | 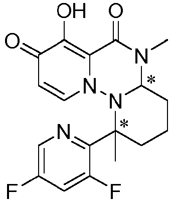 | 377.1 |
| **46B (Peak 2)** | 1-(3,5-difluoropyridin-2-yl)-7-hydroxy-1,5-dimethyl-1,2,3,4,4a,5-hexahydrodipyrido[1,2-b:2',1'-f][1,2,4]triazine-6,8-dione | |
| | Precursor was separated SFC. SFC conditions: Daicel@ chiralpak OD-H column (250*30 mm, 5 µm); 55% EtOH with 0.1% NH₃H₂O; 80 mL/min | |
| **47A (Peak 1)** | 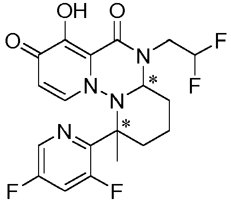 | 427.0 |
| **47B (Peak 2)** | 5-(2,2-difluoroethyl)-1-(3,5-difluoropyridin-2-yl)-7-hydroxy-1-methyl-1,2,3,4,4a,5-hexahydrodipyrido[1,2-b:2',1'-f][1,2,4]triazine-6,8-dione | |
| | Precursor was separated SFC. SFC conditions: Daicel@ chiralpak AD column (250*30 mm, 5 µm); 30% EtOH with 0.1% NH₃H₂O; 70 mL/min | |
| **48A (Peak 1)** | 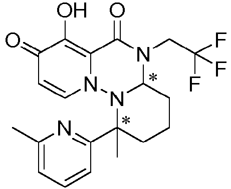 | 423.3 |
| **48B (Peak 2)** | 7-hydroxy-1-methyl-1-(6-methylpyridin-2-yl)-5-(2,2,2-trifluoroethyl)-1,2,3,4,4a,5-hexahydrodipyrido[1,2-b:2',1'-f][1,2,4]triazine-6,8-dione | |
| | Precursor was separated SFC. SFC conditions: Daicel@ chiralpak AS-H column (250*30 mm, 5 µm); 30% EtOH with 0.1% DEA; 70 mL/min | |
| **49A (Peak 1)** | 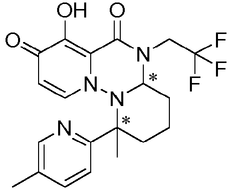 | 423.3 |
| **49B (Peak 2)** | | |
| | 7-hydroxy-1-methyl-1-(5-methylpyridin-2-yl)-5-(2,2,2-trifluoroethyl)-1,2,3,4,4a,5-hexahydrodipyrido[1,2-b:2',1'- | |
| | f][1,2,4]triazine-6,8-dione | |
| | Precursor was separated SFC. SFC conditions: Daicel@ chiralpak OD-H column (250*30 mm, 5 µm); 30% EtOH with 0.1% DEA; 70 mL/min | |
| **50A (Peak 1)** | 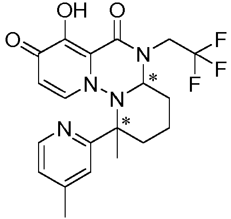 | 423.3 |
| **50B (Peak 2)** | 7-hydroxy-1-methyl-1-(4-methylpyridin-2-yl)-5-(2,2,2-trifluoroethyl)-1,2,3,4,4a,5-hexahydrodipyrido[1,2-b:2',1'-f][1,2,4]triazine-6,8-dione | |
| | Precursor was separated SFC. SFC conditions: Daicel@ chiralpak AS-H column (250*30 mm, 5 µm); 30% EtOH with 0.1% DEA; 70 mL/min | |
| **51A (Peak 1)** | 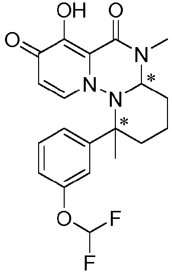 | 406.1 |
| **51B (Peak 2)** | | |
| | 1-(3-(difluoromethoxy)phenyl)-7-hydroxy-1,5-dimethyl-1,2,3,4,4a,5-hexahydrodipyrido[1,2-b:2',1'-f][1,2,4]triazine-6,8-dione | |
| | Precursor was separated SFC. SFC conditions: Daicel@ chiralpak AD-H column (250*30 mm, 5 µm); 40% EtOH with 0.1% NH₃H₂O; 70 mL/min | |
| **52A (Peak 1)** | 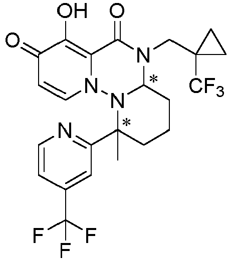 | 517.2 |
| **52B (Peak 2)** | 7-hydroxy-1-methyl-5-((1-(trifluoromethyl)cyclopropyl)methyl)-1-(4-(trifluoromethyl)pyridin-2-yl)-1,2,3,4,4a,5-hexahydrodipyrido[1,2-b:2',1'-f][1,2,4]triazine-6,8-dione | |
| | Precursor was separated SFC. SFC conditions: Daicel@ chiralpak AD-H column (250*30 mm, 10 µm); 20% EtOH with 0.1% NH₃H₂O; 70 mL/min | |
| **53A (Peak 1)** | 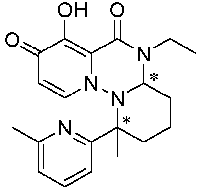 | 369.2 |
| **53B (Peak 2)** | 5-ethyl-7-hydroxy-1-methyl-1-(6-methylpyridin-2-yl)-1,2,3,4,4a,5-hexahydrodipyrido[1,2-b:2',1'-f][1,2,4]triazine-6,8-dione | |
| | Precursor was separated SFC. SFC conditions: Daicel@ chiralpak AS column (250*30 mm, 10 µm); 35% IPA with 0.1% NH₃H₂O; 70 mL/min | |
| **54A (Peak 1)** | 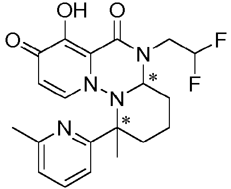 | 405.3 |
| **54B (Peak 2)** | | |
| | 5-(2,2-difluoroethyl)-7-hydroxy-1-methyl-1-(6-methylpyridin-2-yl)-1,2,3,4,4a,5-hexahydrodipyrido[1,2-b:2',1'-f][1,2,4]triazine-6,8-dione | |
| | Precursor was separated SFC. SFC conditions: Daicel@ chiralpak AS column (250*30 mm, 10 µm); 30% EtOH with 0.1% NH₃H₂O; 70 mL/min | |
| **55A (Peak 1)** | 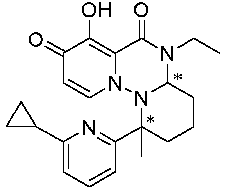 | 395.2 |
| **55B (Peak 2)** | 1-(6-cyclopropylpyridin-2-yl)-5-ethyl-7-hydroxy-1-methyl-1,2,3,4,4a,5-hexahydrodipyrido[1,2-b:2',1'-f][1,2,4]triazine-6,8-dione | |
| | Precursor was separated SFC. SFC conditions: Daicel@ chiralpak AS column (250*30 mm, 10 µm); 40% EtOH with 0.1% NH₃H₂O; 70 mL/min | |

### Example 5

### Preparation of Compounds 56A, 56B, 56C and 56D

### 5-(cyclopropylmethyl)-7-hydroxy-1-phenyl-1,2,3,4,4a,5-hexahydrodipyrido[1,2-b:2',1'-f][1,2,4]triazine-6,8-dione

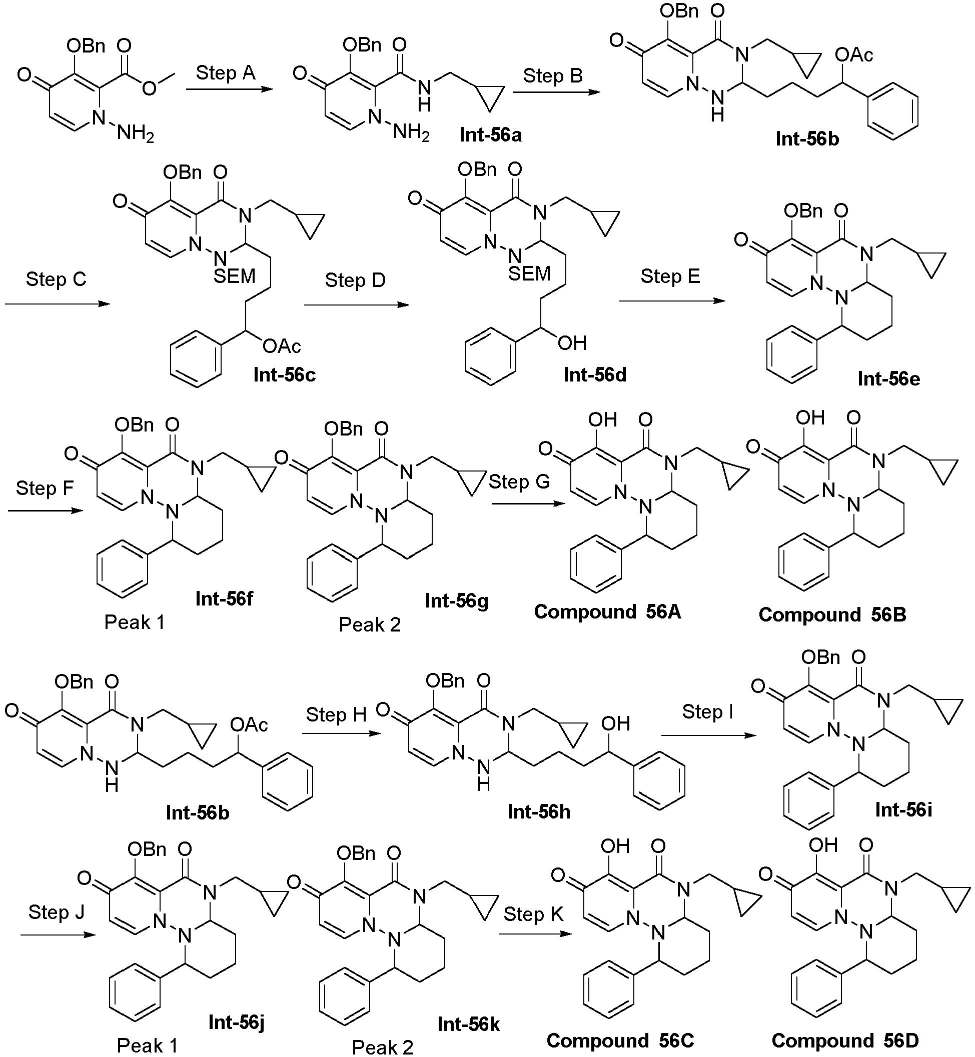

### Step A - Synthesis of Compound Int-56a

A solution of methyl 3-(benzyloxy)-1-((tert-butoxycarbonyl)amino)-4-oxo-1,4-dihydropyridine-2-carboxylate (5 g, 13.36 mmol) in EtOH (50 mL) was added to cyclopropylmethanamine (4.70 g, 40.1 mmol). The resulting reaction was stirred at 80 °C for 8 h. Upon completion, the mixture was concentrated. The residue was purified by a 220 g silica gel flash column with 0-5% MeOH in DCM as eluting solvent to afford Boc-protected intermediate (5.5 g, 11.97 mmol).

The above intermediate (5 g, 12.09 mmol) in DCM (50 mL) was added to TFA (12 mL) at 0 °C. The reaction solution was stirred at 30 °C for 1 h. Upon completion, the mixture was concentrated and purified by a 220 g silica gel flash column eluting with 0-10% MeOH in DCM to provide ***Int-56a.*** LCMS anal. calcd. for C₁₇H₁₉N₃O₃: 313.1; Found: 314.1 (M+H)⁺.

### Step B - Synthesis of Compound Int-56b

To a solution of ***Int-56a*** (200 mg, 0.638 mmol) in DMF (2.0 mL) and AcOH (0.2 mL) was added ***Int-1c*** (211 mg, 0.957 mmol) at 20 °C in a 40 mL sealed tube. The reaction was stirred at 120 °C for 4 h. Upon completion, it was concentrated and purified by a 50 g silica gel flash column with 0-10% MeOH in DCM to provide ***Int-56b.*** LCMS anal. calcd. for C₃₀H₃₃N₃O₅: 515.2; Found: 515.3 (M+H)⁺.

### Step C - Synthesis of Compound Int-56c

To a solution of ***Int-56b*** (600 mg, 1.164 mmol) in THF (10 mL) was added NaHMDS (2.327 mL, 2.327 mmol) at -78 °C under N₂. The resulting mixture was stirred at this temperature for 30 minutes followed by adding SEMCl (0.248 mL, 1.396 mmol). The resulting reaction was slowly warmed up RT for 2 h. Upon completion, it was quenched with NH₄Cl (1 N, 20 mL) and extracted with ethyl acetate (3x30 mL). The combined organic layer was dried over Na₂SO₄, filtered and concentrated. The residue was purified by an 80 g silica gel flash column with 100% EtOAc as eluting solvent to provide ***Int-56c*** and ***Int-56d.*** LCMS anal. calcd. for C₃₆H₄₇N₃O₆Si: 645.3; Found: 646.4 (M+H)⁺.

### Step D - Synthesis of Compound Int-56d

To a solution of ***Int-56c*** (160 mg, 0.248 mmol) in MeOH (3 mL) was added K₂CO₃ (41.1 mg, 0.297 mmol) at 20°C. The resulting mixture was stirred at this temperature for 3 h. Upon completion, water (10 mL) was added. The mixture was extracted with EtOAc (3x10 mL). The combined organic layers were dried over Na₂SO₄, filtered and concentrated to afford ***Int-56d.*** LCMS anal. calcd. for C₃₄H₄₅N₃O₅Si: 603.3; Found: 604.3 (M+H)⁺.

### Step E - Synthesis of Compound Int-56e

To a solution of ***Int-56d*** (145 mg, 0.240 mmol) in DCM (3 mL) was added triethylamine (0.1 mL, 0.720 mmol) and methanesulfonyl chloride (0.054 g, 0.480 mmol) at 0 °C. The resulting reaction was stirred at RT for 30 min. Upon completion, 10 mL DCM was added, and the mixture was washed with 15 mL 0.5 N HCl solution and brine. The organic was dried over Na₂SO₄ and concentrated to provide crude product. The crude was used next reaction without purification.

The above crude (210 mg, 0.337 mmol) in THF (2 mL) was added to TBAF (2.70 mL, 2.70 mmol) at 25 °C. The reaction was stirred at 50 °C for 16 h. Upon completion, it was concentrated and purified by a C18 column (YMC-Actus Triart C18 150*30mm*5um) with 10%-100% ACN in water (with 0.5% TFA modifier) as eluting solvent to provide ***Int-56e.*** LCMS anal. calcd. for C₂₈H₂₉N₃O₃: 455.2; Found: 456.2 M+H)⁺.

### Step F - Synthesis of Compound Int-56f and Int-56g

***Int-23e*** (20 mg, 0.044 mmol) was resolved with SFC to provide the first eluting peak ***Int-56f*** and the second eluting peak ***Int-56g.*** SFC condition: Regis@ Whelk O1 column (250*21 mm, 5 µm); 50% EtOH with 0.1% NH₃H₂O as modifier; 70 mL/min. LCMS anal. calcd. for C₂₈H₂₉N₃O₃: 455.2; Found: 456.2 (M+H)⁺.

### Step G - Synthesis of Compound 56A and 56B

To a stirred mixture of ***Int-56f*** (7 mg, 0.015 mmol) in acetonitrile (1 mL) was added magnesium bromide (2.83 mg, 0.015 mmol) at 20 °C. The mixture was stirred at 20 °C for 2 h under N₂ atmosphere. Upon completion, it was purified by a C18 column (YMC-Actus Triart C18 150*30mm*5um) with 10%-100% ACN in water (with 0.5% TFA modifier) as eluting solvent to provide ***23A.*** LCMS anal. calcd. for C₂₁H₂₃N₃O₃: 365.2; Found: 366.2 (M+H)⁺. ¹H NMR (400 MHz, methanol-d⁴) *δ* 7.30 (br s, 5H), 7.10 (d, *J* = 7.34 Hz, 1H), 5.95 (d, *J* = 7.34 Hz, 1H), 5.53 (br s, 1H), 4.22 (dd, *J* = 2.69, 11.74 Hz, 1H), 4.15 (dd, *J* = 8.07, 14.67 Hz, 1H), 3.25-3.29 (m, 1H), 2.58 (br d, *J* = 15.41 Hz, 1H), 2.24-2.32 (m, 2H), 1.97 (br d, *J* = 13.69 Hz, 1H), 1.85-1.92 (m, 1H), 1.72-1.81 (m, 1H), 1.14-1.21 (m, 1H), 0.54-0.62 (m, 2H), 0.41-0.46 (m, 2H).

Following essentially the method employed to produce compound ***56A*** of example **5,** compound ***56B*** was prepared from ***Int-56g.*** LCMS anal. calcd. for C₂₁H₂₃N₃O₃: 365.2; Found: 366.2 (M+H)⁺. ¹H NMR (400 MHz, methanol-d⁴) δ 7.30 (br s, 5H), 7.07 (d, *J* = 7.58 Hz, 1H), 5.89 (d, *J* = 7.58 Hz, 1H), 5.52 (br s, 1H), 4.12-4.23 (m, 2H), 3.24-3.30 (m, 1H), 2.57 (br d, *J* = 15.41 Hz, 1H), 2.24-2.32 (m, 2H), 1.96 (br d, *J* = 13.69 Hz, 1H), 1.88 (br d, *J* = 13.69 Hz, 1H), 1.74 (q, *J* = 13.78 Hz, 1H), 1.12-1.21 (m, 1H), 0.53-0.63 (m, 2H), 0.41-0.46 (m, 2H).

### Step H - Synthesis of Compound Int-56h

To a solution of ***Int-56b*** (350 mg, 0.679 mmol) in MeOH (5 mL) was added K₂CO₃ (113 mg, 0.815 mmol) and it was stirred at 20°C for 3 h. Upon completion of the reaction, water (10 mL) was added. The mixture was extracted with ethyl acetate (3x20 mL). The combined organic was dried over Na₂SO₄, filtered and concentrated. The residue was purified by an 80 g silica gel flash column with 0-10% MeOH in DCM as eluting solvent to provide ***Int-56h.*** LCMS anal. calcd. for C₂₈H₃₁N₃O₄: 473.2; Found: 474.2 (M+H)⁺.

### Step I - Synthesis of Compound Int-56i

To a solution of ***Int-56h*** (295 mg, 0.623 mmol) in T₃P (1586 mg, 2.492 mmol) was added methanesulfonic acid (59.9 mg, 0.623 mmol) under N₂. The mixture was stirred at 50 °C for 16 h. Upon completion, the reaction mixture was purified by a C18 column (YMC-Actus Triart C18 150*30mm*5um) with 10%-100% ACN in water (with 0.5% TFA modifier) as eluting solvent to provide ***Int-56i.*** LCMS anal. calcd. for C₂₈H₂₉N₃O₃: 455.2; Found: 456.2 (M+H)⁺.

### Step J - Synthesis of Compound Int-56j and Int-56k

***Int-56i*** (80 mg, 0.176 mmol) was resolved by SFC to provide the first eluting peak ***Int-56j*** and the second eluting peak ***Int-56k.*** SFC condition: Daicel@ chiralpak AD-H column (250*30 mm, 10 µm); 30% EtOH with 0.1% NH₃H₂O as modifier; 70 mL/min. LCMS anal. calcd. for C₂₈H₂₉N₃O₃: 455.2; Found: 456.2 (M+H)⁺.

### Step K - Synthesis of Compound 56C and 56D

To a stirred mixture of ***Int-56j*** (30 mg, 0.066 mmol) in acetonitrile (1 mL) was added magnesium bromide (12.12 mg, 0.066 mmol) at 20 °C and it was stirred at 20 °C for 2 h under N₂ atmosphere. Upon completion, the reaction mixture was purified by a C18 column (YMC-Actus Triart C18 150*30mm*5um) with 10%-100% ACN in water (with 0.5% TFA modifier) as eluting solvent to provide ***56C.*** LCMS anal. calcd. for C₂₁H₂₃N₃O₃: 365.2; Found: 366.2 (M+H)⁺. ¹H NMR (400 MHz, methanol-d⁴) *δ* 8.29 (d, *J* = 7.09 Hz, 1H), 7.20-7.27 (m, 1H), 7.15 (br t, *J* = 7.46 Hz, 2H), 7.05 (br d, *J* = 7.58 Hz, 2H), 6.89 (d, *J* = 7.09 Hz, 1H), 5.39-5.44 (m, 1H), 4.71 (br d, *J* = 6.36 Hz, 1H), 3.73 (dd, *J* = 7.58, 14.43 Hz, 1H), 3.28 (br d, *J* = 6.85 Hz, 1H), 2.67 (br d, *J* = 13.45 Hz, 1H), 2.46-2.59 (m, 1H), 2.19-2.43 (m, 3H), 1.87-1.97 (m, 1H), 1.06-1.17 (m, 1H), 0.49-0.62 (m, 2H), 0.38 (br d, *J* = 2.93 Hz, 2H).

Following essentially the method employed to produce compound ***56C*** of example **5,** compound ***56D*** was prepared from ***Int-56k.*** LCMS anal. calcd. for C₂₁H₂₃N₃O₃: 365.2; Found: 366.2 (M+H)⁺. ¹H NMR (400 MHz, methanol-d⁴) *δ* 8.31 (d, *J* = 7.34 Hz, 1H), 7.20-7.27 (m, 1H), 7.15 (br t, *J* = 7.46 Hz, 2H), 7.04 (br d, *J* = 7.58 Hz, 2H), 6.92 (d, *J* = 7.34 Hz, 1H), 5.40-5.44 (m, 1H), 4.71 (br d, *J* = 6.36 Hz, 1H), 3.73 (dd, *J* = 7.58, 14.67 Hz, 1H), 3.26-3.30 (m, 1H), 2.67 (br d, *J* = 13.45 Hz, 1H), 2.47-2.59 (m, 1H), 2.19-2.43 (m, 3H), 1.87-1.97 (m, 1H), 1.05-1.17 (m, 1H), 0.48-0.63 (m, 2H), 0.33-0.43 (m, 2H).

### Example 6

### Preparation of Compounds 57A and 57B

### 4-hydroxy-6-methyl-11-phenyl-6a,7,8,9,10,11-hexahydro-3H-pyrido[1',2':1,6] [1,2,4]triazino[2,3-a]azepine-3,5(6H)-dione

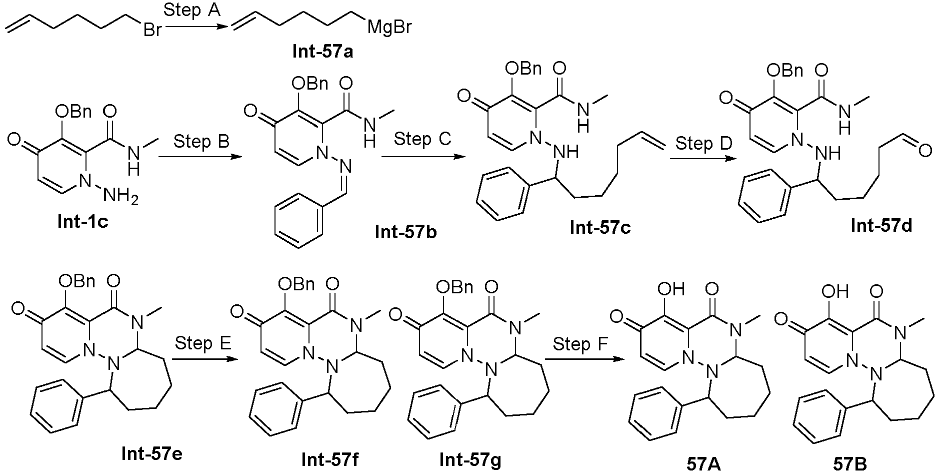

### Step A - Synthesis of Compound Int-57a

To a solution of magnesium (3.35 g, 138 mmol) in anhydrous THF (15 mL) was added I₂ (2.335 g, 9.20 mmol) and 6-bromohex-1-ene (2.5 mL, 18.40 mmol). The resulting mixture was refluxed at 80 °C for 10 min. It was cooled down to room temperature. Another 4 equiv of 6-bromohex-1-ene (10.0 mL, 73.6 mmol) in anhydrous THF (15 mL) was added dropwise. The mixture was stirred at 80 °C for 1 h. The reaction was cooled down to room temperature and it was used directly in next step (28.33 mL in THF about 2.165 M).

### Step B - Synthesis of Compound Int-57b

To a solution of ***Int-1c*** (1 g, 3.66 mmol) in EtOH (10 mL) was added benzaldehyde (0.777 g, 7.32 mmol) under N₂. The reaction was stirred at 80 °C for 10 h. Upon completion, it was concentrated. The residue was purified by an 80 g silica gel flash column with 0-100% MeOH in DCM as eluting solvent to provide ***Int-57b.*** LCMS anal. calcd. for C₂₁H₁₉N₃O₃: 361.1; Found: 362.2 (M+H)⁺.

### Step C - Synthesis of Compound Int-57c

To a solution of ***Int-57b*** (100 mg, 0.277 mmol) in THF (5 mL) was added ***Int-57a** (1* mL, 2.147 mmol, 2.147 M in THF) at 0°C and stirred at this temperature for 1 h. Upon completion, it was quenched with aq. NH₄Cl (10 mL). The mixture was extracted with 2x50 mL EtOAc. The combined organic was dried over MgSO₄ and concentrated. The residue was purified by an 80 g silica gel flash column with 0-10% MeOH in DCM as eluting solvent to provide ***Int-57c.*** LCMS anal. calcd. for C₂₇H₃₁N₃O₃: 445.2; Found: 446.3 (M+H)⁺.

### Step D - Synthesis of Compound Int-57d

To a solution of ***Int-57c*** (630 mg, 1.414 mmol) in water (15 mL) and t-BuOH (15 mL) was added sodium periodate (1512 mg, 7.07 mmol), then potassium osmate (VI) dihydrate (130 mg, 0.353 mmol) at 25 °C. The mixture was stirred at room temperature for 30 min. Upon completion, it was quenched with aq. Na₂SO₃ (50 mL) and extracted with DCM (3x50 mL). The combined organic layers were dried over Na₂SO₄, filtered and concentrated. The residue was purified by a 120 g silica gel flash column with 0-10% MeOH in DCM as eluting solvent to provide ***Int-57d.*** LCMS anal. calcd. for C₂₆H₂₉N₃O₄: 447.2; Found: 448.1 (M+H)⁺.

### Step E - Synthesis of Compound Int-57e

To a solution of ***Int-57d*** (400 mg, 0.894 mmol) in ACN (30 mL) was added MsOH (0.174 mL, 2.68 mmol). The resulting reaction was stirred at 70 °C for 2.5 h. Upon completion, the mixture was concentrated and purified with preparative HPLC using Boston Green ODS (150*30mm*5um) column with water ACN in water (0.1%TFA modifier) to provide ***Int-57e*** (150 mg, 0.349 mmol). LCMS anal. calcd. for C₂₆H₂₇N₃O₃: 447.2; Found: 430.2 (M+H)⁺.

### Step F - Synthesis of Compound Int-57f and Int-57g

***Int-57e*** (250 mg, 0.582 mmol) was resolved by SFC to provide the first eluting peak ***Int-57f*** and the second eluting peak ***Int-57g.*** SFC condition: Daicel^{@} chiralpak IG column (250*30 mm, 10 µm); 55% EtOH with 0.1% NH₃H₂O; 80 mL/min. LCMS anal. calcd. for C₂₆H₂₇N₃O₃: 447.2; Found: 430.2 (M+H)⁺.

### Step G - Synthesis of Compound 57A and 57B

To a solution of ***Int-57f*** (80 mg, 0.186 mmol) in ACN (5 mL) was added MgBr₂ (171 mg, 0.931 mmol) at 25 °C and the mixture was stirred for 4 h. Upon completion, MeOH (1 mL) was added. The mixture was purified by preparative HPLC with a Phenomenex Synergi C18 column (150*30mm*4um) using 0-100% ACN in water (with 0.1%TFA modifier) as eluting solvent to provide ***57A*** (55.57 mg, 0.164 mmol). LCMS anal. calcd. for C₁₉H₂₁N₃O₃: 339.1; Found: 340.2 (M+H)⁺. ¹H NMR (400 MHz, Methanol-d⁴) *δ* 7.85 (d, *J* = 7.3 Hz, 1H), 7.17 (br s, 5H), 6.29 (d, *J* = 7.3 Hz, 1H), 5.33 (dd, J = 7.6, 9.05 Hz, 1H), 4.79 (br d, *J* = 10.8 Hz, 1H), 3.28 (s, 3H), 2.53-2.67 (m, 2H), 2.10-2.27 (m, 2H), 2.05 (br dd, *J* = 3.67, 13.94 Hz, 1H), 1.90-1.99 (m, 2H), 1.59-1.72 (m, 1H).

Following essentially the method employed to produce compound ***57A*** of example **6,** compound ***57B*** was prepared from ***Int-57g.*** LCMS anal. calcd. for C₁₉H₂₁N₃O₃: 339.1; Found: 340.2 (M+H)⁺. ¹H NMR (400 MHz, Methanol-d⁴) *δ* 7.85 (d, *J* = 7.34 Hz, 1H), 7.17 (br s, 5H), 6.27 (d, *J* = 7.3 Hz, 1H), 5.33 (dd, *J* = 7.5, 9.2 Hz, 1H), 4.79 (br d, *J* = 10.5 Hz, 1H), 3.28 (s, 3H), 2.51-2.67 (m, 2H), 2.09-2.25 (m, 2H), 2.05 (br dd, *J* = 3.8, 13.8 Hz, 1H), 1.89-2.00 (m, 2H), 1.60-1.73 (m, 1H).

### Example 7

### Preparation of Compounds 58A, 58B, 58C and 58D

### 4-(benzyloxy)-6-methyl-12-phenyl-6,6a,7,8,9,10,11,12-octahydropyrido[1',2':1,6][1,2,4]triazino[2,3-a]azocine-3,5-dione

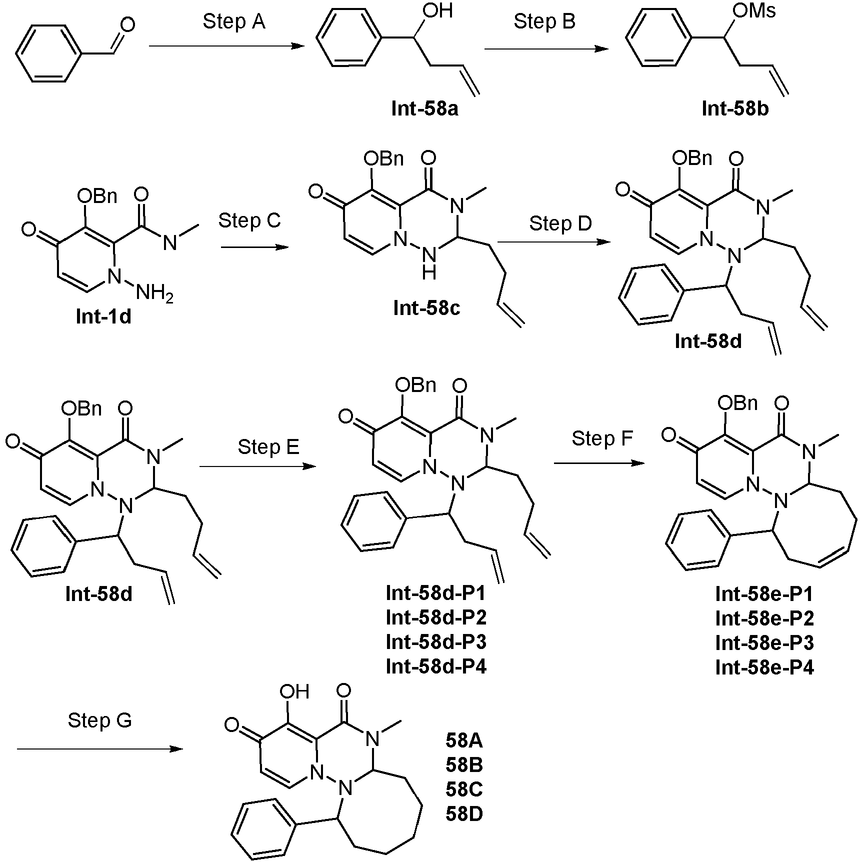

### Step A - Synthesis of Compound Int-58a

To a solution of benzaldehyde (5 g, 47.1 mmol) in THF (50 mL) was added allylmagnesium bromide (141 mL, 141 mmol, 1 M in Et₂O) at 0 °C. The mixture was stirred at 25 °C for 4 h. Upon completion, the mixture was quenched with aq. NH₄Cl (25 mL) and extracted with EtOAc (2x50 mL). The combined the organic layers were washed with brine (30 mL), dried over anhydrous Na₂SO₄, filtered and concentrated. The residue was purified by silica gel column with 0 to 10% EtOAc in petroether to provide ***Int-58a.* ¹H NMR** (400 MHz, Chloroform-d) δ: 7.26~7.35 (m, 5H), 5.77-5.85 (m, 1H), 5.12-5.18 (m, 2H), 4.72 (d, *J* = 8 Hz, 1H), 2.48-2.52 (m, 2H), 2.16 (brs, 1H).

### Step B - Synthesis of Compound Int-58b

To a solution of 1-phenylbut-3-en-1-ol (500 mg, 3.37 mmol) in DCM (10 mL) was added TEA (0.940 mL, 6.75 mmol) and methanesulfonyl chloride (0.394 mL, 5.06 mmol) at 0°C, and the mixture was stirred at 0 °C for 1 h. Upon completion, the mixture was quenched with water (10 mL) and extracted with MTBE (3x10 mL). The combined organic layers were dried over Na₂SO₄, filtered and concentrated to afford 1-phenylbut-3-en-1-yl methanesulfonate, which was used to the next step without further purification.

### Step C - Synthesis of Compound Int-58c

To a solution of ***Int-1d*** (350 mg, 0.859 mmol) in DMF (1 mL) and AcOH (0.1 mL) was added pent-4-enal (38.7 mg, 1.288 mmol) at 25 °C. The solution was stirred at 120 °C for 4 h. Upon completion, the mixture was filtered and concentrated. The crude product was purified by a 12 g flash silica gel column with 0~10% MeOH in DCM as eluting solvent to provide ***Int-58c.*** LCMS anal. calcd. for C₁₉H₂₁N₃O₃: 339.2; Found: 340.2 (M+H)⁺.

### Step D - Synthesis of Compound Int-58d

To a solution of ***Int-58c*** (750 mg, 2.210 mmol) in DMF (10 mL) was added NaH (265 mg, 6.63 mmol) and 1-phenylbut-3-en-1-yl methanesulfonate (750 mg, 3.31 mmol) at 0 °C. The solution was stirred at 0 °C for 1 h. Upon completion, the reaction was quenched with aqueous NH₄Cl (5 mL) and diluted with water (20 mL). The mixture was extracted with DCM (3x30 mL). The combined organic layers were dried over Na₂SO₄, filtered and concentrated. The residue was purified by a 40 g flash silica gel column with 0~10% MeOH in DCM as eluting solvent to provide ***Int-58d.*** LCMS anal. calcd. for C₂₀H₃₁N₃O₃: 469.2; Found: 470.2 (M+H)⁺.

### Step E - synthesis of Compounds Int-58d-P1, Int-58d-P2, Int-58d-P3 and Int-58d-P4

The mixture ***Int-58d*** (200 mg, 0.426 mmol) was resolved to provide a mixture of ***Int-58d-P1*** and ***Int-58d-P2*** (110 mg, 0.223 mmol) as first eluting isomers and a mixture of ***Int-58d-P3*** and ***Int-58d-P4*** (90 mg, 0.168 mmol) as second eluting isomers. SFC condition: REGIS (*s*, *s*) WHELK-O1 column (250 mm * 30 mm, 5 µm); 55% EtOH with 0.1% NH₃H₂O; 60 mL/min.

The mixture of ***Int-58d-P1 and Int-58d-P2*** (110 mg, 0.234 mmol) was further separated by SFC to provide the first eluting peak ***Int-25d-P1*** and the second eluting peak ***Int-58d-P2.*** SFC condition: DAICEL CHIRALPAK AD-H (250 mm * 30 mm, 5 µm); 55% EtOH with 0.1% NH₃H₂O; 65 mL/min. LCMS anal. calcd. for C₂₉H₃₁N₃O₃: 469.2; Found: 470.2 (M+H)⁺.

The mixture of ***Int-58d-P3 and Int-58d-P4*** (90 mg, 0.192 mmol) was further separated by SFC to provide the first eluting peak ***Int-58d-P3*** and the second eluting peak ***Int-58d-P4.*** SFC condition: Phenomenex-Amylose-1 (250 mm * 30 mm, 5 µm); 55% EtOH with 0.1% NH₃H₂O; 50 mL/min. LCMS anal. calcd. for C₂₉R₃₁N₃O₃: 469.2; Found: 470.2 (M+H)⁺.

### Step F - Synthesis of Compounds Int-58e-P1, Int-58e-P2, Int-58e-P3 and Int-58e-P4

To a solution of ***Int-58d-P2*** (50 mg, 0.106 mmol) in DCE (8 mL) was added (1,3-dimesitylimidazolidin-2-ylidene)(2-isopropoxybenzylidene)ruthenium(VI) chloride (13.34 mg, 0.021 mmol). The reaction mixture was stirred at 45 °C for 16 h. Upon completion, the reaction was quenched with water and extracted with DCM (3x10 mL). The combined organic layers were washed with brine (10 mL), dried over Na₂SO₄, filtered and concentrated. The residue was purified by pre-TLC eluting with 0 to10% MeOH in DCM to provide (Z)-4-(benzyloxy)-6-methyl-12-phenyl-6,6a,7,8,11,12-hexahydropyrido[1',2':1,6][1,2,4]triazino[2,3-a]azocine-3,5-dione as a mixture.

The above mixture, combined with another batch (150 mg), was purified by a YMC-Actus Pro C18 (150 mm * 30 mm 5 µm) with ACN in water (with 0.1% TFA modifier) as eluting solvent to provide a mixture which was further purified by pre-TLC eluting with 0 to 10% MeOH in DCM to afford ***Int-58e-P1*** as the first peak and ***Int-58e-P2*** as the second peak. LCMS anal. calcd. for C₂₇H₂₇N₃O₃: 441.2; Found: 442.4 (M+H)⁺.

Following essentially the method employed to produce compounds ***Int-58e-P1*** and ***Int-58e-P2*** of example 7, compounds ***Int-58e-P3*** and ***Int-58e-P4*** were prepared from ***Int-58d-P3.*** LCMS anal. calcd. for C₂₇H₂₇N₃O₃: 441.2; Found: 442.4 (M+H)⁺.

### Step G - Synthesis of Compounds 58A, 58B, 58C and 58D

To a solution of ***Int-58e-P4*** (25 mg, 0.057 mmol) in MeOH (2 mL) was added Pd on carbon (24.10 mg, 0.011 mmol). The reaction mixture was degassed under vacuum and purged with H₂ for 3 times. The reaction mixture was stirred at 20 °C for 1 h under H₂ balloon. Upon completion, the reaction was filtered and purified by preparative HPLC using YMC-Actus Pro C18 (150 mm * 30 mm 5 µm) column with ACN in water (with 0.1% TFA modifier) as eluting solvent to provide ***58D.*** LCMS anal. calcd. for C₂₀H₂₃N₃O₃: 353.2; Found: 354.2 (M+H)⁺. ¹H NMR (500 MHZ, Methanol-d⁴) *δ* 7.96 (dd, *J* = 7.2, 3.7 Hz, 1H), 6.78-7.61 (m, 5H), 6.64 (br t, *J* = 7.2 Hz, 1H), 5.15 (br d, *J* = 11.6 Hz, 1H), 4.57-4.66 (m, 1H), 3.00 (s, 3H), 2.32-2.48 (m, 1H), 1.55-2.09 (m, 8H), 1.23-1.39 (m, 1H).

Following essentially the method employed to produce compound ***58D*** of example **7,** compounds ***58A, 58B*** and ***58C*** were prepared from ***Int-58e-P1*, *Int-58e-P2*** and ***Int-58e-P3.***
***58A:*** LCMS anal. calcd. for C₂₀H₂₃N₃O₃: 353.2; Found: 354.2 (M+H)⁺.¹H NMR (500 MHz, Methanol-d⁴) *δ* 7.83-8.06 (m, 1H), 7.02-7.36 (m, 5H), 6.37-6.66 (m, 1H), 5.13 (br d, *J* = 9.6 Hz, 1H), 4.39-4.65 (m, 1H), 2.97-3.13 (m, 3H), 2.24-2.48 (m, 1H), 1.51-2.07 (m, 9H),
***58B:*** LCMS anal. calcd. for C₂₀H₂₃N₃O₃: 353.2; Found: 354.2 (M+H)⁺. ¹H NMR (500 MHz, Methanol-d⁴) *δ* 7.84-7.99 (m, 1H), 6.98-7.54 (m, 5H), 6.49-6.75 (m, 1H), 5.15 (br dd, *J* = 11.4, 3.0 Hz, 1H), 4.43-4.64 (m, 1H), 2.98-3.10 (m, 3H), 2.30-2.46 (m, 1H), 1.54-2.10 (m, 8H), 1.31 (br d, J=10.7 Hz, 1H),
***58C:*** LCMS anal. calcd. for C₂₀H₂₃N₃O₃: 353.2; Found: 354.2 (M+H)⁺. ¹H NMR (500 MHz, Methanol-d⁴) *δ* 7.65-7.87 (m, 1H), 6.87-7.27 (m, 5H), 6.29-6.55 (m, 1H), 4.90-5.11 (m, 1H), 4.20-4.49 (m, 1H), 2.83-3.01 (m, 3H), 2.12-2.32 (m, 1H), 1.38-1.92 (m, 9H),

### Example 8

### Preparation of Compounds 59A and 59B

### 7-hydroxy-4a,5-dimethyl-1-phenyl-1,2,3,4,4a,5-hexahydrodipyrido[1,2-b:2',1'-f][1,2,4]triazine-6,8-dione

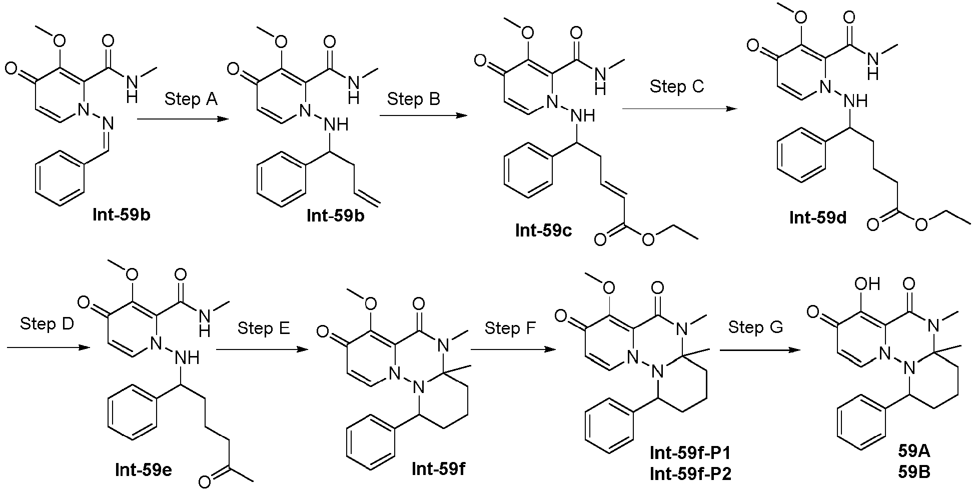

### Step A - Synthesis of Compound Int-59b

To a solution of ***Int-59b*** (1.2g, 4.21 mmol) in THF (130 mL) was added allylmagnesium bromide (12.62 mL, 12.62 mmol) at 0 °C. The reaction was warmed to 0 °C and stirred for 2 h. Upon completion, the reaction mixture was quenched with aq. NH₄Cl then diluted with water (2 mL) and extracted with DCM (2 x10 mL). The combined organic layer was dried over Na₂SO₄, filtered and concentrated. The residue was purified using a 40 g silica gel flash column on ISCO^{®} with 0-10% MeOH in DCM as eluting solvent to provide ***Int-59b.*** LCMS anal. calcd. for C₁₈H₂₁N₃O₃: 327.2; Found: 328.2 (M+H)⁺.

### Step B - Synthesis of Compound Int-59c

To a mixture of ***Int-59b*** (200 mg, 0.611 mmol) in DCE (10 mL) was added ethyl acrylate (0.455 ml, 4.28 mmol) and Grubbs II (207 mg, 0.244 mmol) under N₂. The mixture was stirred at 58 °C for 15 h. Upon completion, the mixture was concentrated. The residue was purified by a 12 g silica gel column on ISCO^{®} with 0~10% MeOH in DCM as eluting solvent to afford ***Int-59c.*** LCMS anal. calcd. for C₂₁H₂₅N₃O₅: 399.2; Found: 400.2 (M+H)⁺.

### Step C - Synthesis of Compound Int-59d

To a mixture of ***Int-59c*** (200 mg, 0.501 mmol) in MeOH (4 mL) was added palladium on carbon (53.3 mg, 0.501 mmol). The mixture was degassed and refilled with H₂ and stirred at 20 °C for 2 h. Upon completion, the mixture was filtered through celite. The filtrate was concentrated. The residue was purified by a 24 g silica gel column on ISCO^{®} with 0-10% MeOH in DCM as eluting solvent to ***Int-59d.*** LCMS anal. calcd. for C₂₁H₂₇N₃O₅: 401.2; Found: 402.2 (M+H)⁺.

### Step D - Synthesis of Compound Int-59e

To a mixture of ***Int-59d*** (200 mg, 0.498 mmol) in THF (4 mL) was added methylmagnesium bromide (1.495 mL, 1.495 mmol, 3M in Et₂O) under N₂. The mixture was stirred at 20 °C for 0.5 h. Upon completion, the reaction mixture was quenched with aqueous NH₄Cl (10 mL). It was extracted with DCM (2x10 mL). The combined organic layers were dried over Na₂SO₄, filtered and concentrated to afford ***Int-59e.*** The crude product was used for next step without further purification. LCMS anal. calcd. for C₂₀H₂₄N₃O₄: 371.2; Found: 372.2 (M+H)⁺.

### Step E - Synthesis of Compound Int-59f

To a mixture of ***Int-59e*** (7 mg, 0.019 mmol) in acetonitrile (1 mL) was added methanesulfonic acid (20 mg, 0.208 mmol) under N₂. The mixture was stirred at 70 °C for 1.5 h. Upon completion, the mixture was concentrated. The residue was purified by prep HPLC using Phenomenex Synergi C18 column (150 mm * 30 mm * 4 µm) with 0-100% ACN in water (with 0.1% TFA modifier) as eluting solvent to provide ***Int-59f.*** LCMS anal. calcd. for C₂₀H₂₃N₃O₃: 353.2; Found: 354.2 (M+H)⁺.

### Step F - Preparation of Compounds Int-59f-P1 and Int-59f-P2

***Int-59f*** (33.5 mg, 0.095 mmol) was resolved by SFC to provide the first eluting peak ***Int-59f -P1*** and the second eluting peak ***Int-59f-P2.*** SFC condition: Daicel^{@} chiralpak AD column (250*30 mm, 10 µm); 40% IPA with 0.1% NH₃H₂O as modifier; 60 mL/min. LCMS anal. calcd. for C₂₀H₂₃N₃O₃: 353.2; Found: 354.2 (M+H)⁺.

### Step G - Synthesis of Compounds 59A and 59B

To a stirred mixture of ***Int-59f -P1*** (15 mg, 0.042 mmol) in ACN (3 mL) was added magnesium bromide (23.44 mg, 0.127 mmol) at 20 °C. The mixture was stirred at 20 °C for 2 h under N₂ atmosphere. Upon completion, the reaction was quenched with MeOH (0.2 mL). It was purified using prep HPLC on Phenomenex Synergi C18 column (150 mm * 30 mm * 4 µm) with 0-100% ACN in water (with 0.1% TFA modifier) as eluting solvent to provide ***59A.*** LCMS anal. calcd. for C₁₉H₂₁N₃O₃: 339.2; Found: 340.2 (M+H)⁺. ¹H NMR (500 MHz, Methanol-d⁴) *δ* 7.01-7.50 (m, 5H), 6.01 (d, *J* = 7.32 Hz, 1H), 4.12 (dd, *J* = 2.90, 11.75 Hz, 1H), 3.26 (s, 3H), 2.59 (br dd, *J* = 2.52, 15.34 Hz, 1H), 2.17-2.30 (m, 1H), 2.03-2.15 (m, 1H), 1.98 (br d, *J* = 13.73 Hz, 1H), 1.86-1.94 (m, 1H), 1.72 (tq, *J* = 3.47, 13.61 Hz, 1H), 1.43 (s, 3H).

Following essentially the method employed to produce compound ***59A*** of example **8,** compound ***59B*** was prepared from ***Int-59f -P2.*** LCMS anal. calcd. for C₁₉H₂₁N₃O₃: 339.2; Found: 340.2 (M+H)⁺. ¹H NMR (500 MHz, Methanol-d⁴) *δ* 7.07-7.46 (m, 5H), 6.01 (d, *J* = 7.32 Hz, 1H), 4.12 (dd, *J* = 2.82, 11.83 Hz, 1H), 3.20-3.29 (m, 3H), 2.59 (dd, *J* = 2.67, 15.34 Hz, 1H), 2.25 (dq, *J* = 4.04, 12.84 Hz, 1H), 2.05-2.16 (m, 1H), 1.98 (td, *J* = 2.86, 13.66 Hz, 1H), 1.85-1.93 (m, 1H), 1.66-1.78 (m, 1H), 1.40-1.46 (m, 3H).

### Example 9

### Preparation of Compounds 60A and 60B

### 6-hydroxy-4-methyl-1-phenyl-2,3,3a,4-tetrahydro-1H-pyrido[2,1-f]pyrrolo[1,2-b][1,2,4]triazine-5,7-dione

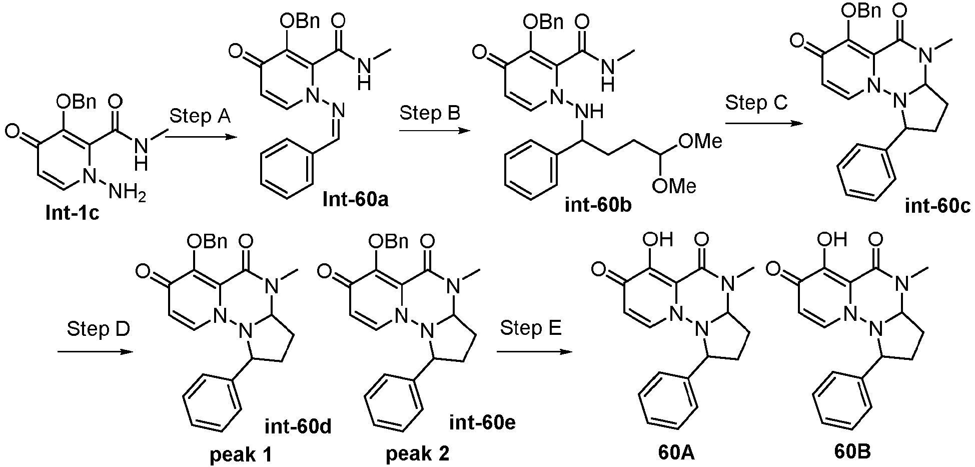

### Step A - Synthesis of Compound Int-60a

To a solution of ***Int-1c*** (1 g, 3.66 mmol) in EtOH (10 mL) was added benzaldehyde (0.777 g, 7.32 mmol) under N₂. The reaction was stirred at 80 °C for 10 h. Upon completion, the reaction was concentrated and purified by a 40 g silica gel flash column with 0~10% MeOH in DCM as eluting solvent to provide ***Int-60a.*** LCMS anal. calcd. for C₂₁H₁₉N₃O₃: 361.1; Found: 362.2 (M+H)⁺.

### Step B - Synthesis of Compound Int-60b

The solution of 10-(3,5-dimethoxyphenyl)-9-mesityl-1,3,6,8-tetramethoxyacridin-10-ium tetrafluoroborate (8.87 mg, 0.014 mmol), Na₂CO₃ (73.3 mg, 0.692 mmol) and ***Int-60a*** (50 mg, 0.138 mmol) in dioxane (1384 µL) was bubbled with N₂ for 2 mins, followed by the addition of 1,1,1,3,3,3-hexamethyl-2-(trimethylsilyl)trisilan-2-ol (36.6 mg, 0.138 mmol) and 3-bromo-1,1-dimethoxypropane (101 mg, 0.553 mmol). The resulting mixture was irradiated with Kessil Lamps (Kessil KSH150B Grow Light Blue (34w) 7 cm away from the reaction vessels for 15 h at 20 °C) for 16 h. Upon completion, the crude product was purified by a C18 reverse phase column (YMC-Actus Triart C18 150*30mm*5um) with 30-100% ACN in water (with 0.1% TFA modifier) to provide ***Int-60b.*** LCMS anal. calcd. for C₂₆H₃₁N₃O₅: 465.2; Found: 466.2 (M+H)⁺.

### Step C - Synthesis of Compound Int-60c

To a solution of ***Int-60b*** (10 mg, 0.021 mmol) in ACN (1 mL) and H₂O (0.2 mL) was added MsOH (4.18 µL, 0.064 mmol). The mixture was stirred at 60 °C for 2 h. Upon completion, the reaction mixture was directly purified by preparative HPLC (Column: YMC-Actus Triart C18 150*30mm*5um) with 30-100% ACN in water (with 0.1% TFA modifier) to provide ***Int-60c.*** LCMS anal. calcd. for C₂₄H₂₃N₃O₃: 401.2; Found: 402.0 (M+H)⁺.

### Step D - Synthesis of Compound Int-60d and Int-60e

***Int-27c*** (35 mg, 0.087 mmol) was resolved by SFC to provide the first eluting peak ***Int-60d*** and the second eluting peak ***Int-60e.*** SFC condition: Daicel^{@} chiralpak AD-H column (250*30 mm, 5 µm); 30% EtOH with 0.1% NH₃H₂O; 60 mL/min. LCMS anal. calcd. for C₂₄H₂₃N₃O₃: 401.2; Found: 402.0 (M+H)⁺.

### Step E - Synthesis of Compound 60A

To a solution of ***Int-60d*** (17 mg, 0.042 mmol) in ACN (2 mL) was added magnesium bromide (39.0 mg, 0.212 mmol) at 25 °C and it was stirred for 16 h. Upon completion, the mixture was diluted with MeOH (0.2 mL) and purified by reverse phase HPLC (Agela DuraShell C18 150 mm * 25 mm * 5µm) with 0-100% ACN in water (with 0.1%TFA modifier) as eluting solvent to provide ***60A.*** LCMS anal. calcd. for C₁₇H₁₇N₃O₃: 311.2; Found: 312.1 (M+H)⁺. ¹H NMR (500 MHz, methanol-d⁴) δ 7.34-7.46 (m, 1H), 7.38 (br d, *J* = 8.24 Hz, 4H), 7.07 (d, *J* = 7.32 Hz, 1H), 6.23 (d, *J* = 7.32 Hz, 1H), 5.41 (d, *J* = 3.97 Hz, 1H), 4.30-4.52 (m, 1H), 2.50-2.72 (m, 3H), 2.12-2.19 (m, 1H).

Following essentially the method employed to produce compound ***60A*** of example **9,** compound ***60B*** was prepared. LCMS anal. calcd. for C₁₇H₁₇N₃O₃: 311.2; Found: 312.1 (M+H)⁺. ¹H NMR (500 MHz, methanol-d⁴) *δ* 7.33-7.49 (m, 5H), 7.09 (d, *J* = 7.48 Hz, 1H), 6.27 (d, *J* = 7.32 Hz, 1H), 5.42 (d, *J* = 4.12 Hz, 1H), 4.36-4.51 (m, 1H), 2.50-2.74 (m, 3H), 2.05-2.25 (m, 1H).

### Example 10

### Preparation of Compounds 61A and 61B

### 6-hydroxy-1,4-dimethyl-1-phenyl-2,3,3a,4-tetrahydro-1H-pyrido[2,1-f]pyrrolo[1,2-b][1,2,4]triazine-5,7-dione

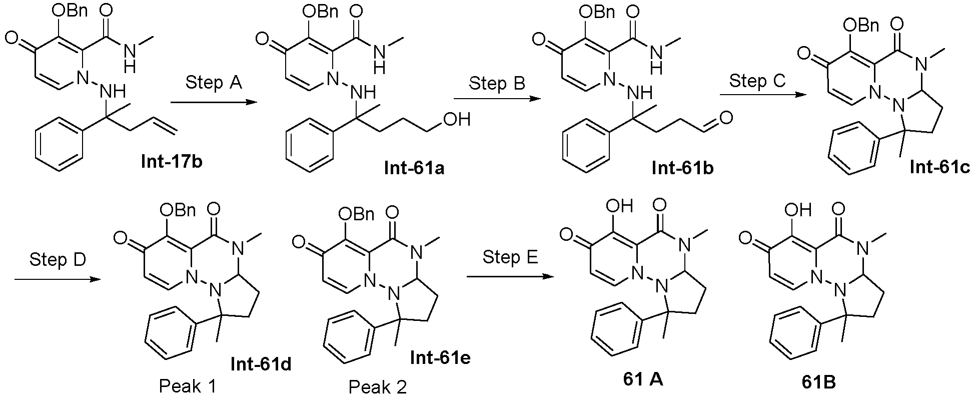

### Step A - Synthesis of Compound Int-61a

To the stirred solution of ***Int-17b*** (420 mg, 1.006 mmol) in anhydrous THF (12 mL) at 0 °C was added 9-borabicyco[3.3.1]nonane (0.5 M in THF) (8.05 mL, 4.02 mmol) dropwise. The resulting solution was stirred at 0 °C for 0.5 h, then stirred at room temperature for 2 h. Additional 9-BBN (0.5 M in THF) (4.02 mL, 2.012 mmol) was added to the reaction. The resulting mixture was stirred at room temperature for another 2 h. Upon completion, to the cooled mixture, a solution of sodium acetate (825 mg, 10.06 mmol) was added dropwise in water (5 mL) and hydrogen peroxide (35% in water) (1.321 mL, 15.09 mmol) at 0 °C. The resulting mixture was stirred at 0 °C for 30 min, then at room temperature for 5 h. The resulting reaction was then partitioned between EtOAc (100 mL) and water (25 mL). The aqueous phase was extracted with EtOAc two more times (2x50 mL). The combined organic phase was washed with 10% Na₂S₂O₃ in water (~ 50 mL), dried over Na₂SO₄, filtered and concentrated. The residue was purified by a 100 g silica gel flash column with 0-100% EtOAc in EtOH (3:1)/hexane as eluting solvent to provide ***Int-61a.*** LCMS m/z calcd for C₂₅H₂₉N₃O₄: 435.2; found 436.4 (M+H)⁺.

### Step B - Synthesis of Compound Int-61b

To the stirred suspension of ***Int-61a*** (240 mg, 0.551 mmol) in DCM (9184 µL) was added Dess-Martin periodinane (280 mg, 0.661 mmol) at 0 °C. The mixture was stirred at room temperature for 2 h. Upon completion, the reaction mixture was diluted with DCM (200 mL) and washed with satd. NaHCO₃ (~ 50 mL), and then with 10% Na₂S₂O₃ in water (~ 50 mL). The organic phase was separated, dried over Na₂SO₄, filtered and concentrated in vacuo to provide ***Int-61b.*** LCMS m/z calcd for C₂₅H₂₇N₃O₄: 433.2; found 434.4 (M+H)⁺.

### Step C - Synthesis of Compound Int-61c

To the stirred solution of ***Int-61b*** (100 mg, 0.231 mmol) in DMF (3 mL) was added AcOH (262 µL) at room temperature. The mixture was then stirred at 120 °C for 2 h. Upon completion, the mixture was cooled to room temperature, then purified by reverse phase HPLC using Isco system (RediSepRf Gold C18 column 100 g) with 0-100% acetonitrile-water (buffered with 0.05 % TFA) as eluting solvent to afford TFA salt form of ***Int-61c*** ( 88 mg, 0.166 mmol). The above product was then redissolved in DCM, neutralized with the addition of TEA (32 ul, 0.23 mmol), and re-purified by flash chromatography using Isco system (40 g silica gel gold column) with 0-100% EtOAc-EtOH (3:1)/hexane as eluting solvent to afford free base form of ***Int-61c.*** LCMS m/z calcd for C₂₅H₂₅N₃O₃: 415.2; found 416.3 (M+H)⁺.

### Step D - Synthesis of Compound Int-61d and Int-61e

***Int-28c*** (32 mg, 0.077 mmol) was resolved by SFC to provide the first eluting peak ***Int-61d*** and the second eluting peak ***Int-61e.*** SFC condition: Daicel^{@} chiralpak OJ-H column (250*21 mm, 5 µm); 30% IPA with 0.2% DIPA; 50 mL/min. LCMS m/z calcd for C₂₅H₂₅N₃O₃: 415.2; found 416.3 (M+H)⁺.

### Step E - Synthesis of Compound 61A

To the round bottom flask was added palladium (10% on carbon) (17.93 mg, 0.017 mmol), followed by addition of ***Int-61d*** (14 mg, 0.034 mmol) in EtOH (12 mL). The flask was equipped with a hydrogen balloon. The system was degassed and flushed with H₂ twice. The mixture was stirred at room temperature under H₂ for 40 min. Upon completion, the catalyst was filtered off, and the filtrate was concentrated. The residue was purified by reverse phase HPLC using Gilson System (Sunfire Prep 30 x 150 mm column) with 5-100% acetonitrile-water (buffered with 0.05 % TFA) as eluting solvent to provide ***61A.*** LCMS m/z calcd for C₁₈H₁₉N₃O₃: 325.1; found 326.3 (M+H)⁺. ¹H NMR (500 MHz, Methanol-d4) *δ* 7.65 (d, *J* = 8.1 Hz, 2H), 7.45 (t, *J* = 7.5 Hz, 2H), 7.35 (t, J = 7.0 Hz, 1H), 7.16 (d, *J* = 7.4 Hz, 1H), 6.35 (d, *J* = 7.4 Hz, 1H), 5.47 (d, *J* = 3.7 Hz, 1H), 3.17 (s, 3H), 2.59 (dd, *J* = 14.0, 7.2 Hz, 1H), 2.51 - 2.41 (m, 1H), 2.40 - 2.30 (m, 1H), 2.23 (dd, *J* = 12.5, 7.8 Hz, 1H), 1.44 (s, 3H).

Following essentially the method employed to produce compound ***61A*** of example **10,** compound ***61B*** was prepared from ***Int-61e.*** LCMS m/z calcd for C₁₈H₁₉N₃O₃: 325.1; found 326.3 (M+H)⁺. ¹H NMR (500 MHz, Methanol-d4) δ 7.65 (d, *J* = 8.1 Hz, 2H), 7.46 (t, *J* = 7.5 Hz, 2H), 7.36 (t, *J* = 7.1 Hz, 1H), 7.23 (d, *J* = 7.4 Hz, 1H), 6.48 (d, *J* = 7.3 Hz, 1H), 5.49 (d, *J* = 3.7 Hz, 1H), 3.33 (s, 9H), 3.18 (s, 3H), 2.60 (dd, *J* = 14.2, 7.3 Hz, 1H), 2.53 - 2.41 (m, 1H), 2.41 - 2.31 (m, 1H), 2.24 (dd, *J* = 12.6, 7.9 Hz, 1H), 1.44 (s, 3H).

The examples in **Table 3** were prepared using procedures similar to those described in **Example 10,** from the appropriate starting material.

**Table 3**

| **Example** | **Structure** | **Observed *m*/*z* [M + H]⁺** |
|---|---|---|
| | **Name** | |
| | **Precursor SFC Separation Condition** | |
| **62A, 62B, 62C, 62D** | 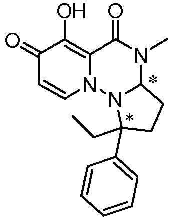 | 340.2 |
| | 1-ethyl-6-hydroxy-4-methyl-1-phenyl-2,3,3a,4-tetrahydro-1H-pyrido[2,1-f]pyrrolo[1,2-b][1,2,4]triazine-5,7-dione | |
| | Precursor was resolved by SFC. SFC conditions: Daicel^{@} chiralpak AD-H(250 mm * 30 mm, 5 µm), Condition: 0.1%NH₃.H₂O, MeOH, 60 mL/min and Phenomenex-Cellulose-2 (250 mm * 30 mm, 10 µm), Condition: 0.1%NH₃.H₂O, MeOH, 70 mL/min | |
| **63A (early peak) 63B (late peak)** | 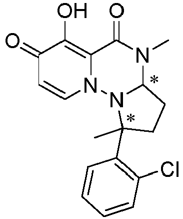 | 360.1 |
| | 1-(2-chlorophenyl)-6-hydroxy-1,4-dimethyl-2,3,3a,4-tetrahydro-1H-pyrido[2,1-f]pyrrolo[1,2-b][1,2,4]triazine-5,7-dione | |
| | Precursor was resolved by SFC. SFC conditions: Daicel^{@} chiralpak OJ-H column (250*21 mm, 10 µm); 30% MeOH with 0.2% DIPA; 70 mL/min | |

### Example 11

### Preparation of Compounds 64A, 64B, 64C and 64D

### 2-allyl-1-(2-chlorophenyl)-7-hydroxy-5-methyl-1,2,3,4,4a,5-hexahydrodipyrido[1,2-b:2',1'-f][1,2,4]triazine-6,8-dione

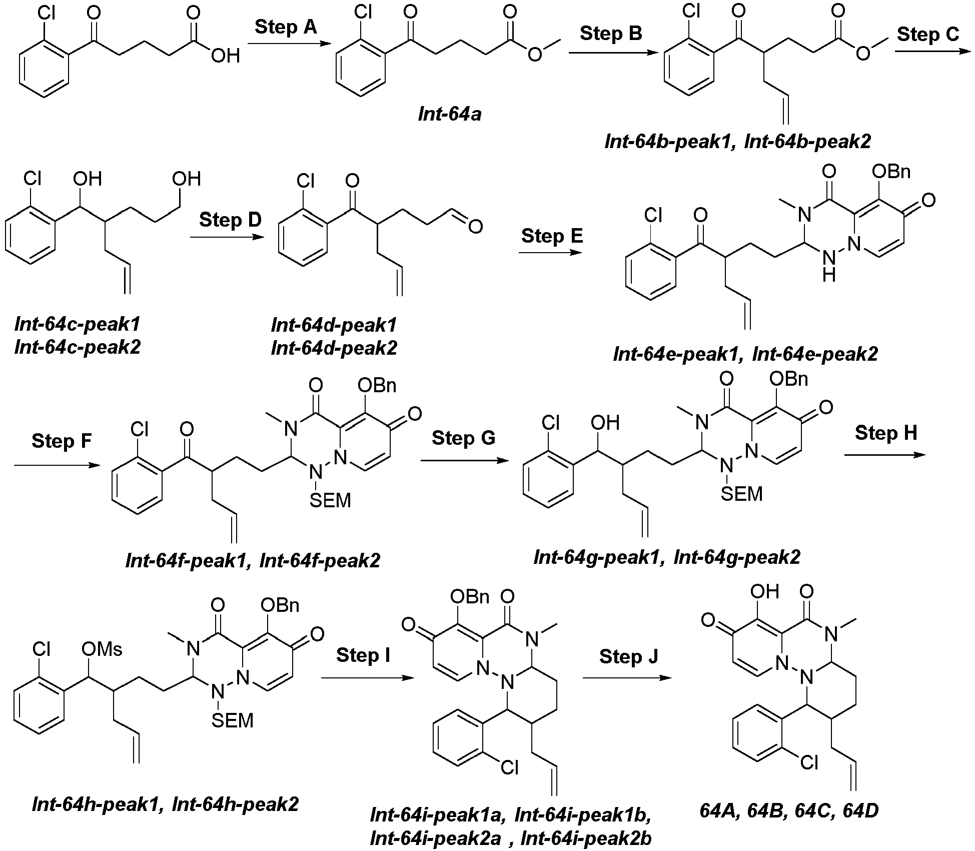

### Step A - Synthesis of Compound Int-64a

Under nitrogen, TMS-Diazomethane (16.68 ml, 33.4 mmol, 2M) was added to a solution of 5-(2-chlorophenyl)-5-oxopentanoic acid (5.04 g, 22.24 mmol) in a mixture of DCM (50 mL) and methanol (16.6 mL) at 0°C. The mixture was stirred at 0 °C for 1 h, then concentrated. The residue was purified by an 80 g silica gel flash column with 0-50% EtOAc in hexane as eluting solvent to provide ***Int-64a.*** LCMS anal. calcd. for C₁₂H₁₃ClO₃: 240.1; Found: 241.2 (M+H)⁺

### Step B - Synthesis of Compound Int-64b

To a solution of ***Int-64a*** (4.64 g, 19.28 mmol) in THF (20 mL) under N₂ was added potassium bis(trimethylsilyl)amide (19.33 mL, 19.33 mmol, 1M) at room temperature, followed by 3-bromoprop-1-ene (3.50 g, 28.9 mmol). The reaction mixture was stirred at room temperature for 16 h. Upon completion, methanol (10 mL) was added. The mixture was concentrated and saturated aqueous ammonium chloride solution (50 mL) was added. The mixture was extracted with ethyl acetate (2x100 mL). The combined organic fractions were dried over Na₂SO₄, filtered and concentrated. The residue was purified on an 80g silica gel flash column with 0-100% EtOAc in hexane as eluting solvent to provide methyl 4-(2-chlorobenzoyl)hept-6-enoate. The material was then resolved on chiral AD-H column (50x 250mm, 5 um) with 10% MeOH as eluting solvent to provide ***Int-64b-peak1*** and ***Int-64b-peak2.*** LCMS anal. calcd. for C₁₅H₁₇ClO₃: 280.1; Found: 281.2 (M+H)⁺ .

### Step C - Synthesis of Compound Int-64c-peak 1

To a solution of ***Int-64b-peak1*** (993 mg, 3.54 mmol) in THF (35.4 mL) was added LAH (189 mg, 4.98 mmol) portionwise at 0 °C and stirred for 1 h. Upon completion, to the above reaction was sequentially added water (0.38 mL), 15% NaOH (0.19 mL), and water (0.95 mL). It was then diluted with EtOAc (10 mL) and anhydrous MgSO₄ (4 g) was added. The mixture was stirred at RT for 0.5 h, filtered and concentrated to provide ***Int-64c-peak 1.*** LCMS anal. calcd. for C₁₄H₁₉ClO₂: 254.1; Found: 277.3 (M+Na)⁺.

Following essentially the method employed to produce compound ***Int-64c-peak 1*** of example **11,** compound ***Int-64c-peak 2*** was prepared. LCMS anal. calcd. for C₁₄H₁₉ClO₂: 254.1; Found: 277.2 (M+Na)⁺.

### Step D - Synthesis of Compound Int-64d-peak1

Dess-Martin periodinane (3090 mg, 7.29 mmol) was added to a solution of ***Int-64c-peak1*** (928 mg, 3.64 mmol) in dichloromethane (36.40 mL) at 0°C. The mixture was stirred at RT for 3 h. Upon completion, the reaction was filtered and concentrated. The residue was purified by a 120 g silica gel flash column with 0-100% EtOAc in hexane as eluting solvent to provide ***Int-64d-peak1**.* LCMS anal. calcd. for C₁₄H₁₅ClO₂: 250.08; Found: 251.15 (M+H)⁺.

Following essentially the method employed to produce compound ***Int-64d-peak 1*** of example **11,** compound ***Int-64d-peak 2*** was prepared. LCMS anal. calcd. for C₁₄H₁₅ClO₂: 250.0; Found: 250.9 (M+H)⁺.

### Step E - Synthesis of Compound Int-64e-peak 1

In a sealed tube, ***Int-64d-peak1*** (710 mg, 2.83 mmol) was added to 1-amino-3-(benzyloxy)-N-methyl-4-oxo-1,4-dihydropyridine-2-carboxamide (774 mg, 2.83 mmol) in DMF (5.5 mL) and AcOH (0.550 mL). The resulting reaction was stirred at 120 °C for 1.5 h. Upon completion, the reaction was cooled down and concentrated. The residue was purified by an 80 g silica gel flash column with 0-10% MeOH in DCM as eluting solvent to provide ***Int-64e-peak 1*** (1902 mg, 2.158 mmol). LCMS anal. calcd. for C₂₈H₂₈ClN₃O₄: 505.18; Found: 506.26 (M+H)⁺.

Following essentially the method employed to produce compound ***Int-64e-peak 1*** of example 11, compound ***Int-64e-peak 2*** was prepared. LCMS anal. calcd. for C₂₈H₂₈ClN₃O₄: 505.2; Found: 506.3 (M+H)⁺.

### Step F - Synthesis of Compound Int-64f-peak 1

To a stirred solution of ***Int-64e-peak 1*** (199 mg, 0.393 mmol) in DCM (10 mL) was added DIEA (0.137 mL, 0.787 mmol), followed by SEMCl (0.070 mL, 0.393 mmol) at 0 °C. The resulting reaction was stirred at RT for 16 h. Upon completion, it was concentrated. The residue was purified by a 40 g silica gel flash column with 0-10% MeOH in DCM as eluting solvent to provide *I**nt-64f-peak 1.*** LCMS anal. calcd. for C₃₄H₄₂ClN₃O₅Si: 635.26; Found: 636.52 (M+H)⁺.

Following essentially the method employed to produce compound *I**nt-64f-peak 1*** of example **11,** compound ***Int-64f-peak 2*** was prepared. LCMS anal. calcd. for : C₃₄H₄₂ClN₃O₅Si: 635.3; Found: 636.4 (M+H)⁺.

### Step G - Synthesis of Compound Int-64g-peak 1

*Under* N₂, *I**nt-64f-peak 1*** (193 mg, 0.303 mmol) in a mixture of dichloromethane (4 mL) and methanol (2 mL) was cooled to -5°C then NaBH₄ (9.18 mg, 0.243 mmol) was added and the mixture was stirred for 1.5 h. Upon completion, the reaction was quenched with 0.4 mL 1N HCl and concentrated. The residue was purified by a 40 g silica gel flash column with 0-10% MeOH in DCM as eluting solvent to provide ***Int-64g-peak 1.*** LCMS anal. calcd. for C₃₄H₄₄ClN₃O₅Si: 637.27; Found: 638.54 (M+H)⁺.

Following essentially the method employed to produce compound ***Int-64g-peak 1*** of example **11,** compound ***Int-64g-peak 2*** was prepared. LCMS anal. calcd. for C₃₄H₄₄ClN₃O₅Si: 637.3; Found: 638.4 (M+H)⁺.

### Step H - Synthesis of Compound Int-64h-peak 1

A solution of ***Int-64g-peak 1*** (166 mg, 0.260 mmol) in 5.0 mL DCM was cooled to 0 °C and triethylamine (0.181 mL, 1.30 mmol) was added, followed by methanesulfonyl chloride (0.07 mL, 0.898 mmol). The reaction was stirred at RT for 1.5 h. Upon completion, the reaction was diluted with dichloromethane (20 mL) and washed with water. The organic layer was separated, dried over Na₂SO₄, filtered and concentrated to provide ***Int-64h-peak1.*** LCMS anal. calcd. for C₃₅H₄₆ClN₃O₇SSi: 715.25; Found: 716.36 (M+H)⁺.

Following essentially the method employed to produce compound ***Int-64h-peak 1*** of example 11, compound ***Int-64h-peak 2*** was prepared. LCMS anal. calcd. for C₃₅H₄₆ClN₃O₇SSi: 715.3; Found: 716.4 (M+H)⁺.

### Step I - Synthesis of Compound Int-64i-peak1a

Tetrabutylammonium fluoride (1.577 mL, 1.577 mmol, 1M) was added to a solution of ***Int-64h-peak 1*** (226 mg, 0.315 mmol) in 5 mL THF. The resulting reaction was heated at 60°C for 2 h. Additional tetrabutylammonium fluoride (1.577 mL, 1.577 mmol, 1M) was added and the mixture was heated for 1.5 h. Upon completion, the resulting reaction was concentrated. The residue was purified by a 24 g silica gel flash column with 0-10% MeOH in DCM as eluting solvent to provide racemic material. The material was resolved using chiral AS-H column (21 x 250mm, 5 µM) with 40% IPA as eluting solvent to provide ***Int-64i-peak1a*** and ***Int-64i -peak1b.*** LCMS anal. calcd. for C₂₈H₂₈ClN₃O₃: 489.18; Found: 490.35 (M+H)⁺.

Following essentially the method employed to produce compound ***Int-31i-peak 1*** of example **11,** compound ***Int-64i-peak 2a*** and ***Int-64i-peak2b*** were prepared. LCMS anal. calcd. for C₂₈H₂₈ClN₃O₃: 489.2; Found: 490.3 (M+H)⁺.

### Step J - Synthesis of Compound 64A

To a solution of ***Int-64i-peak 1a*** (20 mg, 0.041mmol) in 1 mL DMF was added lithium chloride (17.30 mg, 0.408 mmol) and it was stirred at 100 °C for 2.5 h. Upon completion, the reaction was concentrated. The residue was purified by preparative HPLC using reverse phase C18 column with 0-100% acetonitrile in water (0.05% TFA modifier) as eluting solvent to provide compound ***64A.*** LCMS anal. calcd. for C₂₁H₂₂ClN₃O₃: 399.1; Found: 400.3 (M+H)⁺. ¹H NMR (500 MHz, Methanol-*d*⁴) δ 7.83 (dd, *J* = 7.9, 1.6 Hz, 1H), 7.48 (td, *J* = 7.5, 1.4 Hz, 1H), 7.41 - 7.25 (m, 2H), 7.06 (d, *J* = 7.4 Hz, 1H), 5.95 (d, *J* = 7.5 Hz, 1H), 5.65 (dddd, *J* = 16.8, 10.2, 7.9, 6.5 Hz, 1H), 5.33 (t, *J* = 3.0 Hz, 1H), 5.02 - 4.95 (m, 1H), 4.95 - 4.87 (m, 1H), 4.79 (d, *J* = 10.6 Hz, 1H), 3.26 (s, 3H), 2.58 (dq, *J* = 15.4, 3.2 Hz, 1H), 2.44 - 2.22 (m, 2H), 2.07 - 1.93 (m, 1H), 1.89 - 1.72 (m, 2H), 1.48 (qd, *J* = 14.1, 3.4 Hz, 1H).

Following essentially the method employed to produce compound ***64A*** of example **11,** compound ***64B*** was prepared. LCMS anal. calcd. for C₂₁H₂₂ClN₃O₃: 399.13; Found: 400.34 (M+H)⁺. ¹H NMR (500 MHz, Methanol-*d*⁴) δ 7.84 (dd, *J* = 7.8, 1.6 Hz, 1H), 7.49 (td, *J* = 7.6, 1.4 Hz, 1H), 7.39 - 7.25 (m, 2H), 7.10 (d, *J* = 7.4 Hz, 1H), 6.02 (dd, *J* = 7.4, 1.3 Hz, 1H), 5.65 (dddd, *J* = 16.8, 10.2, 7.9, 6.5 Hz, 1H), 5.35 (t, *J* = 3.1 Hz, 1H), 4.98 (ddd, *J* = 10.1, 2.1, 1.0 Hz, 1H), 4.92 (dd, *J* = 17.0, 1.8 Hz, 1H), 4.79 (d, *J* = 10.6 Hz, 1H), 3.27 (s, 3H), 2.59 (dq, *J* = 15.6, 3.2 Hz, 1H), 2.33 (dddt, *J* = 48.0, 15.4, 14.0, 4.0 Hz, 2H), 2.07 - 1.93 (m, 1H), 1.89 - 1.73 (m, 2H), 1.48 (tdd, *J* = 14.0, 11.9, 3.5 Hz, 1H).

Following essentially the method employed to produce compound ***64A*** of example **11,** compound ***64C*** was prepared. LCMS anal. calcd. for C₂₁H₂₂ClN₃O₃: 399.1; Found: 400.2 (M+H)⁺.¹H NMR (500 MHz, Methanol-*d*⁴) *δ* 7.84 (dd, *J* = 7.9, 1.6 Hz, 1H), 7.49 (td, *J* = 7.5, 1.4 Hz, 1H), 7.35 (ddd, *J* = 8.8, 7.2, 1.6 Hz, 1H), 7.29 (dd, *J* = 8.1, 1.4 Hz, 1H), 7.14 (dd, *J* = 7.4, 1.0 Hz, 1H), 6.08 (dd, *J* = 7.4, 1.6 Hz, 1H), 5.65 (dddd, *J* = 16.8, 10.2, 7.9, 6.5 Hz, 1H), 5.36 (t, *J* = 3.1 Hz, 1H), 4.98 (ddt, *J* = 10.1, 2.0, 1.0 Hz, 1H), 4.92 (dd, *J* = 16.8, 1.7 Hz, 1H), 4.80 (d, *J* = 10.6 Hz, 1H), 3.27 (s, 3H), 2.59 (dq, *J* = 15.6, 3.2 Hz, 1H), 2.38 (dtt, *J* = 10.5, 8.1, 4.1 Hz, 1H), 2.29 (ddt, *J* = 17.3, 15.5, 3.7 Hz, 1H), 2.08 - 1.93 (m, 1H), 1.89 - 1.79 (m, 1H), 1.84 - 1.73 (m, 1H), 1.49 (tdd, *J* = 14.1, 11.9, 3.5 Hz, 1H). ).

Following essentially the method employed to produce compound ***64C*** of example **11,** Compound ***64D*** was prepared. LCMS anal. calcd. for C₂₁H₂₂ClN₃O₃: 399.1; Found: 400.2 (M+H)⁺. ¹H NMR (500 MHz, Methanol-*d*₄) δ 7.84 (dd, *J* = 7.9, 1.6 Hz, 1H), 7.49 (td, *J* = 7.6, 1.4 Hz, 1H), 7.35 (ddd, *J* = 8.8, 7.2, 1.6 Hz, 1H), 7.29 (dd, *J* = 8.2, 1.3 Hz, 1H), 7.15 (d, *J* = 7.4 Hz, 1H), 6.09 (d, *J* = 7.4 Hz, 1H), 5.65 (dddd, *J* = 16.8, 10.2, 7.9, 6.5 Hz, 1H), 5.36 (t, *J* = 3.1 Hz, 1H), 5.02-4.94 (m, 1H), 4.99-4.87 (m, 1H), 4.80 (d, *J* = 10.6 Hz, 1H), 3.27 (s, 3H), 2.60 (dq, *J* = 15.5, 3.2 Hz, 1H), 2.39 (dtt, *J* = 10.5, 8.1, 4.1 Hz, 1H), 2.29 (ddt, *J* = 15.5, 14.0, 3.6 Hz, 1H), 2.08- 1.93 (m, 1H), 1.89-1.79 (m, 1H), 1.84-1.73 (m, 1H), 1.49 (tdd, *J* = 14.1, 11.9, 3.5 Hz, 1H).

### Example 12

### Preparation of Compounds 65A and 65B

### 1-(2-chlorophenyl)-7-hydroxy-5-methyl-2-propyl-1,2,3,4,4a,5-hexahydrodipyrido[1,2-b:2',1'-f][1,2,4]triazine-6,8-dione

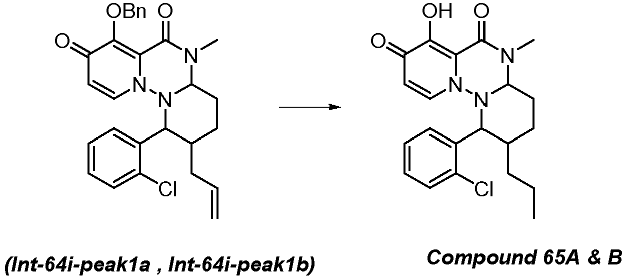

### Step A - Synthesis of Compound 65A

To a solution of ***Int 64i-peak1a*** (33 mg, 0.067 mmol) in 5 mL ethyl acetate was added Pd on C (7.17 mg, 0.067 mmol). The mixture was degassed, flushed with H₂ and stirred under a balloon of H₂ for 5 h. The reaction was filtered and concentrated. The residue was purified by preparative reverse phase HPLC using C18 column with acetonitrile in water (with 0.05% TFA modifier) as eluting solvent to provide ***Compound 65A.*** LCMS anal. calcd. for C₂₁H₂₄ClN₃O₃: 401.2; Found: 402.3 (M+H)⁺. ¹H NMR (500 MHz, Methanol-*d*⁴) *δ* 7.81 (dd, *J* = 7.9, 1.6 Hz, 1H), 7.48 (td, *J* = 7.6, 1.4 Hz, 1H), 7.37 - 7.25 (m, 2H), 7.13 (dd, *J* = 7.4, 3.4 Hz, 1H), 6.09 - 6.02 (m, 1H), 5.35 (t, *J* = 3.1 Hz, 1H), 4.77 (d, *J* = 10.5 Hz, 1H), 3.28 (s, 3H), 2.60 (dq, *J* = 15.5, 3.3 Hz, 1H), 2.35-2.22 (m, 2H), 2.08 (dq, *J* = 14.0, 3.8 Hz, 1H), 1.50-1.29 (m, 2H), 1.23-1.09 (m, 1H), 1.05 (dtd, *J* = 14.4, 9.6, 4.7 Hz, 1H), 0.95 (dddd, *J* = 13.6, 10.0, 6.2, 3.3 Hz, 1H), 0.76 (t, *J* = 7.3 Hz, 3H).

Following essentially the method employed to produce compound ***65A*** of example **12,** compound ***65B*** was prepared from ***Int-64i-peak1b.*** LCMS anal. calcd. for C₂₁H₂₄ClN₃O₃: 401.2; Found: 402.3 (M+H)⁺. ¹H NMR (500 MHz, Methanol-*d*⁴) *δ* 7.82 (dd, *J* = 7.9, 1.6 Hz, 1H), 7.48 (td, *J* = 7.5, 1.4 Hz, 1H), 7.34 (ddd, *J* = 8.7, 7.2, 1.6 Hz, 1H), 7.28 (dd, *J* = 8.1, 1.4 Hz, 1H), 7.15 (d, *J* = 7.4 Hz, 1H), 6.09 (d, *J* = 7.4 Hz, 1H), 5.36 (t, *J* = 3.1 Hz, 1H), 4.77 (d, *J* = 10.5 Hz, 1H), 3.28 (s, 3H), 2.60 (dq, *J* = 15.5, 3.3 Hz, 1H), 2.35-2.23 (m, 2H), 2.08 (dq, *J* = 13.9, 3.7 Hz, 1H), 1.51-1.29 (m, 2H), 1.16 (dddd, *J* = 13.2, 10.0, 7.4, 6.2 Hz, 1H), 1.05 (dtd, *J* = 14.4, 9.6, 4.7 Hz, 1H), 0.95 (dddd, *J* = 13.7, 10.0, 6.2, 3.3 Hz, 1H), 0.76 (t, *J* = 7.3 Hz, 3H).

### Example 13

### Preparation of Compounds 66A and 66B

### 1-(2-chlorophenyl)-7-hydroxy-2-(2-hydroxyethyl)-5-methyl-1,2,3,4,4a,5-hexahydrodipyrido[1,2-b:2',1'-f][1,2,4]triazine-6,8-dione

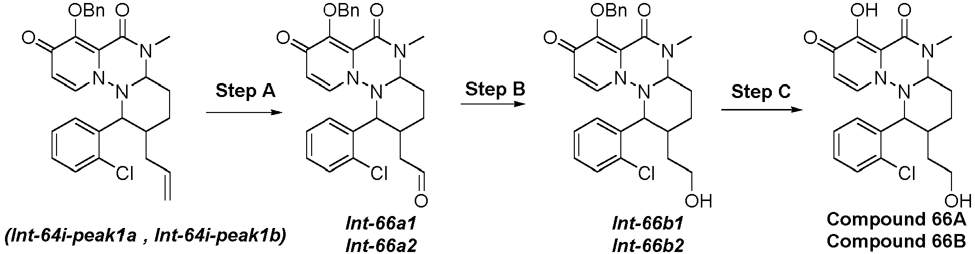

### Step A - Synthesis of Compound Int-66a1

To a solution of ***Int 64i-peak1a*** (33.7 mg, 0.069 mmol) in mixture of acetone (0.5 mL) and water (0.5 mL) under N₂ at 0°C was added sodium periodate (58.8 mg, 0.275 mmol), followed by potassium osmate dihydrate (1.014 mg, 2.75 µmol). The resulting mixture was stirred at RT for 2 h. Upon completion, the reaction was filtered and concentrated. The residue was taken up with ethyl acetate. The organic layer was washed with sodium thiosulfate solution, dried over sodium sulfate, filtered and concentrated to provide ***Int-66a1.*** LCMS anal. calcd. for C₂₇H₂₆ClN₃O₄: 491.2; Found: 492.2 (M+H)⁺ .

Following essentially the method employed to produce compound ***Int-66a1*** of example ***13, Int-66a2*** was prepared. LCMS anal. calcd. for C₂₇H₂₆ClN₃O₄: 491.2; Found: 492.1 (M+H)⁺.

### Step B - Synthesis of Compound Int-66b1

To a solution of ***Int-66a1*** (30 mg, 0.061 mmol) in a mixture of DCM (4 mL) and MeOH (2 mL) was added sodium borohydride (2.307 mg, 0.061 mmol) at 0 °C and stirred for 1.5 h. Upon completion, the reaction was quenched with 1N HCl (0.06 mL) and concentrated. The residue was purified by silica gel flash chromatography with 0-10% MeOH in DCM as eluting solvent to provide ***Int-66b1.*** LCMS anal. calcd. for C₂₇H₂₈ClN₃O₄: 493.2; Found: 494.4 (M+H)⁺ .

Following essentially the method employed to produce compound ***Int-66b1*** of example ***13, Int-66b2*** was prepared. LCMS anal. calcd. for C₂₇H₂₈ClN₃O₄: 493.2; Found: 494.1 (M+H)⁺.

### Step C - Synthesis of Compound 66A

Lithium chloride (45.3 mg, 1.06 mmol) was added to a solution of ***Int-66b1*** (26.4 mg, 0.053 mmol) in 2 mL DMF. The reaction was stirred at 100 °C for 4.5 h. Upon completion, the mixture was cooled and purified by preparative reverse phase HPLC on C18 column with acetonitrile in water (with 0.05% TFA modifier) as eluting solvent to provide compound ***66A**.* LCMS anal. calcd. for C₂₀H₂₂ClN₃O₄: 403.1; Found 404.2: (M+H)⁺. ¹H NMR (500 MHz, Methanol-*d*⁴) *δ* 7.82 (t, *J* = 7.9 Hz, 1H), 7.48 (t, *J* = 7.5 Hz, 1H), 7.41-7.25 (m, 2H), 7.12 (dd, *J* = 9.5, 7.5 Hz, 1H), 6.02 (dd, *J* = 20.0, 7.4 Hz, 1H), 5.36 (d, *J* = 2.7 Hz, 1H), 4.82 (dd, *J* = 14.9, 10.7 Hz, 1H), 4.34-4.24 (m, 1H), 3.47 (t, *J* = 6.5 Hz, 1H), 3.28 (s, 3H), 2.61 (ddd, *J* = 15.4, 7.0, 3.3 Hz, 1H), 2.44 (dt, *J* = 10.5, 5.3 Hz, 1H), 2.36-2.22 (m, 1H), 2.12 (dq, *J* = 10.1, 3.4 Hz, 1H), 1.68-1.38 (m, 2H), 1.25 (q, *J* = 6.6 Hz, 1H).

Following essentially the method employed to produce compound ***66A*** of example **13,** compound ***66B*** was prepared. LCMS anal. calcd. for C₂₀H₂₂ClN₃O₄: 403.1; Found: 404.2 (M+H)⁺. ¹H NMR (500 MHz, Methanol-*d*₄) δ 7.85-7.78 (m, 1H), 7.52-7.42 (m, 1H), 7.42-7.26 (m, 2H), 7.13 (dd, *J* = 10.0, 7.4 Hz, 1H), 6.08-5.99 (m, 1H), 5.36 (q, *J* = 2.8 Hz, 1H), 4.82 (dd, *J* = 14.8, 10.6 Hz, 1H), 4.29 (dd, *J* = 7.5, 5.5 Hz, 1H), 3.50 - 3.42 (m, 1H), 3.28 (s, 3H), 2.61 (ddq, *J* = 13.7, 6.4, 3.2 Hz, 1H), 2.44 (qt, *J* = 9.9, 5.1 Hz, 1H), 2.36-2.24 (m, 1H), 2.12 (ddq, *J* = 13.8, 7.1, 3.7 Hz, 1H), 1.62-1.39 (m, 2H), 1.33-1.21 (m, 1H).

### Example 14

### Preparation of Compound 67

### 1-(2-chlorophenyl)-7-hydroxy-2-(2-methoxyethyl)-5-methyl-1,2,3,4,4a,5-hexahydrodipyrido[1,2-b:2',1'-f][1,2,4]triazine-6,8-dione

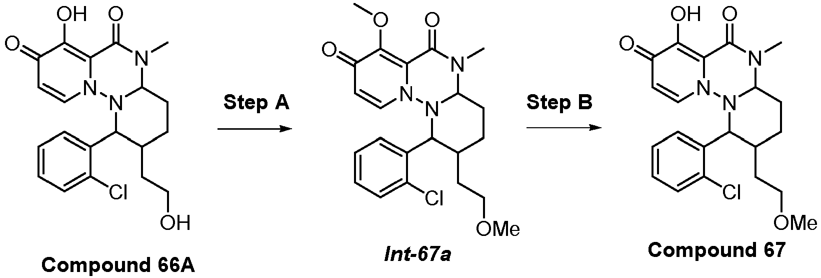

### Step A - Synthesis of Compound Int-67a

To a solution of compound **66A** (3.6 mg, 6.95 µmol) in DMF (1mL) under N₂ was added sodium hydride (1.668 mg, 0.042 mmol), followed by iodomethane (1.912 µL, 0.03 mmol) and it was stirred at RT for 16 h. Upon completion, the resulting reaction was filtered and purified by preparative reverse phase HPLC on C18 column with 0-100% acetonitrile in water (with 0.05% TFA modifier) as eluting solvent to provide ***Int-67a.*** LCMS anal. calcd. for C₂₂H₂₆ClN₃O₄:431.2; Found: 432.2 (M+H)⁺.

### Step B - Synthesis of Compound 67

Lithium chloride (4.658 mg, 0.110 mmol) was added to solution of ***Int-67a*** (2 mg, 0.0037mmol) in 2 mL DMF and it was heated to 100°C for 2.5 h. Upon completion, the mixture was concentrated. The residue was purified by preparative reverse phase HPLC on C18 column with 0-100% acetonitrile in water (with 0.05% TFA modifier) as eluting solvent to provide **Compound 67.** LCMS anal. calcd. for C₂₁H₂₄ClN₃O₄: 417.2; Found: 418.2 (M+H)⁺. ¹H NMR (500 MHz, Methanol-*d*⁴) *δ* 7.80 (dd, *J* = 7.8, 1.6 Hz, 1H), 7.46 (td, *J* = 7.4, 1.3 Hz, 1H), 7.32 (td, *J* = *7.7,* 7.2, 1.6 Hz, 1H), 7.28 (dd, *J* = 8.1, 1.4 Hz, 1H), 7.02 (d, *J* = 7.5 Hz, 1H), 5.87 (d, *J* = 7.5 Hz, 1H), 5.32 (t, *J* = 3.1 Hz, 1H), 4.78 (d, *J* = 10.7 Hz, 1H), 3.28-3.18 (m, 4H), 2.58 (dd, *J* = 15.4, 3.2 Hz, 1H), 2.40 (d, *J* = 11.2 Hz, 1H), 2.33-2.21 (m, 1H), 2.08 (dd, *J* = 13.8, 3.8 Hz, 1H), 1.54-1.42 (m, 1H), 1.31 (s, 2H), 1.31-1.20 (m, 1H).

### Example 15

### Preparation of Compound 68A, 68B, 68C, 68D

### 3,3-difluoro-7'-hydroxy-5'-methyl-1'-phenyl-3',4',4a',5'-tetrahydro-1'H-spiro[cyclopentane-1,2'-dipyrido[1,2-b:2',1'-f][1,2,4]triazine]-6',8'-dione

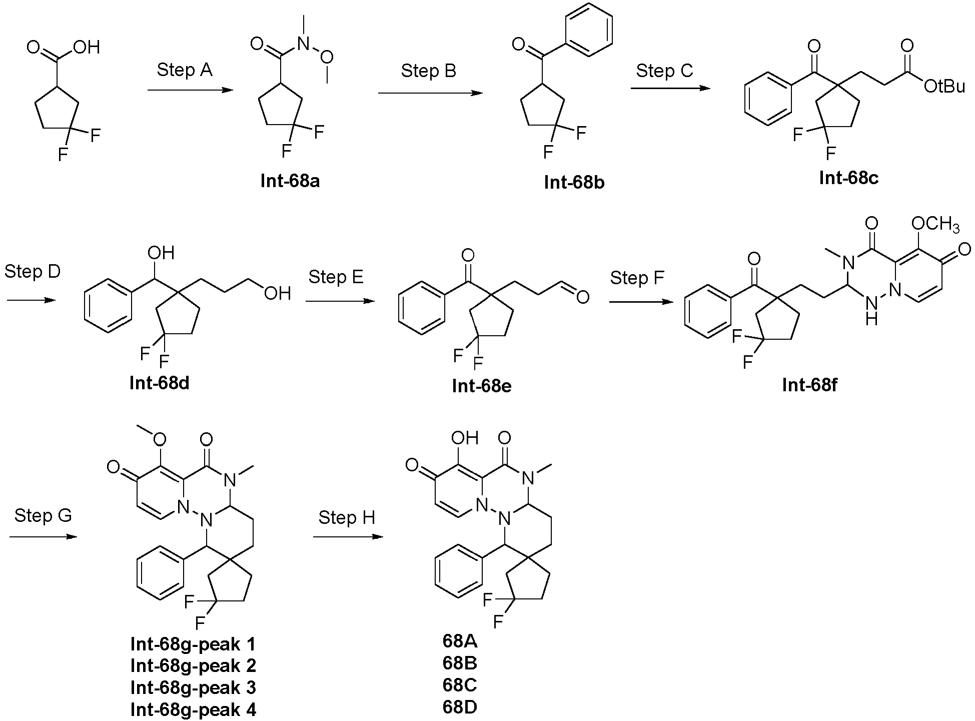

### Step A - Synthesis of Compound Int-68a

To a solution of 3,3-difluorocyclopentane-1-carboxylic acid (10 g, 66.6 mmol) in 250 mL DCM at 0 °C was added HATU (25.3 g, 66.6 mmol) and N-ethyl-N-isopropylpropan-2-amine (25.8 g, 200 mmol). The solution was stirred at RT overnight. Upon completion, the reaction mixture was washed with water (~100 mL), brine (100 mL), dried (Na₂SO₄), filtered and concentrated. The residue was purified by a 220 g silica gel column with 0-80% EtOAc in hexane as eluting solvent to provide ***Int-68a.*** LCMS anal. calcd. for C₈H₁₃F₂NO₂: 193.19; Found: 194.4 (M+H)⁺

### Step B - Synthesis of Compound Int-68b

Under N₂, ***Int-68a*** (10.5 g, 54.3 mmol) was dissolved in 200 mL THF, then phenylmagnesium bromide (70.7 mL,1 M, 70.7 mmol) was added at 0 °C. The reaction was stirred at RT for 24 hrs. Upon completion, sat'd NH₄Cl (~100 mL) was added and he organic layer was extracted with EtOAc (200 mL). The organic layer was washed with brine (50 mL), dried (NaSO4), filtered and concentrated. The residue was purified by a 220 g silica gel column, with 0-80% EtOAc/hexane as eluting solvent to provide ***Int-68b.*** LCMS anal. calcd. for C₁₂H₁₂F₂O: 210.22; Found: 211.1 (M+H)⁺

### Step C - Synthesis of Compound Int-68c

Under N₂, to a solution of ***Int-68b*** (2.508 g, 11.93 mmol) in THF (100 mL) at 0°C was added tert-butyl acrylate (1.77g, 13.50 mmol), followed by sodium t-butoxide (6.75 mL, 2 M, 13.50 mmol). The resulting reaction was stirred for 2hrs. Upon completion, the mixture was poured into aq. NH₄Cl solution and extracted with EtOAc (30 mL). The organic layer was washed with brine (15 mL), dried (Na₂SO₄), filtered and concentrated. The residue was purified by an 80 g silica gel column with 0-50% EtOAc in hexane as eluting solvent to provide ***Int-68c.*** LCMS anal. calcd. for C₁₉H₂₄F₂O3: 338.39; Found: 361.01(M+Na)⁺.

### Step D - Synthesis of Compound Int-68d

To a solution of ***Int-68c*** (1.705 g, 5.04 mmol) in THF (45 mL) at 0 °C was added LAH (0.269 g, 7.09 mmol). The reaction was stirred at room temperature for 90 mins. Upon completion, it was quenched with 0.54 mL H₂O, 0.27 mL 15% NaOH, 1.35 mL H₂O, then diluted with EtOAc (25 mL) and stirred with MgSO₄ (5 g) at RT for 1 h. It was filtered and concentrated to provide ***Int-68d.*** LCMS anal. calcd. for C₁₅H₂₀F₂O₂: 270.32; Found: 253.2 (M-OH)⁺

### Step E - Synthesis of Compound Int-68e

Dess-Martin periodinane (4971 mg, 11.72 mmol) was added to a solution of ***Int-68d*** (1440 mg, 5.33 mmol) in dichloromethane (50 mL) at 0°C. The mixture was stirred at RT for 2 h. Upon completion, the reaction was filtered and concentrated. The residue was purified by an 80 g silica gel flash column with 0-100% EtOAc in hexane as eluting solvent to provide ***Int-68e** .* LCMS anal. calcd. for C₁₅H₁₆F₂O₂: 266.11; Found: 267.2(M+H)⁺.

### Step F - Synthesis of Compound Int-68f

To a solution of ***Int-68e*** (528 mg, 1.98 mmol) in DMF (8.5 mL) was added acetic acid (0.9 mL) followed by 1-amino-3-methoxy-N-methyl-4-oxo-1,4-dihydropyridine-2-carboxamide (391 mg, 1.983 mmol) and heated at 120 °C for 2.5 h. Upon completion, the mixture was concentrated. The residue was purified by an 80 g silica gel column with 0-10% MeOH in CH₂Cl₂ to provide ***Int-1f.*** LCMS anal. calcd. for C₂₃H₂₅F₂N₃O₄: 445.18; Found: 446.2 (M+H)⁺.

### Step G - Synthesis of Compound Int-68g

To a solution of ***Int-68f*** (633 mg, 1.42 mmol) in trifluoroethanol (15 mL) was added triethyl silane (1.362 mL, 8.53 mmol) and triflic acid (0.757 mL, 8.53 mmol) then the mixture was stirred at 35°C for 16 h. The reaction was cooled and neutralized with sat'd aqueous NaHCO₃ (16 mL). The mixture was extracted with DCM. The organic layer was dried over MgSO₄, filtered and concentrated. The residue was first purified by a 120 g silica gel column with 0-10% MeOH in CH₂Cl₂ then preparative HPLC using SunFire C18 OBD Prep Column (19x100 mm, 5 µm) with ACN in water (with 0.05% TFA modifier) to provide racemic ***Int-68g.*** The material was resolved using AS-H (21x250mm, 5 µm) chiral column with 70 mL/min of 25% EtOH (with 0.2% DIPA) as eluting solvent to provide ***Int-68g-peak1, Int-68g-peak2, Int-68g-peak3*** and ***Int-68g-peak4.*** LCMS anal. calcd. for C₂₃H₂₅F₂N₃O₃: 429.19; Found: 430.2 (M+H)⁺ .

### Step H - Synthesis of Compound 68A, 68B, 68C and 68D

Lithium chloride (47 mg, 1.1 mmol) was added to a stirred mixture of ***Int-68g-peak1** (4* mg, 9.31 µmol) in DMF (1 mL) at room temperature. The mixture was stirred at 100 °C for 5 hrs. Upon completion, the mixture was cooled down. The residue was purified by preparative HPLC on SunFire C18 OBD Prep Column (19x100 mm, 5 µm) with ACN in water (with 0.05% TFA modifier) as eluting solvent to provide compound ***68A.*** LCMS anal. calcd. for C₂₂H₂₃F₂N₃O₃: 415.17; Found: 416.17 (M+H)⁺. ¹H NMR (500 MHz, Methanol-*d*₄) δ 7.93 (d, *J* = 7.9 Hz, 1H), 7.77 (d, *J* = 7.4 Hz, 1H), 7.43 (t, *J* = 7.5 Hz, 1H), 7.35 (t, *J* = 7.4 Hz, 1H), 7.21 (t, *J* = 7.5 Hz, 1H), 6.93 (d, *J* = 7.6 Hz, 1H), 6.02 (d, *J* = 7.4 Hz, 1H), 5.37 (t, *J* = 2.8 Hz, 1H), 4.40 (s, 1H), 3.26 (s, 3H), 2.91 (ddd, *J* = 13.6, 9.0, 4.0 Hz, 1H), 2.50 - 2.34 (m, 2H), 2.09 (t, *J* = 13.0 Hz, 1H), 2.00 - 1.88 (m, 3H), 1.88 - 1.68 (m, 3H).

Following essentially the method employed to produce ***68A*** of example **15, *68B*** was prepared. LCMS anal. calcd. for C₂₂H₂₃F₂N₃O₃: 415.17; Found: 416.17 (M+H)⁺. ¹H NMR (500 MHz, Methanol-*d*₄) δ 7.95 - 7.79 (m, 2H), 7.41 (t, *J* = 7.6 Hz, 1H), 7.33 (t, *J* = 7.4 Hz, 1H), 7.21 (t, *J* = 7.4 Hz, 1H), 7.00 (d, *J* = 7.5 Hz, 1H), 6.06 (d, *J* = 7.4 Hz, 1H), 5.37 (t, *J* = 2.9 Hz, 1H), 4.40 (s, 1H), 3.26 (s, 3H), 3.22 - 2.97 (m, 1H), 2.51 (dq, *J* = 15.8, 3.1 Hz, 1H), 2.44 - 2.22 (m, 2H), 1.97 - 1.71 (m, 3H), 1.60 (t, *J* = 7.5 Hz, 2H), 1.11 (tdd, *J* = 21.0, 13.0, 8.6 Hz, 1H).

Following essentially the method employed to produce ***68A*** of example **15, *68C*** was prepared. LCMS anal. calcd. for C₂₂H₂₃F₂N₃O₃: 415.17; Found: 416.17 (M+H)⁺. ¹H NMR (500 MHz, Methanol-*d*₄) δ 7.93 (d, *J* = 7.7 Hz, 1H), 7.77 (d, *J* = 7.4 Hz, 1H), 7.43 (t, *J* = 7.6 Hz, 1H), 7.35 (t, *J* = 7.4 Hz, 1H), 7.21 (t, *J* = 7.4 Hz, 1H), 6.93 (d, *J* = 7.5 Hz, 1H), 6.01 (d, *J* = 7.4 Hz, 1H), 5.37 (t, *J* = 2.8 Hz, 1H), 4.40 (s, 1H), 3.25 (s, 3H), 2.91 (ddd, *J* = 13.6, 9.1, 4.0 Hz, 1H), 2.52 - 2.32 (m, 2H), 2.18 - 2.03 (m, 1H), 2.01 - 1.90 (m, 2H), 1.90 - 1.64 (m, 4H).

Following essentially the method employed to produce ***68A*** of example **15, *68D*** was prepared. LCMS anal. calcd. for C₂₂H₂₃F₂N₃O₃: 415.17; Found: 416.19 (M+H)⁺. ¹H NMR (500 MHz, Methanol-*d*₄) δ 7.86 (d, *J* = 7.4 Hz, 2H), 7.41 (t, *J* = 7.5 Hz, 1H), 7.34 (t, *J* = 7.4 Hz, 1H), 7.21 (t, *J* = 7.3 Hz, 1H), 7.00 (d, *J* = 7.5 Hz, 1H), 6.07 (d, *J* = 7.4 Hz, 1H), 5.37 (t, *J* = 2.8 Hz, 1H), 4.41 (s, 1H), 3.27 (s, 3H), 3.18- 3.06 (m, 1H), 2.51 (dq, *J* = 15.9, 3.1 Hz, 1H), 2.39 - 2.25 (m, 2H), 1.91-1.74 (m, 3H),1.60 (t, *J* = 7.6 Hz, 2H), 1.11 (tdd, *J* = 21.1, 13.3, 8.7 Hz, 1H).

The examples in **Table 4** were prepared using procedures similar to those described in **Example 15,** from the appropriate starting material.

**Table 4**

| **Example** | **Structure** | |
|---|---|---|
| | **Name** | |
| | **Precursor SFC Separation Condition** | |
| **69A (Peak1)** | 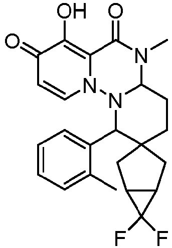 | 442.2 |
| **69B (Peak2)** | | |
| **69C (Peak3)** | | |
| | 6,6-difluoro-7'-hydroxy-5'-methyl-1'-(o-tolyl)-3',4',4a',5'-tetrahydro-1'H-spiro[bicyclo[3.1.0]hexane-3,2'-dipyrido[1,2-b:2',1'-f][1,2,4]triazine]-6',8'-dione | |
| | Precursor was resolved by SFC. SFC conditions: Daicel^{@} chiralpak AD-H(250 mm *21 mm, 5 µm), Condition:40% IPA with 0.2% DIPA, 50 mL/min | |
| **70A (peak 1)** | 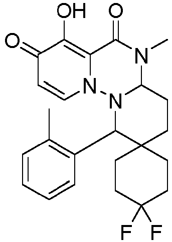 | 444.2 |
| **70B (peak 2)** | | |
| | 4,4-difluoro-7'-hydroxy-5'-methyl-1'-(o-tolyl)-3',4',4a',5'-tetrahydro-1'H-spiro[cyclohexane-1,2'dipyrido[1,2-b:2',1'-f][1,2,4]triazine]-6',8'-dione, | |
| | Precursor was resolved by SFC. SFC conditions: Daicel^{@} chiralpak AS-H column (250*21 mm, 10 µm); 40% EtOH; 50 mL/min | |
| **71A (peak 1)** | 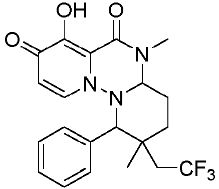 | 422.2 |
| **71B (peak 2)** | 7-hydroxy-2,5-dimethyl-1-phenyl-2-(2,2,2-trifluoroethyl)-1,2,3,4,4a,5-hexahydrodipyrido[1,2-b:2',1'-f][1,2,4]triazine-6,8-dione | |
| | Precursor was resolved by SFC. SFC conditions: Daicel^{@} chiralpak AS (250 mm * 30 mm, 5 µm), Condition: 0.1%NH₃.H₂O, 30% MeOH, 60 mL/min | |
| **72A (peak 1)** | 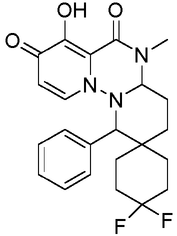 | 430.2 |
| **72B (peak 2)** | | |
| | 4,4-difluoro-7'-hydroxy-5'-methyl-1'-phenyl-3',4',4a',5'-tetrahydro-1'H-spiro[cyclohexane-1,2'-dipyrido[1,2-b:2',1'-f][1,2,4]triazine]-6',8'-dione | |
| | Precursor was resolved by SFC. SFC conditions: Phenomenex-Cellulose-2 (250 mm * 30 mm, 5 µm), Condition: 0.1%NH₃.H₂O, 45% EtOH, 60 mL/min | |
| **73A (peak 1)** | 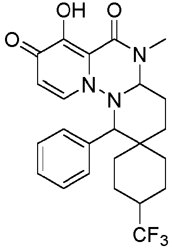 | 462.3 |
| **73B (peak 2)** | | |
| **73C (peak 3)** | | |
| **73D (peak 4)** | 7'-hydroxy-5'-methyl-1'-phenyl-4-(trifluoromethyl)-3',4',4a',5'-tetrahydro-1'H-spiro[cyclohexane-1,2'-dipyrido[1,2-b:2',1'-f][1,2,4]triazine]-6',8'-dione | |
| | Precursor was resolved by SFC. SFC conditions: Daicel^{@} chiralpak AD-H column (250*21 mm, 10 µm); 30% EtOH; 0.2% DIPA, 50 mL/min | |
| **74A (peak 1)** | 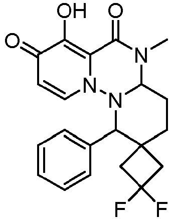 | 402.3 |
| **74B (peak 2)** | | |
| **74C (peak 3)** | | |
| **74D (peak 4)** | 3,3-difluoro-7'-hydroxy-5'-methyl-1'-phenyl-3',4',4a',5'-tetrahydro-1'H-spiro[cyclobutane-1,2'-dipyrido[1,2-b:2',1'-f][1,2,4]triazine]-6',8'-dione | |
| | Precursor was resolved by SFC. SFC conditions: Daicel^{@} chiralpak AS-H column (250*21 mm, 10 µm); 30% MeOH; 0.2% DIPA, 70 mL/min | |
| **75A (peak 1)** | 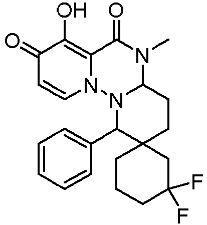 | 430.4 |
| **75B (peak 2)** | 3,3-difluoro-7'-hydroxy-5'-methyl-1'-phenyl-3',4',4a',5'-tetrahydro-1'H-spiro[cyclohexane-1,2'-dipyrido[1,2-b:2',1'-f][1,2,4]triazine]-6',8'-dione | |
| **75C (peak 3)** | | |
| **75D (peak 4)** | | |
| | Precursor was separated by SFC. SFC conditions: Daicel@chiralpak AD-H column (250*21 mm, 10 µm); 35% EtOH, 50g/min for peak1 and 2; Precursor was separated by SFC. SFC conditions: Daicel@chiralpak AS-H column (250*21 mm, 10 µm); 25% EtOH, 50g/min for peak 3 and 4 | |
| **76A (peak 1)** | 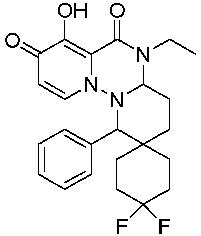 | 444.2 |
| **76B (peak 2)** | | |
| | 5'-ethyl-4,4-difluoro-7'-hydroxy-1'-phenyl-3',4',4a',5'-tetrahydro-1'H-spiro[cyclohexane-1,2'-dipyrido[1,2-b:2',1'-f][1,2,4]triazine]-6',8'-dione | |
| | Precursor was resolved by SFC. SFC conditions: Daicel@chiralpak AD-H column (250 mm * 30 mm, 5 µm), Condition: 0.1%NH₃.H₂O, 50% EtOH, 60 mL/min | |
| **77A (peak 1)** | 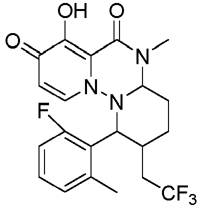 | 440.2 |
| **77B (peak 2)** | | |
| **77C (peak 3)** | | |
| **77D (peak 4)** | 1-(2-fluoro-6-methylphenyl)-7-hydroxy-5-methyl-2-(2,2,2-trifluoroethyl)-1,2,3,4,4a,5-hexahydrodipyrido[1,2-b:2',1'-f][1,2,4]triazine-6,8-dione | |
| | Precursor was resolved by SFC. SFC conditions: Daicel@chiralpak IG column (250 mm * 30 mm, 5 µm), Condition: 0.1%NH₃.H₂O, 20% EtOH, 60 mL/min | |
| **78A (peak 1)** | 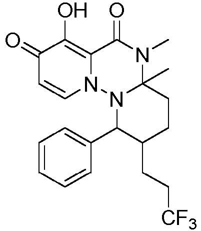 | 436.2 |
| **78B (peak 2)** | 7-hydroxy-4a,5-dimethyl-1-phenyl-2-(3,3,3-trifluoropropyl)-1,2,3,4,4a,5-hexahydrodipyrido[1,2-b:2',1'-f][1,2,4]triazine-6,8-dione | |
| | Precursor was resolved by SFC. SFC conditions: Daicel@chiralpak AD column (250 mm * 30 mm, 5 µm), Condition: 0.1%NH₃.H₂O, 25% EtOH, 60 mL/min | |
| **79A (peak 1)** | 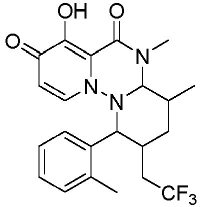 | 436.3 |
| **79B (peak 2)** | 7-hydroxy-4,5-dimethyl-1-(o-tolyl)-2-(2,2,2-trifluoroethyl)-1,2,3,4,4a,5-hexahydrodipyrido[1,2-b:2',1'-f][1,2,4]triazine-6,8-dione | |
| | Precursor was separated by SFC. SFC conditions: Daicel@chiralpak AS-H column (250*21 mm, 10 µm); 30% EtOH, 0.2% DIPA, 60 mL/min | |
| **80A (peak 1)** | 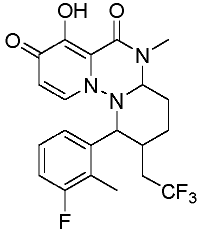 | 440.1 |
| **80B (peak 2)** | | |
| | 1-(3-fluoro-2-methylphenyl)-7-hydroxy-5-methyl-2-(2,2,2-trifluoroethyl)-1,2,3,4,4a,5-hexahydrodipyrido[1,2-b:2',1'-f][1,2,4]triazine-6,8-dione | |
| | Precursor was resolved by SFC. SFC conditions: Daicel@ chiralpak AS-H column (250*21 mm, 5 µm); 20% EtOH; 70 mL/min | |
| **81A (peak 1)** | 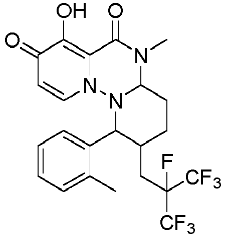 | 522.1 |
| **81B (peak 2)** | 7-hydroxy-5-methyl-2-(2,3,3,3-tetrafluoro -2-(trifluoromethyl)propyl)-1-(o-tolyl)-1,2, 3,4,4a,5-hexahydrodipyrido[1,2-b:2',1'-f] [1,2,4]triazine-6,8-dione | |
| | Precursor was resolved by SFC. SFC conditions: Daicel@ chiralcel OD-H column (250*21 mm, 5 µm); 25%EtOH; 50 mL/min | |
| **82A (peak 1)** | 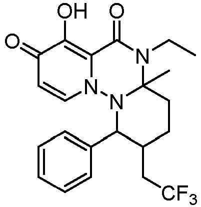 | 436.1 |
| **82B (peak 2)** | 5-ethyl-7-hydroxy-4a-methyl-1-phenyl-2-(2,2,2-trifluoroethyl)-1,2,3,4,4a,5-hexahydrodipyrido[1,2-b:2',1'-f][1,2,4]triazine-6,8-dione | |
| | Precursor was resolved by SFC. SFC conditions: Daicel@chiralpak AD column (250 mm * 30 mm, 10 µm), Condition: 0.1%NH₃.H₂O, 45% EtOH, 60 mL/min | |
| **83A (peak 1)** | 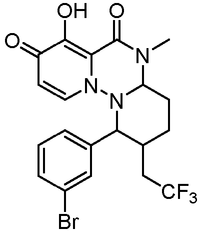 | 488.2, 489.3 |
| **83B (peak 2)** | | |
| | 1-(3-bromophenyl)-7-hydroxy-5-methyl-2-(2,2,2-trifluoroethyl)-1,2,3,4,4a,5-hexahydrodipyrido[1,2-b:2',1'-f][1,2,4]triazine-6,8-dione | |
| | Precursor was resolved by SFC. SFC conditions: Daicel@chiralpak AS-H column (250 mm * 30 mm, 10 µm), 30% EtOH, 60 mL/min | |
| **84A (peak 1)** | 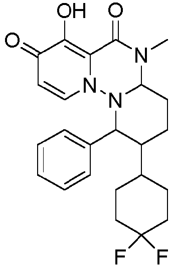 | 442.3 |
| **84B (peak 2)** | | |
| | 2-(4,4-difluorocyclohexyl)-7-hydroxy-5-methyl-1-phenyl-1,2,3,4,4a,5-hexahydrodipyrido[1,2-b:2',1'-f][1,2,4]triazine-6,8-dione | |
| | Precursor was resolved by SFC. SFC conditions: Daicel@chiralpak IC column (250 mm * 30 mm, 10 µm), Condition: 0.1%NH₃.H₂O, 50% EtOH, 60 mL/min | |
| **85A (peak 1)** | 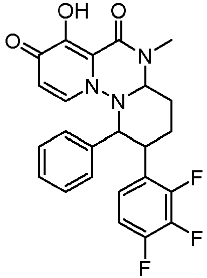 | 456.3 |
| **85B (peak 2)** | | |
| **85C (peak 3)** | | |
| **85D (peak 4)** | 7-hydroxy-5-methyl-1-phenyl-2-(2,3,4-trifluorophenyl)-1,2,3,4,4a,5-hexahydrodipyrido[1,2-b:2',1'-f][1,2,4]triazine-6,8-dione | |
| | Precursor was resolved by SFC. SFC conditions: Daicel@chiralpak AD column (250 mm * 30 mm, 10 µm), Condition: 0.1%NH₃.H₂O, 30% EtOH, 60 mL/min | |
| **86A (peak 1)** | 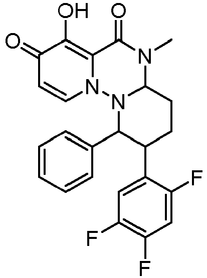 | 456.3 |
| **86B (peak 2)** | | |
| **86C (peak 3)** | | |
| **86D (peak 4)** | | |
| | 7-hydroxy-5-methyl-1-phenyl-2-(2,4,5-trifluorophenyl)-1,2,3,4,4a,5-hexahydrodipyrido[1,2-b:2',1'-f][1,2,4]triazine-6,8-dione | |
| | Precursor was resolved by SFC. SFC conditions: Daicel@chiralpak AD column (250 mm * 30 mm, 10 µm), Condition: 0.1%NH₃.H₂O, 45% EtOH, 60 mL/min | |
| **87A (peak 1)** | 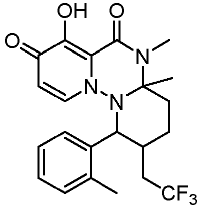 | 435.8 |
| **87B (peak 2)** | 7-hydroxy-4a,5-dimethyl-1-(o-tolyl)-2-(2,2,2-trifluoroethyl)-1,2,3,4,4a,5-hexahydrodipyrido[1,2-b:2',1'-f][1,2,4]triazine-6,8-dione | |
| | Precursor was resolved by SFC. SFC conditions: Daicel@chiralpak AD column (250 mm * 30 mm, 10 µm), Condition: 0.1%NH₃.H₂O, 20% EtOH, 60 mL/min | |
| **88A (peak 1)** | 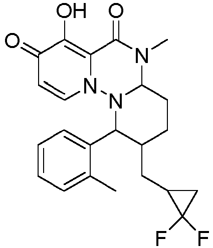 | 430.1 |
| **88B (peak 2)** | | |
| **88C (peak 3)** | | |
| **88D (peak 4)** | 2-((2,2-difluorocyclopropyl)methyl)-7-hydroxy-5-methyl-1-(o-tolyl)-1,2,3,4,4a,5-hexahydrodipyrido[1,2-b:2',1'-f][1,2,4]triazine-6,8-dione | |
| | Precursor was resolved by SFC. SFC conditions: Phenomenex-Cellulose-2 column (250 mm * 30 mm, 10 µm), Condition: 0.1%NH₃.H₂O, 50% EtOH, 60 mL/min | |
| **89A (peak 1)** | 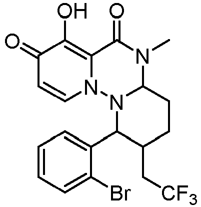 | 500.2, 502.3 |
| **89B (peak 2)** | | |
| | 1-(2-bromophenyl)-7-hydroxy-5-methyl-2-(2,2,2-trifluoroethyl)-1,2,3,4,4a,5-hexahydrodipyrido[1,2-b:2',1'-f][1,2,4]triazine-6,8-dione | |
| | Precursor was resolved by SFC. SFC conditions: Daicel@chiralpak AD-H column (250 mm * 30 mm, 10 µm), Condition: 30% EtOH, 60 mL/min | |
| **90A (peak 1)** | 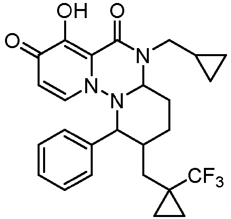 | 488.1 |
| **90B (peak 2)** | 5-(cyclopropylmethyl)-7-hydroxy-1-phenyl-2-((1-(trifluoromethyl)cyclopropyl)methyl)-1,2,3,4,4a,5-hexahydrodipyrido[1,2-b:2',1'-f][1,2,4]triazine-6,8-dione | |
| | Precursor was resolved by SFC. SFC conditions: Daicel@chiralpak IC column (250 mm * 30 mm, 10 µm), Condition: 0.1%NH₃.H₂O, 45% MeOH, 60 mL/min | |
| **91A (peak 1)** | 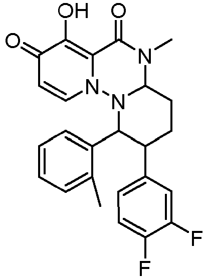 | 452.1 |
| **91B (peak 2)** | | |
| | 2-(3,4-difluorophenyl)-7-hydroxy-5-methyl-1-(o-tolyl)-1,2,3,4,4a,5-hexahydrodipyrido[1,2-b:2',1'-f][1,2,4]triazine-6,8-dione | |
| | Precursor was resolved by SFC. SFC conditions: Daicel@chiralpak AD column (250 mm * 30 mm, 10 µm), Condition: 0.1%NH₃.H₂O, 40% EtOH, 60 mL/min | |
| **92A (peak 1)** | 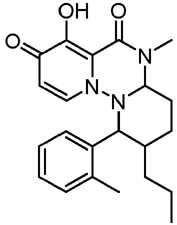 | 382.1 |
| **92B (peak 2)** | | |
| | 7-hydroxy-5-methyl-2-propyl-1-(o-tolyl)-1,2,3,4,4a, 5-hexahydrodipyrido[1,2-b:2',1'-f][1,2,4]triazine-6,8-dione | |
| | Precursor was resolved by SFC. SFC conditions: Daicel@chiralpak AS column (250 mm * 30 mm, 10 µm), Condition: 0.1%NH₃.H₂O, 40% EtOH, 60 mL/min | |
| **93A (peak 1)** | 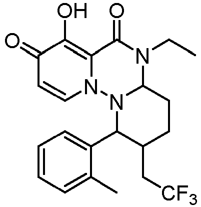 | 436.1 |
| **93B (peak 2)** | 5-ethyl-7-hydroxy-1-(o-tolyl)-2-(2,2,2-trifluoroethyl)-1,2,3,4,4a,5-hexahydrodipyrido[1,2-b:2',1'-f][1,2,4]triazine-6,8-dione | |
| | Precursor was resolved by SFC. SFC conditions: Daicel@chiralpak AS column (250 mm * 30 mm, 10 µm), Condition: 0.1%NH₃.H₂O, 50% EtOH, 60 mL/min | |
| **94A (peak 1)** | 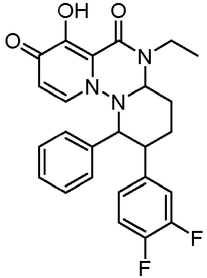 | 452.3 |
| **94B (peak 2)** | | |
| | 2-(3,4-difluorophenyl)-5-ethyl-7-hydroxy-1-phenyl-1,2,3,4,4a,5-hexahydrodipyrido[1,2-b:2',1'-f][1,2,4]triazine-6,8-dione | |
| | Precursor was separated by SFC. SFC conditions: Daicel@ chiralpak AD-H column (250*21 mm, 10 µm); 20% MeOH; 70 mL/min | |
| **95A (peak 1)** | 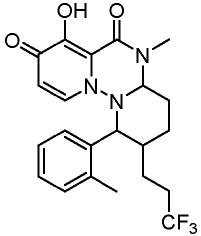 | 436.2 |
| **95B (peak 2)** | | |
| | 7-hydroxy-5-methyl-1-(o-tolyl)-2-(3,3,3-trifluoropropyl)-1,2,3,4,4a,5-hexahydrodipyrido[1,2-b:2',1'-f][1,2,4]triazine-6,8-dione | |
| | Precursors were separated by SFC. SFC conditions: Daicel@ chiralpak OD-H column (250*21 mm, 10 µm); 20% IPA, 0.2% DIPA; 70 mL/min | |
| **96A (peak 1)** | 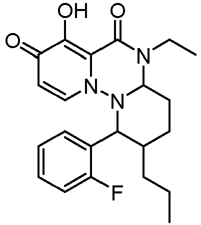 | 400.1 |
| **96B (peak 2)** | 5-ethyl-1-(2-fluorophenyl)-7-hydroxy-2-propyl-1,2,3,4,4a,5-hexahydrodipyrido[1,2-b:2',1'-f][1,2,4]triazine-6,8-dione | |
| | Precursor was resolved by SFC. SFC conditions: Phenomenex-Cellulose-2 column (250 mm * 30 mm, 10 µm), Condition: 0.1%NH₃.H₂O, 45% EtOH, 60 mL/min | |
| **97A (peak 1)** | 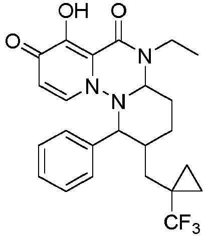 | 462.1 |
| **97B (peak 2)** | | |
| | 5-ethyl-7-hydroxy-1-phenyl-2-((1-(trifluoromethyl)cyclopropyl)methyl)-1,2,3,4,4a,5-hexahydrodipyrido[1,2-b:2',1'-f][1,2,4]triazine-6,8-dione | |
| | Precursor was resolved by SFC. SFC conditions: Daicel@ chiralpak IC column (250 mm * 30 mm, 10 µm), Condition: 0.1%NH₃.H₂O, 50% EtOH, 60 mL/min | |
| **98A (peak 1)** | 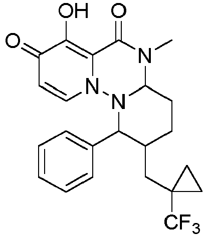 | 462.2 |
| **98B (peak 2)** | | |
| | 7-hydroxy-5-methyl-1-phenyl-2-((1-(trifluoromethyl)cyclopropyl)methyl)-1,2,3,4,4a,5-hexahydrodipyrido[1,2-b:2',1'-f][1,2,4]triazine-6,8-dione | |
| | Precursor was resolved by SFC. SFC conditions: Daicel@ chiralpak OD-H column (250 mm * 30 mm, 10 µm), Condition: 0.1%NH₃.H₂O, 25% EtOH, 60 mL/min | |
| **99A (peak 1)** | 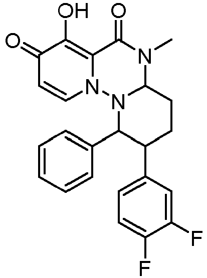 | 438.2 |
| **99B (peak 2)** | | |
| | 2-(2,3-difluorophenyl)-7-hydroxy-5-methyl-1-phenyl-1,2,3,4,4a,5-hexahydrodipyrido[ 1,2-b:2',1'-f][1,2,4]triazine-6,8-dione | |
| | Precursors were separated by SFC. SFC conditions: Daicel@ chiralpak AD-H column (250*21 mm, 10 µm); 35% MeOH, 70 mL/min | |
| **100A (peak 1)** | 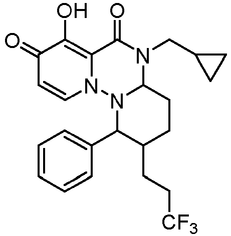 | 462.1 |
| **100B (peak 2)** | | |
| | 5-(cyclopropylmethyl)-7-hydroxy-1-phenyl-2-(3,3,3-trifluoropropyl)-1,2,3,4,4a,5-hexahydrodipyrido[1,2-b:2',1'-f][1,2,4]triazine-6,8-dione | |
| | Precursor was resolved by SFC. SFC conditions: Daicel@ chiralpak IC column (250 mm * 30 mm, 10 µm), Condition: 0.1%NH₃.H₂O, 40% EtOH, 60 mL/min | |
| **101A (peak 1)** | 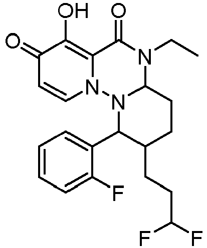 | 436.1 |
| **101B (peak 2)** | | |
| | 2-(3,3-difluoropropyl)-5-ethyl-1-(2-fluorophenyl)-7-hydroxy-1,2,3,4,4a,5-hexahydrodipyrido[1,2-b:2',1'-f][1,2,4]triazine-6,8-dione | |
| | Precursor was resolved by SFC. SFC conditions: Daicel@ chiralpak IC column (250 mm * 30 mm, 10 µm), Condition: 0.1%NH₃.H₂O, 55% MeOH, 60 mL/min | |
| **102A (peak 1)** | 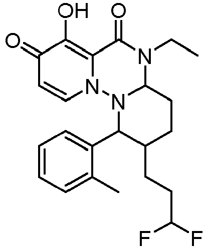 | 432.4 |
| **102B (peak 2)** | 2-(3,3-difluoropropyl)-5-ethyl-7-hydroxy-1-(o-tolyl)-1,2,3,4,4a,5-hexahydrodipyrido[1,2-b:2',1'-f][1,2,4]triazine-6,8-dione | |
| | Precursor was resolved by SFC. SFC conditions: Daicel@ chiralpak OD column (250 mm * 30 mm, 10 µm), Condition: 20% EtOH, 60 mL/min | |
| **103A (peak 1)** | 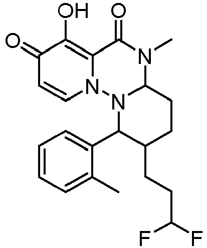 | 418.4 |
| **103B (peak 2)** | 2-(3,3-difluoropropyl)-7-hydroxy-5-methyl-1-(o-tolyl)-1,2,3,4,4a,5-hexahydrodipyrido[1,2-b:2',1'-f][1,2,4]triazine-6,8-dione | |
| | Precursor was resolved by SFC. SFC conditions: Daicel@ chiralpak AD column (250 mm * 30 mm, 10 µm), Condition: 20% EtOH, 60 mL/min | |
| **104A (peak 1)** | 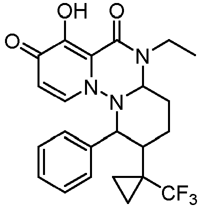 | 448.2 |
| **104B (peak 2)** | | |
| | 5-ethyl-7-hydroxy-1-phenyl-2-(1-(trifluoromethyl)cyclopropyl)-1,2,3,4,4a,5-hexahydrodipyrido[1,2-b:2',1'-f][1,2,4]triazine-6,8-dione | |
| | Precursor was resolved by SFC. SFC conditions: Daicel@ chiralpak OD-H column (250 mm * 30 mm, 10 µm), Condition: 0.1%NH₃.H₂O, 25% EtOH, 60 mL/min | |
| **105A (peak 1)** | 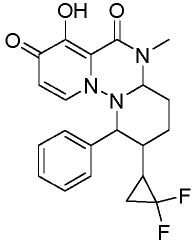 | 402.1 |
| **105B (peak 2)** | 2-(2,2-difluorocyclopropyl)-7-hydroxy-5-methyl-1-phenyl-1,2,3,4,4a,5-hexahydrodipyrido[1,2-b:2',1'-f][1,2,4]triazine-6,8-dione | |
| | Precursor was resolved by SFC. SFC conditions: Daicel@ chiralpak AD column (250 mm * 30 mm, 10 µm), Condition: 0.1%NH₃.H₂O, 25% EtOH, 60 mL/min | |
| **106A (peak 1)** | 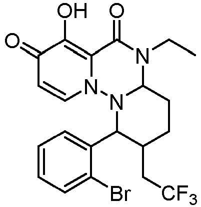 | 500.2, 502.3 |
| **106B (peak 2)** | 1-(2-bromophenyl)-5-ethyl-7-hydroxy-2-(2,2,2-trifluoroethyl)-1,2,3,4,4a,5-hexahydrodipyrido[1,2-b:2',1'-f][1,2,4]triazine-6,8-dione | |
| | Precursor was resolved by SFC. SFC conditions: Daicel@ chiralpak OD-H column (250 mm * 30 mm, 10 µm), Condition: 15% EtOH, 60 mL/min | |
| **107A (peak 1)** | 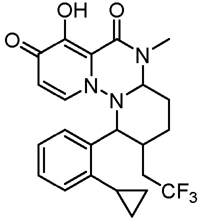 | 448.1 |
| **107B (peak 2)** | | |
| | 1-(2-cyclopropylphenyl)-7-hydroxy-5-methyl-2-(2,2,2-trifluoroethyl)-1,2,3,4,4a,5-hexahydrodipyrido[1,2-b:2',1'-f][1,2,4]triazine-6,8-dione | |
| | Precursor was resolved by SFC. SFC conditions: Daicel@ chiralpak AD column (250 mm * 30 mm, 10 µm), Condition: 0.1%NH₃.H₂O, 25% EtOH, 60 mL/min | |
| **108A (peak 1)** | 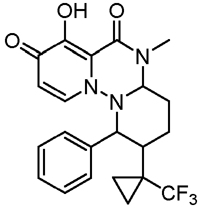 | 434.1 |
| **108B (peak 2)** | 7-hydroxy-5-methyl-1-phenyl-2-(1-(trifluoromethyl)cyclopropyl)-1,2,3,4,4a,5-hexahydrodipyrido[1,2-b:2',1'-f][1,2,4]triazine-6,8-dione | |
| | Precursor was resolved by SFC. SFC conditions: Daicel@ chiralpak OD-H column (250 mm * 30 mm, 10 µm), Condition: 0.1%NH₃.H₂O, 25% EtOH, 60 mL/min | |
| **109A (peak 1)** | 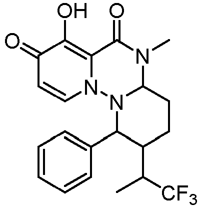 | 422.2 |
| **109B (peak 2)** | | |
| | 7-hydroxy-5-methyl-1-phenyl-2-(1,1,1-trifluoropropan-2-yl)-1,2,3,4,4a,5-hexahydrodipyrido[1,2-b:2',1'-f][1,2,4]triazine-6,8-dione | |
| **110A (peak 1)** | 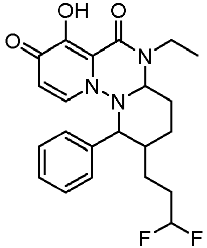 | 418.2 |
| **110B (peak 2)** | 2-(3,3-difluoropropyl)-5-ethyl-7-hydroxy-1-phenyl-1,2,3,4,4a,5-hexahydrodipyrido[1,2-b:2',1'-f][1,2,4]triazine-6,8-dione | |
| | Precursor was resolved by SFC. SFC conditions: Daicel@ chiralpak AD-H column (250 mm * 30 mm, 10 µm), Condition: 0.1%NH₃.H₂O, 40% EtOH, 60 mL/min | |
| **111A (peak 1)** | 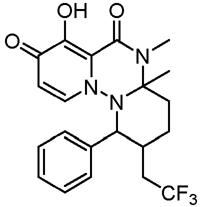 | 436.1 |
| **111B (peak 2)** | 7-hydroxy-4a,5-dimethyl-1-phenyl-2-(2,2,2-trifluoroethyl)-1,2,3,4,4a,5-hexahydrodipyrido[1,2-b:2',1'-f][1,2,4]triazine-6,8-dione | |
| | Precursor was resolved by SFC. SFC conditions: Daicel@ chiralpak AD-H column (250 mm * 30 mm, 10 µm), Condition: 0.1%NH₃.H₂O, 20% EtOH, 60 mL/min | |
| **112A (peak 1)** | 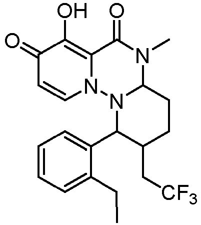 | 436.2 |
| **112B (peak 2)** | 1-(2-ethylphenyl)-7-hydroxy-5-methyl-2-(2,2,2-trifluoroethyl)-1,2,3,4,4a,5-hexahydrodipyrido[1,2-b:2',1'-f][1,2,4]triazine-6,8-dione | |
| | Precursors were separated by SFC. SFC conditions: Daicel@chiralcel OD-H column (250*21 mm, 10 µm); 25% MeOH, 50 mL/min | |
| **113A (peak 1)** | 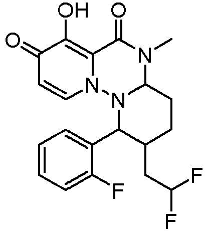 | 408.2 |
| **113B (peak 2)** | 2-(2,2-difluoroethyl)-1-(2-fluorophenyl)-7-hydroxy-5-methyl-1,2,3,4,4a,5-hexahydrodipyrido[1,2-b:2',1'-f][1,2,4]triazine-6,8-dione | |
| | Precursor was resolved by SFC. SFC conditions: Daicel@ chiralpak AS column (250 mm * 30 mm, 10 µm), Condition: 0.1%NH₃.H₂O, 30% EtOH, 60 mL/min | |
| **114A (peak 1)** | 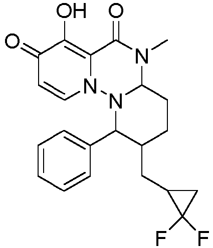 | 416.1 |
| **114B (peak 2)** | | |
| **114C (Peak 3)** | 2-((2,2-difluorocyclopropyl)methyl)-7-hydroxy-5-methyl-1-phenyl-1,2,3,4,4a,5-hexahydrodipyrido[1,2-b:2',1'-f][1,2,4]triazine-6,8-dione | |
| **114D (Peak 4)** | | |
| | Precursor was resolved by SFC. SFC conditions: Daicel@ chiralpak AS column (250 mm * 30 mm, 10 µm), Condition: 30% EtOH, 60 mL/min | |
| **115A (peak 1)** | 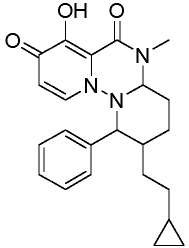 | 394.2 |
| **115B (peak 2)** | 2-(2-cyclopropylethyl)-7-hydroxy-5-methyl-1-phenyl-1,2,3,4,4a, 5-hexahydro dipyrido[1,2-b:2',1'-f][1,2,4]triazine-6,8-dione | |
| **115C (peak 3)** | | |
| **115D (peak 4)** | | |
| | Precursor was resolved by SFC. SFC conditions: Daicel@ chiralpak AS-H column (250*21 mm, 5 µm); 35% EtOH with 0.2% DIPA; 70 mL/min Daicel@ chiralpak AS-H column (250*21 mm, 5 µm); 50% EtOH with 0.2% DIPA; 50 mL/min | |
| **116A (peak 1)** | 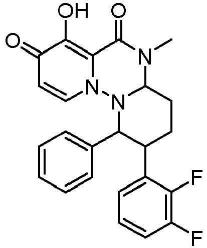 | 438.2 |
| **116B (peak 2)** | | |
| | 2-(2,3-difluorophenyl)-7-hydroxy-5-methyl-1-phenyl-1,2,3,4,4a,5-hexahydrodipyrido[1,2-b:2',1'-f][1,2,4]triazine-6,8-dione | |
| | Precursors were separated by SFC. SFC conditions: Daicel@ chiralpak AD-H column (250*21 mm, 10 µm); 35% MeOH, 70 mL/min | |
| **117A (peak 1)** | 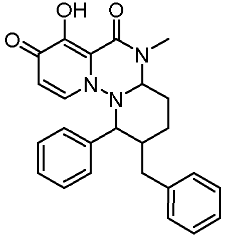 | 416.3 |
| **117B (peak 2)** | | |
| | 2-benzyl-7-hydroxy-5-methyl-1-phenyl-1,2,3,4,4a,5-hexahydrodipyrido[1,2-b:2',1'-f][1 ,2,4]triazine-6,8-dione | |
| | Precursors were separated by SFC. SFC conditions: Daicel@ chiralpak AS-H column (250*21 mm, 10 µm); 30% EtOH, 70 mL/min | |
| **118A (peak 1)** | 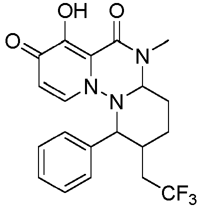 | 408.2 |
| **118B (peak 2)** | 7-hydroxy-5-methyl-1-phenyl-2-(2,2,2-trifluoroethyl)-1,2,3,4,4a,5-hexahydrodipyrido[1,2-b:2',1'-f][1,2,4]triazine-6,8-dione | |
| | Precursor was resolved by SFC. SFC conditions: Daicel@ chiralpak AS column (250*21 mm, 5 µm); 30% EtOH with 0.2% NH₃H₂O; 70 mL/min | |
| **119A (peak 1)** | 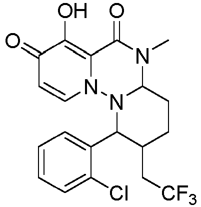 | 442.0 |
| **119B (peak 2)** | | |
| | 1-(2-chlorophenyl)-7-hydroxy-5-methyl-2-(2,2,2-trifluoroethyl)-1,2,3,4,4a,5-hexahydrodipyrido[1,2-b:2',1'-f][1,2,4]triazine-6,8-dione | |
| | Precursor was resolved by SFC. SFC conditions: Daicel@ chiralpak AD column (250*21 mm, 5 µm); 30% EtOH with 0.2% NH₃H₂O; 70 mL/min | |
| **120A (peak 1)** | 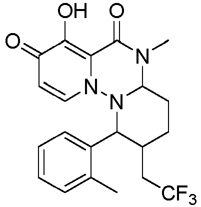 | 422.1 |
| **120B (peak 2)** | 7-hydroxy-5-methyl-1-(o-tolyl)-2-(2,2,2-trifluoroethyl)-1,2,3,4,4a,5-hexahydrodipyrido[1,2-b:2',1'-f][1,2,4]triazine-6,8-dione | |
| | Precursor was resolved by SFC. SFC conditions: Daicel@ chiralpak AD column (250*21 mm, 5 µm); 25% MeOH; 70 mL/min | |
| **121A (peak 1)** | 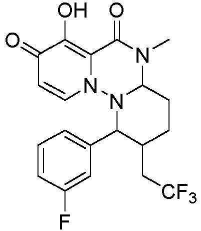 | 426.3 |
| **121B (peak 2)** | 1-(3-fluorophenyl)-7-hydroxy-5-methyl-2-(2,2,2-trifluoroethyl)-1,2,3,4,4a,5-hexahydrodipyrido[1,2-b:2',1'-f][1,2,4]triazine-6,8-dione | |
| | Precursors were separated by SFC. SFC conditions: Daicel@chiralpak AS-H column (250*21 mm, 10 µm); 25% EtOH; 50 mL/min | |
| **122A (peak 1)** | 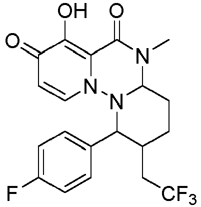 | 426.3 |
| **122B (peak 2)** | 1-(4-fluorophenyl)-7-hydroxy-5-methyl-2-(2,2,2-trifluoroethyl)-1,2,3,4,4a,5-hexahydrodipyrido[1,2-b:2',1'-f][1,2,4]triazine-6,8-dione | |
| | Precursor was resolved by SFC. SFC conditions: Daicel@ chiralpak AD-H(250 mm* 21 mm, 5 µm), Condition: 15%EtOH, 70 mL/min | |
| **123A (peak 1)** | 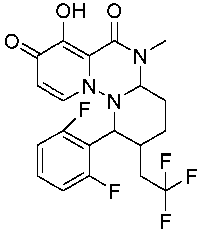 | 444.2 |
| **123B (peak 2)** | | |
| **123C (peak 3)** | 1-(2,6-difluorophenyl)-7-hydroxy-5-methyl-2-(2,2,2-trifluoroethyl)-1,2,3,4,4a,5-hexahydrodipyrido[1,2-b:2',1'-f][1,2,4]triazine-6,8-dione | |
| **123D (peak 4)** | | |
| | Precursor was resolved by SFC. SFC conditions: Daicel@ chiralpak AS-H column (250*21 mm, 5 µm); 25% EtOH with 0.2% DIPA; 70 mL/min | |
| **124A (peak 1)** | 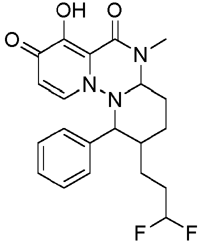 | 404.2 |
| **124B (peak 2)** | | |
| | 2-(3,3-difluoropropyl)-7-hydroxy-5-methyl-1-phenyl-1,2,3,4,4a,5-hexahydrodipyrido[1,2-b:2',1'-f][1,2,4]triazine-6,8-dione | |
| | Precursor was resolved by SFC. SFC conditions: Daicel@ chiralpak AD-H column (250*21 mm, 5 µm); 15% MeOH with 0.2% DIPA; 70 mL/min | |
| **125A (peak 1)** | 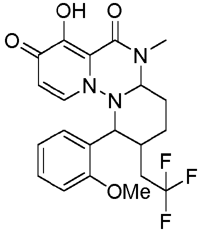 | 438.3 |
| **125B (peak 2)** | | |
| | 7-hydroxy-1-(2-methoxyphenyl)-5-methyl-2-(2,2,2-trifluoroethyl)-1,2,3,4,4a,5-hexahydrodipyrido[1,2-b:2',1'-f][1,2,4]triazine-6,8-dione | |
| | Precursor was resolved by SFC. SFC conditions: Daicel@ chiralpak AS-H column (250*21 mm, 5 µm); 25% MeOH; 70 mL/min | |
| **126A (peak 1)** | 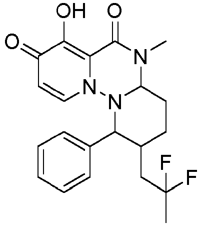 | 404.3 |
| **126B (peak 2)** | 2-(2,2-difluoropropyl)-7-hydroxy-5-methyl-1-phenyl-1,2,3,4,4a,5-hexahydrodipyrido[1,2-b:2',1'-f][1,2,4]triazine-6,8-dione | |
| | Precursor was resolved by SFC. SFC conditions: Daicel@ chiralpak AD-H column (250*21 mm, 5 µm); 20% MeOH, 0.1% DEA; 70 mL/min | |
| **127A (peak 1)** | 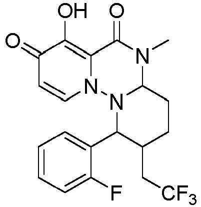 | 426.3 |
| **127B (peak 2)** | 1-(2-fluorophenyl)-7-hydroxy-5-methyl-2-(2,2,2-trifluoroethyl)-1,2,3,4,4a,5-hexahydrodipyrido[1,2-b:2',1'-f][1,2,4]triazine-6,8-dione | |
| | Precursor was resolved by SFC. SFC conditions: Daicel@ chiralpak IC column (250*21 mm, 5 µm); 50% MeOH with 0.1% NH₃H₂O; 70 mL/min | |
| **128A (peak 1)** | 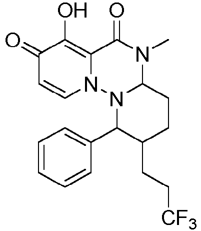 | 422.1 |
| **128B (peak 2)** | | |
| | 7-hydroxy-5-methyl-1-phenyl-2-(3,3,3-trifluoropropyl)-1,2,3,4,4a,5-hexahydrodipyrido[1,2-b:2',1'-f][1,2,4]triazine-6,8-dione | |
| | Precursor was resolved by SFC. SFC conditions: Daicel@ chiralpak AD column (250*21 mm, 5 µm); 25% EtOH with 0.1% NH₃H₂O; 70 mL/min | |
| **129A (peak 1)** | 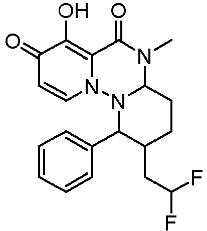 | 390.2 |
| **129B (peak 2)** | | |
| **129C (peak 3)** | | |
| **129D (peak 4)** | 2-(2,2-difluoroethyl)-7-hydroxy-5-methyl-1-phenyl-1,2,3,4,4a,5-hexahydrodipyrido[1,2-b:2',1'-f][1,2,4]triazine-6,8-dione | |
| | Precursor was resolved by SFC. SFC conditions: Daicel@ chiralpak OD column (250*21 mm, 5 µm); 25% EtOH with 0.1% NH₃H₂O; 70 mL/min | |

### Example 16

### Preparation of Compounds 130A and 130B

### 7-hydroxy-3-isobutyl-5-methyl-1-phenyl-1,2,3,4,4a,5-hexahydrodipyrido[1,2-b:2',1'-f][1,2,4]triazine-6,8-dione

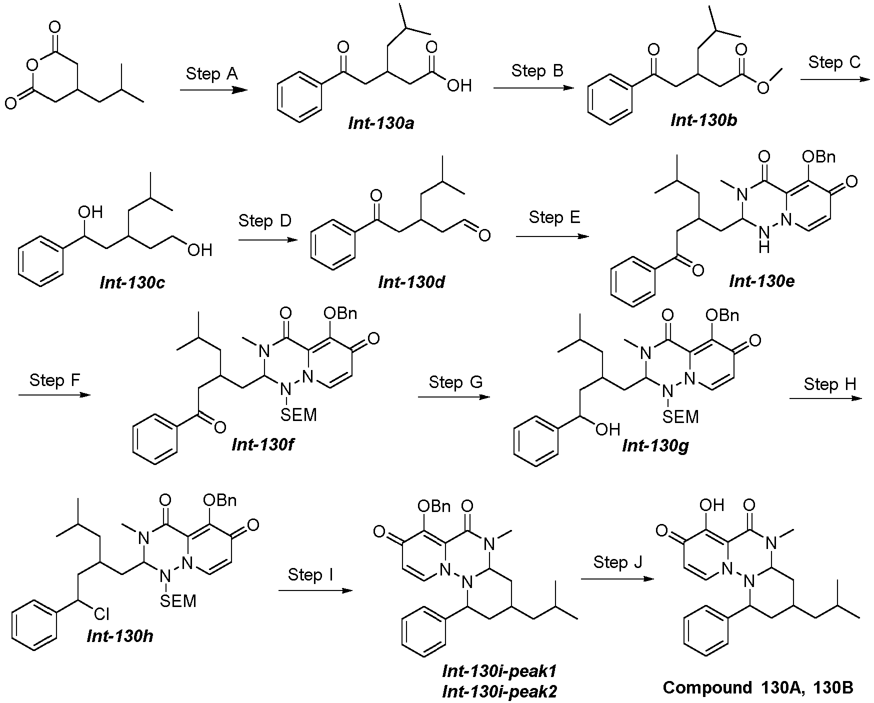

### Step A - Synthesis of Compound Int-130a

A solution of 4-isobutyldihydro-2h-pyran-2,6(3h)-dione (0.8 g, 4.70 mmol) in toluene (25 mL) was added to a mixture of (-)-sparteine (1.404 mL, 6.11 mmol) and phenylmagnesium chloride (3.06 mL, 6.11 mmol) in toluene (15 mL) under N₂ at -78 °C. The mixture was stirred for 7 h before it was quenched with saturated ammonium chloride solution (12 mL) and 2 N NaOH (47 mL). The mixture was extracted with 50 mL ether. The aqueous layer was acidified with 6 *N* HCl to pH 2. It was extracted with EtOAc (2x50 mL). The combined organic layers were washed with brine, dried over sodium sulfate, filtered and concentrated to provide ***Int-130a.*** LCMS anal. calcd. for C₁₅H₂₀O₃ 248.1; Found: 249.4 (M+H)⁺ .

### Step B - Synthesis of Compound Int-130b

TMS-Diazomethane (3.56 mL, 7.13 mmol) was added to ***Int-130a*** (885mg, 3.56mmol) in a mixture of DCM (9.0 mL) and MeOH (3.0 mL) at 0 °C. The resulting reaction was stirred for 1 hour. Upon completion, it was concentrated. The residue was purified by silica gel flash chromatography with 0-100% EtOAc in hexane as eluting solvent to provide 510 mg racemic material. The racemic compound was resolved on a chiral AD-H (21x250mm, 5 µm) column with 5% IPA (0.1% DIPA) as eluting solvent to provide ***Int-130b-peak1*** and ***Int-130b-peak2.*** LCMS anal. calcd. for C₁₆H₂₂O₃: 262.2; Found: 263.3 (M+H)⁺.

### Step C - Synthesis of Compound Int-130c

Lithium aluminum hydride (39 mg, 1.028 mmol) was added to a solution of ***Int-130b-peak1*** (181.4 mg, 0.691 mmol) in THF (10 mL) under N₂ at 0 °C. The mixture was stirred at this temperature for 1 h. Upon completion, water (0.08 mL), 15% NaOH solution (0.04 mL) and water (0.2 mL) were added sequentially. It was diluted with ethyl acetate (50 mL) and MgSO₄ (2 g) was added. The mixture was stirred at RT for 0.5 h. It was filtered and concentrated to provide ***Int-130c.*** LCMS anal. calcd. for C₁₅H₂₄O₂: 236.2; Found:260.2 (M+Na)⁺.

### Step D - Synthesis of Compound Int-130d

Dess-Martin periodinane (607 mg, 1.430mmol) was added to a solution of ***Int-130c*** (169 mg, 0.715 mmol) in DCM (10 mL) at 0 °C. The resulting reaction was stirred at RT for 1 h. Upon completion, the reaction was filtered and concentrated. The residue was purified by silica gel flash chromatography with 0-100% EtOAc in hexane as eluting solvent to provide ***Int-130d.*** LCMS anal. calcd. for C₁₅H₂₀O₂: 232.2; Found: 233.3 (M+H)⁺.

### Step E - Synthesis of Compound Int-130e

A solution of ***Int-130d*** (0.0536 g, 0.231 mmol) and 1-amino-3-(benzyloxy)-N-methyl-4-oxo-1,4-dihydropyridine-2-carboxamide (0.063 g, 0.231 mmol) in DMF (2.5 mL) with acetic acid (0.25 mL) was heated in a sealed tube at 120 °C for 2 h. Upon completion, the reaction mixture was concentrated. The residue was purified by silica gel flash chromatography with 0-10% MeOH in DCM as eluting solvent to provide ***Int-130e.*** LCMS anal. calcd. for C₂₉H₃₃N₃O₄: 487.3; Found:488.4 (M+H)⁺.

### Step F - Synthesis of Compound Int-130f

To a solution of ***Int-130e*** (41 mg, 0.084 mmol) in DCM (5.0 mL) was added DIEA (0.032 mL, 0.185 mmol), followed by SEMCl (0.016 mL, 0.092 mmol) at 0 °C. The reaction was stirred at RT for 1.5 h. Upon completion, the reaction was concentrated. The residue was purified by silica gel flash chromatography with 0-10% MeOH in DCM as eluting solvent to provide ***Int-130f.*** LCMS anal. calcd. for C₃₅H₄₇N₃O₅Si:617.3; Found: 618.5 (M+H)⁺.

### Step G - Synthesis of Compound Int-130g

***Int-130f*** (39.5mg, 0.064 mmol) in a mixture of DCM (2 mL) and MeOH (1 mL) was at 0 °C and NaBH₄ (4.84 mg, 0.128 mmol) was added. The resulting mixture was stirred at this temperature for 2 h. Upon completion, the reaction was concentrated. The residue was purified by silica gel flash chromatography with 0-10% MeOH in DCM as eluting solvent to provide ***Int-130g.*** LCMS anal. calcd. for C₃₅H₄₉N₃O₅Si: 619.3; Found: 620.3 (M+H)⁺.

### Step H - Synthesis of Compound Int-130h

***Int-130g*** (37.2 mg, 0.060 mmol) in DCM (3.0 mL) was cooled to 0°C, followed by adding triethylamine (0.042 mL, 0.300 mmol) and methanesulfonyl chloride (0.014 mL, 0.180 mmol). The reaction was stirred at RT for 1.5 h. Upon completion, the reaction was diluted with DCM (15 mL) and washed with water and brine, dried over Na₂SO₄, filtered and concentrated to provide ***Int-130h.*** LCMS anal. calcd. for C₃₅H₄₈ClN₃O₄Si: 437.3; Found: 638.3 (M+H)⁺.

### Step I - Synthesis of Compound Int-130i

Tetrabutylammonium fluoride (0.501 mL, 0.501 mmol) was added to a solution of ***Int-130h*** (64 mg, 0.100 mmol) in THF (5 mL) at RT. The resulting mixture was heated at 60 °C for 16 h. Upon completion, the reaction was concentrated. The residue was purified by silica gel flash chromatography with 0-10% MeOH in DCM as eluting solvent to provide ***Int-130i*** (20 mg, 0.057 mmol). ***Int-130i*** was resolved using chiral AD-H column (21 x 250mm, 5 µm) with 30% IPA (0.2% DIPA) as eluting solvent to provide ***Int-130i -peak1*** and ***Int-130i-peak2.*** LCMS anal. calcd. for C₂₉H₃₃N₃O₃: 471.2; Found: 472.3 (M+H)⁺.

### Step J - Synthesis of Compound 130A

To a solution of ***Int-130i-peak 1*** (3 mg, 0.0064 mmol) in DMF (1 mL) was added lithium chloride (8.08 mg, 0.19 mmol) and the mixture was stirred at 100 °C for 3 h. Upon completion, the reaction was concentrated. The residue was purified by preparative reverse phase HPLC on C18 column with ACN in water (0.05% TFA modifier) as eluting solvent to provide Compound ***130A.*** LCMS anal. calcd. for C₂₂H₂₇N₃O₃: 381.2; Found: 382.3 (M+H)⁺. ¹H NMR (500 MHz, Methanol-d⁴) *δ* 7.30 (s, b, 5H), 7.13 (d, *J =* 7.4 Hz, 1H), 5.86 (d, *J =* 7.4 Hz, 1H), 5.35 - 5.25 (m, 1H), 4.40 (dd, *J =* 11.6, 2.5 Hz, 1H), 3.20 (s, 3H), 2.59-2.41 (m, 3H), 2.21 (s, b, 1H), 1.95-1.85 (m, 1H), 1.70 (dt, *J =* 13.6, 6.6 Hz, 1H), 1.61 (ddd, *J =* 15.2, 9.2, 6.1 Hz, 1H), 1.49 (dt, *J =* 13.9, 7.2 Hz, 1H), 0.98 (dd, *J =* 19.8, 6.5 Hz, 6H).

Following essentially the method employed to produce Compound ***130A*** of example **16,** compound ***130B*** was prepared. LCMS anal. calcd. for C₂₂H₂₇N₃O₃: 381.2 Found: 382.3 (M+H)⁺. ¹H NMR (500 MHz, Methanol-d⁴) *δ* 7.72 (s, 1H), 7.46 (s, 1H), 7.32 (t, *J =* 7.3 Hz, 1H), 7.14 (s, 1H), 7.07 (d, *J =* 7.4 Hz, 1H), 6.75 (s, 1H), 5.92 (d, *J =* 7.3 Hz, 1H), 5.34 (s, 1H), 4.31 (d, *J =* 10.5 Hz, 1H), 3.26 (s, 3H), 2.54 (d, *J =* 12.6 Hz, 1H), 2.07-1.86 (m, 4H), 1.76 (tt, *J =* 13.7, 6.8 Hz, 1H), 1.31 (t, *J =* 6.4 Hz, 2H), 0.98 (t, *J =* 6.3 Hz, 6H).

### Example 17

### Preparation of Compounds 131A, 131B, 131C, 131D, 131E and 131F

### 5-ethyl-7-hydroxy-10-(methoxymethyl)-1-phenyl-2-(2,2,2-trifluoroethyl)-1,2,3,4,4a,5-hexahydrodipyrido[1,2-b:2',1'-f][1,2,4]triazine-6,8-dione

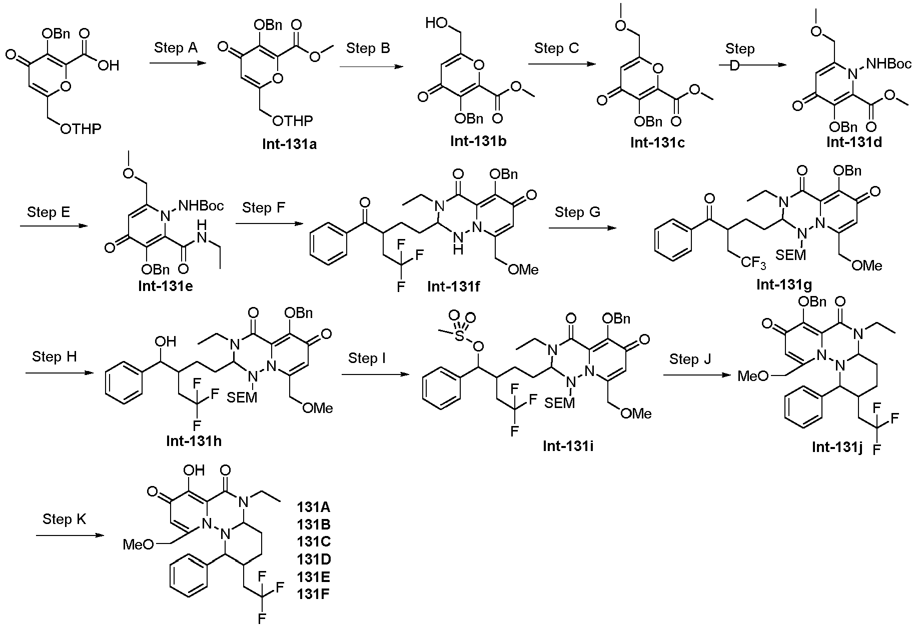

### Step A - Synthesis of Compound Int-131a

Under N₂, 3-(benzyloxy)-4-oxo-6-(((tetrahydro-2H-pyran-2-yl)oxy)methyl)-4H-pyran-2-carboxylic acid (5 g, 13.87 mmol) was dissolved in 100 mL 1: 1 MeOH /CH₂Cl₂ and then cooled to 0°C. (Trimetjulsilyl)dizaomethane (6.94 ml, 13.87 mmol) was added dropwise. The resulting mixture was stirred at for 0 °C. Upon completion, it was concentrated to provide ***Int-131a.*** The crude was used without further purification. LCMS anal. calcd. for C₂₀H₂₂O₇: 374.3; Found: 375.2 (M+H)⁺.

### Step B - Synthesis of Compound Int-131b

To a solution of ***Int-131a*** (5.1 g, 13.62 mmol) in MeOH (15 mL) at RT under N₂ was added hydrogen chloride in dioxane (10.22 mL, 40.9 mmol). The reaction was stirred at this temperature for 1 hour. Upon completion, it was concentrated. The residue was diluted with 100 mL EtOAc, followed by washing with 100 mL sat. NaHCO₃ aqueous solution. The organic layer was dried over MgSO₄ and concentrated to provide ***Int-131b.*** LCMS anal. calcd. for C₁₅H₁₄O₆: 290.2; Found: 291.0 (M+H) ⁺.

### Step C - Synthesis of Compound Int-131c

To a stirred solution of ***Int-131b*** (5 g, 17.23 mmol) in DMF (150 mL) at 0 °C was added iodomethane (2.93 g, 20.67 mmol), followed by sodium hydride (0.827 g, 20.67 mmol). The resulting mixture was stirred at this temperature for 1 hour. Upon completion, it was slowly quenched with 100 mL water. The mixture was extracted with 2x100 mL EtOAc. The combined organic was dried over MgSO₄ and concentrated. The residue was purified by a 120 g silica gel column with 0-10% MeOH in DCM as eluting solvent to provide ***Int-131c.*** LCMS anal. calcd. for C₁₆H₁₆O₆: 304.3; Found: 305.0 (M+H)⁺.

### Step D - Synthesis of Compound Int-131d

A solution of pyridinium-p-toluenesulfonate (4.68 g, 18.63 mmol) and ***Int-131d*** (2.1 g, 6.90 mmol) in DMA (40 mL) was heated up to 60 °C. Thereafter, a solution of tert-butyl carbazate (1.277 g, 9.66 mmol) in DMA (10 mL) was slowly added to the reaction solution over 10 min. The reaction solution was stirred at 60 °C for 48 hrs. Upon completion, the mixture was added 50 mL water. It was extracted with 2x50 mL EtOAc. The combined organic layer was dried over MgSO₄, filtered and concentrated. The residue was purified with a 120 g silica gel column using 0-100% EtOAc/hexane as eluing solvent to provide ***Int-131d** .* LCMS anal. calcd. for C₂₁H₂₆N₂O₇: 418.4; Found: 419.2(M+H)⁺.

### Step E - Synthesis of Compound Int-131e

The mixture of ***Int-131d*** (1.2 g, 2.87 mmol) and ethanamine in THF (14.34 mL, 28.7 mmol) was heated at 80 °C overnight. Upon completion, it was concentrated. To above residue, 5 mL DCM and 5 mL TFA was added. It was stirred at room temperature for 2 hrs before it was concentrated. The resulting residue was loaded onto a 100 g C18 reverse pahse column with 0-40% ACN in water (0.05% TFA modifier) to provide ***Int-131e.*** LCMS anal. calcd. for C₁₇H₂₁N₃O₄ :331.4 Found: 332.1 (M+H)⁺.

### Step F - Synthesis of Compound Int-131f

***Int-131e*** (240 mg, 0.724 mmol) and 4-benzoyl-6,6,6-trifluorohexanal was mixed in a 20 mL sealed microwave tube. The reaction was stirred at 120 °C for 2.5 hrs. Upon completion, the reaction was cooled down and concentrated. The residue was purified by MPLC ISCO Combi-flash on ISCO Redi-Sep 24 column, eluting with 0-10% CH₂Cl₂/MeOH to provide ***Int-131f.*** LCMS anal. calcd. for C₃₀H₃₂F₃N₃O₅: 571.6; Found: 572.4 (M+H)⁺.

### Step G - Synthesis of Compound Int-131g

Under N₂, to a solution of ***Int-131f*** (590 mg, 1.032 mmol) in DCM (20 mL) was added DIEA (0.397 mL, 2.271 mmol) and SEM-Cl (0.201 mL, 1.135 mmol) at 0 °C. The reaction was stirred at 25 °C overnight before it was concentrated. The residue was purified by MPLC ISCO Combi-flash on ISCO Redi-Sep 40 g column, eluting with 0-10% CH₂Cl₂/MeOH, to provide ***Int-131g.*** LCMS anal. calcd. for C₃₆H₄₆F₃N₃O₆Si: 304; Found: 305.0 (M+H)⁺.

### Step H - Synthesis of Compound Int-131h

Under N₂, to a solution of ***Int-131g*** (680 mg, 0.969 mmol) in DCM (10 mL)/MeOH (5 mL) was added NaBH₄ (36.7 mg, 0.969 mmol) at 0 °C. The reaction was stirred at room temperature for 1.5 hours. Upon completion, the reaction was cooled back to 0°C and quenched with 1N HCl (0.5 mL) then concentrated. The residue was purified by MPLC ISCO Combi-flash on ISCO Redi-Sep 40 g column, eluting with 0-10% CH₂Cl₂/MeOH to provde ***Int-131h.*** LCMS anal. calcd. for C₃₆H₄₈F₃N₃O₆Si: 703.8; Found: 704.6(M+H)⁺.

### Step I - Synthesis of Compound Int-131i

Under N₂, to a solution of ***Int-131h*** (605 mg, 0.860 mmol) in DCM (10 mL) was added TEA (599 µL, 4.30 mmol), followed by Ms-Cl (201 µL, 2.58 mmol) at 0°C. The reaction was stirred at room temperature for 1.0 h. Upon completion, the reaction was diluted with DCM (15 mL) and then washed water, brine, dried over sodium sulfate, filtered and concentrated to yield ***Int-131i*** which was used for next step without purification. LCMS anal. calcd. for C₃₇H₅₀F₃N₃O₈SSi: 781.9 Found: 782.4 (M+H)⁻.

### Step J- Synthesis of Compound Int-131j

Under N₂, to a solution of ***Int-131i*** (712 mg, 0.911 mmol) in THF (10 mL) was added TBAF in THF (3.821 µL, 3.821 mmol) at 60 °C. The reaction was stirred at this temperature for 4.0 hrs, cooled and concentrated. The residue was purified by MPLC ISCO Combi-flash on ISCO Redi-Sep 40 g column, eluting with 0-20% CH₂Cl₂/MeOH to afford 2 fractions. Both fractions were subject to SFC resolution. The less polar fraction yielded 2 peaks from SFC resolution ***Int-131j-peak1*** and ***Int-131j-peak2.*** The more polar fraction yielded 4 peaks from SFC resolution ***Int-131j-peak3, Int-131j-peak4, Int-131j-peak5*** and ***Int-131j-peak6.*** (SFC conditions: Daicel@ chiralpak OD-H column (250*21 mm, 5 µm); 35% EtOH with 0.1% DIEA; 70 mL/min). LCMS anal. calcd. for C₃₀H₃₂F₃N₃O₄: 555.6 Found: 556.5(M+H)⁺.

### Step K- Synthesis of Compound Int-131h

***Int-131j-peakl*** (7 mg, 0.013 mmol) was dissolved in MeOH (1 mL). The flask was purged with nitrogen twice, followed by the addition of Pd/C (9.39 mg, 8.82 µmol). It was purged with hydrogen 3 times and was allowed to stir under H₂ balloon for 1 hour. The resulting reaction mixture was filtered through a syringe filter before concentrated. The residue was purified by preparative HPLC Reverse phase (C-18 column), eluting with 0-90% Acetonitrile/Water (with 0.1% TFA modifier) to afford ***131A.*** LCMS anal. calcd. for C₂₃H₂₆F₃N₃O₄: 465.4; Found: 466.3 (M+H)⁺. ¹H NMR (500 MHz, Methanol-d⁴) δ 7.34 - 6.85 (m, 6 H), 5.27 (s, 1 H), 4.93 (d, *J =* 13.2 Hz, 1 H), 4.63 (d, *J =* 13.2 Hz, 1 H), 4.57 (s, 1 H), 3.59 (s, 3 H), 2.93 - 2.81 (m, 2 H), 2.51 (s, 3 H), 1.97 (d, *J =* 9.7 Hz, 1H).

Following essentially the method employed to produce Compound ***131A*** of example **17,** compound ***131B*** was prepared. LCMS anal. calcd. for C₂₃H₂₆F₃N₃O₄: 465.4; Found: 466.3 (M+H)⁺. ¹H NMR (500 MHz, Methanol-d⁴) *δ* 7.33 - 6.92 (m, 6 H), 5.29 (s, 1 H), 4.95 (d, *J =* 13.4 Hz, 1 H), 4.68 (d, *J =* 13.4 Hz, 1 H), 4.56 (s, 1 H), 3.60 (s, 3 H), 2.93 - 2.68 (m, 3 H), 2.52 (s, 3 H), 1.98 (d, *J =* 9.2 Hz, 1 H).

Following essentially the method employed to produce Compound ***131A*** of example **17,** compound ***131C*** was prepared. LCMS anal. calcd. for C₂₃H₂₆F₃N₃O₄: 465.4; Found: 466.3 (M+H)⁺. ¹H NMR (500 MHz, Methanol-d⁴) *δ* 7.84 - 6.57 (m, 6 H), 6.18 (s, 1 H), 5.36 (s, 1 H), 4.52 (d, *J =* 14.6 Hz, 1 H), 4.14 (d, *J =* 10.9 Hz, 1 H), 3.90 (d, *J =* 14.6 Hz, 1 H), 3.26 (s, 3 H), 2.86 (q, *J =* 10.4 Hz, 1 H), 2.64 - 1.52 (m, 7 H).

Following essentially the method employed to produce Compound ***131A*** of example **17,** compound ***131D*** was prepared. LCMS anal. calcd. for C₂₃H₂₆F₃N₃O₄: 465.4; Found: 466.3 (M+H)⁺. ¹H NMR (500 MHz, Methanol-d⁴) *δ* 7.53 - 7.22 (m, 7 H), 6.29 (s, 1 H), 5.42 (s, 1 H), 4.76 - 4.64 (m, 2 H), 4.22 (d, *J =* 14.3 Hz, 1 H), 3.38 (s, 4 H), 3.33 (s, 5 H), 2.84 - 2.67 (m, 2 H), 2.68 - 2.44 (m, 4 H), 2.10 - 1.96 (m, 2 H).

Following essentially the method employed to produce Compound ***131A*** of example **17,** compound ***131E*** was prepared. LCMS anal. calcd. for C₂₃H₂₆F₃N₃O₄: 465.4; Found: 466.3 (M+H)⁺. ¹H NMR (500 MHz, Methanol-d⁴) *δ* 7.30 (tt, *J =* 15.8, 6.8 Hz, 5 H), 6.24 (s, 1 H), 5.41 (s, 1 H), 4.76 - 4.64 (m, 2 H), 4.22 (d, *J =* 14.2 Hz, 1 H), 3.50 - 3.34 (m, 5 H), 2.83 - 2.66 (m, 2 H), 2.65 - 2.43 (m, 3 H), 2.10 - 1.92 (m, 2 H).

Following essentially the method employed to produce Compound ***131A*** of example **17,** compound ***131F*** was prepared. LCMS anal. calcd. for C₂₃H₂₆F₃N₃O₄: 465.4; Found: 466.3 (M+H)⁺. ¹H NMR (500 MHz, Chloroform-d) δ 7.79 (d, *J* = 7.7 Hz, 1 H), 7.54 (d, *J =* 6.9 Hz, 1 H), 7.42 (d, *J =* 7.5 Hz, 1 H), 7.17 (d, *J =* 7.1 Hz, 1 H), 6.73 (d, *J =* 7.2 Hz, 1 H), 6.29 (d, *J =* 15.0 Hz, 1 H), 5.42 (s, 1 H), 4.63 - 4.38 (m, 2 H), 4.19 (t, *J =* 12.5 Hz, 1 H), 3.96 (t, *J =* 14.8 Hz, 1 H), 3.36 - 3.26 (m, 6 H), 2.92 (d, *J =* 10.1 Hz, 1 H), 2.61 (d, *J =* 15.0 Hz, 1 H), 2.48 - 1.48 (m, 5 H).

The examples in **Table 5** were prepared using procedures similar to those described in **Example 17,** from the appropriate starting material.

**Table 5**

| **Example** | **Structure** | **Observed *m*/*z* [M + H]⁺** |
|---|---|---|
| | **Name** | |
| | **Precursor SFC Separation Condition** | |
| **132A (Peak1)** | 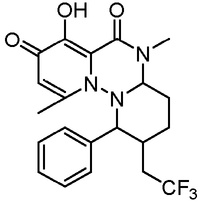 | 422.2 |
| **132B (Peak2)** | | |
| **132C (Peak3)** | | |
| **132D (Peak 4)** | 7-hydroxy-5,10-dimethyl-1-phenyl-2-(2,2,2-trifluoroethyl)-1, 2,3,4,4a,5-hexahydrodipyrido[1,2-b:2',1'-f][1,2,4]triazine-6 ,8-dione | |
| | Precursor was resolved by SFC. SFC conditions: Daicel@ chiralpak AS-H column (250*21 mm, 5 µm); 50%EtOH; 50 mL/min and Daicel@ chiralpak AD-H column (250*21 mm, 5 µm); 25%EtOH with 0.2% DIPA; 70 mL/min | |

### Example 18

### Preparation of Compounds 133

### 7-hydroxy-5-methyl-6,8-dioxo-1-phenyl-2-(2,2,2-trifluoroethyl)-1,2,3,4,4a,5,6,8-octahydrodipyrido[1,2-b:2',1'-f][1,2,4]triazine-9-carbonitrile

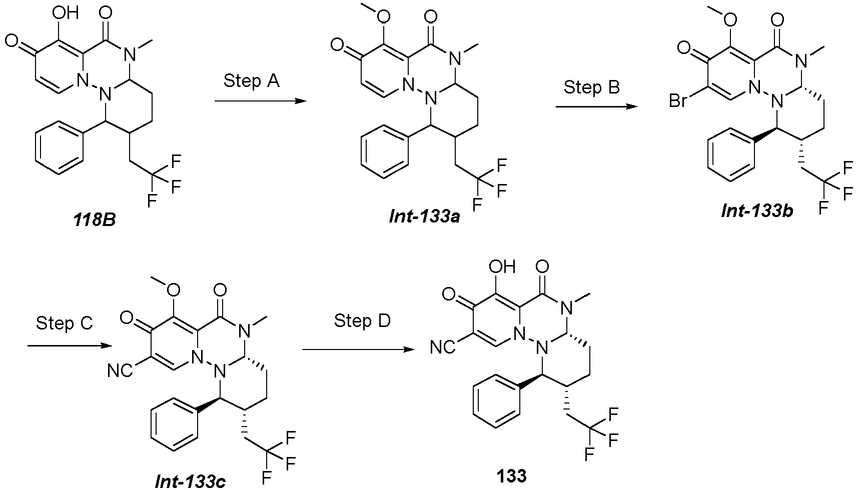

### Step A - Synthesis of Compound Int-133a

In a flame-dried flask under an atmosphere of N₂, ***118B*** (320 mg, 0.785 mmol) was dissolved in anhydrous DCM (10 mL) and anhydrous MeOH (3 mL). TMS-Diazomethane (2M in ether) (707 µL, 1.414 mmol) was added dropwise over 15 minutes and the reaction was stirred at RT. After 2 hours, TMS-Diazomethane (2M in ether) (450 µL) was added and stirred at RT for another hour. More TMS-Diazomethane (2M in ether) (500 µL) was added and the reaction was stirred for another hour at RT. The reaction was quenched with acetic acid (180 µL, 3.14 mmol) and stirred for several minutes before diluting with water and DCM. It was extracted with DCM (3x) and the combined organics were washed with brine, dried over Na₂SO₄, filtered, and concentrated under reduced pressure. The residue was dissolved in DCM and purified on normal phase silica gel (24 g ISCO gold column) eluting under gradient conditions 0-100% (26% EtOH-EtOAc / EtOAc). The appropriate fractions were collected and concentrated under reduced pressure. The residue was dried by concentrating under reduced pressure with toluene/ACN and then dried under high vacuum to provide ***Int-133a.*** LCMS anal. calcd. for C₂₁H₂₂F₃N₃O₃: 421.4; Found: 422.2 (M+H)⁺.

### Step B - Synthesis of Compound Int-133b

***Int-133a*** (255 mg, 0.605 mmol) under an atmosphere of N₂ was dissolved in anhydrous DCM (10 mL). To this solution was added *N*-Bromosuccinimide (162 mg, 0.908 mmol) and the reaction was stirred at RT for 90 minutes. More *N*-Bromosuccinimide (115 mg) was added and the reaction was stirred at RT for another 45 minutes. Upon completion, the reaction volume was reduced under reduced pressure and loaded directly onto normal phase silica gel (24g ISCO gold column) eluting under gradient conditions 0-40% (26% EtOH-EtOAc / EtOAc). The appropriate fractions were collected and concentrated under reduced pressure and then dried under high vacuum to provide ***Int-133b.*** LCMS anal. calcd. for C₂₁H₂₁BrF₃N₃O₃: 499.1; Found: 500.0 (M+H)⁺.

### Step C - Synthesis of Compound Int-133c

To a microwave reaction vial under an atmosphere of N₂, ***Int-133b*** (175 mg, 0.350 mmol) and zinc cyanide (205 mg, 1.749 mmol) were mixed in anhydrous DMF (4 mL) and purged with a stream of N₂ gas. Bis(tri-t-butylphosphine)palladium(0) (89 mg, 0.175 mmol) was added and the reaction was sealed and heated at 100 °C in preheated block for 3 hours. The reaction was cooled and partitioned between DCM and water. The mixture was extracted with DCM (4x) and the combined organic layers were washed with brine, dried over Na₂SO₄, filtered, and concentrated under reduced pressure. The residue was dissolved in DCM/EtOAc and purified on normal phase silica gel (12 g ISCO gold column) eluting under gradient conditions 0-20% (26% EtOH-EtOAc / EtOAc). The appropriate fractions were collected and concentrated under reduced pressure and then dried under high vacuum to provide ***Int-133c.*** LCMS anal. calcd. for C₂₂H₂₁F₃N₄O₃: 446.4; Found: 447.2 (M+H)⁺.

### Step D - Synthesis of Compound 133

***Int-133c*** (9 mg, 0.020 mmol) and magnesium bromide (12 mg, 0.065 mmol) was mixed in anhydrous THF (3 mL) and stirred at RT for 2 hours. The reaction was then concentrated under reduced pressure. The residue was redissolved in 5:1 DMF/water, and purified by gradient elution on reverse-phase (25-75 % CH₃CN/ water (0.1% TFA modifier) on Sunfire Prep column (C18, 30 x 150mm) over 12 min. The appropriate fractions were lyophilized to provide 133. LCMS anal. calcd. for C₂₁H₁₉F₃N₄O₃: 432.4; Found: 433.2 (M+H)⁺. ¹H NMR (500 MHz, Acetonitrile-d3) *δ* 12.36 (s, 1H), 7.67 (d, *J =* 7.8 Hz, 1H), 7.54 (t, *J =* 7.6 Hz, 1H), 7.41 (t, *J* = 7.5 Hz, 1H), 7.21 (t, *J =* 7.5 Hz, 1H), 7.13 (s, 1H), 6.81 (d, *J =* 7.3 Hz, 1H), 5.19 (s, 1H), 4.00 (d, *J =* 10.6 Hz, 1H), 3.14 (s, 3H), 2.63 - 2.54 (m, 1H), 2.50 - 2.43 (m, 1H), 2.23 - 2.14 (m, 1H), 2.13 - 2.07 (m, 1H), 2.04 - 1.96 (m, 1H), 1.78 - 1.66 (m, 1H), 1.57 - 1.47 (m, 1H).

### Example 19

### Preparation of Compound 134

### 7-hydroxy-5-methyl-1-phenyl-9-(1H-tetrazol-5-yl)-2-(2,2,2-trifluoroethyl)-1,2,3,4,4a,5-hexahydrodipyrido[1,2-b:2',1'-f][1,2,4]triazine-6,8-dione

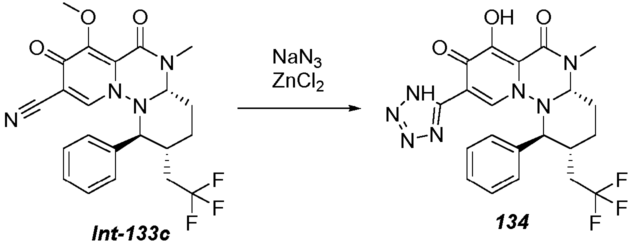

In a vial, ***Int-133c*** (11 mg, 0.025 mmol), sodium azide (3.20 mg, 0.049 mmol), and zinc chloride (3.36 mg, 0.025 mmol) were mixed in DMF (1 mL). The vial was sealed and heated at 100 °C in a preheated block for 3 hours. The reaction was then recharged with sodium azide (16 mg) and zinc chloride (2 mg) and stirred overnight at 100 °C. Upon completion, the reaction was diluted with water and purified by gradient elution on reverse-phase (15-65 % CH₃CN/ water (0.1%TFA) on Sunfire Prep C18 column (30 x 150mm) over 12 min. The appropriate fractions were lyophilized to provide the title compound. LCMS anal. calcd. for C₂₁H₂₀F₃N₇O₃: 475.4; Found: 476.2 (M+H)⁺. ¹H NMR (500 MHz, Acetonitrile-d³) *δ* 12.30 (s, 1H), 7.77 (d, *J =* 7.7 Hz, 1H), 7.71 (s, 1H), 7.46 (t, J = 7.5 Hz, 1H), 7.11 (t, *J =* 7.4 Hz, 1H), 7.02 (t, *J =* 7.4 Hz, 1H), 6.78 (d, *J =* 7.5 Hz, 1H), 5.27 (s, 1H), 4.06 (d, *J =* 10.7 Hz, 1H), 3.17 (s, 3H), 2.67 - 2.57 (m, 1H), 2.53 - 2.46 (m, 1H), 2.29 - 2.21 (m, 1H), 2.19 - 2.10 (m, 1H), 2.06 - 1.97 (m, 1H), 1.79 - 1.67 (m, 1H), 1.62 - 1.52 (m, 1H).

### Example 20

### Preparation of Compounds 135A and 135B

### 2,7-dihydroxy-5-methyl-1-phenyl-1,2,3,4,4a,5-hexahydrodipyrido[1,2-b:2',1'-f][1,2,4]triazine-6,8-dione

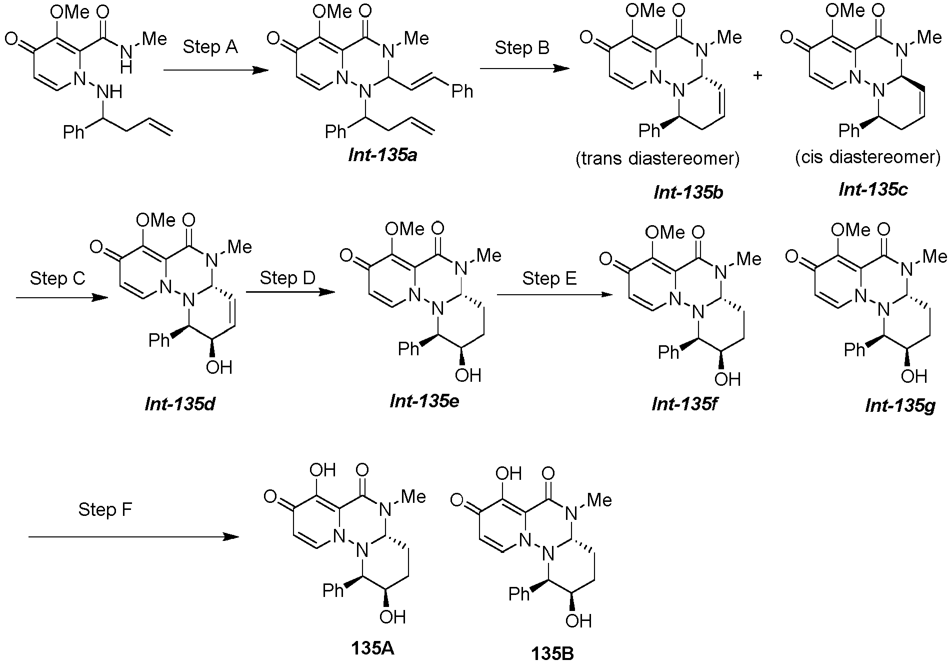

### Step A - Synthesis of Compound Int-135a

A mixture of 3-methoxy-N-methyl-4-oxo-1-((1-phenylbut-3-en-1-yl)amino)-1,4-dihydropyridine-2-carboxamide (154.00 g, 471.553 mmol,), cinnamal (124.50 g, 943.106 mmol) and trifluoroacetic acid (80.64 g, 707.3301 mmol,) in DCE (1.00 L) was stirred at 120 °C for 3 days. After cooling, the reaction mixture was concentrated. The residue was applied onto a silica gel column and eluted with (30% EtOH/EA)/PE=0-60% to provide ***Int-135a.*** LCMS anal. calcd. for C₂₇H₂₇N₃O₃: 441.2; Found: 442.2 (M+H)⁺.

### Step B - Synthesis of Compound Int-135b and Int-135c

A mixture of ***Int-135a*** (10.00 g, 22.648 mmol), TFA (2.59 g, 22.715 mmol) and Hoveyda-Grubbs 2^{nd} generation catalyst (5.79 g, 6.820 mmol) was stirred for 4 hrs at 80 °C. After cooling, the reaction mixture was concentrated. The residue was applied onto a silica gel column with 0-15% dichloromethane/methanol to afford a mixture (110 g) which was further purified by Sunfire Prep C18 column with 30-80% ACN in water (0.05% TFA modifier) as eluting solvent to provide a 40 g mixture. The mixture was further purified by Prep-SFC with CHIRAL ART Cellulose-SB column (S-5um 50*250 mm, 50 mm*250 mm, 5um; mobile phase, CO₂ (50%) and MeOH) to afford ***Int-1b*** (22.52 g, 5.562 mmol) and ***Int-1c*** (3.17 g, 0.783 mmol). LCMS anal. calcd. for C₁₉H₁₉N₃O₃: 337.2; Found: 338.3 (M+H)⁺.

### Step C - Synthesis of Compound Int-135d

To a solution of ***Int-135c*** (0.300 g, 0.89 mmol) in dioxane (9 mL) was added selenium dioxide (0.197 g, 1.78 mmol). The resulting reaction mixture was stirred at 95 °C overnight. After cooling, the mixture was concentrated and purified by ISCO reverse phase C18 column chromatography using 0-50% H₂O/ACN with 0.05% TFA modifier as eluting solvent to afford ***Int-135d*** (0.09 g, 0.276 mmol). LCMS anal. calcd. for C₁₉H₁₉N₃O₄: 353.1; Found: 354.1 (M+H)⁺.

### Step D - Synthesis of Compound Int-135e

To a solution of ***Int-135d*** (0.142 g, 0.40 mmol) in MeOH (5 mL) was added 10% Pd-C (0.070 g) at 25 °C with stirring. The resulting black suspension was placed under an atmosphere of hydrogen (balloon) for 3 hrs. The reaction mixture was filtered through a pad of celite and the solid thoroughly washed with MeOH. The combined filtrate was concentrated to provide ***Int-135e.*** LCMS anal. calcd. for C₁₉H₂₁N₃O₄: 355.2; Found: 356.3 (M+H)⁺.

### Step E - Preparation of Compounds Int-135f and Int-135g.

***Int-135e*** (0.57 g) was separated by SFC using an AD-H column (50 x 250mm) and 40% MeOH as co-solvent to afford ***Int-135f*** (Peak 1) and ***Int-135g*** (Peak 2). LCMS anal. calcd. for C₁₉H₂₁N₃O₄: 355.2; Found: 356.3 (M+H)⁺.

### Step F - Syntheses of compounds 135A and 135B

To a solution of ***Int-135f*** (10.0 mg, 0.04 mmol) in ACN (1 mL) was added MgBr₂ (36.8 mg, 0.20 mmol) at 25 °C. The reaction was stirred at 25 °C for 3 hrs. The volatiles were removed under reduced pressure. The residue was purified by HPLC (Sunfire Prep C18 column) using 0.05% TFA as modifier to give ***135A*** (10.6mg). LCMS anal. calcd. for C₁₈H₁₉N₃O₄: 341.2; Found: 342.6 (M+H)⁺.

Following essentially the method employed to produce Compound ***135A*** of Step F, compound ***135B*** was prepared. LCMS anal. calcd. for C₁₈H₁₉N₃O₄: 341.2; Found: 342.6 (M+H)⁺.

### Example 21

### Preparation of Compound 136A and 136B

### 2,7-dihydroxy-5-methyl-1-phenyl-1,2,3,4,4a,5-hexahydrodipyrido[1,2-b:2',1'-f][1,2,4]triazine-6,8-dione

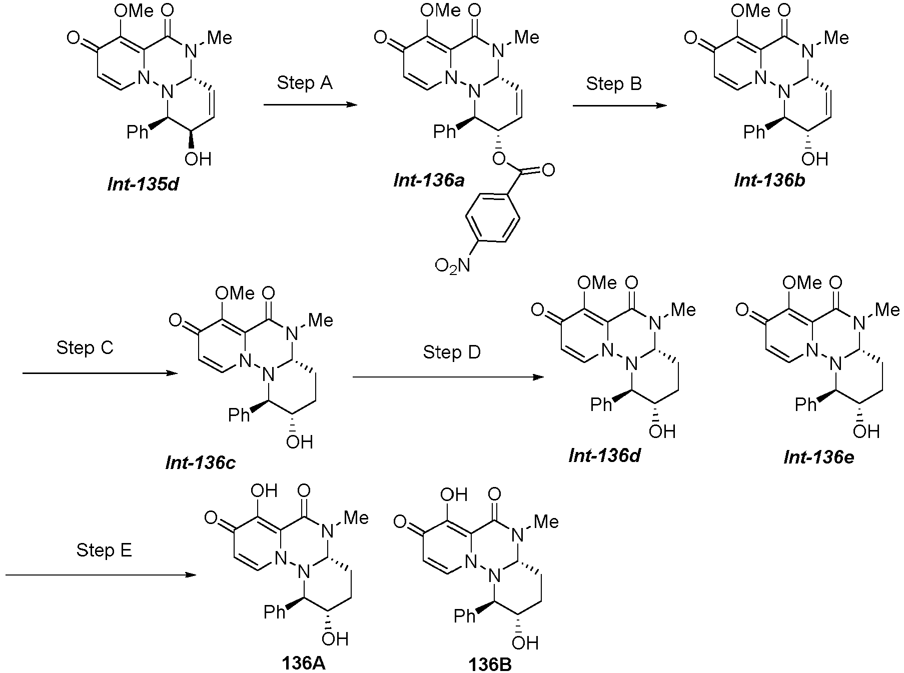

### Step A - Synthesis of Compound Int-136a

A solution of DEAD (0.20 mL of a 40% solution in toluene, 0.458 mmol) was added dropwise to a mixture of ***Int-135d*** (54mg, 0.153 mmol), 4-nitrobenzoic acid (77mg, 0.453 mmol) and triphenylphosphine (120 mg, 0.453 mmol) in anhydrous THF (3 mL) at room temperature under an atmosphere of nitrogen. The resulting reaction mixture was stirred overnight. The volatiles were removed under reduced pressure. The residue was purified by silica gel column chromatography (ISCO 4 g column) using 0 to 5% MeOH in DCM as eluent to provide ***Int-136a.*** LCMS anal. calcd. for C₂₆H₂₂N₄O₇: 502.2; Found: 503.2 (M+1)⁺.

### Step B - Synthesis of Compound Int-136b

To a solution of ***Int-136a*** (48.8 mg, 0.097 mmol) in MeOH (1 mL) was added potassium carbonate (1 mg) at room temperature. The resulting reaction mixture was stirred at room temperature overnight. The volatiles were removed under reduced pressure. The residue was purified by silica gel column chromatography (ISCO 12g column) using 0 to 10% MeOH in dichloromethane as eluent to afford ***Int-136b.*** LCMS anal. calcd. for C₁₉H₁₉N₃O₄: 353.1; Found: 354.3 (M+H)⁺.

### Step C, D, E - Preparation of compounds 136A and 136B

Following essentially the method employed to produce compounds ***135A*** and ***135B,*** compounds ***136A*** and ***136B*** were prepared. LCMS anal. calcd. for C₁₈H₁₉N₃O₄: 341.1; Found: 342.3 (M+1)⁺.

### Example 22

### Preparation of Compound 137A, 137B, 137C and 137D

### 2,7-dihydroxy-1,5-dimethyl-1-phenyl-1,2,3,4,4a,5-hexahydrodipyrido[1,2-b:2',1'-f][1,2,4]triazine-6,8-dione

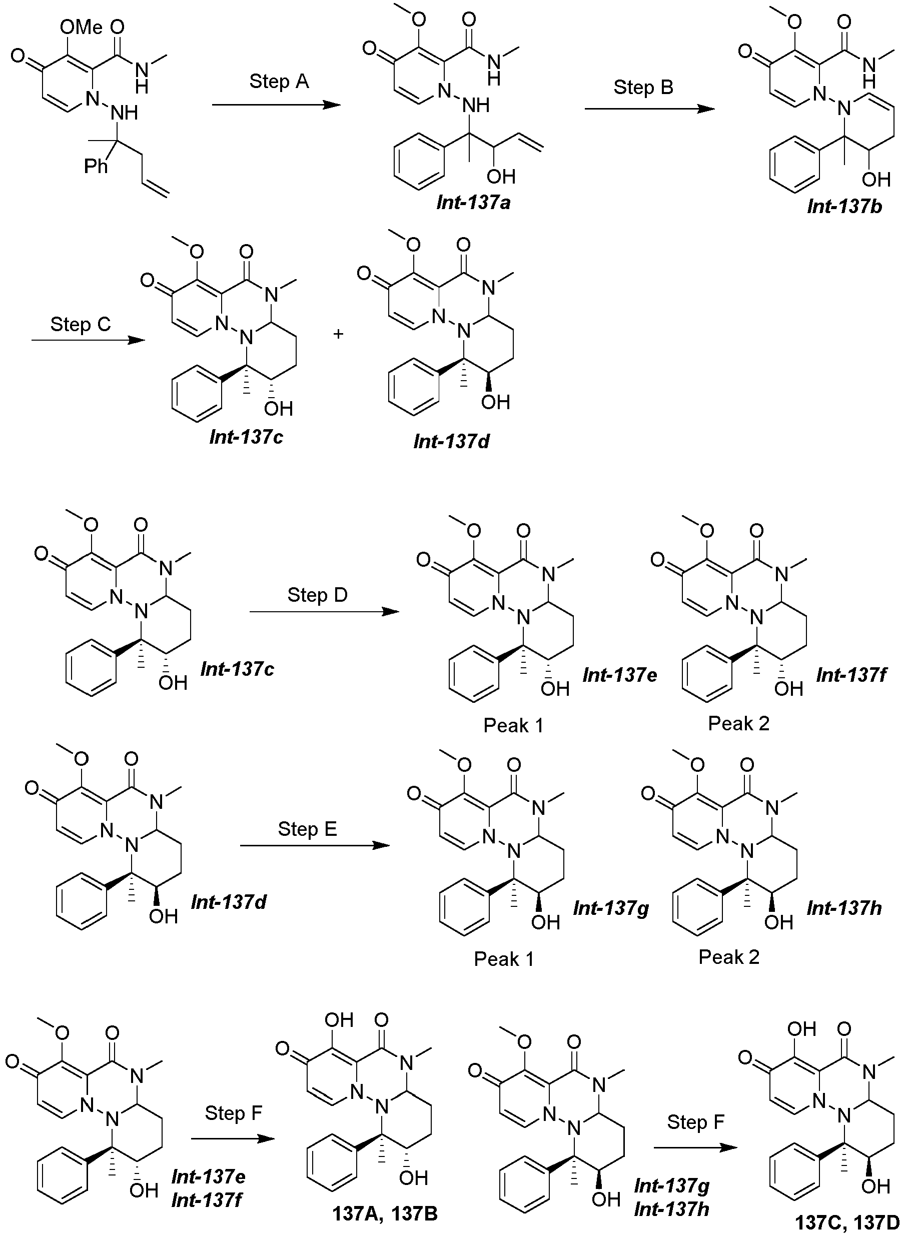

### Step A - Synthesis of Compound Int-137a

A solution of 3-methoxy-N-methyl-4-oxo-1-((2-phenylpent-4-en-2-yl)amino)-1,4-dihydropyridine-2-carboxamide (97.00 g, 284.115 mmol), SeO₂ (157.63 g, 1420.58 mmol), tert-Butyl hydroperoxide (285 mL in decane, 5 equiv) in DCM (2.91 L) was stirred for 32 hrs at 40 degrees °C under an atmosphere of nitrogen. The reaction mixture was cooled to -10 °C with an ice/salt bath. It was then quenched by the addition of 2 L of Na₂S₂O₃ aqueous solution. The solids were filtered out. The resulting solution was extracted with dichloromethane three times and the combine organic layer was concentrated. The residue was applied onto a 330 g silica gel column with dichloromethane/methanol (20:1) as eluting solvent to provide ***Int-137a.*** LCMS anal. calcd. for C₁₉H₂₃N₃O₄: 357.4; Found: 358.3 (M+H)⁺.

### Step B - Synthesis of Compound Int-137b

To a 2-L high pressure tank reactor, was placed ACN (680.00 mL), ***Int-137a*** (34.00 g, 95.129 mmol), BiPhePhos (1.50 g, 1.906 mmol), Carbonyltris(triphenylphosphine)rhodium(I) hydride (1.75 g, 1.903 mmol), CO (0.5 Mpa). To the above reactor, H₂(g) (0.5MPa) was introduced at 80 °C. The resulting mixture was stirred at 80 °C for 20 hrs. Upon completion, it was cooled down. The reaction mixture was concentrated to 10 V and used to next step directly.

### Step C - Synthesis of Compounds Int-137c and Int-137d

To a 1-L 4-necked round-bottom flask was placed the reaction mixture from the previous step and AcOH (68 g). The resulting solution was stirred at 80 °C for 3 days. Upon completion, the resulting mixture was concentrated. The residue was diluted with ACN and purified by Flash-Prep-HPLC using reverse phase C18 (220 g) with 10-100% ACN in H₂O as eluting solvent to provide ***Int-137c*** and ***Int-137d.*** LCMS anal. calcd. for C₂₀H₂₃N₃O₄: 369.2; Found: 370.2 (M+H)⁺.

### Step D - Preparation of Compounds Int-137e and Int-137f

***Int-137c*** (1.0g) was resolved by SFC using an OD-H column (21 X 250mm) with 35% MeOH as co-solvent to provide ***Int-137e*** (Peak 1: 0.433g) and ***Int-137f*** (*Peak* 2: 0.423g). LCMS anal. calcd. for C₂₀H₂₃N₃O₄: 369.2; Found: 370.2 (M+H)⁺.

### Step E - Preparation of Compounds Int-137g and Int-137h.

***Int-137d*** (1.0g) was resolved by SFC using an OJ-H column (21 X 250mm) and 30% MeOH and 0.2% DIPA as co-solvent to provide ***Int-137g*** (Peak 1) and ***Int-137f*** (Peak 2). LCMS anal. calcd. for C₂₀H₂₃N₃O₄: 369.2; Found: 370.3 (M+H)⁺.

### Step F- Synthesis of Compounds 137A, 137B, 137C and 137D.

Following essentially the method employed to produce compounds ***135A*** and ***135B,*** compounds ***137A, 137B, 137C*** and ***137D*** were prepared. LCMS anal. calcd. for C₁₉H₂₁N₃O₄: 355.2; Found: 356.2 (M+H)⁺.

### Example 23

### Preparation of Compounds 138A and Compound 138B.

### 7-hydroxy-1,5-dimethyl-1-phenyl-3,4,4a,5-tetrahydrodipyrido[1,2-b:2',1'-f][1,2,4]triazine-2,6,8(1H)-trione

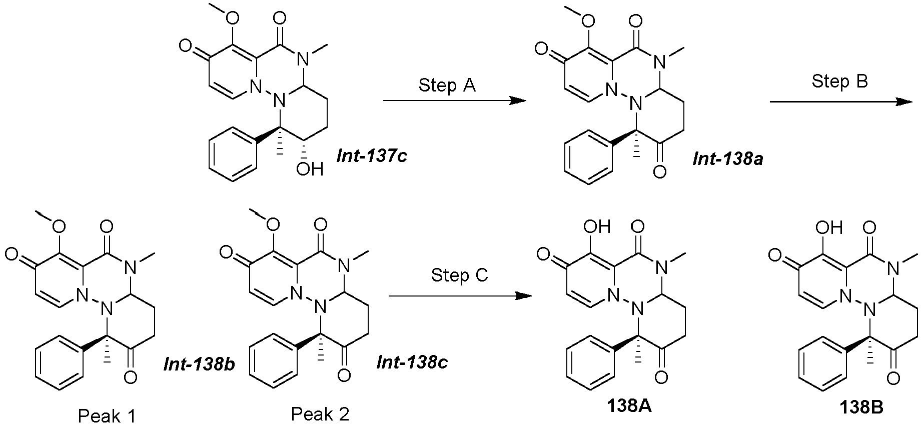

### Step A - Synthesis of Compound Int-138a

Dess- Martin periodinane (0.230 g, 0.541 mmol) was added to a solution of ***Int-137c*** (0.100 g, 0.271 mmol) in dichloromethane (5 mL). The resulting reaction mixture was stirred at room temperature for 3 hrs. The volatiles were removed under reduced pressure. The residue was purified by silica gel column chromatography using 0 to 5% MeOH in dichloromethane as eluent to provide ***Int-138a*** (0.083 g, 0.228 mmol). LCMS anal. calcd. for C₂₀H₂₁N₃O₄: 367.2; Found: 368.3 (M+H)⁺.

### Step B - Preparation of Compounds Int-138b and Int-138c.

***Int-138a*** (0.083 g) was resolved by SFC using an AD-H column (21 X 250mm) and 30% IPA and 0.2% DIPA as co-solvent to provide ***Int-138b*** (Peak 1) and ***Int-138c*** (Peak 2). LCMS anal. calcd. for C₂₀H₂₁N₃O₄: 367.2; Found: 368.3 (M+1)⁺.

### Step C - Synthesis of Compounds 138A and 138B.

Following essentially the method employed to produce compounds ***135A*** and ***135B,*** compounds ***138A*** and ***138B*** were prepared. LCMS anal. calcd. for C₁₉H₁₉N₃O₄: 355.2; Found: 354.2 (M+H)⁺.

### Example 24

### Preparation of Compound 139

### (1R,2 S,4aS)-2-azido-7-hydroxy-5-methyl-1-phenyl-1,2,3,4,4a,5-hexahydrodipyrido[1,2-b:2',1'-f][1,2,4]triazine-6,8-dione

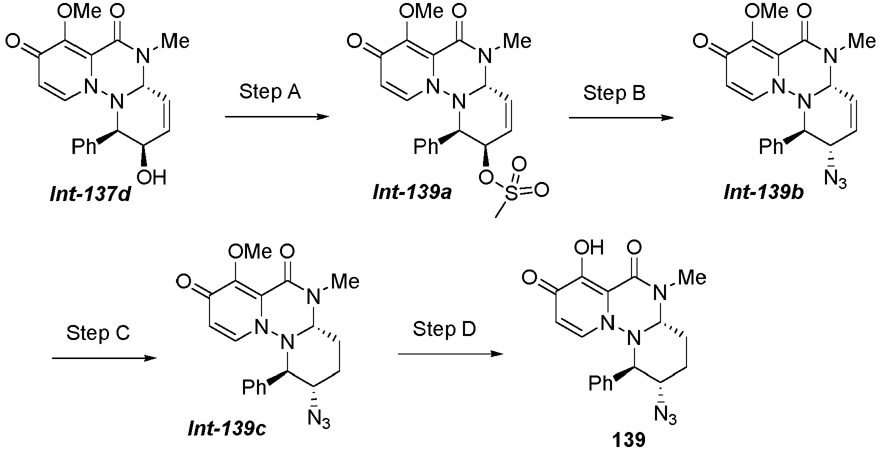

### Step A - Synthesis of Compound Int-139a

Triethylamine (0.197 mL, 1.42 mmol) was added to a solution of ***Int-137d*** (0.250 g, 0.707 mmol) and methylsulfonyl chloride (0.082 mL, 1.05mmol) in anhydrous dichloromethane (1 mL). The resulting reaction mixture was stirred at room temperature for 1 h, and additional portions of triethylamine (0.197 mL) and methane sulfonyl chloride (0.082 mL) were added. After stirring for another 1 h., the volatiles were removed under reduced pressure. The residue purified by silica gel column chromatography using 0 to 5% MeOH in dichloromethane as eluent to provide ***Int-139a.*** LCMS anal. calcd. for C₂₀H₂₁N₃O₆S: 432.1; Found: 432.3 (M+H)⁺.

### Step B - Synthesis of Compound Int-139b

Sodium azide (0.113 g, 1.74 mmol) was added to a solution of ***Int-139a*** (0.250 g, 0.579 mmol) in anhydrous DMF (1 mL). The resulting reaction mixture was stirred at 40 °C overnight. After cooling, the volatiles were removed under reduced pressure. The residue was purified by silica gel column chromatography using 0 to 5% MeOH in dichloromethane as eluent to give ***Int-139b.*** LCMS anal. calcd. for C₁₉H₁₈N₆O₃: 378.2; Found: 379.3 (M+H)⁺.

### Step C - Synthesis of Compound Int-139c

Wilkinson's catalyst (0.012 g, 1.74 mmol,) was added to a solution of ***Int-139b*** (0.024 g, 0.063 mmol) in a mixture of THF (1 mL) and methanol (1 mL). The resulting reaction mixture was stirred at room temperature under an atmosphere of hydrogen overnight. The volatiles were removed under reduced pressure. The residue was purified by silica gel column chromatography using 0 to 5% MeOH in dichloromethane as eluent to provide ***Int-139c.*** LCMS anal. calcd. for C₁₉H₂₀N₆O₃: 380.3; Found: 381.3 (M+H)⁺.

### Step D - Synthesis of Compound 139.

Following essentially the method employed to produce compounds ***135A*** and ***135B,*** compound ***139*** was prepared. LCMS anal. calcd. for C₁₈H₁₈N₆O₃: 366.2; Found: 367.3(M+H)⁺.

### Example 25

### Preparation of Compound 140

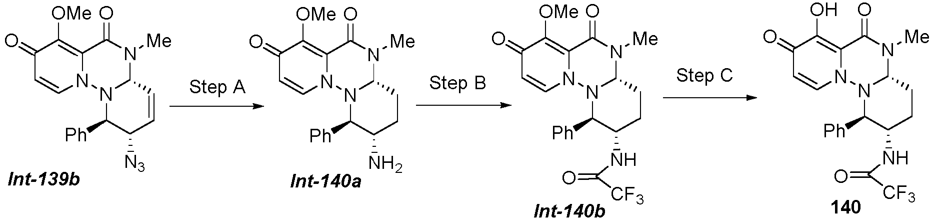

### Step A - Synthesis of Compound Int-140a

Pd-C (10%, 10 mg) was added to a solution of ***Int-139b*** (19.5 mg, 0.052 mmol) in methanol (3 mL). The resulting black suspension was placed under an atmosphere of hydrogen (balloon) overnight. The reaction was filtered through a pad of celite. The solid was thoroughly washed with methanol. The combined filtrate was concentrated under reduced pressure to provide ***Int-140a**.* LCMS anal. calcd. for C₁₉H₂₂N₄O₃: 354.2; Found: 355.3 (M+H)⁺.

### Step B - Synthesis of Compound Int-140b

Trifluoroacetic anhydride (0.007 mL, 0.053 mmol) was added to a solution of ***Int-140a*** (12.5 mg, 0.035 mmol) and triethylamine (0.009 mL, 0.07 mmol) in anhydrous dichloromethane (1 mL). The resulting reaction mixture was stirred at room temperature overnight. The volatiles were removed under reduced pressure. The residue was purified by silica gel column chromatography using 0 to 10% MeOH in dichloromethane as eluent to provide ***Int-140b.*** LCMS anal. calcd. for C₂₁H₂₁F₃N₄O₄: 450.2; Found: 451.3 (M+H)⁺.

### Step C - Synthesis of Compound 140.

Following essentially the method employed to produce compounds ***135A*** and ***135B,*** compound ***140*** was prepared. LCMS anal. calcd. for C₂₀H₁₉F₃N₄O₄: 436.2; Found: 437.3 (M+H)⁺.

### Example 26

### Preparation of Compound 141

### (1R,2S,4aS)-7-hydroxy-2-(4-(hydroxymethyl)-1H-1,2,3-triazol-1-yl)-5-methyl-1-phenyl-1,2,3,4,4a,5-hexahydrodipyrido[1,2-b:2',1'-f][1,2,4]triazine-6,8-dione

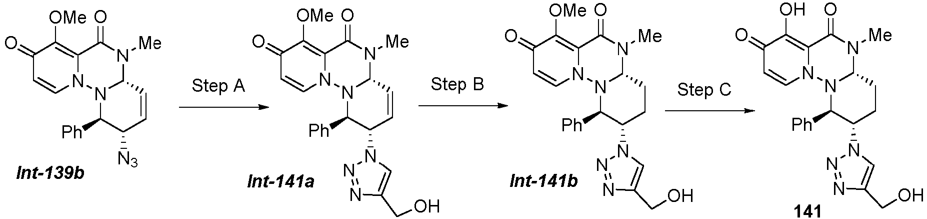

### Step A - Synthesis of Compound Int-141a

A mixture of copper (II) sulfate (264mg, 1.05 mmol) and sodium (*R*)-2-((*S*)-1,2-dihydroxyethyl)-4-hydroxy-5-oxo-2,5-dihydrofuran-3-olate (2.094 mg, 10.57 µmol) in water (1 mL) was added to a mixture of ***Int-5b*** (40.0 mg, 0.106 mmol) and propargyl alcohol (6.79 mL, 0.116 mmol) in ^{t}BuOH (1 mL) at room temperature. The resulting reaction mixture was stirred overnight. Additional portions of copper (II) sulfate (264mg, 1.05 mmol) and sodium (*R*)-2-((*S*)-1,2-dihydroxyethyl)-4-hydroxy-5-oxo-2,5-dihydrofuran-3-olate (2.094 mg, 10.57 µmol) were added in water (1 mL) and stirring was continued for a further 24 hrs. The volatiles were removed under reduced pressure. The residue was purified by silica gel column chromatography using 0 to 10% MeOH in dichloromethane as eluent to provide ***Int-141a.*** LCMS anal. calcd. for C₂₂H₂₂N₆O₄: 434.2; Found: 435.3 (M+H)⁺.

### Step B - Synthesis of Compound Int-141b

Pd-C (10%, 10 mg) was added to a solution of ***Int-141a*** (20 mg, 0.046 mmol) in methanol (3 mL). The resulting suspension was placed under an atmosphere of hydrogen (balloon) overnight. The reaction was filtered through a pad of celite and was thoroughly washed with methanol. The combined filtrate was concentrated under reduced pressure to provide ***Int-141b.*** LCMS anal. calcd. for C₂₂H₂₄N₆O₄: 436.2; Found: 437.3 (M+H)⁺.

### Step C - Synthesis of Compound 141.

Following essentially the method employed to produce compounds ***135A*** and ***135B,*** compound ***141*** was prepared. LCMS anal. calcd. for C₂₁H₂₂N₆O₄: 422.2; Found: 423.3 (M+H)⁺.

### Example 27

### Preparation of Compound 142

### (1R,2S,4aS)-2-azido-7-hydroxy-5-methyl-1-phenyl-1,2,3,4,4a,5-hexahydrodipyrido[1,2-b:2',1'-f][1,2,4]triazine-6,8-dione

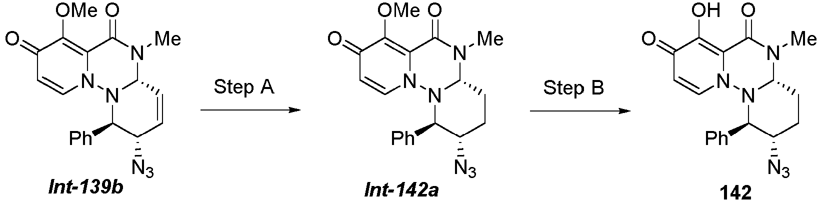

### Step A - Synthesis of Compound Int-142a

Wilkinson's catalyst (12 mg, 0.013mmol) was added to a solution of ***Int-139b*** (24 mg, 0.063 mmol) in methanol (1 mL) and THF (1mL). The resulting mixture was placed under an atmosphere of hydrogen (balloon) overnight. The volatiles were removed under reduced pressure. The residue was purified by reverse phase HPLC using 0-100% ACN in water as eluent (0.05% TFA as the modifier). The combined fractions were concentrated under reduced pressure to provide ***Int-142a**.* LCMS anal. calcd. for C₁₉H₂₀N₆O₃: 380.2; Found: 381.3 (M+H)⁺.

### Step B - Synthesis of Compound 142

Following essentially the method employed to produce compounds ***135A*** and ***135B,*** compound ***142*** was prepared. LCMS anal. calcd. for C₁₈H₁₈N₆O₃: 366.1; Found: 367.3 (M+1)⁺.

### Example 28

### Preparation of Compounds 143A and 143B

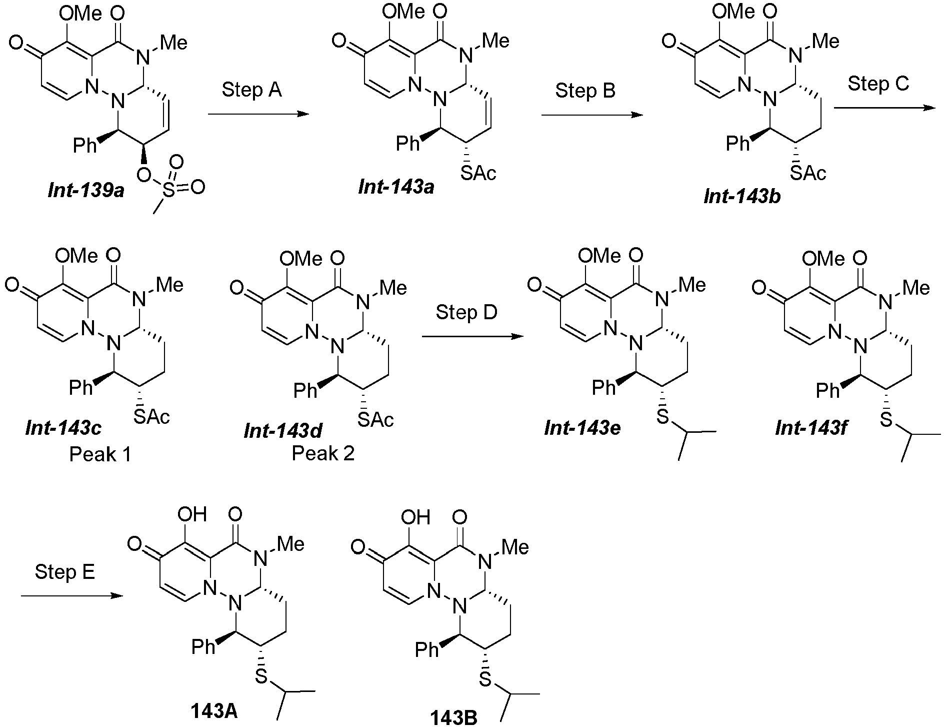

### Step A - Synthesis of Compound Int-143a

Potassium thioacetate (338 mg, 2.96 mmol) was added to a solution **of *Int-139a*** (426 mg, 0.987 mmol) in anhydrous DMF (25 mL) and THF (1 mL). The resulting mixture was heated to 40 °C under an atmosphere of nitrogen for 4 hrs. The volatiles were removed under reduced pressure. The residue was purified by silica gel column chromatography using 0 to 10% MeOH in dichloromethane as eluent to provide ***Int-143a.*** LCMS anal. calcd. for C₂₁H₂₁N₃O₄S: 411.1; Found: 411.2 (M+H)⁺.

### Step B - Synthesis of Compound Int-143b

Wilkinson's catalyst (112 mg, 0.122 mmol) was added to a solution of ***Int-143a*** (50 mg, 0.122 mmol) in methanol (4 mL) and THF (4mL). The resulting mixture was placed under an atmosphere of hydrogen (balloon) overnight. The volatiles were removed under reduced pressure. The residue was purified by silica gel column chromatography using 0 to 5% MeOH in dichloromethane as eluent to provide ***Int-143b.*** LCMS anal. calcd. for C₂₁H₂₃N₃O₄S: 413.1; Found: 414.2 (M+H)⁺.

### Step C - Preparation of Compounds Int-143c and Int-143d.

***Int-143b*** (0.016 g) was resolved by SFC using an AD-H column (21 x 250mm) and 30% EtOH as co-solvent to provide ***Int-9c*** (Peak 1) and ***Int-9d*** (Peak 2). LCMS anal. calcd. for C₂₁H₂₃N₃O₄S: 413.1; Found: 414.2 (M+H)⁺.

### Step D - Synthesis of Compounds Int-143e and Int-143f

Aqueous 1 M sodium hydroxide (0.034 ml, 0.034 mmol) was added to a solution of ***Int-143c*** (7 mg, 0.017 mmol) in THF (1 mL). The resulting mixture was heated to 40 °C overnight. The volatiles were removed under reduced pressure to provide ***Int-143e.*** The residue was used in the next step without purification. LCMS anal. calcd. for C₂₂H₂₇N₃O₃S: 413.2; Found: 414.2 (M+H)⁺.

Following essentially the method employed to produce compound ***Int-143e,*** compound ***Int-143f*** was prepared. LCMS anal. calcd. for C₂₂H₂₇N₃O₃S: 413.2; Found: 414.2 (M+1)⁺.

### Step E - Synthesis of Compounds 143A and 143B

Following essentially the method employed to produce compounds ***135A*** and ***135B,*** compounds ***143A*** and ***143B*** were prepared. LCMS anal. calcd. for C₂₁H₂₅N₃O₃S: 399.1; Found: 400.1 (M+H)⁺.

### Example 29

### Preparation of Compounds 144 and 145

### N-((1S,4S,4aS)-7-hydroxy-5-methyl-6,8-dioxo-1-phenyl-1,2,3,4,4a,5,6,8-octahydrodipyrido[1,2-b:2',1'-f][1,2,4]triazin-4-yl)acetamide and N-((1S,4S,4aS)-7-hydroxy-5-methyl-6,8-dioxo-1-phenyl-1,2,3,4,4a,5,6,8-octahydrodipyrido[1,2-b:2',1'-f][1,2,4]triazin-4-yl)-N-methylacetamide

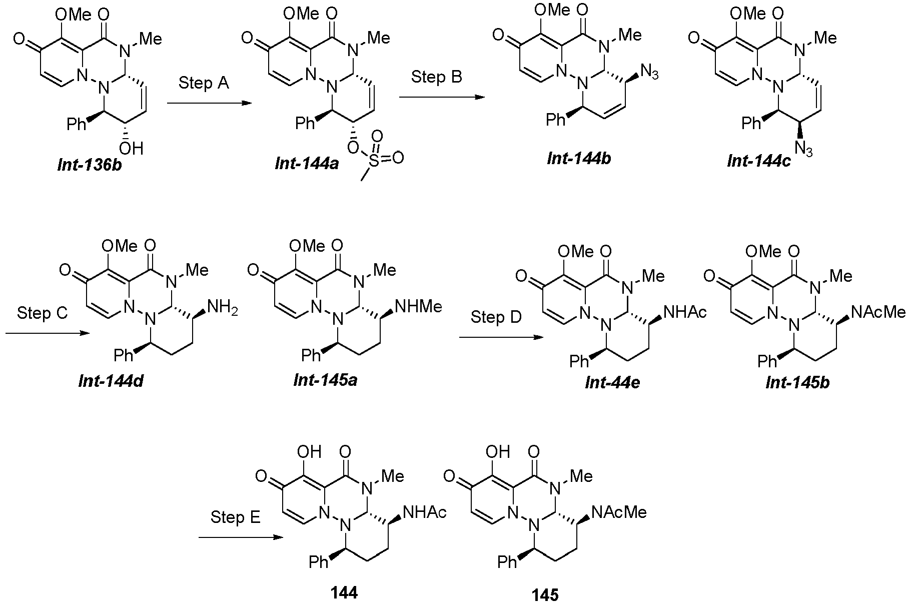

### Step A - Synthesis of Compound Int-144a

Triethylamine (0.059 mL, 0.424 mmol) was added to a solution of ***Int-136b*** (0.250 g, 0.707 mmol) and methylsulfonyl chloride (0.022 mL, 0.283 mmol) in anhydrous dichloromethane (2 mL). The resulting reaction mixture was stirred at room temperature for 2 hrs. The volatiles were removed under reduced pressure. The residue was purified by silica gel column chromatography using 0 to 10% MeOH in dichloromethane as eluent to provide ***Int-144a.*** LCMS anal. calcd. for C₂₀H₂₁N₃O₆S: 431.2; Found: 432.6 (M+H)⁺.

### Step B - Synthesis of Compound Int-144b and Compound Int-144c

Sodium azide (25.8 mg, 0.396 mmol) was added to a solution of ***Int-144a*** (57 mg, 0.707 mmol) in anhydrous DMF (2 mL). The resulting reaction mixture was stirred at 40 °C overnight. The volatiles were removed under reduced pressure. The residue was purified by reverse phase HPLC using 0-100% ACN in water as eluent (0.05% TFA as the modifier) to provide ***Int-144b*** and ***Int-144c.*** LCMS anal. calcd. for C₁₉H₁₈N₆O₃ 378.1; Found: 379.2 (M+H)⁺.

### Step C - Synthesis of Compound Int-144d and Int-145a

Pd-C (10%, 5 mg) was added to a solution of ***Int-144b*** (14 mg, 0.037 mmol) in methanol (3 mL). The resulting suspension was placed under an atmosphere of hydrogen (balloon) overnight. The reaction was filtered through a pad of celite and the solid was thoroughly washed with methanol. The combined filtrate was concentrated under reduced pressure to give a mixture of ***Int-144d*** and ***Int-145a.*** It was used in the next step without purification.

### Step D - Synthesis of Compound Int-144e and Int-145b

Acetic anhydride (1 drop from a pipette) was added to a mixture of ***Int-144d*** and ***145a** (13* mg) and triethylamine (0.05 mL) in anhydrous dichloromethane (1 mL). The resulting reaction mixture was stirred at room temperature overnight. The reaction was concentrated under reduced pressure. The residue was purified by reverse phase HPLC using 0-100% ACN in water as eluent (0.05% TFA as modifier) to provide ***Int-144e,*** LCMS anal. calcd. for C₂₁H₂₄N₄O₄: 386.2; Found: 397.2 (M+H)⁺, and ***Int-145b,*** LCMS anal. calcd. for C₂₂H₂₆N₄O₄: 410.2; Found: 411.3 (M+H)⁺, respectively.

### Step E - Synthesis of Compounds 144 and 145

Following essentially the method employed to produce compounds ***135A*** and ***135B,*** compounds ***144*** and ***145*** were prepared. ***144:*** LCMS anal. calcd. for C₂₀H₂₂N₄O₄: 382.2; Found: 383.3 (M+1)⁺. ***145*:** LCMS anal. calcd. for C₂₁H₂₄N₄O₄: 396.2; Found: 397.3 (M+H)⁺.

### Example 30

### Preparation of Compounds 146A and 146B

### 2-(isopropylthio)-7-methoxy-5-methyl-1-phenyl-1,2,3,4,4a,5-hexahydrodipyrido[1,2-b:2',1'-f][1,2,4]triazine-6,8-dione

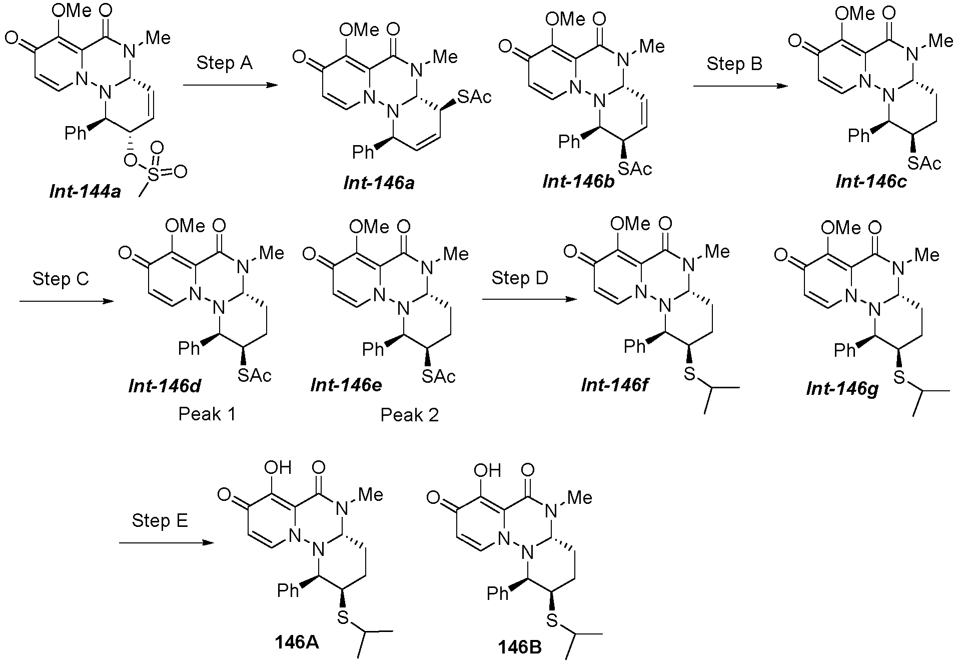

### Step A - Synthesis of Compounds Int-146a and Int-146b

Potassium thioacetate (144 mg, 1.259 mmol) was added to a solution of ***Int-144a** (181* mg, 0.420 mmol) in anhydrous DMF (8 mL). The resulting reaction mixture was stirred at 40 °C for 4 hrs. After cooling, the volatiles were removed under reduced pressure. The residue was purified by silica gel column chromatography using 0 to 10% MeOH in dichloromethane as eluent to provide ***Int-146a*** and ***Int-146b.*** LCMS anal. calcd. for C₂₁H₂₁N₃O₄S: 411.2; Found: 412.3 (M+H)⁺.

### Step B - Synthesis of Compound Int-146c

Wilkinson's catalyst (117 mg, 0.126 mmol) was added to a solution of ***Int-146b*** (52 mg, 0.126 mmol) in methanol (4 mL) and THF (4 mL). The resulting mixture was placed under an atmosphere of hydrogen (balloon) overnight. The volatiles were removed under reduced pressure. The residue was purified by silica gel column chromatography using 0 to 10% MeOH in dichloromethane as eluent to provide ***Int-146c.*** LCMS anal. calcd. for C₂₁H₂₃N₃O₄S: 413.2; Found: 414.3 (M+H)⁺.

### Step C - Preparation of Compounds Int-146d and Int-146e.

***Int-146c*** (0.048 g) was resolved by SFC using an OD column (21 x 250mm) and 35% EtOH as co-solvent to provide ***Int-146d*** (Peak 1) and ***Int-146e*** (Peak 2). LCMS anal. calcd. for C₂₁H₂₃N₃O₄S: 413.2; Found: 414.3 (M+H)⁺.

### Step D - Synthesis of Compounds Int-146f and Int-146g

Aqueous 1 M sodium hydroxide (0.076 mL, 0.076 mmol) was added to a solution of ***Int-146d*** (15.7 mg, 0.038 mmol) and isopropyl iodide (4 mL, 0.038 mmol) in THF (1 mL). The resulting mixture was stirred for overnight. The volatiles were removed under reduced pressure to provide ***Int-146f*** The residue was used in the next step without purification. LCMS anal. calcd. for C₂₂H₂₂N₃O₃S: 413.2; Found: 414.3 (M+H)⁺.

Following essentially the method employed to produce compound ***Int-146f,*** compound ***Int-146g*** was prepared. LCMS anal. calcd. for C₂₂H₂₂N₃O₃S: 413.2; Found: 414.3 (M+H)⁺.

### Step E - Synthesis of Compound 146A and 146B

Following essentially the method employed to produce compounds ***135A*** and ***135B,*** compounds ***146A*** and ***146B*** were prepared. LCMS anal. calcd. for C₂₁H₂₅N₃O₃S: 399.2; Found: 400.2 (M+H)⁺.

### Example 31

### Preparation of Compound 147

### (1S,4S,4aS)-7-hydroxy-5-methyl-4-(methylthio)-1-phenyl-1,2,3,4,4a,5-hexahydrodipyrido[1,2-b:2',1'-f][1,2,4]triazine-6,8-dione

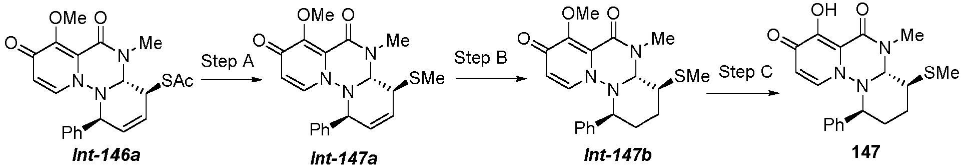

### Step A - Synthesis of Compounds Int-147a

Aqueous 1 M sodium hydroxide (0.170 mL, 0.170 mmol) was added to a solution of ***Int-146a*** (35 mg, 0.085 mmol) and iodomethane (11 mL, 0.170 mmol) in THF (2 mL). The resulting mixture was heated at 40 °C overnight. The volatiles were removed under reduced pressure. The residue was used in the next step without purification. LCMS anal. calcd. for C₂₀H₂₁N₃O₃S: 383.1; Found: 384.7 (M+H)⁺.

### Step B - Synthesis of Compound Int-147b

Wilkinson's catalyst (74.8 mg, 0.081 mmol) was added to a solution of ***Int-147a*** (Crude product from Step A) in methanol (3 mL) and THF (3 mL). The resulting mixture was placed under an atmosphere of hydrogen (balloon) overnight. The volatiles were removed under reduced pressure. The residue was purified by reverse phase HPLC using 0-100% ACN in water as eluent (0.05% TFA as modifier) to provide ***Int-147b.*** LCMS anal. calcd. for C₂₀H₂₃N₃O₃S: 385.2; Found: 386.2 (M+H)⁺.

### Step C - Synthesis of Compound 147

Following essentially the method employed to produce compounds ***135A*** and ***135B,*** compound ***147*** was prepared. LCMS anal. calcd. for C₁₉H₂₁N₃O₃S: 371.2; Found: 372.3 (M+H)⁺.

### Example 32

### Preparation of Compound 148

### 7-hydroxy-5,12a-dimethyl-2a,3,4,4a,5,12a-hexahydro-1H-isochromeno[3',4':5,6]pyrido[1,2-b]pyrido[2,1-f][1,2,4]triazine-6,8-dione

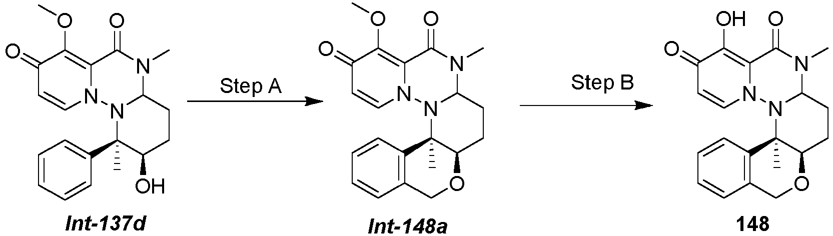

### Step A - Synthesis of Compounds Int-148a

Zinc Chloride (29.5 mg, 0.217 mmol) was added to a solution of ***Int-137d*** (20 mg, 0.054 mmol) and MOMCl (0.107 mL, 1.41 mmol) in anhydrous dichloromethane (1 mL). The resulting reaction mixture was stirred at room temperature overnight. The volatiles were removed under reduced pressure. The residue was purified by silica gel column chromatography using 0 to 10% MeOH in dichloromethane as eluent to provide ***Int-148a.*** LCMS anal. calcd. for C₂₁H₂₃N₃O₄: 381.2; Found: 382.3 (M+H)⁺.

### Step B - Synthesis of Compound 148.

Following essentially the method employed to produce compounds ***135A*** and ***135B,*** compound ***148*** was prepared. LCMS anal. calcd. for C₁₈H₁₉N₃O₃S: 367.2; Found: 368.3 (M+H)⁺.

### Example 33

### Preparation of Compound 149

### (1R,2R)-4-((E)-benzylidene)-2,7-dihydroxy-1,5-dimethyl-1-phenyl-1,2,3,4,4a,5-hexahydrodipyrido[1,2-b:2',1'-f][1,2,4]triazine-6,8-dione

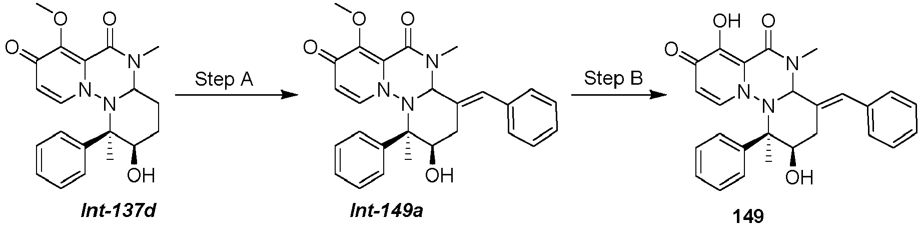

### Step A - Synthesis of Compound Int-149a

*p*-TsOH (5.15 mg, 0.03 mmol) was added to a solution of ***Int-137d*** (20 mg, 0.054 mmol) and benzaldehyde (0.100 mL, 0.987 mmol) in anhydrous 1,2-dichloroethane (1 mL) in a 5 mL microwave vial. The vial was capped and placed in a microwave reactor at 120 °C for 2 hrs. After cooling, the volatiles were removed under reduced pressure. The residue was purified by silica gel column chromatography using 0 to 10% MeOH in dichloromethane as eluent to provide ***Int-149a.*** LCMS anal. calcd. for C₂₇H₂₇N₃O₄: 457.2; Found: 458.4 (M+H)⁺.

### Step B - Synthesis of Compound 149

Following essentially the method employed to produce compounds ***135A*** and ***135B,*** compound ***148*** was prepared. LCMS anal. calcd. for C₂₆H₂₅N₃O₄: 443.2; Found: 444.3 (M+H)⁺.

### Example 34

### Preparation of Compound 150

### Cis-16-hydroxy-2-methyl-2,2a,3,4,4a,5,6,10b-octahydrobenzo[h]pyrido[1',2':1,6][1,2,4]triazino[2,3-a]quinoline-1,15-dione

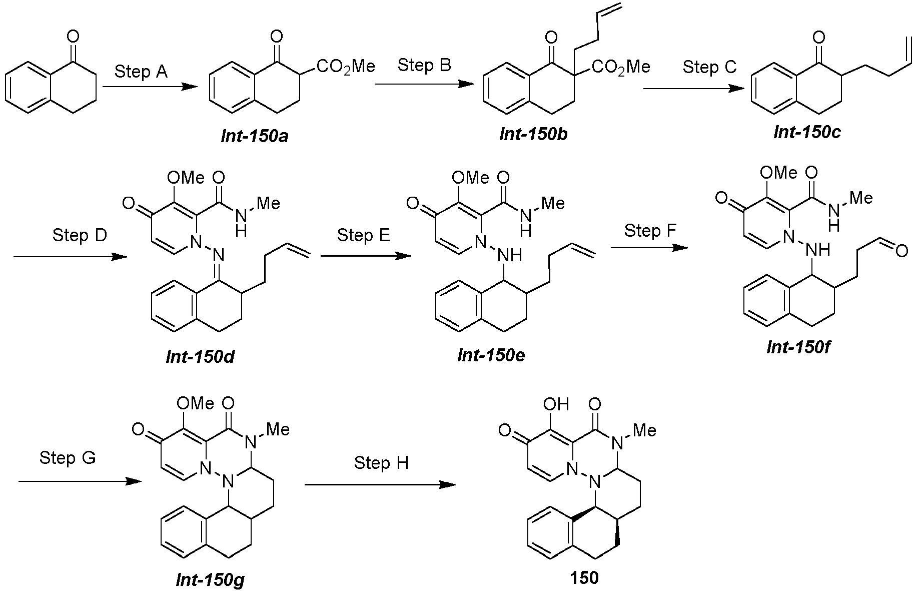

### Step A - Synthesis of Compound Int-150a

Tetralone (2.66 mL, 20.0 mmol) in anhydrous THF (20 mL) was added to sodium hydride (2.48g, 60% suspension in mineral oil, 62.0 mmol) under an atmosphere of nitrogen. The resulting mixture was stirred at room temperature for 1 h. A solution of dimethyl carbonate (4.25 mL, 50.0 mmol) in anhydrous THF (20 mL) was added dropwise while the reaction was being heated to 80 °C. When the addition was complete, the reaction was maintained at the temperature for 2 hrs. After cooling, the mixture was partitioned between dichloromethane and aq. 1N HCl. The aqueous phase was separated and further extracted with dichloromethane (2x). The combined organic phases were dried (MgSO₄). The volatiles were removed under reduced pressure. The residue was purified by silica gel column chromatography using 0 to 10% EtOAc in hexanes as eluent to provide ***Int-150a.*** LCMS anal. calcd. for C₁₂H₁₂O₃: 204.1; Found: 205.1 (M+H)⁺.

### Step B - Synthesis of Compound Int-150b

***Int-150a*** (1.60g, 7.83 mmol) in anhydrous DMF (10 mL) was added to sodium hydride (0.94 g, 60% suspension in mineral oil, 23.5 mmol) under an atmosphere of nitrogen. The resulting mixture stirred at room temperature for 1 h, and 4-bromobut-1-ene was added in one portion. The resulting reaction mixture was stirred at 40 °C overnight. After cooling, the mixture was partitioned between dichloromethane and sat. aq. ammonium chloride. The aqueous phase was separated and further extracted with dichloromethane (2x). The combined organic phases were dried (MgSO₄), filtered and concentrated. The residue was purified by silica gel column chromatography with 0-100% EtOAc in hexane as eluent to provide ***Int-150b.*** LCMS anal. calcd. for C₁₆H₁₈O₃: 258.1; Found: 259.2 (M+H)⁺.

### Step C - Synthesis of Compound Int-150c

Aqueous 1.0 M sodium hydroxide (9.68 ml, 9.68 mmol) was added to a solution of ***Int-150b*** (0.500g, 1.94 mmol) in THF (15 mL). The resulting reaction mixture was heated at 100 °C overnight. After cooling, the mixture was partitioned between dichloromethane and aq. 1N HCl. The aqueous phase was separated and further extracted with dichloromethane (2x). The combined organic phases were dried (MgSO₄) and the volatiles were removed under reduced pressure. The residue was purified by silica gel column chromatography using 0 to 10% EtOAc in hexanes as eluent to give ***Int-150c.*** LCMS anal. calcd. for C₁₄H₁₆O: 200.1; Found: 201.2 (M+H)⁺.

### Step D - Synthesis of Compound Int-150d

Triflic acid (0.141 mL, 1.59 mmol) was added to a solution of 1-amino-3-methoxy-N-methyl-4-oxo-1,4-dihydropyridine-2-carboxamide (0.157 g, 0.80 mmol), ***Int-150c*** (0.159 g, 0.80 mmol) and palladium diacetate (0.65 mg, 2.89 mmol) in anhydrous dioxane (5 mL) under an atmosphere of nitrogen. The resulting reaction mixture was stirred at room temperature overnight. The volatiles were removed under reduced pressure. The residue was purified by silica gel column chromatography using 0 to 10% MeOH in dichloromethane as eluent to provide ***Int-150d**.* LCMS anal. calcd. for C₂₂H₂₅N₃O₃: 379.2; Found: 380.3 (M+H)⁺.

### Step E - Synthesis of Compound Int-150e

Sodium borohydride (61.8 mg, 1.63 mmol) was added to a solution of ***Int-150d*** (0.062 g, 0.334 mmol) in methanol (1 mL) under an atmosphere of nitrogen. The resulting reaction mixture was stirred at room temperature overnight. The volatiles were removed under reduced pressure. The residue was purified by silica gel column chromatography using 0 to 10% MeOH in dichloromethane as eluent to provide ***Int-150e.*** LCMS anal. calcd. for C₂₂H₂₇N₃O₃: 381.2; Found: 382.4 (M+H)⁺.

### Step F - Synthesis of Compound Int-150f

Osmium tetroxide (9 mL, 4% solution in ^{t}BuOH, 1.41 mmol) followed by sodium periodate (45.4 mg, 0.212 mmol) was added to a solution of ***Int-150e*** (27 mg, 0.07 mmol) in a mixture of THF (1 mL) and water (0.3 mL) at room temperature under an atmosphere of nitrogen. The resulting reaction mixture was stirred overnight then quenched by the addition of aq. 10% sodium thiosulfate and stirred for 1 h. The volatiles were removed under reduced pressure. The residue was thoroughly extracted with 20% MeOH in dichloromethane (50 mL). The combined extract was dried (sodium sulfate). The volatiles removed under reduced pressure. The residue was purified by silica gel column chromatography using 0 to 10% MeOH in dichloromethane as eluent to provide ***Int-150f.*** LCMS anal. calcd. for C₂₁H₂₅N₃O₄: 383.2; Found: 384.4 (M+H)⁺.

### Step G - Synthesis of Compound Int-150g

***Int-150f** (13* mg, 0.034 mmol) in a mixture of DMF (1 mL) and AcOH (0.1 mL) was heated at 120 °C for 1 h. After cooling, the volatiles were removed under reduced pressure. The residue was purified by silica gel column chromatography using 0 to 10% MeOH in dichloromethane as eluent to provide ***Int-150g.*** LCMS anal. calcd. for C₂₁H₂₃N₃O₃: 365.2; Found: 366.1 (M+H)⁺.

### Step H - Synthesis of Compound 150.

Following essentially the method employed to produce compounds ***135A*** and ***135B,*** compound ***150*** was prepared. LCMS anal. calcd. for C₂₀H₂₁N₃O₃: 351.2; Found: 352.1 (M+1)⁺.

### Example 35

### Preparation of Compound 151

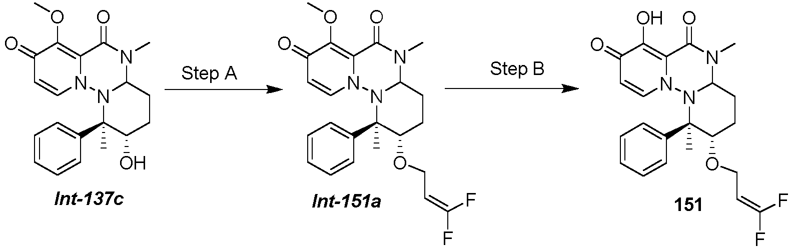

### Step A - Synthesis of Compound Int-151a

***Int-137c*** (20 mg, 0.054 mmol) was added to a suspension of sodium hydride (6.5 mg, 60% suspension in mineral oil, 0.162 mmol) in anhydrous THF under an atmosphere of nitrogen. The resulting mixture was stirred at room temperature for 1 h. 3-Bromo-3,3-difluoroprop-1-ene (8.5 mg (0.054 mmol) in anhydrous THF (0.5ml) was added to above reaction. The resulting reaction mixture was stirred overnight. Upon completion, it was quenched by the addition of sat. aq. ammonium chloride (0.5 ml). The volatiles removed under reduced pressure. The residue was purified by reverse phase HPLC to provide ***Int-151a.*** LCMS anal. calcd. for C₂₃H₂₅F₂N₃O₄: 445.2; Found: 446.4 (M+H)⁺.

### Step B - Synthesis of Compound 151

Following essentially the method employed to produce compounds ***135A*** and ***135B,*** compound ***151*** was prepared. LCMS anal. calcd. for C₂₂H₂₃F₂N₃O₄: 431.2; Found: 432.3 (M+1)⁺.

### Example 36

### Preparation of Compounds 152A and 152B

### 2-ethoxy-7-hydroxy-5-methyl-1-phenyl-1,2,3,4,4a,5-hexahydrodipyrido[1,2-b:2',1'-f][1,2,4]triazine-6,8-dione

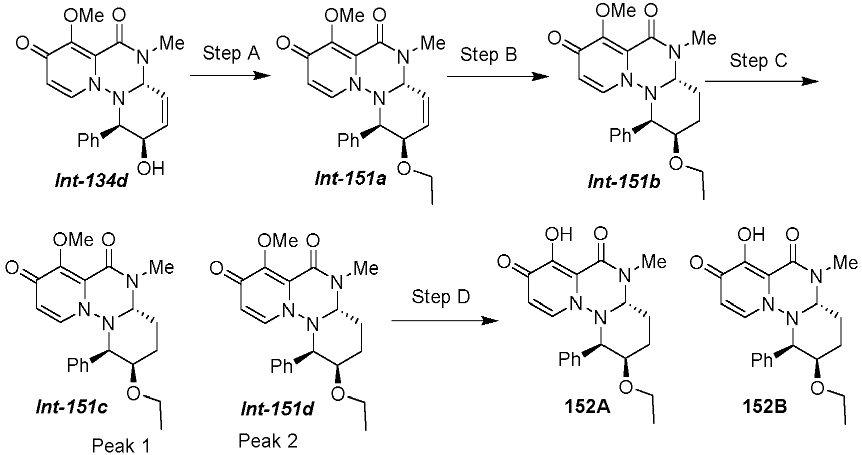

### Step A - Synthesis of Compound Int-151a

Sodium hydride (5.7 mg, 60% suspension in mineral oil, 0.143 mmol) was added to a solution of ***Int-134d*** (50 mg, 0.142 mmol) in anhydrous DMF (2.4 mL) cooled in an ice bath under an atmosphere of nitrogen. The resulting mixture was stirred at room temperature for 10 min. Iodoethane (0.024 mL, 0.297 mmol) was added. The resulting reaction mixture was allowed to warm to room temperature for 3 hrs. The mixture was partitioned between EtOAc and water. The organic phase was separated, washed with brine, dried (sodium sulfate). The volatiles were removed under reduced pressure. The residue was purified by silica gel column chromatography using 0 to 10% MeOH in dichloromethane as eluent to provide ***Int-151a.*** LCMS anal. calcd. for C₂₁H₂₃N₃O₄: 381.2; Found: 382.2 (M+H)⁺.

### Step B - Synthesis of Compound Int-151b

Pd-C (10%, 12.6 mg) was added to a solution of ***Int-151a*** (45 mg, 0.118 mmol) in MeOH (2.4 mL). The resulting suspension was placed under an atmosphere of hydrogen (balloon) for 5 hrs. The mixture was filtered and washed with MeOH (1 mL). The filtrate was concentrated to provide ***Int-151b*** which was used in the next step without further purification.

### Step C - Preparation of Compounds Int-151c and Int-151d

***Int-151b*** (crude from step B) was separated by SFC using an AD-H column (21 X 250 mm) and 20% MeOH as co-solvent to provide ***Int-151c*** and ***Int-151d.*** LCMS anal. calcd. for C₂₁H₂₅N₃O₄: 383.2; Found: 384.3 (M+H)⁺.

### Step D - Synthesis of Compound 152A and 152B

Lithium chloride (1.66 mg, 0.039 mmol) was added to a solution of ***Int-151c*** (3 mg, 7.82 mmol) in DMF (0.16 mL). The resulting reaction mixture was heated at 100 °C for 3 hrs. After cooling, the solution was directly purified by reverse phase HPLC using 0-100% ACN in water as eluent (0.05% TFA as modifier) to provide ***152A.*** LCMS anal. calcd. for C₂₀H₂₃N₃O₄: 369.2; Found: 370.3 (M+H)⁺.

Following essentially the method employed to produce compound ***152A,*** compound ***152B*** was prepared. LCMS anal. calcd. for C₂₀H₂₃N₃O₄: 369.2; Found: 370.3 (M+H)⁺.

The examples in **Table 6** were prepared using procedures similar to those described in **Example 36,** from the appropriate starting material.

**Table 6**

| **Example** | **Structure** | **Observed *m*/*z* [M + H]⁺** |
|---|---|---|
| | **Name** | |
| | **Precursor SFC Separation Condition** | |
| **153A (Peak1)** | 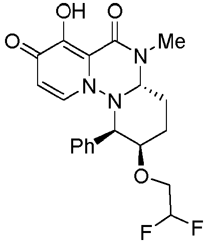 | 406.3 |
| **153B (Peak2)** | | |
| | (1R,2R,4aS)-2-(2,2-difluoroethoxy)-7-hydroxy-5-methyl-1-phenyl-1,2,3,4,4a, 5-hexahydrodipyrido[1,2-b:2',1'-f][1,2,4]triazine-6,8-dione | |
| | Precursor was separated SFC. SFC conditions: Daicel@ chiralpak AD-H column (250*21 mm, 10 µm); 20% MeOH; 70 mL/min | |
| **154** | 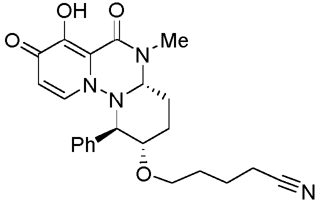 | 423.2 |
| | 5-(((1*R*,2*S*,4a*S*)-7-hydroxy-5-methyl-6,8-dioxo-1-phenyl-1,2,3,4,4a,5,6,8-octahydrodipyrido[1,2-b:2',1'-f][1,2,4]triazin-2-yl)oxy)pentanenitrile | |
| ***155*** | 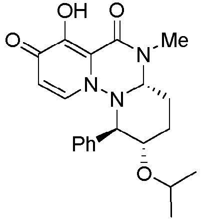 | 384.2 |
| | (1*R*,2*S*,4a*S*)-7-hydroxy-2-isopropoxy-5-methyl-1-phenyl-1,2,3,4,4a,5-hexahydrodipyrido[1,2-b:2',1'-f][1,2,4]triazine-6,8-dione | |
| **156** | 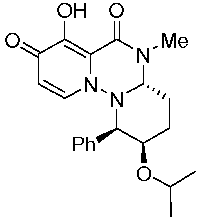 | 384.2 |
| | (1*R*,2*R*,4a*S*)-7-hydroxy-2-isopropoxy-5-methyl-1-phenyl-1,2,3,4,4a,5-hexahydrodipyrido[1,2-b:2',1'-f][1,2,4]triazine-6,8-dione | |
| **157** | 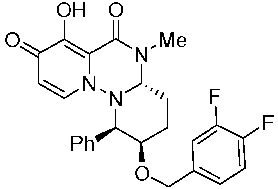 | 468.2 |
| | (1*R*,2*R*,4a*S*)-2-((3,4-difluorobenzyl)oxy)-7-hydroxy-5-methyl-1-phenyl-1,2,3,4,4a,5-hexahydrodipyrido[1,2-b:2',1'-f][1,2,4]triazine-6,8-dione | |
| **158** | 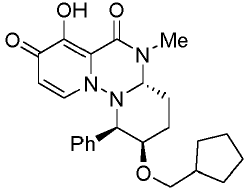 | 424.2 |
| | (1*R*,2*R*,4a*S*)-2-(cyclopentylmethoxy)-7-hydroxy-5-methyl-1-phenyl-1,2,3,4,4a,5-hexahydrodipyrido[1,2-b:2',1'-f][1,2,4]triazine-6,8-dione | |
| **159** | 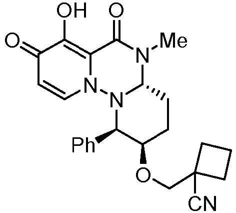 | 435.2 |
| | 1-((((1*R*,2*R*,4a*S*)-7-hydroxy-5-methyl-6,8-dioxo-1-phenyl-1,2,3,4,4a,5,6,8-octahydrodipyrido[1,2-b:2',1'-f][1,2,4]triazin-2-yl)oxy)methyl)cyclobutane-1-carbonitrile | |
| **160** | 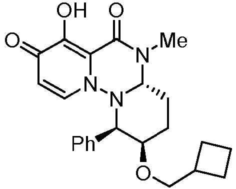 | 410.2 |
| | (1*R*,2*R*,4a*S*)-2-(cyclobutylmethoxy)-7-hydroxy-5-methyl-1-phenyl-1,2,3,4,4a,5-hexahydrodipyrido[1,2-b:2',1'-f][1,2,4]triazine-6,8-dione | |
| **161** | 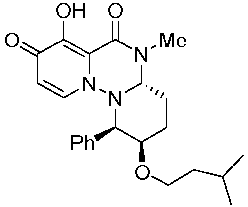 | 412.2 |
| | (1*R*,2*R*,4a*S*)-7-hydroxy-2-(isopentyloxy)-5-methyl-1-phenyl-1,2,3,4,4a,5-hexahydrodipyrido[1,2-b:2',1'-f][1,2,4]triazine-6,8-dione | |
| **162** | 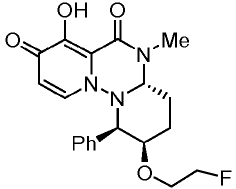 | 388.2 |
| | (1*R*,2*R*,4a*S*)-2-(2-fluoroethoxy)-7-hydroxy-5-methyl-1-phenyl-1,2,3,4,4a,5-hexahydrodipyrido[1,2-b:2',1'-f][1,2,4]triazine-6,8-dione | |
| **163** | 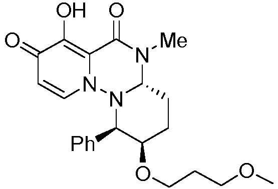 | 414.2 |
| | (1*R*,2*R*,4a*S*)-7-hydroxy-2-(3-methoxypropoxy)-5-methyl-1-phenyl-1,2,3,4,4a,5-hexahydrodipyrido[1,2-b:2',1'-f][1,2,4]triazine-6,8-dione | |

### Example 38

### Preparation of Compounds 165A and 165B

### 1,7-dihydroxy-5-methyl-1-phenyl-1,3,4,4a,5,11a-hexahydro-2H-pyrido[1,2-a]quinoxaline-6,8-dione

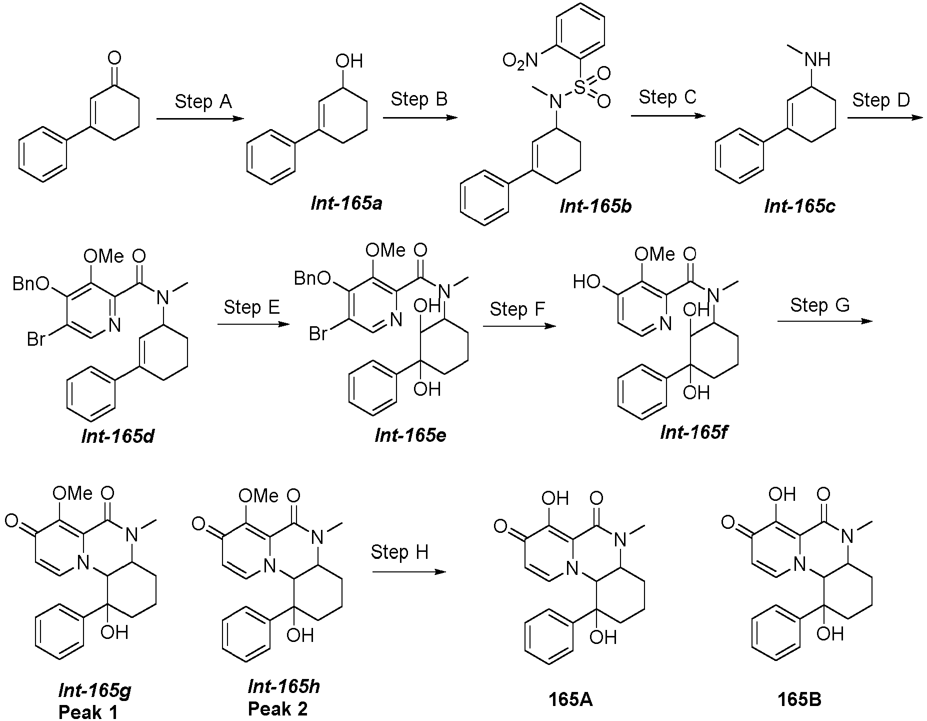

### Step A - Synthesis of Compound Int-165a

Sodium borohydride (52.7 mg, 1.394 mmol) was added to a solution of 5,6-dihydro-[1,1'-biphenyl]-3(4H)-one (400 mg, 2.323 mmol) in MeOH (4.0 mL) at 0 °C in two equal portions over a 5 min period. After the mixture was stirred for 30 min at ambient temperature, it was diluted with 20 ml EtOAc, washed with 1 *N* HCl (2 mL), water (2x3 mL), brine and dried over sodium sulfate, and filtered. The filtrate was concentrated. The resulting residue was purified by column chromatography on silica gel column with 0-15% EtOAc in hexane as eluting solvent to provide ***Int-165a.*** LCMS anal. calcd. for C₁₂H₁₄O: 174.2; Found: 175.2 (M+H)⁺.

### Step B - Synthesis of Compound Int-165b

DIAD (603 µl, 3.10 mmol) was added to a stirred mixture of N-methyl-2-nitrobenzenesulfonamide (447 mg, 2.066 mmol), ***Int-165a*** (360 mg, 2.066 mmol) and 2-(diphenylphosphaneyl)pyridine (816 mg, 3.10 mmol) in DCM (20 mL). The mixture was stirred at RT for 4 h. Without workup, the mixture was loaded onto a 100 g silica gel column with 0-90% EtOAc in hexane as eluting solvent to provide ***Int-165b.*** LCMS anal. calcd. for C₁₉H₂₀ N₂O₄S: 372.4; Found: 395.2 (M+Na)⁺.

### Step C - Synthesis of Compound Int-165c

Benzenethiol (1302 mg, 11.81 mmol) was added to a mixture of ***Int-165b*** (2.2 g, 5.89 mmol) and potassium carbonate (3266 mg, 23.63 mmol) in acetonitrile (100 mL) at RT. The mixture was stirred at RT overnight. Upon completion, it was concentrated. The residue was redissolved in DCM (100mL), filtered, concentrated and purified by a 100 g silica gel column with 0-5% EtOAc in MeOH as eluting solvent to provide ***Int-165c.*** LCMS anal. calcd. for C₁₃H₁₇N: 187.3; Found: 188.2 (M+H)⁺.

### Step D - Synthesis of Compound Int-165d

To a stirred solution of ***Int-165c*** (300 mg, 1.6 mmol) and 4-(benzyloxy)-5-bromo-3-methoxypicolinic acid (650 mg, 1.922 mmol) in DMF (16 mL) was added *N*-ethyl*-N-*isopropylpropan-2-amine (621 mg, 4.81 mmol), followed by HATU (914 mg, 2.403 mmol) at room temperature. The mixture was stirred at room temperature for 1 h. Upon completion, the reaction was loaded onto a 100 g silica gel column with 0-100% 30% EtOH in EtOAc/hexane as eluting solvent to provide ***Int-165d.*** LCMS anal. calcd. for C₂₇H₂₇BrN₂O₃: 507.4; Found: 507.3, 509.3 (M+H)⁺.

### Step E - Synthesis of Compound Int-165e

To a stirred solution of ***Int-165d*** (670 mg, 1.320 mmol) in THF (11 mL) and water (2.2 mL) was added 4-methylmorpholine 4-oxide (309 mg, 2.64 mmol), osmium(VIII) oxide (839 µL, 0.132 mmol). The resulting solution was heated at 50 °C for 24 h. Upon completion, it was cooled down, and 0.5 g sodium metabisulfite was added and the mixture was stirred for 1 h before it was filtered. The filtrate was concentrated. The residue was loaded onto a 120 g silica gel column with 0-100% 30% EtOH in EtOAc/hexane as eluting solvent to provide ***Int-165e.*** LCMS anal. calcd. for C₂₇H₂₉BrN₂O₅: 541.4; Found: 541.3, 543.3 (M+H)⁺.

### Step F - Synthesis of Compound Int-165f

To a stirred solution of ***Int-165e*** (650 mg, 1.20 mmol) in MeOH (12 mL) was added palladium on carbon (128 mg, 0.120 mmol). The resulting mixture was flushed with N₂ and connected with H₂ balloon for 1 h. Upon completion, the reaction mixture was filtered through a pad of celite. The filtrate was concentrated to provide ***Int-165f.*** LCMS anal. calcd. for C₂₀H₂₄N₂O₅: 372.4; Found: 373.4 (M+H)⁺.

### Step G - Synthesis of Compound Int-165g and Int-165h

To a stirred solution of ***Int-165**f* (410 mg, 1.101 mmol) in DCM (11 mL) was added triethylamine (334 mg, 3.30 mmol) and methanesulfonyl chloride (277 mg, 2.422 mmol). The resulting mixture was stirred at room temperature for 3 hrs. Upon completion, it was concentrated to remove most of the DCM. To the residue, 20 mL EtOAc and 5 mL 0.2 N HCl aqueous solution was added. The organic layer was separated, dried over MgSO₄ and concentrated to provide the crude product.

To the crude product was added 10 mL DMF and potassium carbonate (761 mg, 5.50 mmol). The mixture was stirred at 100 °C for 4 hrs. Upon completion, it was filtered. The filtrate was loaded onto a C18 column (220 g) with 0-100% ACN in water (with 0.05% TFA modifier) as eluting solvent to racemic material (82 mg, 0.231 mmol). The racemic material was further resolved using SFC on an AD-H (4.6x250mm, 5 µm) column with 30% MeOH as eluting solvent to provide the earlier eluting peak ***Int-165g*** and later eluting peak ***Int-165h.*** LCMS anal. calcd. for C₂₀H₂₂N₂O₄: 354.4; Found: 355.4 (M+H)⁺.

### Step H - Synthesis of Compound 165A

Lithium chloride (11.96 mg, 0.282 mmol) was added to a stirred mixture of ***Int-165g** (10* mg, 0.028 mmol) in DMF (1 mL) at room temperature. The mixture was stirred at 100 °C overnight. Upon completion, the mixture was cooled down. It was purified by reverse phase preparative HPLC with a 40 g C18 column with ACN in water (with 0.1% TFA modifier) to provide compound ***165A.*** LCMS anal. calcd. for C₁₉H₂₀N₂O₄: 340.4; Found: 341.4 (M+H)⁺. ¹H NMR (500 MHz, Methanol-d⁴) *δ* 8.3-8.1 (br, 1H), 7.3-6.95 (m, 6H), 4.85 (s,1H), 4.37 (s, 1H), 2.98 (s, 3H), 2.75-2.62(br, 1H), 2.62-2.39 (br,1H), 2.27-2.02(m, 4H).

Following essentially the method employed to produce compound ***165A,*** compound ***165B*** was prepared from ***Int-165h.*** LCMS anal. calcd. for C₁₉H₂₀N₂O₄: 340.4; Found: 341.3 (M+H)⁺. ¹H NMR (500 MHz, Methanol-d⁴) *δ* 8.27-8.07 (br, 1H), 7.3-6.9 (m, 6H), 4.83 (s,1H), 4.36 (s, 1H), 2.97 (s, 3H), 2.73-2.62 (br, 1H), 2.62-2.39 (br, 1H), 2.26-2.04 (m, 4H).

### Example 39

### Preparation of Compounds 166A and 166B

### 1,7-dihydroxy-5-methyl-1-phenyl-2-propyl-1,3,4,4a,5,11a-hexahydro-2H-pyrido[1,2-a]quinoxaline-6,8-dione

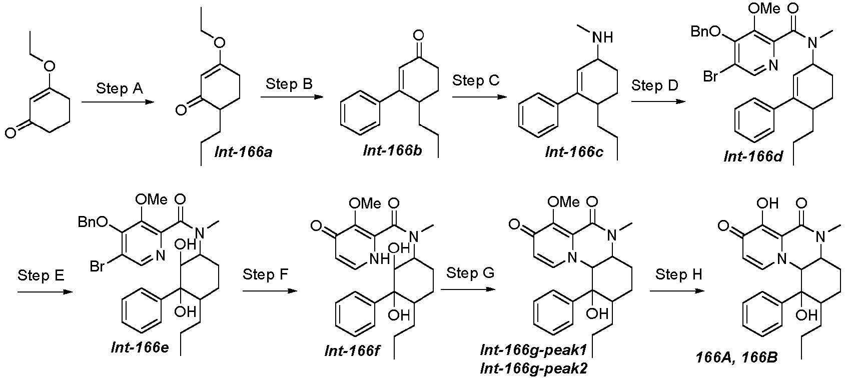

### Step A - Synthesis of Compound Int-166a

A mixture of 3-ethoxycyclohex-2-en-1-one (5 g, 35.7 mmol), hexamethylphosphoramide (12.78 g, 71.3 mmol) and 1-iodopropane (7.28 g, 42.8 mmol) in THF (178 mL) was added lithium diisopropylamide (35.7 mL, 71.3 mmol) dropwise at -78 °C under N₂. The mixture was slowly warmed to room temperature for 2 hrs. The reaction was quenched with aqueous ammonium chloride solution at 0 °C, and warmed to room temperature, with stirring. The mixture was extracted with ether. The combined organic layer was washed with brine, dried over MgSO₄ and concentrated. The residue was chromatographed on silica gel (10:3 hexane-ethyl acetate) to provide ***Int-166a**.* LCMS anal. calcd. for C₁₁H₁₈O₂: 182.2; Found: 183.2 (M+H)⁺.

### Step B - Synthesis of Compound Int-166b

To a solution of ***Int-166a*** (3.3 g, 18.11 mmol) in THF (91 mL) was added phenylmagnesium bromide (23.54 mL, 23.54 mmol) at -78 °C. The resulting mixture was stirred at this temperature for 10 min before being warmed to 0 °C for 30 minutes. Upon completion, it was quenched with 100 mL NH₄Cl aqueous solution. The mixture was extracted with 3x100 mL EtOAc. The combined organic layer was dried over MgSO₄ and concentrated to provide 5 g crude product.

The above crude was added to 100 mL MeOH and 50 mL water, followed by the addition of 2 mL TFA. The resulting mixture was stirred at room temperature for 24 hours. Upon completion, it was concentrated to remove most of MeOH. The residue was added to 100 mL EtOAc. The organic layer was separated and dried over MgSO₄ and concentrated. The residue was loaded onto a 220 g silica gel column with 20% EtOAc in hexane as eluent to provide ***Int-166b.*** LCMS anal. calcd. for C₁₅H₁₈O: 214.3; Found: 215.6 (M+H)⁺.

### Step C - Synthesis of Compound Int-166c

The mixture of ***Int-166b*** (3.5 g, 16.33 mmol), methanamine in MeOH (40.8 ml, 82 mmol) was added to 0.2 mL TFA and 5 g anhydrous MgSO₄. The resulting reaction was stirred at room temperature for 4 hours. It was filtered and the filtrate was concentrated. To the above residue was added 100 mL DCM and 10 mL MeOH, followed by sodium borohydride (0.989 g, 26.1 mmol) portionwise. Upon completion, it was quenched with 10 mL 2 N HCl aqueous solution. The reaction mixture was dried over MgSO₄ and concentrated. The residue was purified by a 120 g silica gel column with 0-20% MeOH in DCM as eluent to provide ***Int-166c**.* LCMS anal. calcd. for C₁₆H₂₃N: 229.4 Found: 230.4 (M+H)⁺.

### Step D - Synthesis of Compound Int-166d

A mixture of ***Int-166c*** (1700 mg, 7.41 mmol), 4-(benzyloxy)-5-bromo-3-methoxypicolinic acid (2506 mg, 7.41 mmol), triethylamine (1500 mg, 14.82 mmol) and HATU (3382 mg, 8.89 mmol) in DCM (100 mL) was stirred at room temperature 5 hours. Upon completion, 100 mL 0.2 HCl aqueous solution was added. The organic layer was separated, dried over MgSO₄ and concentrated. The residue was loaded onto a 120 g silica gel column with 0-100% EtOAc in hexane to provide ***Int-166d.*** LCMS anal. calcd. for C₃₀H₃₃BrN₂O₃: 549.5 Found: 550.4 (M+H)⁺.

### Step E - Synthesis of Compound Int-166e

To a stirred solution of ***Int-166d*** (2.3 g, 4.19 mmol) in THF (35.9 mL)/Water (5.98 mL) was added 4-methylmorpholine 4-oxide (0.981 g, 8.37 mmol) and osmium (VIII) oxide (2.66 mL, 0.419 mmol). The resulting mixture was stirred at 50 °C overnight. Upon completion, 5 g solid sodium metabisulfite and 5 g MgSO₄ was added. The resulting mixture was stirred at room temperature for 1 hour. It was filtered and the filtrate was concentrated. The residue was purified by a 120 g C18 column with 0-100% ACN in water with 0.05% TFA modifier to provide ***Int-166e.*** LCMS anal. calcd. for C₃₀H₃₅BrN₂O₅: 583.5; Found: 584.4 (M+H)⁺.

### Step F - Synthesis of Compound Int-166f

To a stirred solution of ***Int-166e*** (1.1 g, 1.885 mmol) in methanol (20 mL)/DCM (2 mL) was added palladium on carbon (0.201 g, 0.189 mmol). The resulting mixture was hydrogenated under H₂ balloon at room temperature for 1 hour. Upon completion, it was filtered, and the filtrate was concentrated to provide ***Int-166f**.* LCMS anal. calcd. for C₂₃H₃₀N₂O₅: 414.5; Found: 415.4 (M+H)⁺.

### Step G - Synthesis of Compound Int-166g

To a stirred solution of ***Int-166f*** (760 mg, 1.834 mmol) in 20 mL THF was added triethylamine (649 mg, 6.42 mmol) and methanesulfonyl chloride (462 mg, 4.03 mmol) at 0 °C. The resulting mixture was stirred at this temperature for 1 hour. At completion, it was filtered, and the filtrate was concentrated and 50 mL EtOAc was added. It was washed with 0.1 N aqueous HCl solution and dried over MgSO₄. It was concentrated to afford crude product.

To the above crude product was added DMF (20 mL) and potassium carbonate (1267 mg, 9.17 mmol). The resulting mixture was stirred at 100 °C overnight. Upon completion, it was cooled down and 100 mL water and 100 mL EtOAc was added. The organic layer was separated and dried over MgSO₄. It was concentrated and the residue was loaded onto a 100 g C18 column with 0-100% ACN in water (0.05% TFA modifier) as eluent to provide ***Int-166g*** (320 mg, 0.807 mmol), which was subjected to SFC resolution (AD-H, 4.6x250mm, EtOH) to provide ***Int-166g-peak1*** and ***Int-166g-peak2**.* LCMS anal. calcd. for C₂₃H₂₈N₂O₄: 396.5; Found: 397.4 (M+H)⁺.

### Step H - Synthesis of Compound 166A and 166B

***Int-166g-peak1*** (15 mg, 0.038 mmol) was dissolved in the suspension of LiCl (16.04 mg, 0.378 mmol) in DMF (1000 µL) and the mixture was stirred at 100 °C for 1 hour. The suspension was filtered. The filtrate was purified by preparative HPLC Reverse phase (on SunFire C18 OBD Prep Column), eluting with 10-90% Acetonitrile/Water with 0.1% TFA modifier to provide 166***A***. LCMS anal. calcd. for C₂₂H₂₆N₂O₄: 382.5; Found: 383.2 (M+H)⁺. ¹H NMR (500 MHz, Methanol-d⁴) *δ* 8.14 (s, 1H), 7.26 - 6.94 (m, 5H), 4.68 (s, 1H), 4.38 (s, 1H), 3.06 (s, 3H), 2.67 (d, *J* = 12.4 Hz, 1H), 2.30 - 2.00 (m, 5H), 1.68 - 1.26 (m, 5H).

Following essentially the method employed to produce compound ***166A**,* compound ***166B*** was prepared from ***Int-166g-peak2.*** LCMS anal. calcd. for C₂₂H₂₆N₂O₄: 382.5; Found: 383.2 (M+H)⁺. ¹H NMR (500 MHz, Methanol-d⁴) *δ* 8.14 (s, 1H), 7.26 - 6.94 (m, 5H), 4.68 (s, 1H), 4.38 (s, 1H), 3.06 (s, 3H), 2.67 (d, *J =* 12.4 Hz, 1H), 2.30 - 2.00 (m, 5H), 1.68 - 1.28 (m, 5H).

### Example 40

### Preparation of Compounds 167

### 7-hydroxy-1-methoxy-5-methyl-1-phenyl-1,3,4,4a,5,11a-hexahydro-2H-pyrido[1,2-a]quinoxaline-6,8-dione

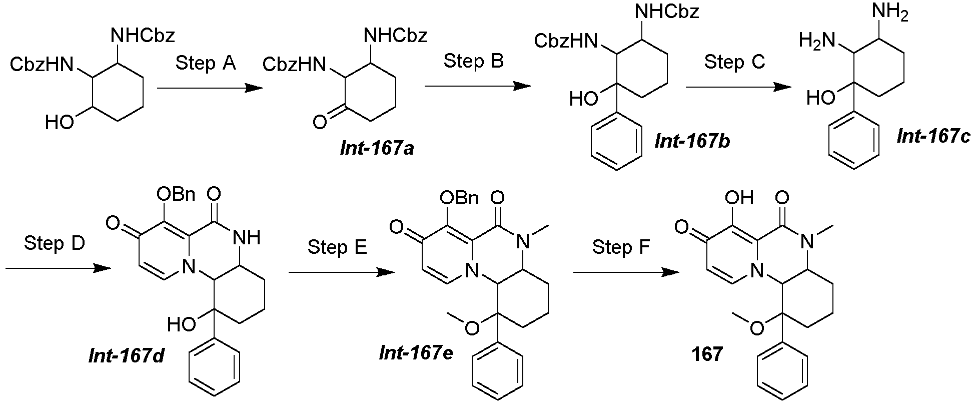

### Step A - Synthesis of Compound Int-167a

To a stirred solution of dibenzyl (3-hydroxycyclohexane-1,2-diyl)dicarbamate (2 g, 5.02 mmol) in CH₂Cl₂ (50.2 mL) was added Dess-Martin periodinane (3.19 g, 7.53 mmol). The resulting mixture was stirred at room temperature for 2 hours. A drop of water was added and the precipitate was filtered through a pad of celite. The filtrate was concentrated. The residue was loaded onto a 120 g silica gel column with 0-100% 30% EtOH in EtOAc/hexane as eluent to provide ***Int-167a**.* LCMS anal. calcd. for C₂₂H₂₄N₂O₅: 396.4; Found: 397.2(M+H)⁺.

### Step B - Synthesis of Compound Int-167b

To a stirred solution of ***Int-167a*** (1800 mg, 4.54 mmol) in THF (50 mL) was added phenyllithium (9559 µL, 18.16 mmol) at -78 °C. The resulting mixture was slowly warmed up to 0 °C for 1 hour. Upon completion, it was quenched with 20 mL NH₄Cl aqueous solution. The mixture was extracted with 2x30 mL EtOAc. The combined organic layers were washed with brine, dried over Na₂SO₄ and concentrated. The residue was purified by a 100 g silica gel column with 0-100% 30% EtOH in EtOAc/hexane to provide ***Int-167b.*** It was moved to next step without further purification. LCMS anal. calcd. for C₂₈H₃₀N₂O₅ : 474.5; Found: 475.3(M+H)⁺.

### Step C - Synthesis of Compound Int-167c

To a stirred solution of ***Int-167b*** (700 mg, 1.475 mmol) in MeOH (20 mL) was added palladium on carbon (157 mg, 0.148 mmol). The resulting mixture was hydrogenated under H₂ balloon for 3 hours. It was filtered through a pad of celite. The filtrate was concentrated to provide ***Int-167c**.* LCMS anal. calcd. for C₁₂H₁₈N₂O: 206.3; Found 207.3 (M+H)⁺.

### Step D - Synthesis of Compound Int-167d

A stirred solution of ***Int-167c*** (399 mg, 1.454 mmol), 2,3-diamino-1-phenylcyclohexan-1-ol (300 mg, 1.454 mmol) in n-methylimidazole (5 mL) was heated at 80 °C overnight. Upon completion, it was cooled down. The reaction was loaded onto a 100 g C18 column with 0-100% ACN in water as eluent to provide crude product. The crude product was then loaded onto Gilson using SunFire C18 OBD Prep Column with 0-100% ACN in water (0.1% TFA modifier) as eluent to provide ***Int-167d.*** LCMS anal. calcd. for C₂₅H₂₄N₂O₄: 416.5; Found 417.2 (M+H)⁺.

### Step E - Synthesis of Compound Int-167e

To a stirred solution of ***Int-167d*** (40 mg, 0.096 mmol) in DMF (960 µL) was added iodomethane (40.9 mg, 0.288 mmol) and sodium hydride (11.52 mg, 0.288 mmol) at 0 °C. The resulting mixture was stirred at this temperature for 1 hour. Upon completion, it was quenched with 2 drops of 1 N ammonium chloride aqueous solution. The mixture was loaded onto a 50 g C18 column with 0-100% ACN in water (0.05% TFA as modifier) as eluent to provide ***Int-167e.*** LCMS anal. calcd. for C₂₇H₂₈N₂O₄: 444.5; Found 445.2(M+H)⁺.

### Step F - Synthesis of Compound 167

Pd/C (5.75 mg, 5.40 µmol) was added to a stirred, cooled room temperature mixture of ***Int-167e*** (24 mg, 0.054 mmol) in methanol. The mixture was stirred at room temperature for 3 hrs. The reaction mixture was then filtered. The filtrate was loaded onto a 50 g C18 column eluting with 0-100% acetonitrile/water (0.05% TFA) to provide **167.** LCMS anal. calcd. for C₂₀H₂₂N₂O₄: 354.4; Found: 355.2 (M+H)⁺. ¹H NMR (500 MHz, Methanol-d⁴) *δ* 7.38 (s, 2H), 4.73 (s, 1H), 4.41 (s, 1H), 3.33 (s, 13H), 3.25 (s, 3H), 3.13 (s, 3H), 2.05 (s, 2H).

### Flu A Neuraminidase Antiviral Assay

A fluorescent assay monitoring the activity of influenza-derived neuraminidase (NA) enzyme in infected cells was developed to evaluate antiviral compounds against Flu A. NA activity enables the release of influenza virions from infected cells but is also functional in cell culture systems that retain infectious virus at the cell surface like Madin-Darby canine kidney (MDCK) epithelial cells which require chemical intervention for release of virus. NA activity can be monitored by the increase in fluorescence of MUNAN (4-methylumbelliferone) released as product from enzymatic substrate cleavage of 2'-(4-methylumbelliferyl)-α-D-N-acetylneuraminic acid (MUNANA). The amount of fluorescence is directly proportional to the amount of NA enzyme activity which increases with viral replication.

MDCK (Sigma) cells are incubated at 37°C in an atmosphere of 5% CO₂, and >85% humidity in growth medium of DMEM with Glutamax and pyruvate (Thermo Fisher) with 5% heat-inactivated fetal bovine serum (Thermo Fisher), and 1% Pen-Strep (Thermo-Fisher). On the day of assay, cells are washed with 15-20 ml PBS (Thermo Fisher) followed by the addition of 1.5 ml 0.25% Trypsin-EDTA (Thermo Fisher) solution and incubation at 37°C for 2-5 min. After the cells have dislodged from the plate, 6-8 mL of growth medium is added to resuspend cells. Cells are counted in a ViCell Counter (Beckman), and the cell density is adjusted to 80,000 cells/mL by adding growth medium. FLU A (PR/8/34) at 3.4E+08 pfu/ml is diluted 1:25,000 in to the MDCK cell suspension.

Compounds diluted in DMSO are titrated (10-point, 3-fold dilution) and added by acoustic dispense (200 nL, Labcyte Echo) into a 384-well black polystyrene tissue-culture treated microplate (Corning). The cell and virus suspension (25 µL) is dispensed into each well of the assay plate. Plates are briefly centrifuged to (300 rpm x 30 s) and incubated at 37°C in an atmosphere of 5% CO₂, and >85% humidity for 48 h. For detection of NA activity, the MUNANA substrate (MP Biomedical) is diluted in dH₂O at a concentration of 2.5 mM. The substrate is further diluted in assay buffer (66.6 mM MES, 8 mM CaCl₂, pH 6.5) to a concentration of 200 µM. A volume of 6 µl of the substrate dilution is added to each well of the assay plate, shaken for 1 min to mix and returned to the incubator for 1 h at 37°C. Then, 25 µL of MUNANA Stop Solution (0.2 M sodium carbonate, Fisher) in dH₂O was added to each well followed by shaking for 1 min. An Envision plate reader (Perkin Elmer) was used to measure the fluorescence intensity (Ex = 355 nm, Em = 460 nm) of each well.

Raw data from each test well is normalized to the average signal of 100% inhibited wells (Max Effect; 100% inhibition) and virus infected cells only (Min Effect; 0% inhibition) to calculate % inhibition using the following formula: % inhibition = 100*(Test Cmpd - Max Effect)/(Min Effect - Max Effect). Data are analyzed using ActivityBase (IDBS), and dose-response curves were generated by plotting percent inhibition (Y-axis) vs. Log₁₀ compound concentrations (X-axis). IC₅₀ values are calculated using a non-linear regression, four-parameters sigmoidal dose-response model.

The compounds of the instant invention were tested in the assay described and the results appear in the table below.

| Examples | Flu A EC50 | Examples | Flu A EC50 | Examples | Flu A EC50 |
|---|---|---|---|---|---|
| 1A | >8100 | 56A | >8100 | 104A | 8 |
| 1B | 39.6 | 56B | 6853 | 104B | >810 |
| 2A | 109 | 56C | >8100 | 105A | 413.2 |
| 2B | 5920 | 56D | 37.7 | 105B | >810 |
| 3A | 1016 | 57A | 95.5 | 106A | 6.4 |
| 3B | >2700 | 57B | >8100 | 106B | 87.8 |
| 4A | >8100 | 58A | >8100 | 107A | 4799 |
| 4B | >8100 | 58B | >8100 | 107B | 14.4 |
| 5A | 1530 | 58C | >8100 | 108A | 5423 |
| 5B | 1700 | 58D | >8100 | 108B | 9.4 |
| 6A | 372.2 | 59A | 7284 | 109A | 802.9 |
| 6B | 7756 | 59B | 12.1 | 109B | 5.4 |
| 7A | >8100 | 60A | >8100 | 110A | 1119 |
| 7B | 255.9 | 60B | 883.9 | 110B | 5.5 |
| 8A | >8100 | 61A | 122.5 | 111A | >8100 |
| 8B | 65.6 | 61B | 2733 | 111B | 3.3 |
| 9A | 6206 | 62A | >8100 | 112A | 3.7 |
| 9B | 165.9 | 62B | >8100 | 112B | 161.5 |
| 9C | >8100 | 62C | 7487 | 113A | 3445 |
| 9D | >8100 | 62D | 7966 | 113B | 7.3 |
| 10A | 1609 | 63A | >8100 | 114A | >8100 |
| 10B | 86.5 | 63B | 160.2 | 114B | >8100 |
| 11A | >8100 | 64A | 3786 | 114C | 5.3 |
| 11B | >8100 | 64B | 28.4 | 114D | 2.3 |
| 12A | >8100 | 64C | 673.5 | 115A | >8100 |
| 12B | >8100 | 64D | 4.6 | 115B | >8100 |
| 13A | >8100 | 65A | 396.1 | 115C | 2.7 |
| 13B | 783.1 | 65B | 2.8 | 115D | 6.9 |
| 14A | >8100 | 66A | >8100 | 116A | 6199 |
| 14B | >8100 | 66B | 182.3 | 116B | 6.9 |
| 15A | >8100 | 67 | 16.4 | 117A | 3531 |
| 15B | 1447 | 68A | 349.3 | 117B | 112.6 |
| 16 | 4100 | 68B | >810 | 118A | >8100 |
| 17A | 3508 | 68C | 11.4 | 118B | 3.1 |
| 17B | 102.6 | 68D | 11.8 | 119A | 3078 |
| 18A | 7.1 | 69A | 2.3 | 119B | 2.7 |
| 18B | 355.9 | 69B | 57 | 120A | 2723 |
| 19A | >8100 | 69C | 88.4 | 120B | 1.9 |
| 19B | 348.4 | 70A | 299 | 121A | 431.4 |
| 20A | 564 | 70B | 2 | 121B | 5.8 |
| 20B | 4.6 | 71A | 5906 | 122A | 343 |
| 21A | >8100 | 71B | 18.5 | 122B | 5.2 |
| 21B | 2.6 | 72A | >810 | 123A | >8100 |
| 21C | 1601 | 72B | 3.8 | 123B | 4.2 |
| 21D | 1551 | 73A | >810 | 123C | >8100 |
| 22A | >8100 | 73B | >810 | 123D | 26.9 |
| 22B | 5287 | 73C | 104.6 | 124A | 870.8 |
| 22C | >8100 | 73D | 6.6 | 124B | 1.6 |
| 22D | 11 | 74A | >810 | 125A | 1019 |
| 23A | >8100 | 74B | 15.7 | 125B | 2.5 |
| 23B | 2.6 | 74C | 32.5 | 126A | >8100 |
| 24A | 2.1 | 74D | 442 | 126B | 16.1 |
| 24B | 1236 | 75A | >810 | 127A | 3503 |
| 25A | 3.5 | 75B | 8 | 127B | 3 |
| 25B | 70.7 | 75C | 139.9 | 128A | 799.3 |
| 26A | 8.4 | 75D | 19.2 | 128B | 1.5 |
| 26B | 1801 | 76A | >810 | 129A | 2.3 |
| 27A | 859 | 76B | 2.0 | 129B | 196.3 |
| 27B | 17.1 | 77A | 3.3 | 129C | 6.6 |
| 28A | 5.2 | 77B | 3.2 | 129D | 92 |
| 28B | 278.4 | 77C | >810 | 130A | >8100 |
| 29A | 1861 | 77D | 1.9 | 130B | 1411 |
| 29B | 286.5 | 78A | >810 | 131A | >810 |
| 30A | 16.6 | 78B | 2.9 | 131B | >810 |
| 30B | 5300 | 79A | 83.8 | 131C | >810 |
| 31A | 282.6 | 79B | 2.3 | 131D | >810 |
| 31B | >8100 | 80A | 59.6 | 131E | 6.8 |
| 32A | >8100 | 80B | 3.3 | 131F | 12.5 |
| 32B | 156.2 | 81A | 19.1 | 132A | >810 |
| 33A | 49.5 | 81B | 744.1 | 132B | 21.3 |
| 33B | 1883 | 82A | >810 | 132C | >810 |
| 33C | 1315 | 82B | 2.3 | 132D | >810 |
| 33D | 2.3 | 83A | 152.1 | 133 | >810 |
| 34A | 3091 | 83B | 2.2 | 134 | 453.2 |
| 34B | 4.9 | 84A | >810 | 135A | 428 |
| 35A | 137.6 | 84B | 42 | 135B | >8100 |
| 35B | >8100 | 85A | >810 | 136A | 1919 |
| 36A | 3.2 | 85B | 9.7 | 136B | >8100 |
| 36B | 1290 | 85C | >810 | 137A | 790.2 |
| 37A | 11.4 | 85D | 218 | 137B | >8100 |
| 37B | 1235 | 86A | 4.3 | 137C | >8100 |
| 38A | >8100 | 86B | >810 | 137D | 49.3 |
| 38B | 5.8 | 86C | 229.8 | 138A | 19.7 |
| 39A | >8100 | 86D | >810 | 138B | 1715 |
| 39B | 10.1 | 87A | >810 | 139 | 23.6 |
| 40A | 14.4 | 87B | 3.6 | 140 | 2396 |
| 40B | >8100 | 88A | >810 | 141 | >8100 |
| 41A | >8100 | 88B | >810 | 142 | 23.6 |
| 41B | 11.1 | 88C | 4 | 143A | >8100 |
| 42A | >8100 | 88D | 2.9 | 143B | 15.3 |
| 42B | 49.5 | 89A | 298.2 | 144 | >810 |
| 43A | 1167 | 89B | 2.4 | 145 | >810 |
| 43B | 1105 | 90A | 772.1 | 146A | 18.7 |
| 43C | 14.1 | 90B | 29.3 | 146B | 237.2 |
| 43D | 7.8 | 91A | 485.1 | 147 | 16.8 |
| 44A | >8100 | 91B | 7.3 | 148 | 38 |
| 44B | 15.2 | 92A | 4573 | 149 | >810 |
| 45A | >8100 | 92B | 3.3 | 150 | 69.6 |
| 45B | 10.6 | 93A | 474.9 | 151 | >810 |
| 46A | 40.3 | 93B | 2.9 | 152A | 4305 |
| 46B | >8100 | 94A | 397.1 | 152B | 36.1 |
| 47A | 25.7 | 94B | 8.6 | 153A | 3063 |
| 47B | 4376 | 95A | 137.1 | 153B | 94.4 |
| 48A | >8100 | 95B | 7.5 | 154 | >810 |
| 48A | 7.8 | 96A | >810 | 155 | 185 |
| 49A | >8100 | 96B | 2.2 | 156 | 38 |
| 49B | 26.5 | 97A | >810 | 157 | 335.5 |
| 50A | >8100 | 97B | 24.2 | 158 | 102.5 |
| 50B | 9 | 98A | 21.9 | 159 | 210.2 |
| 51A | >8100 | 98A | >810 | 160 | 18.1 |
| 51B | 16.3 | 99A | 565.2 | 161 | 119.9 |
| 52A | 113.8 | 99B | 7.4 | 162 | 194.6 |
| 52B | >2700 | 100A | >810 | 163 | 269.5 |
| 53A | >810 | 100B | 5.4 | 165A | >8100 |
| 53B | 8.3 | 101A | 148.4 | 165B | >8100 |
| 54A | >810 | 101B | 5.6 | 166A | >8100 |
| 54B | 24.9 | 102A | 2.9 | 166B | >8100 |
| 55A | >810 | 102B | 36.5 | 167 | 109.4 |
| 55B | 18.5 | 103A | >810 | | |
| | | 103B | 5.3 | | |

## Claims

1. A compound of the formula 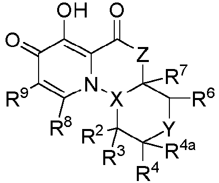 wherein X is N or CH;
Y is absent, CHR⁵, -CH₂-CHR⁵-, -CH₂-CHR⁵-CH₂-, S, SO or SO₂;
Z is NR¹ or CR¹R^{1a};
R¹ is selected from the group consisting of hydrogen, C₁₋₆ alkyl, C₁₋₃ alkyl(C₃₋₇ cycloalkyl), C₁₋₃ alkyl(heterocycyl), (C₁₋₆ alkyl)OR^{x} and C₁₋₃ haloalkyl, wherein said cycloalkyl group can be monocyclic or bicyclic, and is optionally substituted with one or two substituents independently selected from the group consisting of halo and R^{x};
R^{1a} is selected from the group consisting of hydrogen, C₁₋₆ alkyl, C₁₋₃ alkyl(cyclopropyl), (C₁₋₆ alkyl)OR^{x} and C₁₋₃ haloalkyl;
R² is aryl, heteroaryl, cycloalkyl or heterocyclyl, wherein said aryl may be monocyclic or bicyclic, wherein said aryl, heteroaryl, cycloalkyl and heterocyclyl are optionally substituted with one to three substituents independently selected from the group consisting of halo, cyano, cyclopropyl, R^{x}, R^{y}, SR^{x} and OR^{x};
R³ is hydrogen, hydroxy, C₁₋₆ alkyl, OR^{x} or C₁₋₃ haloalkyl;
or R² and R³ can be taken with the carbon atom or heteroatom to which they are attached to form dihydroindene, 1,2,3,4-tetrahydronaphthalene, chromane, 2,3-dihydrobenzo[b]thiophene, dihydrobenzofuran or thiochromane;
R⁴ is selected from the group consisting of hydrogen, hydroxy, N₃, NH(C=O)R^{x}, SR^{x}, C₁₋₆ alkyl, C₂₋₆ alkenyl, O(C₁₋₆ alkyl), O(C₂₋₆ alkenyl), (C₁₋₃ alkyl)R^{y}, O(C₁₋₃ alkyl)R^{y}, R^{y} and heteroaryl, wherein said alkyl groups are optionally substituted with one to three substituents independently selected from the group consisting of halo, R^{x}, R^{y}, OR^{x}, cyano and phenyl, wherein said heteroaryl and alkenyl groups are optionally substituted with one to three substituents independently selected from the group consisting of halo, R^{x} and (C₁₋₃ alkyl)OR^{x};
or R² and R⁴ can be taken with the carbon atom or heteroatom to which they are attached to form dihydroindene, 1,2,3,4-tetrahydronaphthalene, 2,3-dihydrobenzo[b]thiophene, dihydrobenzofuran, chromane or thiochromane;
R^{4a}is selected from the group consisting of hydrogen and C₁₋₆ alkyl, wherein said alkyl group is optionally substituted with one to three substituents independently selected from the group consisting of halo and OR^{x};
or R⁴ and R^{4a} can be taken with the carbon atom or heteroatom to which they are attached to form an oxo group or a C₄₋₆ cycloalkyl group, which can be monocyclic or bicyclic, which is optionally substituted with one to three substituents independently selected from the group consisting of halo, C₁₋₃ alkyl and C₁₋₃ haloalkyl;
R⁵ is selected from the group consisting of hydrogen, C₁₋₆ alkyl, C₁₋₃ alkyl(cyclopropyl) and (C₁₋₆ alkyl)OR^{x};
R⁶ is hydrogen, C₁₋₆ alkyl, SR^{x}, NR^{x}(C=O)C₁₋₆ alkyl or (C₂₋₃ alkenyl)R^{y};
or R⁴ and R⁶ can be taken with the carbon atoms to which they are attached to form a C₄₋₆ cycloalkyl group;
R⁷ is hydrogen or C₁₋₃ alkyl;
R⁸ is hydrogen, C₁₋₆ alkyl or (C₁₋₆ alkyl)OR^{x};
R⁹ is hydrogen, cyano or C₁₋₆ alkyl, wherein said alkyl is optionally substituted with one to three halo;
R^{x} is selected from the group consisting of hydrogen and C₁₋₆ alkyl, wherein said alkyl is optionally substituted with one to three halo;
R^{y} is selected from the group consisting of phenyl and C₃₋₆ cycloalkyl, wherein said phenyl and cycloalkyl groups are optionally substituted with one to three substituents independently selected from the group consisting of halo, cyano and R^{x}
or a pharmaceutically acceptable salt thereof.

2. The compound of Claim 1 wherein X is N; or a pharmaceutically acceptable salt thereof.

3. The compound of Claim 1 or 2 wherein R¹ is methyl; or a pharmaceutically acceptable salt thereof.

4. The compound of any of Claims 1 to 3 wherein Y is CH₂; or a pharmaceutically acceptable salt thereof.

5. The compound of any of Claims 1 to 4 wherein R² is phenyl, which is optionally substituted with one or two substituents independently selected from the group consisting of halo, CH₃, CF₃, OCHF₂, and OCH₃; or a pharmaceutically acceptable salt thereof.

6. The compound of any of Claims 1 to 5 wherein R³ is hydrogen, methyl, ethyl or hydroxy; or a pharmaceutically acceptable salt thereof.

7. The compound of any of Claims 1 to 6 wherein R⁴ is hydrogen, methyl, ethyl, propyl, trifluoroethyl, CH₂CH₂OH, CH₂CH₂OCH₃ or cyclopropylmethyl; or a pharmaceutically acceptable salt thereof.

8. The compound of any of Claims 1 to 7 wherein R⁴ and R⁶ can be taken with the carbon atoms to which they are attached to form a C₄₋₆ cycloalkyl group; or a pharmaceutically acceptable salt thereof.

9. The compound of Claim 1 selected from 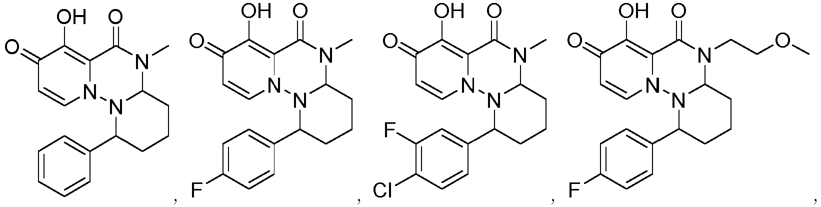 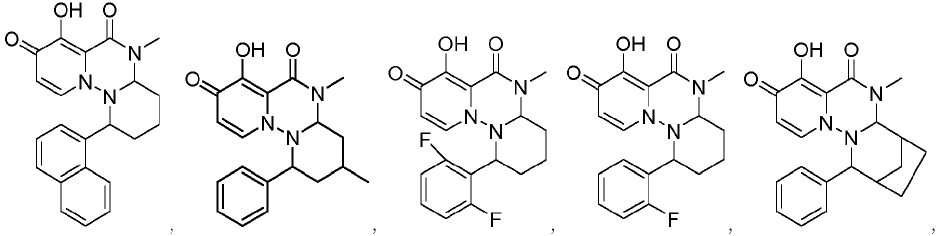 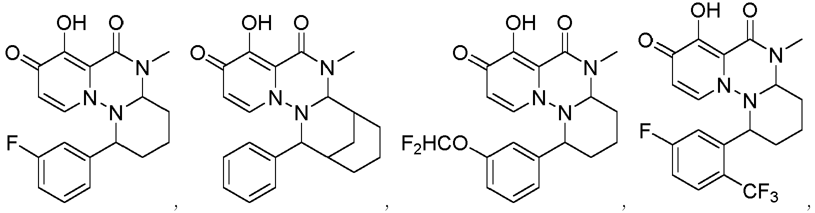 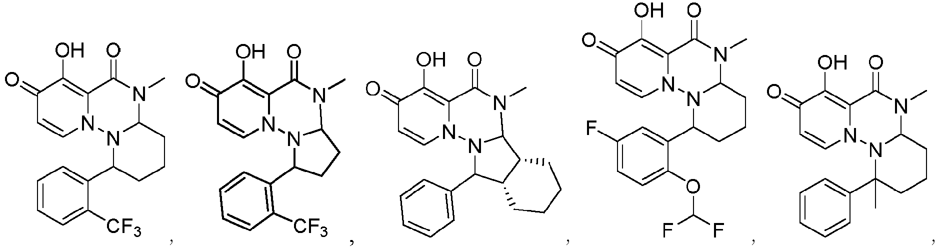 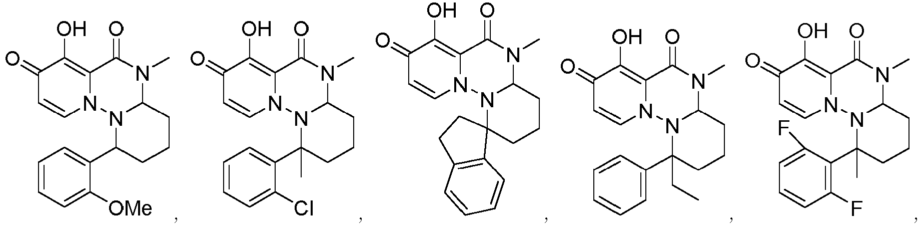 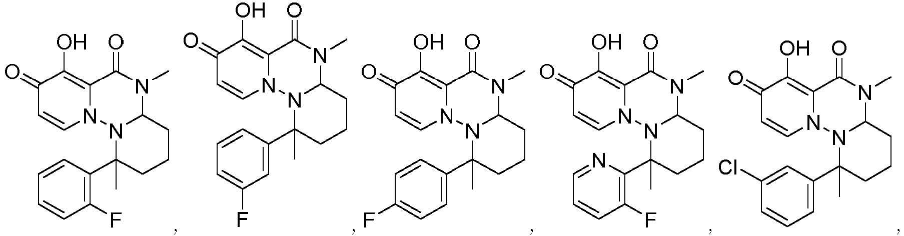 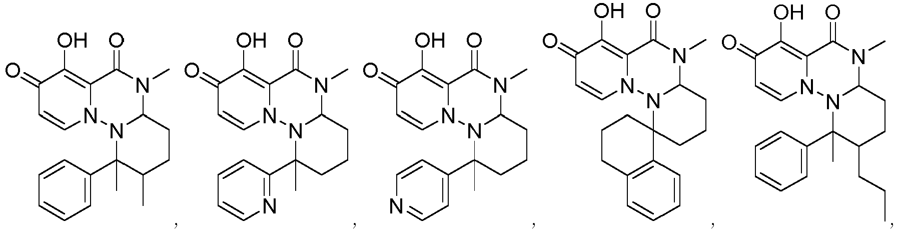 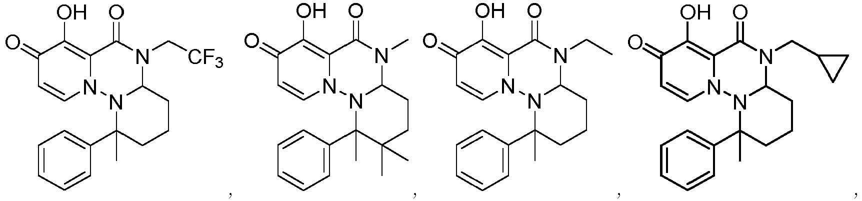 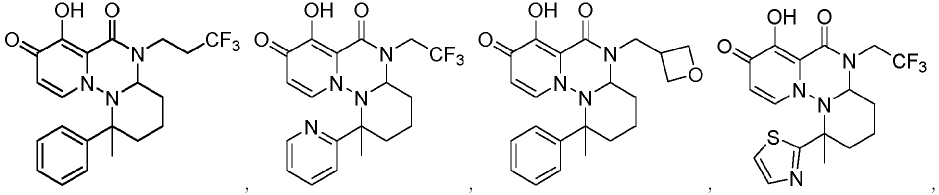 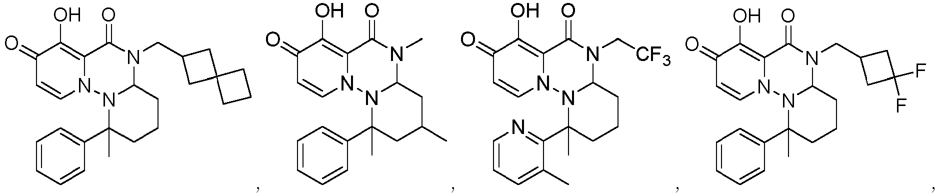 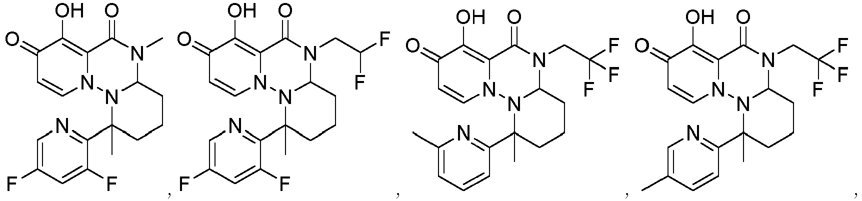 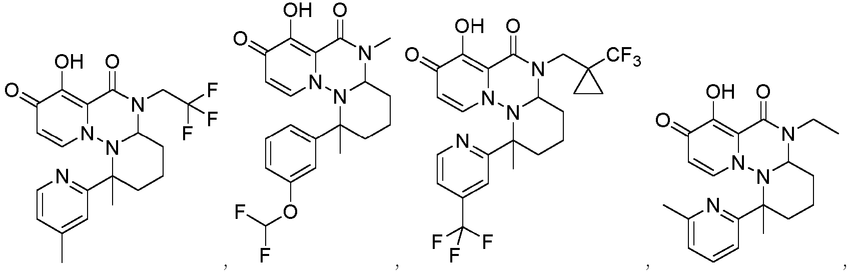 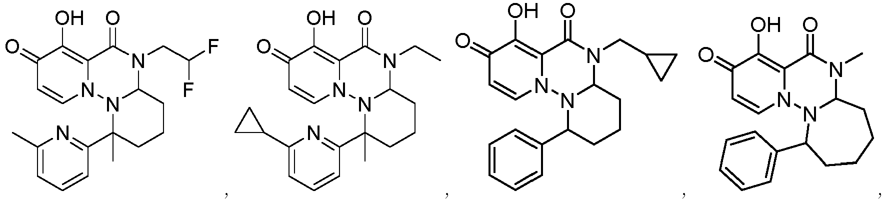 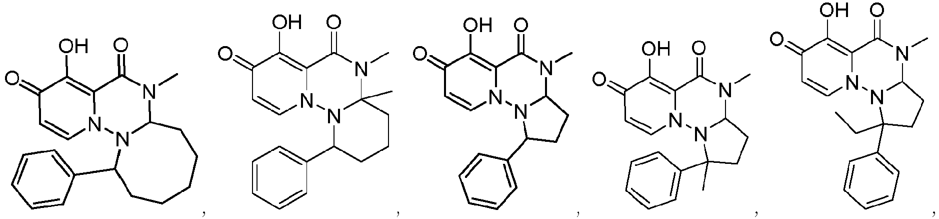 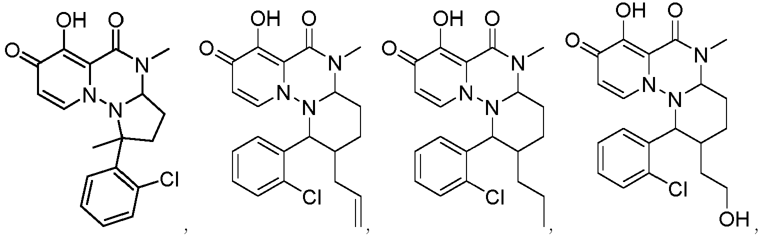 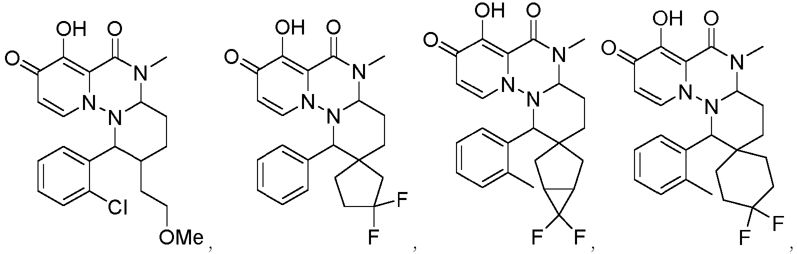 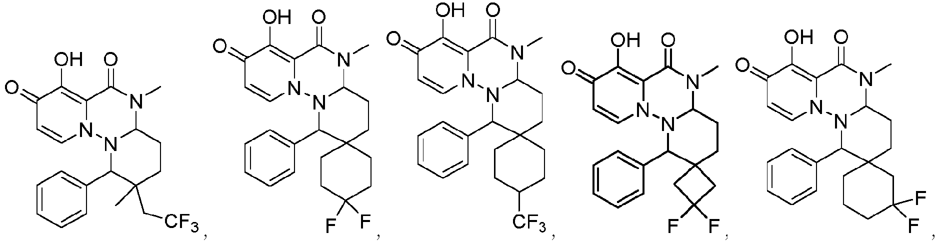 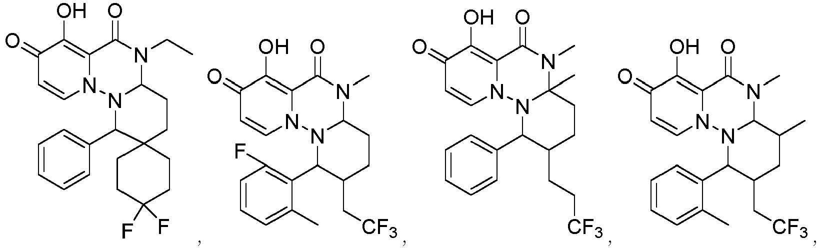 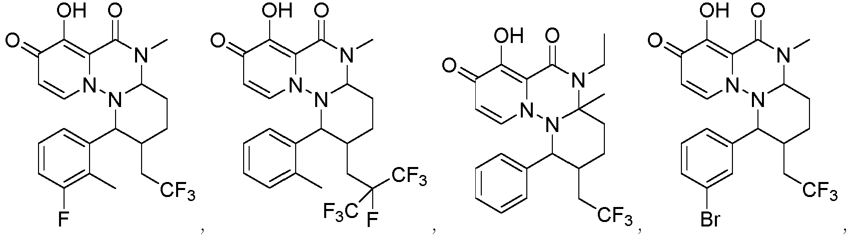 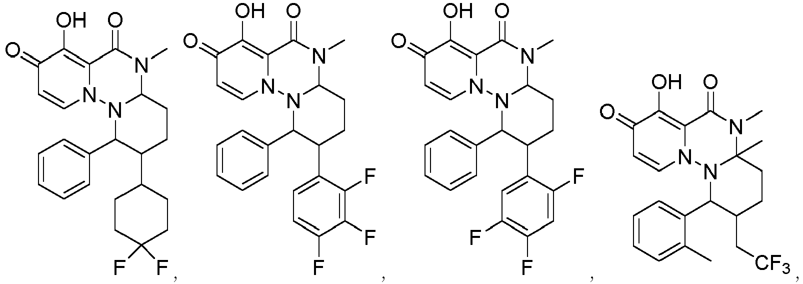 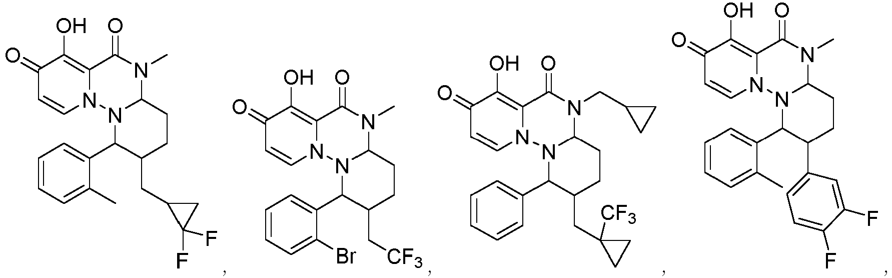 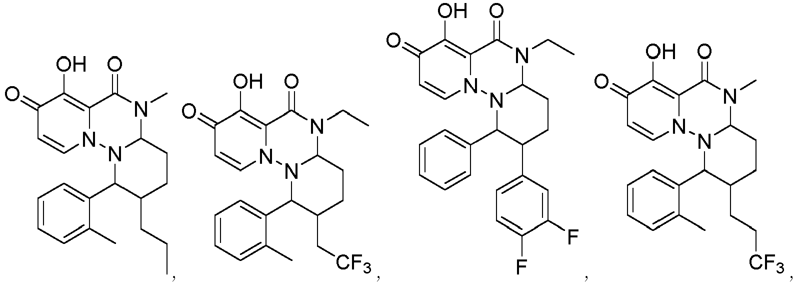 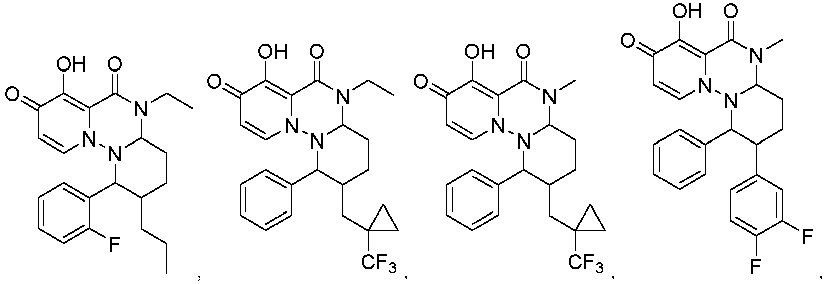 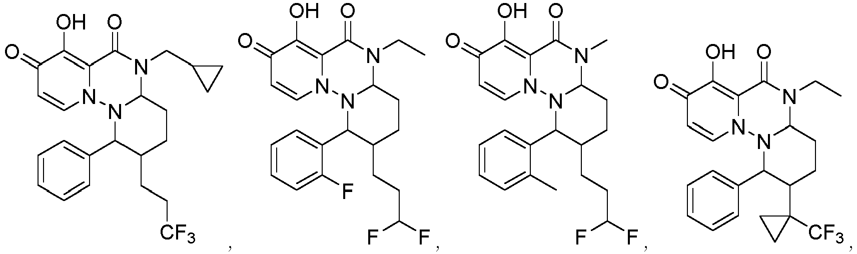 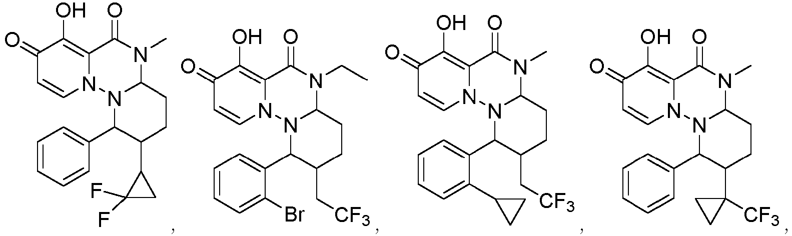 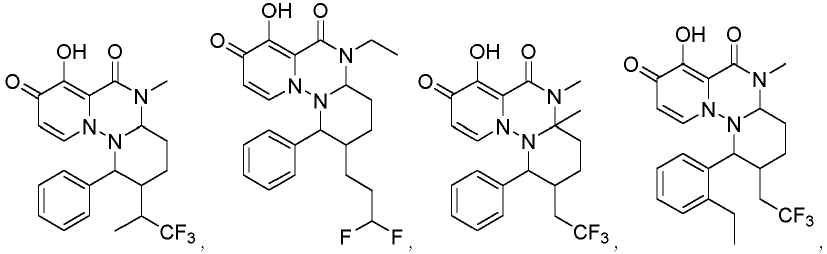 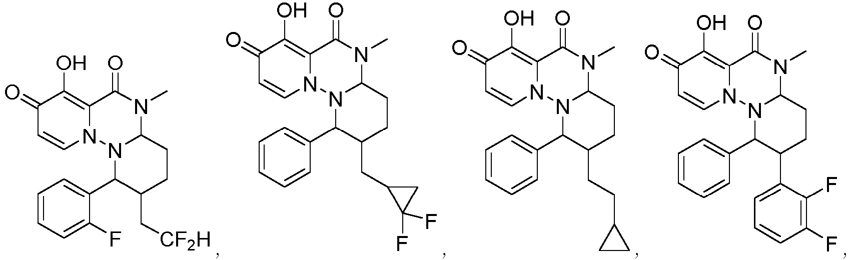 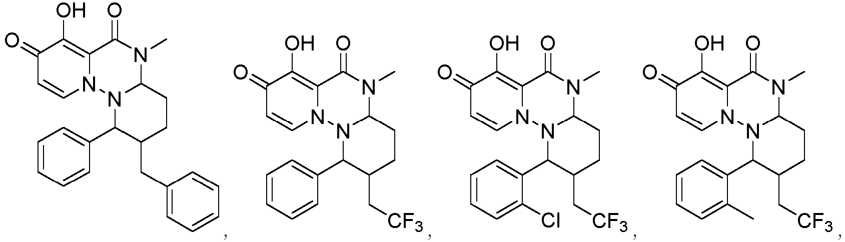 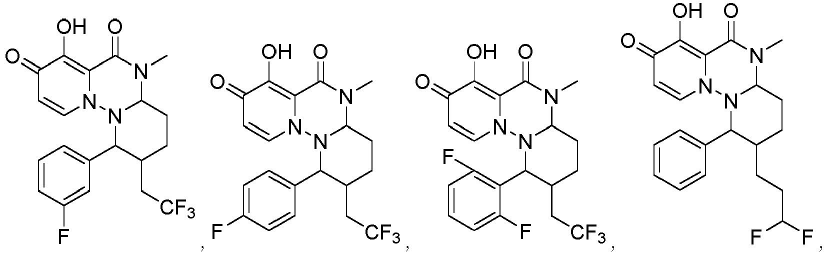 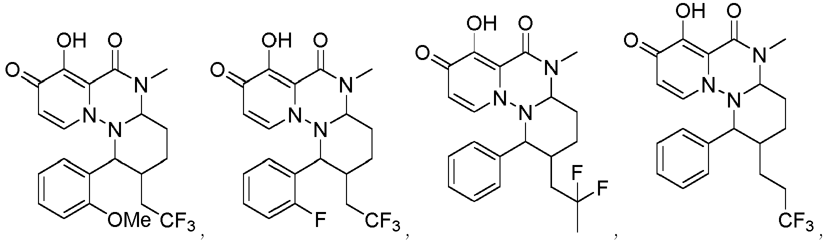 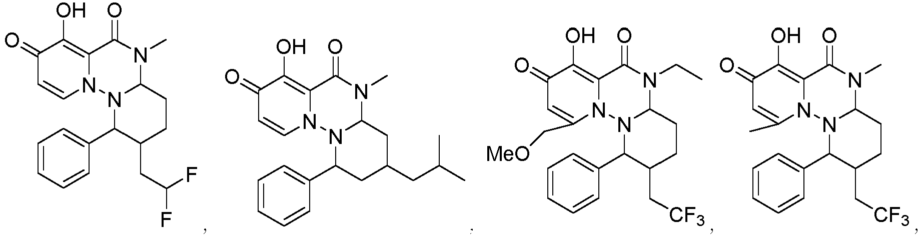 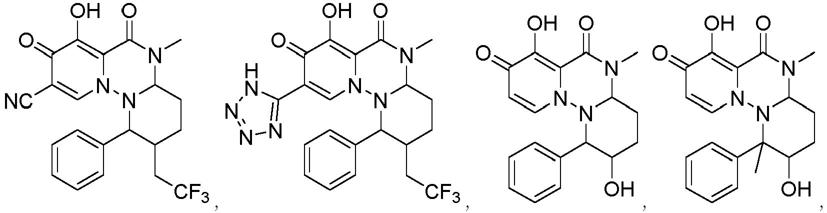 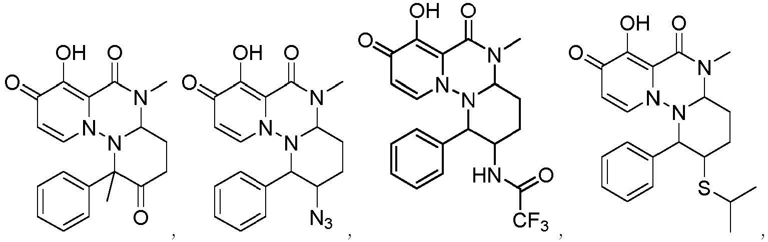 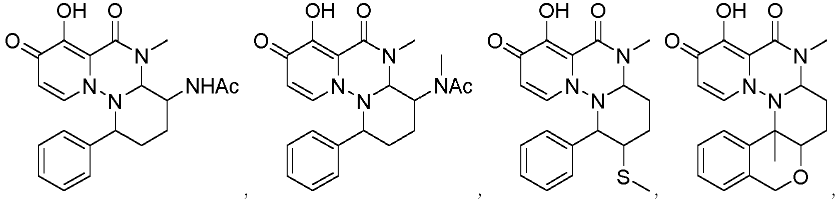 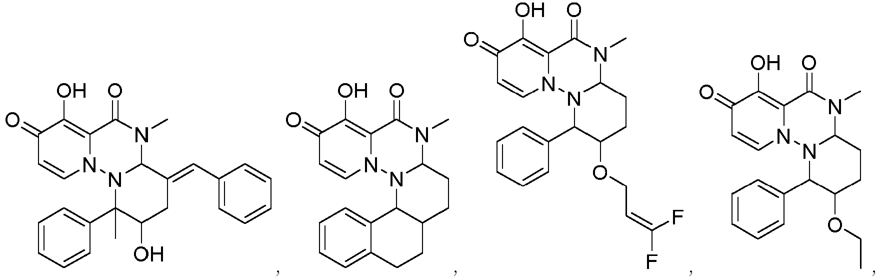 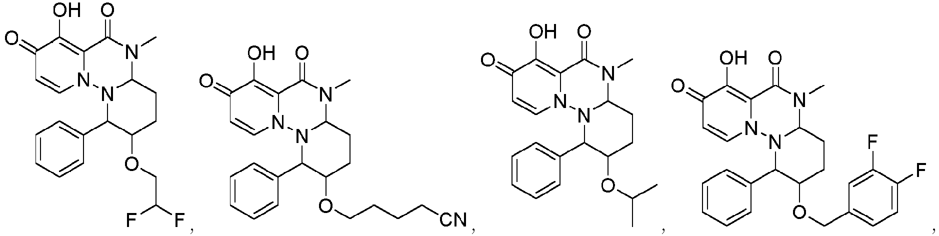 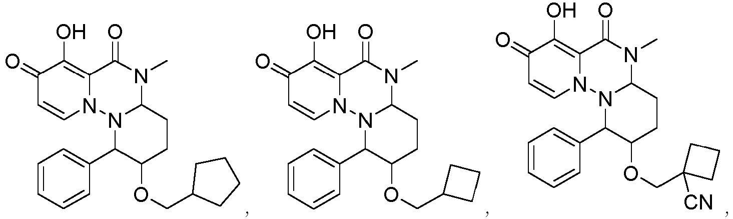 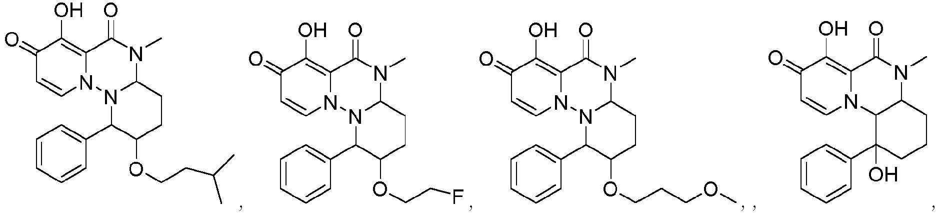 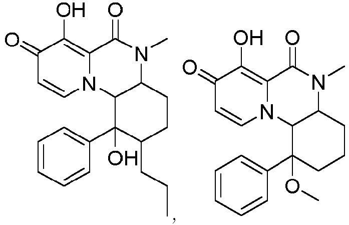 or a pharmaceutically acceptable salt thereof.

10. A pharmaceutical composition comprising a compound of any of Claims 1 to 9 or a pharmaceutically acceptable salt thereof and a pharmaceutically acceptable carrier.

11. A compound of any of Claims 1 to 9 or a pharmaceutically acceptable salt thereof or a pharmaceutical composition of claim 10, for use in a method for treating a disease caused by a virus having a cap-dependent endonuclease, the method comprising administering the compound, pharmaceutically acceptable salt, or pharmaceutical composition to a mammal in need of thereof.

12. A compound of any of Claims 1 to 9 or a pharmaceutically acceptable salt thereof or a pharmaceutical composition of claim 10, for use in a method for treating or preventing influenza in a mammal, the method comprising administering the compound, pharmaceutically acceptable salt, or pharmaceutical composition to a mammal in need thereof.

13. The compound, pharmaceutically acceptable salt or pharmaceutical composition for use according to claim 12, wherein the mammal is human.

14. The compound according to any of Claims 1 to 9, or a pharmaceutically acceptable salt thereof, for use in therapy.

15. A combination comprising a compound according to any one of Claims 1 to 9, or a pharmaceutically acceptable salt thereof, and additional therapeutic agents, for use in a therapy.

## Patentansprüche

1. Eine Verbindung der Formel 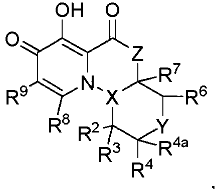 wobei X N oder CH ist,
Y fehlt, CHR⁵, -CH₂-CHR⁵-, -CH₂-CHR⁵-CH₂-, S, SO oder SO₂ ist,
Z NR¹ oder CR¹R^{1a} ist,
R¹ ausgewählt ist aus der Gruppe bestehend aus Wasserstoff, C₁₋₆-Alkyl, C₁₋₃-Alkyl(C₃₋₇-cycloalkyl), C₁₋₃-Alkyl(heterocyclyl), (C₁₋₆-Alkyl)OR^{x} und C₁₋₃-Halogenalkyl, wobei die Cycloalkylgruppe monocyclisch oder bicyclisch sein kann und gegebenenfalls substituiert ist mit einem oder zwei Substituenten, unabhängig ausgewählt aus der Gruppe bestehend aus Halogen und R^{x},
R^{1a} ausgewählt ist aus der Gruppe bestehend aus Wasserstoff, C₁₋₆-Alkyl, C₁₋₃-Alkyl(cyclopropyl), (C₁₋₆-Alkyl)OR^{x} und C₁₋₃-Halogenalkyl,
R² Aryl, Heteroaryl, Cycloalkyl oder Heterocyclyl ist, wobei das Aryl monocyclisch oder bicyclisch sein kann, wobei das Aryl, Heteroaryl, Cycloalkyl und Heterocyclyl gegebenenfalls substituiert sind mit einem bis drei Substituenten, unabhängig ausgewählt aus der Gruppe bestehend aus Halogen, Cyano, Cyclopropyl, R^{x}, R^{y}, SR^{x} und OR^{x},
R³ Wasserstoff, Hydroxy, C₁₋₆-Alkyl, OR^{x} oder C₁₋₃-Halogenalkyl ist,
oder R² und R³ mit dem Kohlenstoffatom oder Heteroatom, an das sie gebunden sind, zusammengenommen sein können unter Bildung von Dihydroinden, 1,2,3,4-Tetrahydronaphthalin, Chroman, 2,3-Dihydrobenzo[b]thiophen, Dihydrobenzofuran oder Thiochroman,
R⁴ ausgewählt ist aus der Gruppe bestehend aus Wasserstoff, Hydroxy, N₃, NH(C=O)R^{x}, SR^{x}, C₁₋₆-Alkyl, C₂₋₆-Alkenyl, O(C₁₋₆-Alkyl), O(C₂₋₆-Alkenyl), (C₁₋₃-Alkyl)R^{y}, O(C₁₋₃-Alkyl)R^{y}, R^{y} und Heteroaryl, wobei die Alkylgruppen gegebenenfalls substituiert sind mit einem bis drei Substituenten, unabhängig ausgewählt aus der Gruppe bestehend aus Halogen, R^{x}, R^{y}, OR^{x}, Cyano und Phenyl, wobei die Heteroaryl- und Alkenylgruppen gegebenenfalls substituiert sind mit einem bis drei Substituenten, unabhängig ausgewählt aus der Gruppe bestehend aus Halogen, R^{x} und (C₁₋₃-Alkyl)OR^{x},
oder R² und R⁴ mit dem Kohlenstoffatom oder Heteroatom, an das sie gebunden sind, zusammengenommen sein können unter Bildung von Dihydroinden, 1,2,3,4-Tetrahydronaphthalin, 2,3-Dihydrobenzo[b]thiophen, Dihydrobenzofuran, Chroman oder Thiochroman,
R^{4a} ausgewählt ist aus der Gruppe bestehend aus Wasserstoff und C₁₋₆-Alkyl, wobei die Alkylgruppe gegebenenfalls substituiert ist mit einem bis drei Substituenten, unabhängig ausgewählt aus der Gruppe bestehend aus Halogen und OR^{x},
oder R⁴ und R^{4a} mit dem Kohlenstoffatom oder Heteroatom, an das sie gebunden sind, zusammengenommen sein können unter Bildung einer Oxogruppe oder einer C₄₋₆-Cycloalkylgruppe, die monocyclisch oder bicyclisch sein kann, die gegebenenfalls substituiert ist mit einem bis drei Substituenten, unabhängig ausgewählt aus der Gruppe bestehend aus Halogen, C₁₋₃-Alkyl und C₁₋₃-Halogenalkyl,
R⁵ ausgewählt ist aus der Gruppe bestehend aus Wasserstoff, C₁₋₆-Alkyl, C₁₋₃-Alkyl(cyclopropyl) und (C₁₋₆-Alkyl)OR^{x},
R⁶ Wasserstoff, C₁₋₆-Alkyl, SR^{x}, NR^{x}(C=O)C₁₋₆-Alkyl oder (C₂₋₃-Alkenyl)R^{y} ist,
oder R⁴ und R⁶ mit den Kohlenstoffatomen, an die sie gebunden sind, zusammengenommen sein können unter Bildung einer C₄₋₆-Cycloalkylgruppe,
R⁷ Wasserstoff oder C₁₋₃-Alkyl ist,
R⁸ Wasserstoff, C₁₋₆-Alkyl oder (C₁₋₆-Alkyl)OR^{x} ist,
R⁹ Wasserstoff, Cyano oder C₁₋₆-Alkyl ist, wobei das Alkyl gegebenenfalls substituiert ist mit einem bis drei Halogen,
R^{x} ausgewählt ist aus der Gruppe bestehend aus Wasserstoff und C₁₋₆-Alkyl, wobei das Alkyl gegebenenfalls substituiert ist mit einem bis drei Halogen,
R^{y} ausgewählt ist aus der Gruppe bestehend aus Phenyl und C₃₋₆-Cycloalkyl, wobei die Phenyl- und Cycloalkylgruppen gegebenenfalls substituiert sind mit einem bis drei Substituenten, unabhängig ausgewählt aus der Gruppe bestehend aus Halogen, Cyano und R^{x},
oder ein pharmazeutisch annehmbares Salz davon.

2. Die Verbindung nach Anspruch 1, wobei X N ist, oder ein pharmazeutisch annehmbares Salz davon.

3. Die Verbindung nach Anspruch 1 oder 2, wobei R¹ Methyl ist, oder ein pharmazeutisch annehmbares Salz davon.

4. Die Verbindung nach einem der Ansprüche 1 bis 3, wobei Y CH₂ ist, oder ein pharmazeutisch annehmbares Salz davon.

5. Die Verbindung nach einem der Ansprüche 1 bis 4, wobei R² Phenyl ist, das gegebenenfalls substituiert ist mit einem oder zwei Substituenten, unabhängig ausgewählt aus der Gruppe bestehend aus Halogen, CH₃, CF₃, OCHF₂ und OCH₃, oder ein pharmazeutisch annehmbares Salz davon.

6. Die Verbindung nach einem der Ansprüche 1 bis 5, wobei R³ Wasserstoff, Methyl, Ethyl oder Hydroxy ist, oder ein pharmazeutisch annehmbares Salz davon.

7. Die Verbindung nach einem der Ansprüche 1 bis 6, wobei R⁴ Wasserstoff, Methyl, Ethyl, Propyl, Trifluorethyl, CH₂CH₂OH, CH₂CH₂OCH₃ oder Cyclopropylmethyl ist, oder ein pharmazeutisch annehmbares Salz davon.

8. Die Verbindung nach einem der Ansprüche 1 bis 7, wobei R⁴ und R⁶ mit den Kohlenstoffatomen, an die sie gebunden sind, zusammengenommen sein können unter Bildung einer C₄₋₆-Cycloalkylgruppe, oder ein pharmazeutisch annehmbares Salz davon.

9. Die Verbindung nach Anspruch 1, ausgewählt aus 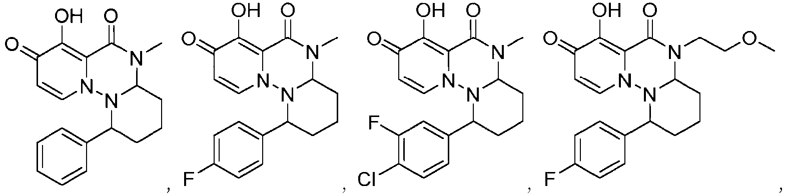 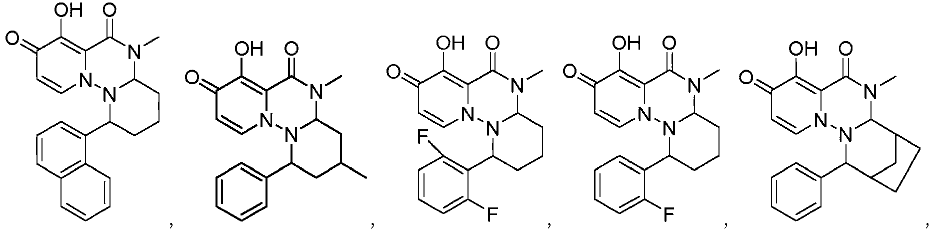 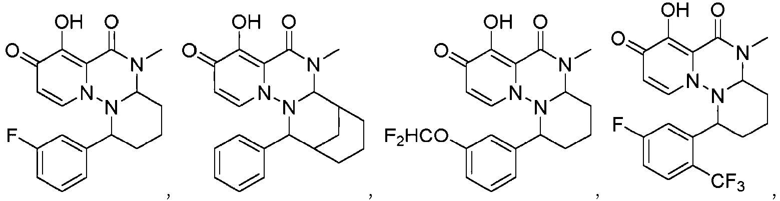 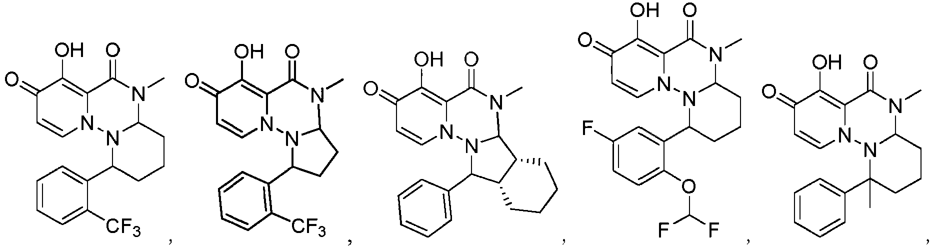 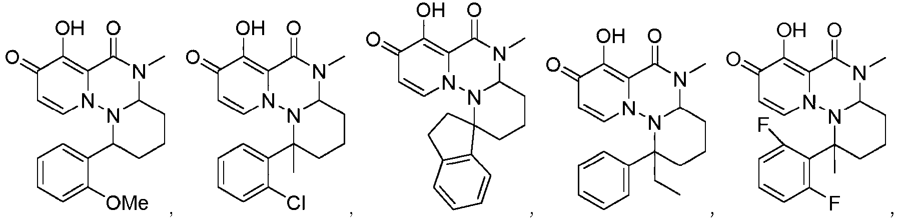 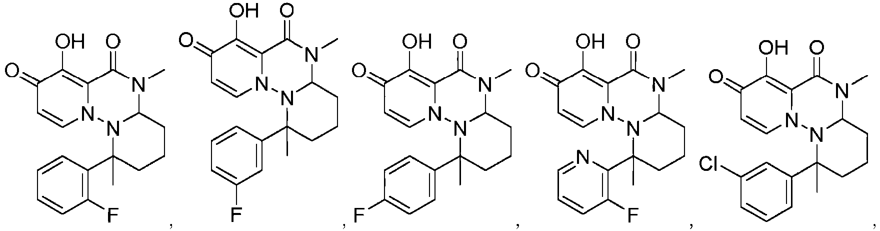 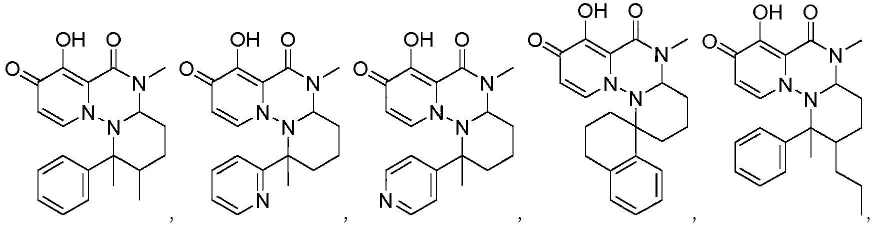 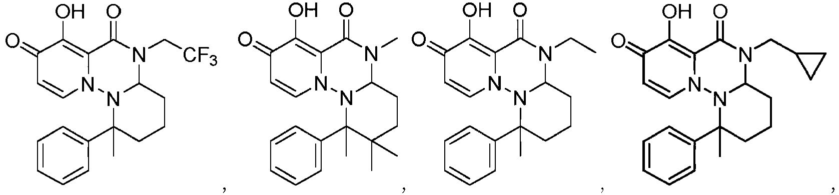 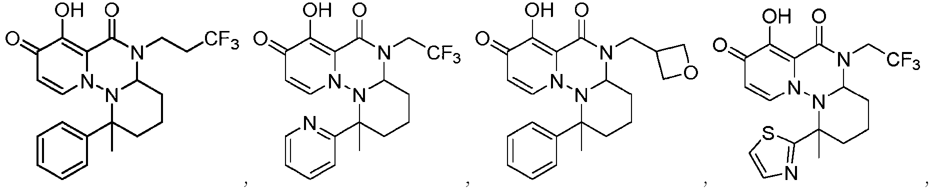 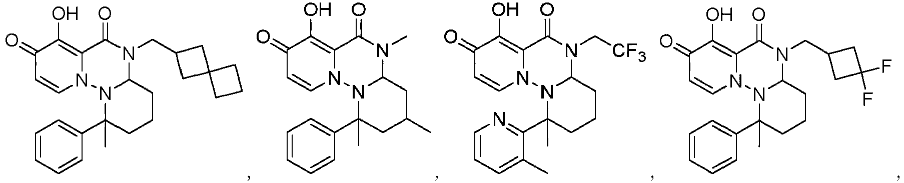 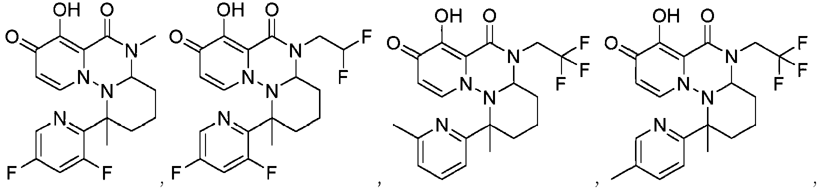 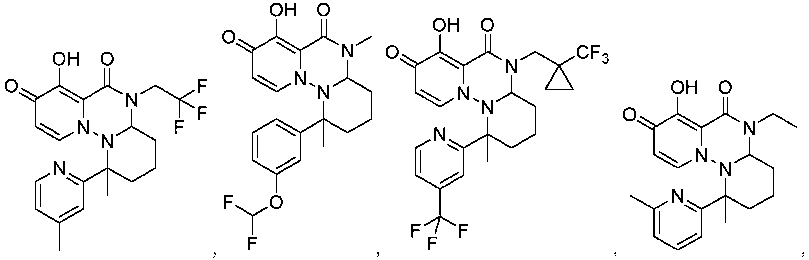 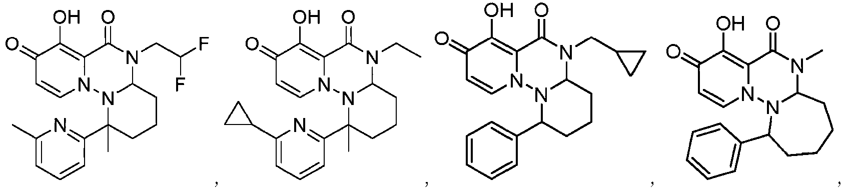 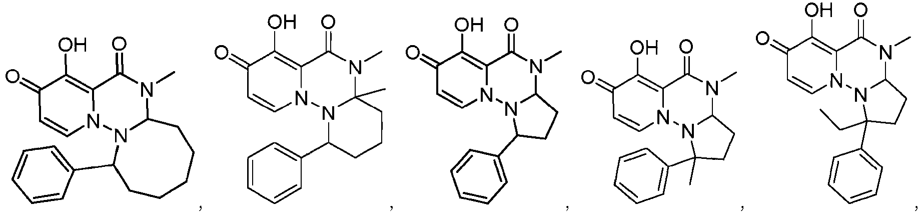 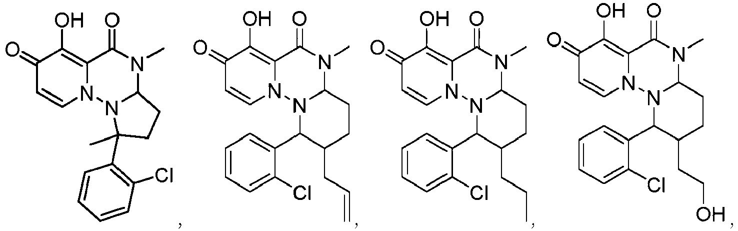 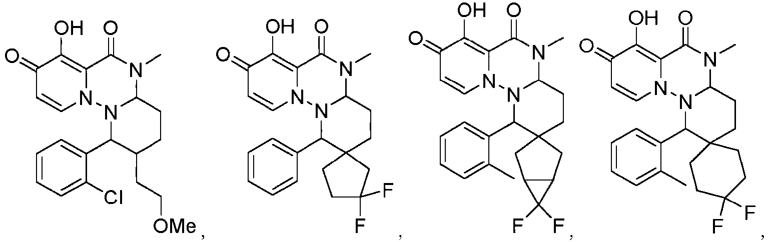 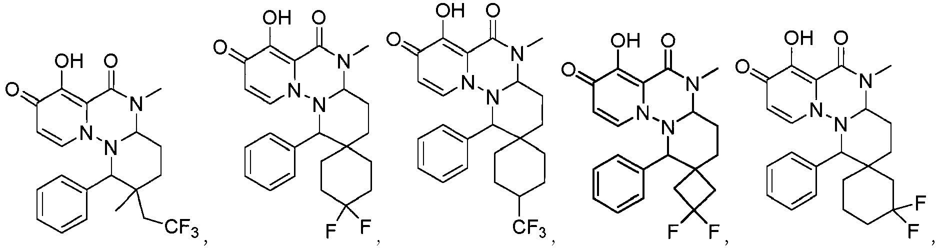 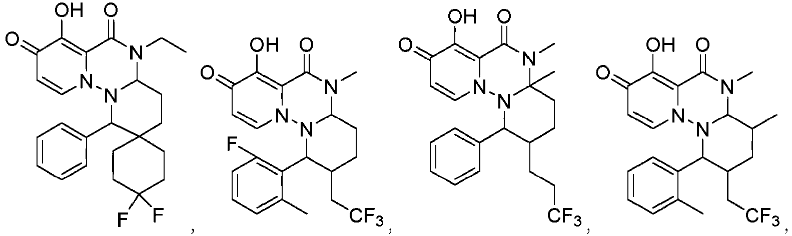 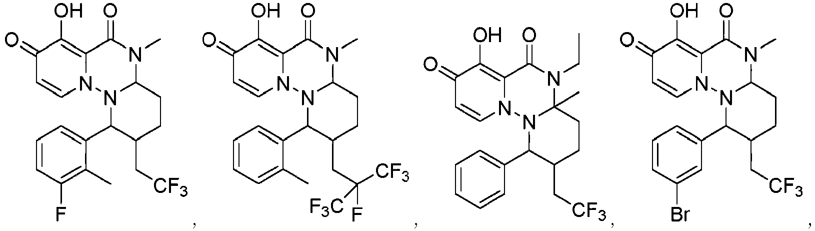 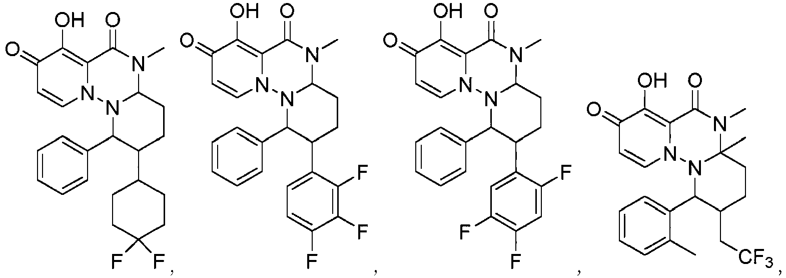 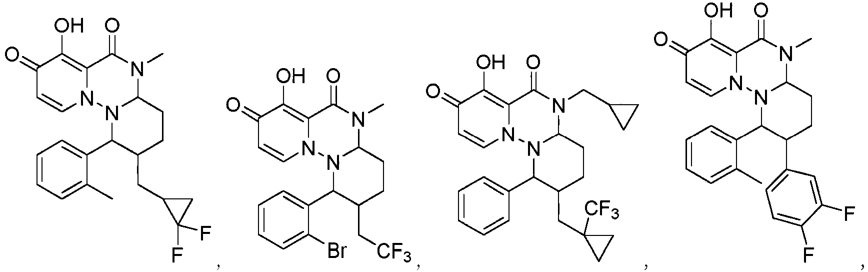 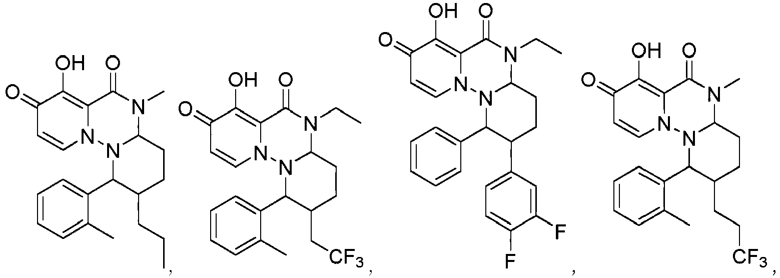 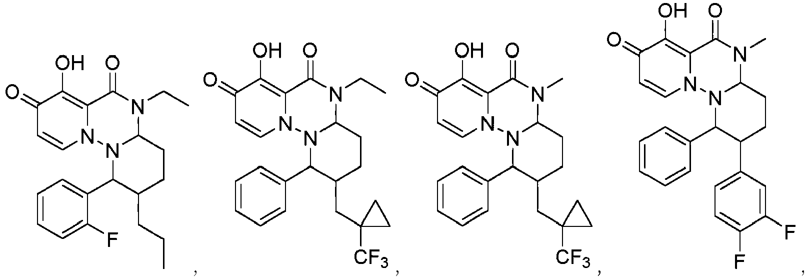 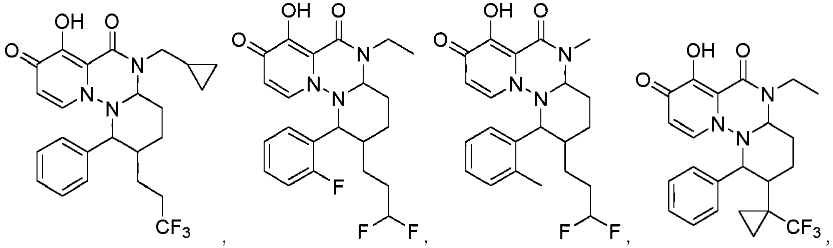 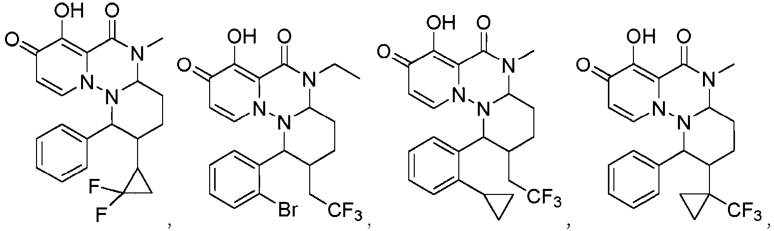 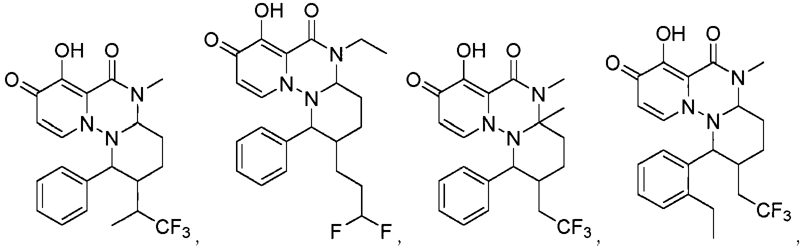 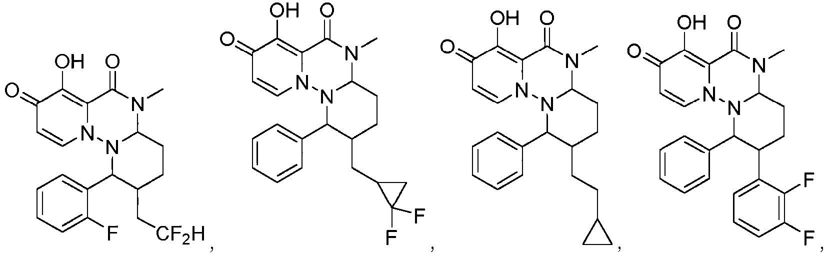 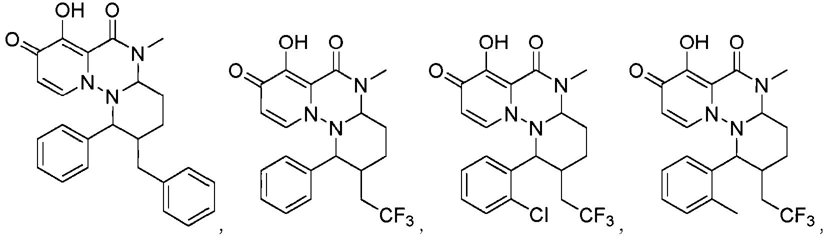 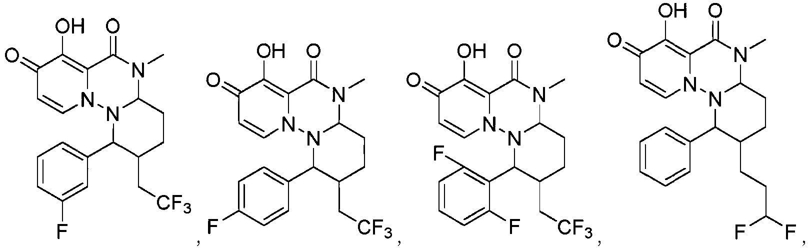 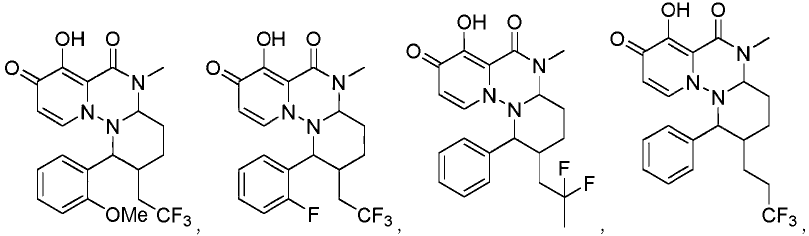 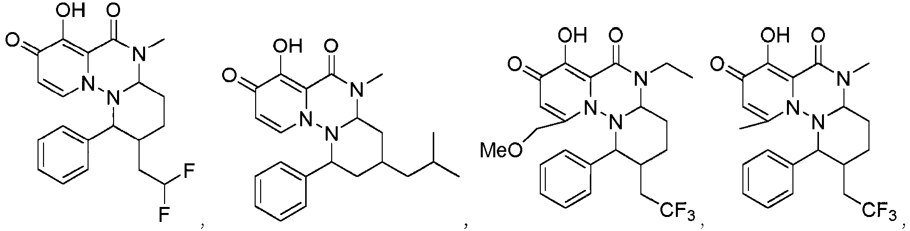 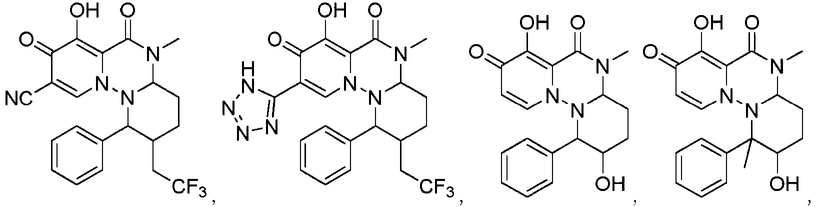 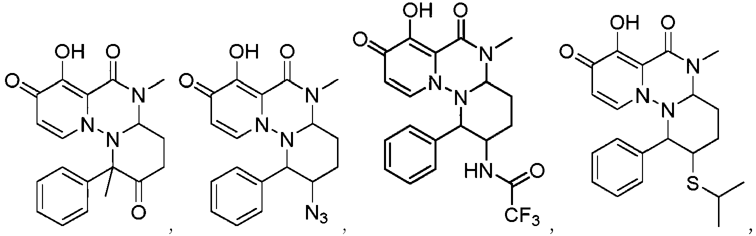 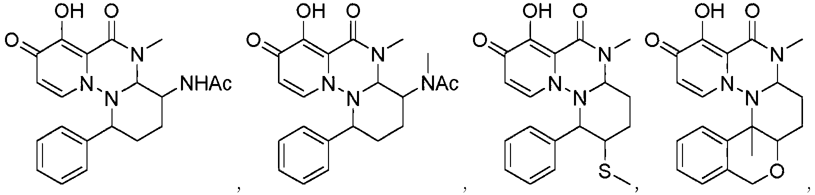 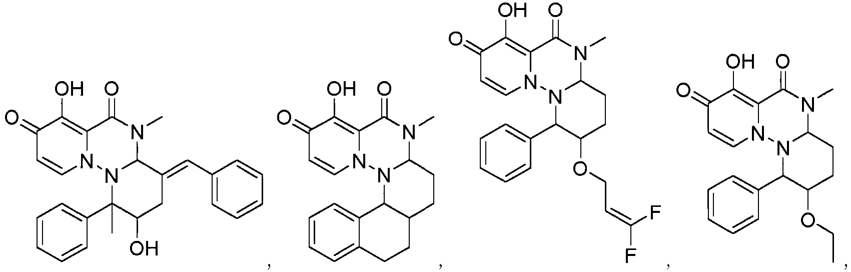 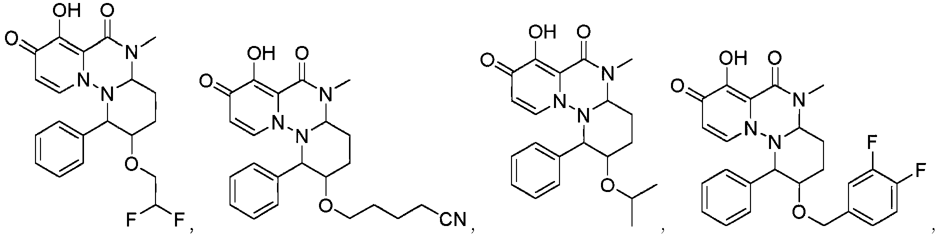 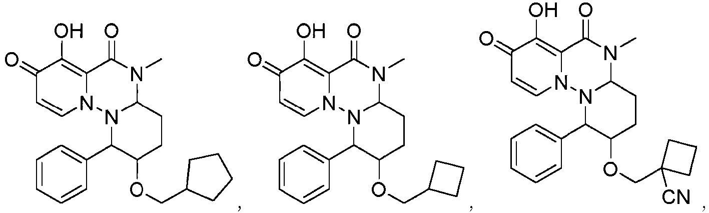 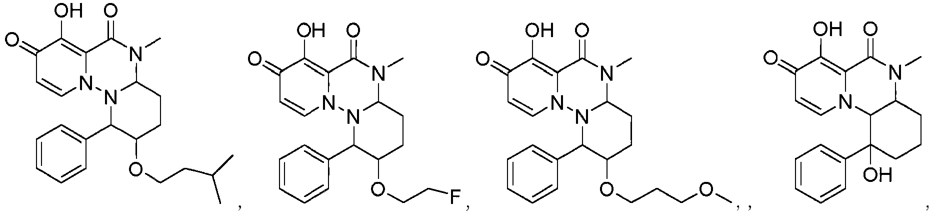 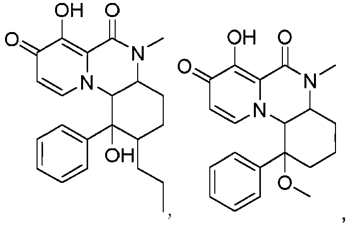 oder ein pharmazeutisch annehmbares Salz davon.

10. Eine pharmazeutische Zusammensetzung, die eine Verbindung nach einem der Ansprüche 1 bis 9 oder ein pharmazeutisch annehmbares Salz davon und einen pharmazeutisch annehmbaren Träger umfasst.

11. Eine Verbindung nach einem der Ansprüche 1 bis 9 oder ein pharmazeutisch annehmbares Salz davon oder eine pharmazeutische Zusammensetzung nach Anspruch 10 zur Verwendung bei einem Verfahren zur Behandlung einer Erkrankung, die durch ein Virus mit einer Cap-abhängigen Endonuklease hervorgerufen wird, wobei das Verfahren die Verabreichung der Verbindung, des pharmazeutisch annehmbaren Salzes oder der pharmazeutischen Zusammensetzung an einen Säuger, der dies benötigt, umfasst.

12. Eine Verbindung nach einem der Ansprüche 1 bis 9 oder ein pharmazeutisch annehmbares Salz davon oder eine pharmazeutische Zusammensetzung nach Anspruch 10 zur Verwendung bei einem Verfahren zur Behandlung oder Prävention von Influenza bei einem Säuger, wobei das Verfahren die Verabreichung der Verbindung, des pharmazeutisch annehmbaren Salzes oder der pharmazeutischen Zusammensetzung an einen Säuger, der dies benötigt, umfasst.

13. Die Verbindung, das pharmazeutisch annehmbare Salz oder die pharmazeutische Zusammensetzung zur Verwendung gemäß Anspruch 12, wobei der Säuger ein Mensch ist.

14. Die Verbindung gemäß einem der Ansprüche 1 bis 9 oder ein pharmazeutisch annehmbares Salz davon zur Verwendung in der Therapie.

15. Eine Kombination, die eine Verbindung gemäß einem der Ansprüche 1 bis 9 oder ein pharmazeutisch annehmbares Salz davon und zusätzliche therapeutische Mittel umfasst, zur Verwendung in einer Therapie.

## Revendications

1. Composé de la formule 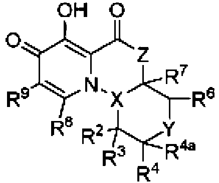 dans lequel X est N ou CH ;
Y est absent, CHR⁵, -CH₂-CHR⁵-, -CH₂-CHR⁵-CH₂-, S, SO ou SO₂ ;
Z est NR¹ ou CR¹R^{1a} ;
R¹ est sélectionné dans le groupe consistant en hydrogène, alkyle en C₁₋₆, alkyle en C₁₋₃ (cycloalkyle en C₃₋₇), alkyle en C₁₋₃ (hétérocyclyle), (alkyle en C₁₋₆)OR^{x} et haloalkyle en C₁₋₃, dans lequel le groupe cycloalkyle peut être monocyclique ou bicyclique, et est éventuellement substitué par un ou deux substituants sélectionnés indépendamment dans le groupe consistant en halo et R^{x} ;
R^{1a} est sélectionné dans le groupe consistant en hydrogène, alkyle en C₁₋₆, alkyle en C₁₋₃ (cyclopropyle), (alkyle en C₁₋₆)OR^{x} et haloalkyle en C₁₋₃ ;
R² est aryle, hétéroaryle, cycloalkyle ou hétérocyclyle, dans lequel ledit aryle peut être monocyclique ou bicyclique, dans lequel lesdits aryle, hétéroaryle, cycloalkyle et hétérocyclyle sont éventuellement substitués par un à trois substituants sélectionnés indépendamment dans le groupe consistant en halo, cyano, cyclopropyle, R^{x}, R^{y}, SR^{x} et OR^{x} ;
R³ est hydrogène, hydroxy, alkyle en C₁₋₆, OR^{x} ou haloalkyle en C₁₋₃ ;
ou R² et R³ peuvent être pris avec l'atome ou l'hétéroatome de carbone auquel ils sont liés pour former du dihydroindène, du 1,2,3,4-tétrahydronaphtalène, du chromane, du 2,3-dihydrobenzo[b]thiophène, du dihydrobenzofurane ou du thiochromane ;
R⁴ est sélectionné dans le groupe consistant en hydrogène, hydroxyle, N₃, NH(C=O)R^{x}, SR^{x}, alkyle en C₁₋₆, alcényle en C₂₋₆, O(alkyle en C₁₋₆), O(alcényle en C₂₋₆), (alkyle en C₁₋₃)R^{y}, O(alkyle en C₁₋₃)R^{y}, R^{y} et hétéroaryle, dans lequel lesdits groupes alkyle sont éventuellement substitués par un à trois substituants sélectionnés indépendamment dans le groupe consistant en halo, R^{x}, R^{y}, OR^{x}, cyano et phényle, dans lequel lesdits groupes hétéroaryle et alcényle sont éventuellement substitués par un à trois substituants sélectionnés indépendamment dans le groupe consistant en halo, R^{x} et (alkyle en C₁₋₃)OR^{x};
ou R² et R⁴ peuvent être pris avec l'atome ou l'hétéroatome de carbone auquel ils sont liés pour former du dihydroindène, du 1,2,3,4-tétrahydronaphtalène, du 2,3-dihydrobenzo[b]thiophène, du dihydrobenzofurane, du chromane ou du thiochromane ;
R^{4a} est sélectionné dans le groupe consistant en hydrogène et alkyle en C₁₋₆, dans lequel ledit groupe alkyle est éventuellement substitué par un à trois substituants sélectionnés indépendamment dans le groupe consistant en halo et OR^{x} ;
ou R⁴ et R^{4a} peuvent être pris avec l'atome ou l'hétéroatome de carbone auquel ils sont liés pour former un groupe oxo ou un groupe cycloalkyle en C₄₋₆, qui peut être monocyclique ou bicyclique, lequel est éventuellement substitué par un à trois substituants sélectionnés indépendamment dans le groupe consistant en halo, alkyle en C₁₋₃ et haloalkyle en C₁₋₃ ;
R⁵ est sélectionné dans le groupe consistant en hydrogène, alkyle en C₁₋₆, alkyle en C₁₋₃ (cyclopropyle) et (alkyle en C₁₋₆)OR^{X} ;
R⁶ est hydrogène, alkyle en C₁₋₆, SR^{x}, NR^{x}(C=O) alkyle en C₁₋₆ ou (alcényl en C₂₋₃)R^{y};
ou R⁴ et R⁶ peuvent être pris avec les atomes de carbone auxquels ils sont liés pour former un groupe cycloalkyle en C₄₋₆ ;
R⁷ est hydrogène ou alkyle en C₁₋₃ ;
R⁸ est hydrogène, alkyle en C₁₋₆ ou (alkyle en C₁₋₆)OR^{x} ;
R⁹ est hydrogène, cyano ou alkyle en C₁₋₆, dans lequel ledit alkyle est éventuellement substitué par un à trois halo ;
R^{x} est sélectionné dans le groupe consistant en hydrogène et alkyle en C₁₋₆, dans lequel ledit alkyle est éventuellement substitué par un à trois halo ;
R^{y} est sélectionné dans le groupe consistant en phényle et cycloalkyle en C₃₋₆, dans lequel lesdits groupes phényle et cycloalkyle sont éventuellement substitués par un à trois substituants sélectionnés indépendamment dans le groupe consistant en halo, cyano et R^{x} ;
ou sel pharmaceutiquement acceptable de celui-ci.

2. Composé selon la revendication 1 dans lequel X est N ; ou sel pharmaceutiquement acceptable de celui-ci.

3. Composé selon la revendication 1 ou 2 dans lequel R¹ est méthyle ; ou sel pharmaceutiquement acceptable de celui-ci.

4. Composé selon l'une quelconque des revendications 1 à 3, dans lequel Y est CH₂; ou sel pharmaceutiquement acceptable de celui-ci.

5. Composé selon l'une quelconque des revendications 1 à 4, dans lequel R² est phényle, qui est éventuellement substitué par un ou deux substituants sélectionnés indépendamment dans le groupe consistant en halo, CH₃, CF₃, OCHF₂ et OCH₃; ou sel pharmaceutiquement acceptable de celui-ci.

6. Composé selon l'une quelconque des revendications 1 à 5 dans lequel R³ est hydrogène, méthyle, éthyle ou hydroxy ; ou sel pharmaceutiquement acceptable de celui-ci.

7. Composé selon l'une quelconque des revendications 1 à 6 dans lequel R⁴ est hydrogène, méthyle, éthyle, propyle, trifluoroéthyle, CH₂CH₂OH, CH₂CH₂OCH₃ ou cyclopropylméthyle ; ou sel pharmaceutiquement acceptable de celui-ci.

8. Composé selon l'une quelconque des revendications 1 à 7 dans lequel R⁴ et R⁶ peuvent être pris avec les atomes de carbone auxquels ils sont liés pour former un groupe cycloalkyle en C₄₋₆ ; ou sel pharmaceutiquement acceptable de celui-ci.

9. Composé selon la revendication 1 sélectionné parmi 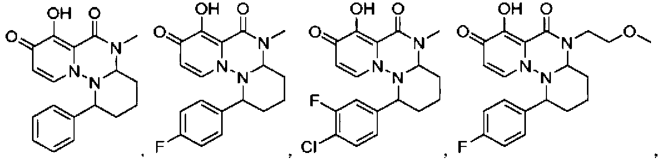 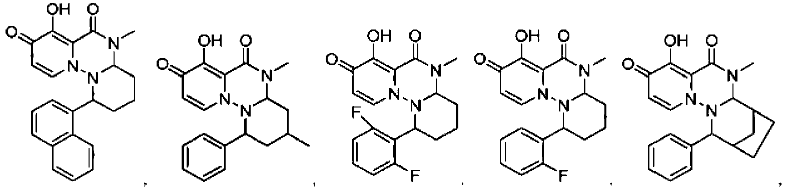 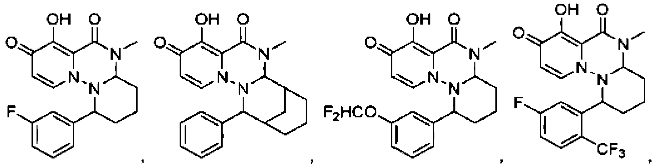 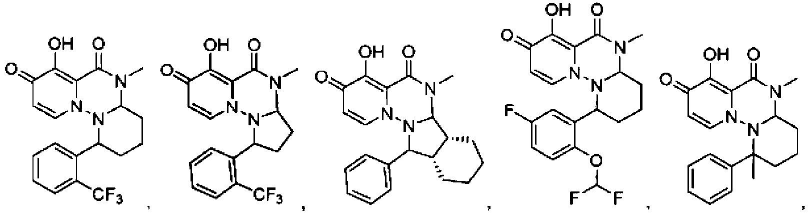 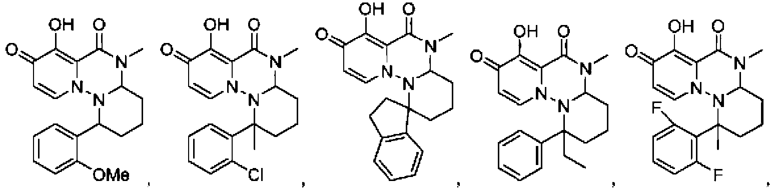 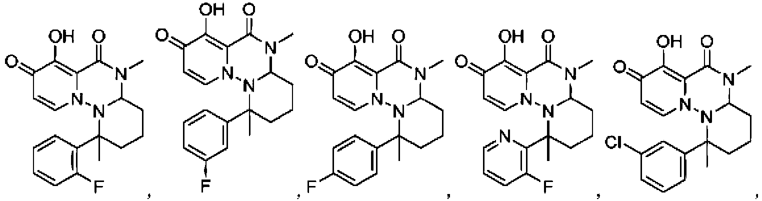 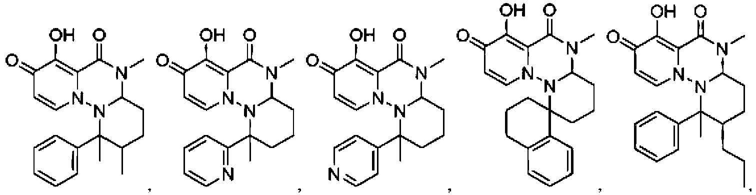 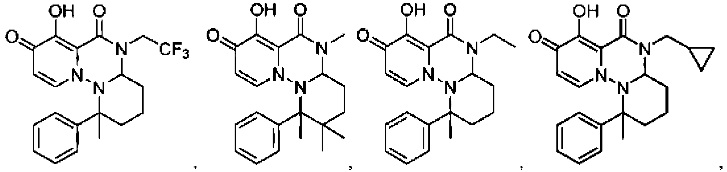 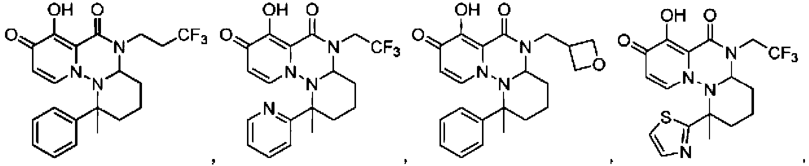 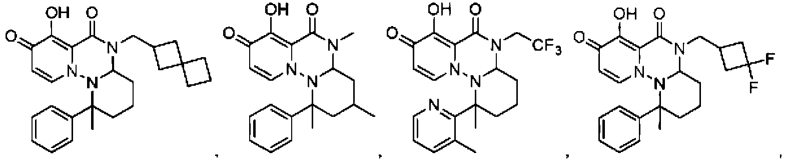 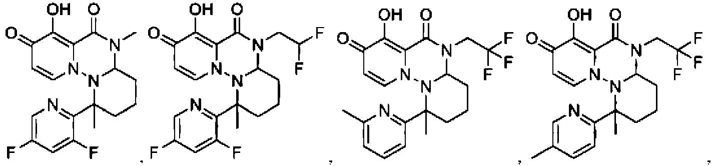 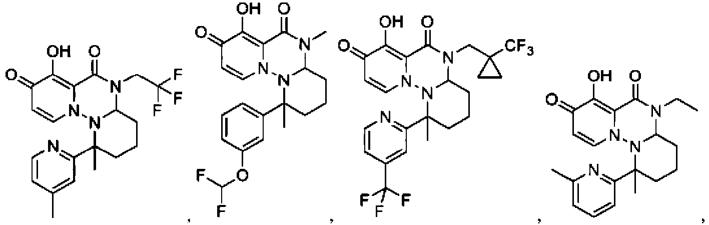 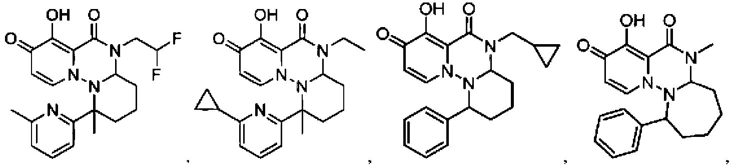 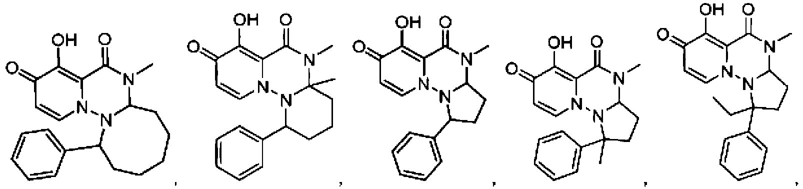 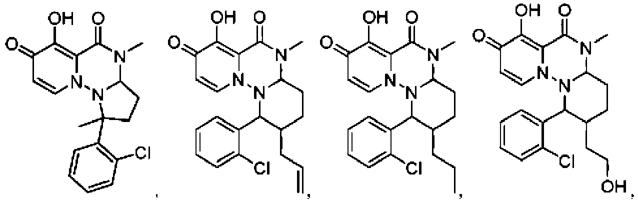 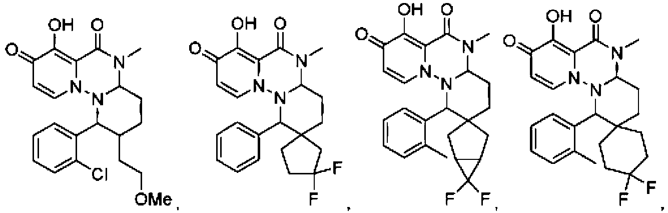 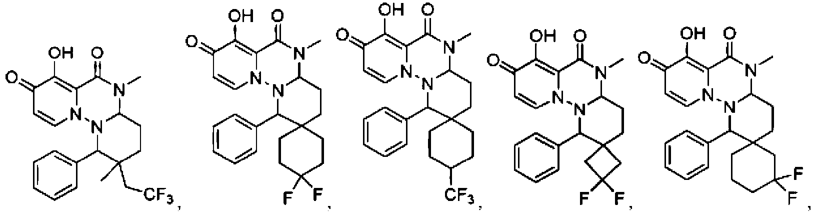 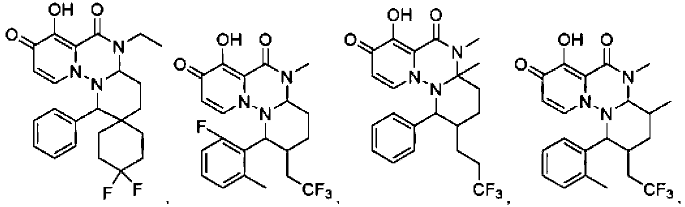 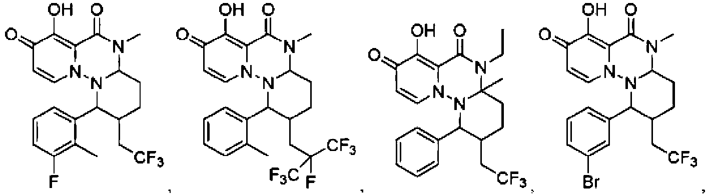 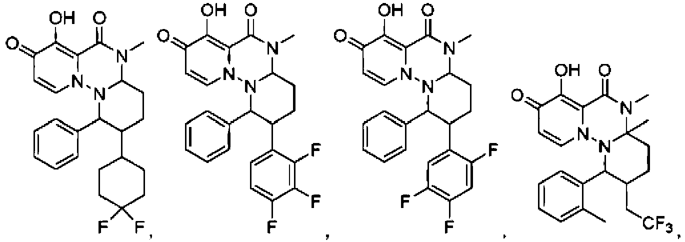 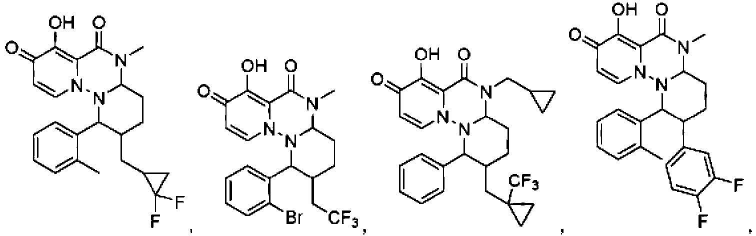 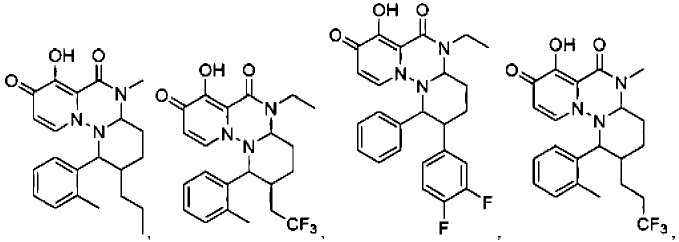 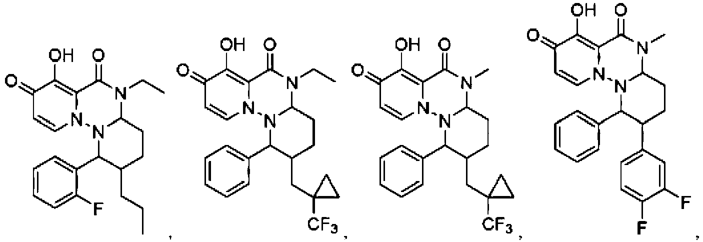 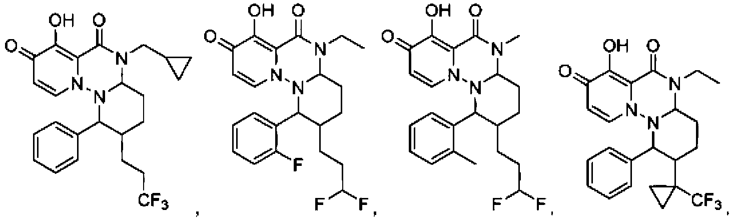 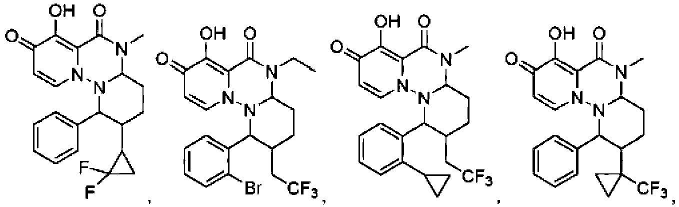 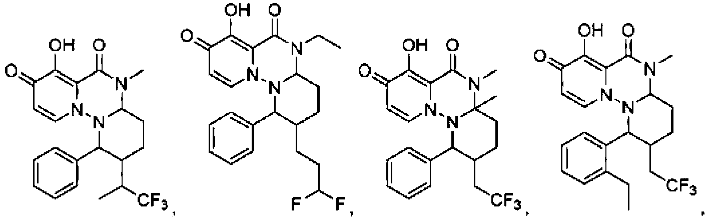 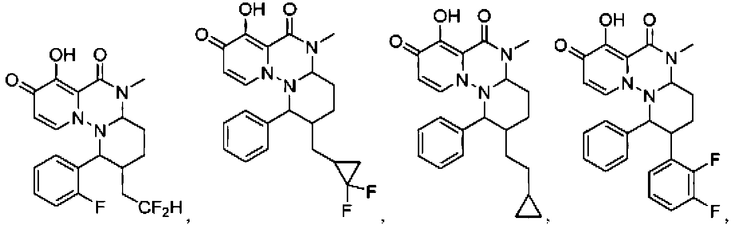 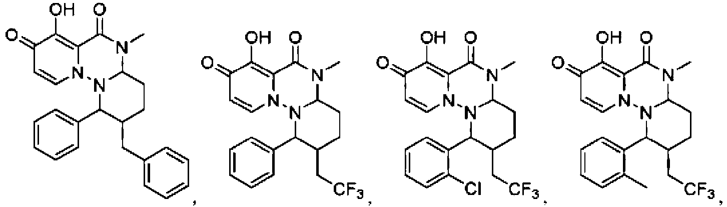 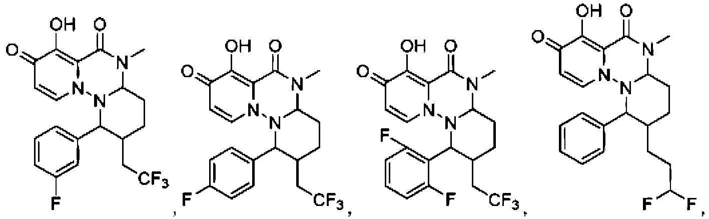 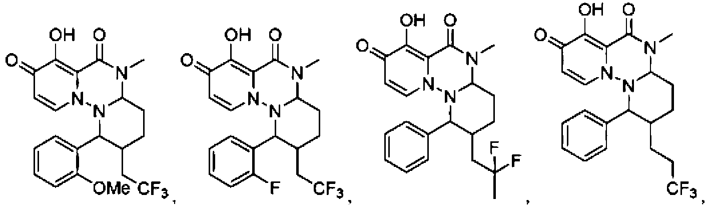 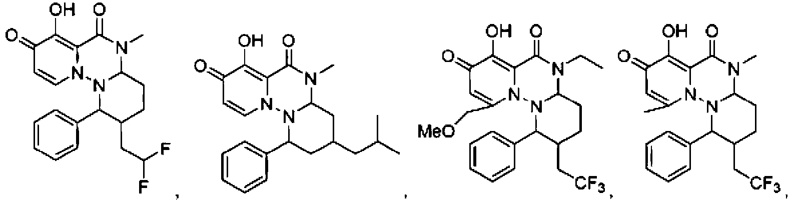 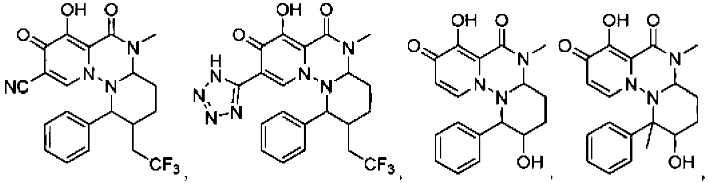 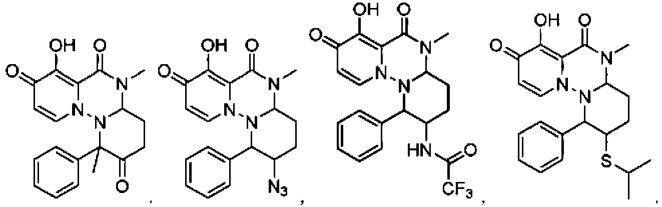 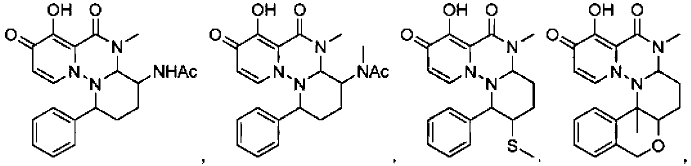 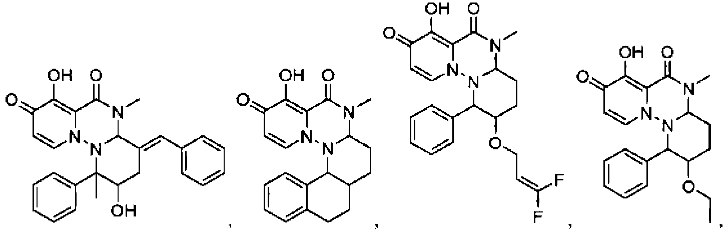 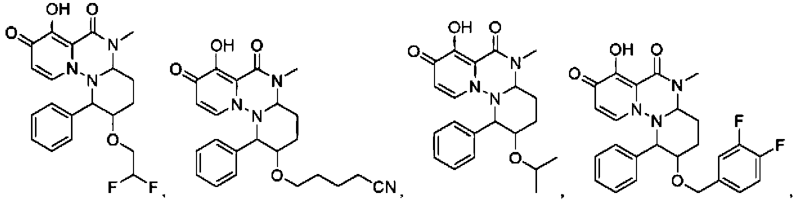 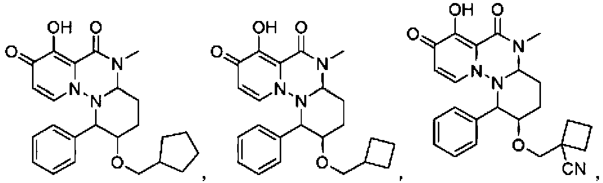 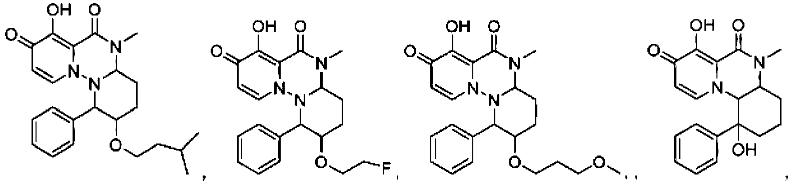 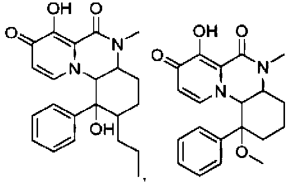 ou sel pharmaceutiquement acceptable de celui-ci.

10. Composition pharmaceutique comprenant un composé selon l'une quelconque des revendications 1 à 9 ou un sel pharmaceutiquement acceptable de celui-ci, et un excipient pharmaceutiquement acceptable.

11. Composé selon l'une quelconque des revendications 1 à 9 ou sel pharmaceutiquement acceptable de celui-ci ou composition pharmaceutique selon la revendication 10, destiné(e) à être utilisé(e) dans un procédé de traitement d'une maladie causée par un virus présentant une endonucléase dépendante de la coiffe, le procédé comprenant l'administration du composé, du sel pharmaceutiquement acceptable ou de la composition pharmaceutique à un mammifère en ayant besoin.

12. Composé selon l'une quelconque des revendications 1 à 9 ou sel pharmaceutiquement acceptable de celui-ci ou composition pharmaceutique selon la revendication 10, destiné(e) à être utilisé(e) dans un procédé de traitement ou de prévention de la grippe chez un mammifère, le procédé comprenant l'administration du composé, du sel pharmaceutiquement acceptable ou de la composition pharmaceutique à un mammifère en ayant besoin.

13. Composé, sel pharmaceutiquement acceptable ou composition pharmaceutique destiné(e) à être utilisé(e) selon la revendication 12, dans lequel/laquelle le mammifère est un être humain.

14. Composé selon l'une quelconque des revendications 1 à 9 ou sel pharmaceutiquement acceptable de celui-ci, destiné à être utilisé en thérapie.

15. Combinaison comprenant un composé selon l'une quelconque des revendications 1 à 9, ou un sel pharmaceutiquement acceptable de celui-ci, et des agents thérapeutiques supplémentaires, destinée à être utilisée en thérapie.
